# EUROPEAN PATENT APPLICATION

(11) **EP 1 466 902 A1**
(43) Date of publication of application: **13.10.2004**
(21) Application number: 02790889.6
(22) Date of filing: 26.12.2002
(51) Int. Cl.: C07D 207/34, A61K 31/40, A61K 45/06, A61P 13/08, A61P 35/00, A61P 43/00

(54) **ANDROGEN RECEPTOR ANTAGONISTS**

(30) Priority: 28.12.2001 JP 2001399143
(71) Applicant: Takeda Chemical Industries, Ltd., Osaka-shi, Osaka 541-0045 (JP)
(72) Inventor: FURUYA, Shuichi, Tsukuba-shi, Ibaraki 305-0821 (JP); MATSUNAGA, Nobuyuki, Osaka-shi, Osaka 545-0021 (JP); KUSAKA, Masami, Kobe-shi, Hyogo 651-2102 (JP); HARA, Takahito, Osaka-shi, Osaka 532-0013 (JP); MIYAZAKI, Junichi, Kobe-shi, Hyogo 658-0073 (JP)
(74) Representative: Rickard, Timothy Mark Adrian
(86) International application number: PCT/JP2002/013652
(87) International publication number: WO 2003/057669

(57) **Abstract**

The present invention provides an androgen receptor antagonistic agent and a superior prophylactic or therapeutic agent for hormone-sensitive cancer, which contain a compound of the formula: 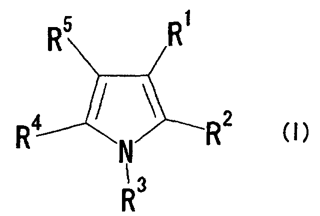 wherein, R¹ is a hydrogen atom, a group binding through a carbon atom, a group binding through a nitrogen atom, a group binding through an oxygen atom or a group binding through a sulfur atom; R² is a hydrogen atom, a group binding through a carbon atom, a group binding through a nitrogen atom, a group binding through an oxygen atom or a group binding through a sulfur atom, R³ is a hydrogen atom, a hydrocarbon group which may have substituent (s) , an acyl group or a heterocyclic group which may have substituent(s), R⁴ is a hydrogen atom, a group binding through a carbon atom, a group binding through a nitrogen atom, a group binding through an oxygen atom or a group binding through a sulfur atom, and R⁵ is a cyclic group which may have substituent(s); or a salt thereof, or its prodrug.

## Description

### Technical Field

The present invention relates to an androgen receptor antagonistic drug which has prophylactic and therapeutic effects against androgen-dependent diseases by inhibiting male sex hormone androgen receptor (AR), and whose effects are not influenced by the mutation of AR.

### Background

For the therapy of prostate cancer (its proliferation at the early stage is controlled by androgen which is a type of steroid hormone, and therefore, such kinds of prostate cancer are said to be "an androgen (hormone)-dependent") which is one of the most serious diseases among the male sex hormone-dependent diseases, the first choice aiming at the radical cure is the treatment by means of surgical operation and/or radiotherapy. However, the endocrine therapy with an LH-RH agonist is mainly selected when surgical operation and radiotherapy cannot be adopted or when a recurrence has occurred after the operation. Further, the adjuvant therapy by using an androgen receptor (AR) antagonist solely and the MAB (Maximum Androgen Block) therapy, in which an LHRH agonist and an androgen receptor (AR) antagonist are used in combination, are carried out, and the efficacy of these treatments are surprisingly remarkable. However, endocrine therapy is not a radical cure therapy, and there is a possibility that a hormone-independent recurrent carcinoma is caused as a result of the generation of endocrine therapy-resistance. (As the mechanism of this resistance generation, it is thought that cancer cells change to have the ability to use other steroid hormones or the androgen receptor antagonists which are used in the therapy as an agonist-like ligand. It can be said that such kinds of prostate cancer are not androgen (hormone)-dependent but androgen(hormone)-independent. Further, in the sense that the cancer cells react to other steroid hormones or androgen receptor antagonists used for the therapy and proliferate, it can be said that they are "hormone-sensitive". If the cancer cells change further, they will no longer react to the hormones at all, and in this case it can be said that they have become hormone-insensitive.

Flutamide and bicalutamide, which are both androgen receptor (AR) antagonists available in the market, are said to become considerably less effective against a hormone-independent recurrent cancer which has acquired a system adapted to a mutant AR or a low concentration of androgen, and even to change to an agonist. Further, chemotherapeutic agents are not expected to be effective against such type of hormone-independent cancers, and this is a major problem in the treatment of the prostate cancer.

In recent years, based on the research progress on hormone-independent diseases, it has come to be known that, in the disease called hormone-independent disease, proliferation proceeds by using the signal transduction system via AR in the same manner as that in the hormone-dependent diseases. It came to be known that change in the signal transduction system causes an apparent hormone-independent phenomenon. Concretely speaking, when AR mutates, for example, other than androgen, glucocorticoid and currently used AR antagonist themselves come to act as agonists. As a result of some change including the change of expression level and/or mutation of AR, the sensitivity of AR increases, whereby a strong proliferation signal comes to be transmitted by a small amount of androgen. By these changes, presently used AR antagonists are being found to be considerably less effective.

If a compound having a strong antagonistic effect against an mutated AR and an AR having increased sensitivity can be found, the compound is expected to become a drug effective with prostate cancer in the hormone-independent stage.

### Disclosure of Invention

The present inventors conducted intensive investigations and found a compound having an excellent antagonistic activity against a natural-type AR as well as a strong inhibitory activity against a mutant AR. In addition, the inventors found that these compounds can be administered orally, show extremely low toxicity, and therefore are sufficiently satisfactory as pharmaceuticals with AR antagonistic effects. This invention was completed on the basis of these findings.

The present invention relates to:
(1) An androgen receptor antagonistic agent containing a compound of the formula: 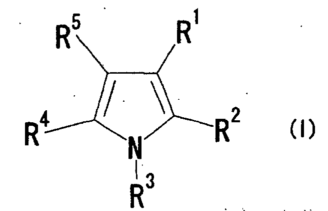 wherein, R¹ is a hydrogen atom, a group binding through a carbon atom, a group binding through a nitrogen atom, a group binding through an oxygen atom or a group binding through a sulfur atom, R² is a hydrogen atom, a group binding through a carbon atom, a group binding through a nitrogen atom, a group binding through an oxygen atom or a group binding through a sulfur atom, R³ is a hydrogen atom, a hydrocarbon group which may have substituent (s) , an acyl group or a heterocyclic group which may have substituent(s), R⁴ is a hydrogen atom, a group binding through a carbon atom, a group binding through a nitrogen atom, a group binding through an oxygen atom or a group binding through a sulfur atom, R⁵ is a cyclic group which may have substituent (s), or a salt thereof, or its prodrug,
(2) An agent as defined in (1) above, wherein R¹ is a hydrogen atom, a cyano group, a hydrocarbon group which may have substituent(s), a group represented by the formula: -COOR⁶¹ (wherein _{R}⁶¹ is a hydrogen atom or a hydrocarbon group which may have substituent(s)), a group represented by the formula: - CONR⁷¹R⁸¹ (wherein R⁷¹ and R⁸¹ are the same or different, each of them is a hydrogen atom or a hydrocarbon group which may have substituent(s), and R⁷¹ and R⁸¹ are combined with each other together with the adjacent nitrogen atom to form a cyclic group which may have substituent(s)), a group represented by the formula: -COR⁹¹ (wherein R⁹¹ is a hydrogen atom or a hydrocarbon group which may have substituent (s)) or a group represented by the formula: -OR¹³¹ (wherein R¹³¹ is a hydrogen atom or a hydrocarbon group which may have substituent(s));
(3) An agent as defined in (1) above, wherein R¹ is a cyano group or a group represented by the formula: -COOR⁶¹ (wherein R⁶¹ is a hydrocarbon group which may have substituent(s));
(4) An agent as defined in (1) above, wherein R² is a hydrogen atom, a cyano group, a hydrocarbon group which may have substituent(s), a group represented by the formula: -COOR⁶²: (wherein R⁶² is a hydrocarbon group which may have substituent (s)), a group represented by the formula: -CONR⁷²R⁸² (wherein R⁷² and R⁸² are the same or different, each of them is a hydrogen atom or a hydrocarbon group which may have substituent (s), R⁷² and R⁸² are combined with each other together with the adjacent nitrogen atom to form a cyclic group which may have substituent(s)), a group represented by the formula: -COR⁹² (wherein R⁹² is a hydrogen atom or a hydrocarbon group which may have substituent (s)), or a group represented by the formula: -OR¹³² (wherein R¹³² is a hydrogen atom or a hydrocarbon group which may have substituent(s));
(5) An agent as defined in (1) above, wherein R² is a C₁₋₆ alkyl group which may have substituent(s);
(6) An agent as defined in (1) above, wherein R³ is a C₁₋₆ alkyl group which may have substituent (s), a C₇₋₁₅ aralkyl group which may have substituent (s) , a heterocyclic group-C₁₋₆ alkyl group which may have substituent (s) , a C₁₋₆ alkyl group-sulfonyl group which may.have substituent (s) , a C₆₋₁₄ aryl group-sulfonyl group which may have substituent (s) , a heterocyclic group-sulfonyl group which may have substituent(s), a C₁₋₆ alkyl group-carbonyl group which may have substituent (s), a C₆₋₁₄ aryl group-carbonyl group which may have substituent(s) or a heterocyclic group-carbonyl group which may have substituent(s);
(7) An agent as defined in (1) above, wherein R³ is a benzyl group which may have substituent(s) or a pyridylmethyl group which may have substituent(s);
(8) An agent as defined in (1) above, wherein R³ is a heterocyclic group-methyl group which may have substituent(s));
(9) An agent as defined in (1) above, wherein R⁴ is a hydrogen atom, a cyano group, a hydrocarbon group which may have substituent(s), a group represented by the formula: -COOR⁶⁴ (wherein R⁶⁴ is a hydrogen atom or a hydrocarbon group which may have substituent(s)), a group represented by the formula: -CONR⁷⁴R⁸⁴ (wherein R⁷⁴ and R⁸⁴ are the same or different, each of them is a hydrogen atom or a hydrocarbon group which may have substituent (s) , R⁷⁴ and R⁸⁴ are combined to each other together with the adjacent nitrogen atom to form a cyclic group which may have substituent(s)), a group represented by the formula: -COR⁹⁴ (wherein R⁹⁴ is a hydrogen atom or a hydrocarbon group which may have substituent(s)) or a group represented by the formula: -OR¹³⁴ (wherein R¹³⁴ is a hydrogen atom or a hydrocarbon group which may have substituent(s));
(10) An agent as defined in (1) above, wherein R⁴ is a C₁₋₆ alkyl group which may have substituent(s);
(11) An agent as defined in (1) above, wherein R⁵ is a cyclic hydrocarbon group which may have substituent(s);
(12) An agent as defined in (1) above, wherein R⁵ is a C₆₋₁₄ aryl group which may have substituent(s) or a heterocyclic group which may have substituent(s);
(13) An agent as defined in (1) above, wherein R⁵ is a C₆₋₁₄ aryl group which has at least one substituent selected from a group consisting of a cyano group and a nitro group;
(14) An agent as defined in (1) above, wherein R¹ is a cyano group, a C₁₋₆ alkyl group which may have hydroxy, a group represented by the formula: -COOR^{6'1} (wherein R^{6'1} is a C₁₋₆ alkyl group), a group represented by the formula: -CONR^{7'1}R^{8'1} (wherein R^{7'1} and R^{8'1} are the same or different, each of them is a hydrogen atom, a C₁₋₆ alkyl group or a C₆₋₁₀ aryl group, R^{7'1} and R^{8'1} are taken together to form a cyclic group which may have substituent(s)), a group represented by the formula: -COR^{9''1} (wherein R^{9''1} is a C₁₋₆ alkyl group, a C₃₋₈ cyclo alkyl group or a heterocyclic group which may have substituent (s)), R² and R⁴ are the same or different, and each of them is a hydrogen atom, a cyano group or a C₁₋₆ alkyl group, R³ is a hydrogen atom or a hydrocarbon group which may have substituent(s) or a heterocyclic group which may have substituent(s), R⁵ is an aryl group which may have substituent(s), a C₃₋₈ cyclo alkyl group which may have substituent (s) or a heterocyclic group which may have substituent(s);
(15) An agent as defined in (1) above, wherein R¹ is a cyano group or a group represented by the formula: -COOR^{6'1} (wherein R^{6'1} is a C₁₋₆ alkyl group), R² is a C₁₋₆ alkyl group which may have substituent(s), R³ is a C₁₋₆ alkyl group which may have substituent(s), a C₇₋₁₅ aralkyl group which may have substituent (s) , a C₁₋₆ alkyl group-sulfonyl group which may have substituent(s) or a C₆₋₁₄ aryl group-carbonyl group which may have substituent(s), a R⁴ is a C₁₋₆ alkyl group which may have substituent (s) , R⁵ is a C₆₋₁₄ aryl group which may have substituent(s);
(16) An agent as defined in (1) above, wherein R¹ is a cyano group or a group represented by the formula: -COOR^{6'1} (wherein R^{6'1} is a C₁₋₆ alkyl group), R² is a C₁₋₆ alkyl group, R³ is a C₁₋₆ alkyl group which may have hydroxy, a C₇₋₁₅ aralkyl group, a C₁₋₆ alkyl group-sulfonyl group or a C₆₋₁₄ aryl group-carbonyl group, R⁴ is a C₁₋₆ alkyl group, and R⁵ is a C₆₋₁₄ aryl group which may have a nitro group, a cyano group or a C₁₋₃ acyl group;
(17) An agent as defined in (1) above, wherein the compound is a group represented by the formula: 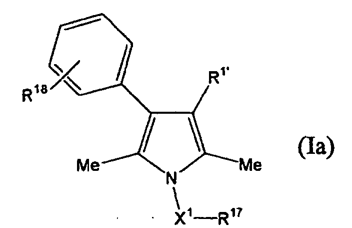 wherein R^{1'} is a cyano group, a C₁₋₆ alkyl group, a group represented by the formula: -COOR^{6'1'} (wherein R^{6'1'} is a C₁₋₆ alkyl group), a group represented by the formula: -CONR^{7'1'}R^{8'1'} (wherein R^{7'1'} and R^{8'1'} are the same or different, and each of them is a hydrogen atom, a C₁₋₆ alkyl group or a C₆₋₁₀ aryl group), a group represented by the formula: -C(OH)R^{161'}R^{16'1'} (R^{161'} and R^{16'1'} are the same or different, and each of them is a hydrogen atom or a C₁₋₆ alkyl) or a group represented by the formula: - COR^{9''1'} (wherein R^{9''1'} is a C₁₋₆ alkyl group, a C₃₋₈ cyclo alkyl group or a heterocyclic group which may have substituent (s) ), R¹⁷ is a C₆₋₁₄ aryl group which may have substituent(s) or a heterocyclic group which may have substituent (s), R¹⁸ is a nitro group, a C₁₋₆ alkyl group which may have substituent(s), a halogen atom, a C₁₋₆ alkoxy group, a cyano group, a carboxyl group, a carboxylic acid ester group, a hydroxy group or an amide group, X¹ is a bivalent group in which the number of straight-chained carbon atom(s) is 1 to 5 which may have substituent(s); or a salt thereof, or its prodrug;
(18) An agent as defined in (1) above, wherein the compound is represented by the formula: 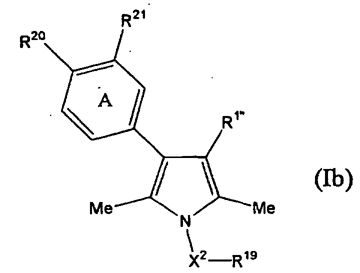 wherein R^{1''} is a cyano group or a group represented by the formula: -COOR^{6''1''} (wherein R^{6''1''} is a methyl group or a ethyl group), R¹⁹ is a C₆₋₁₄ aryl group which may have substituent (s) or a heterocyclic group which may have substituent (s) , at least one of R²⁰ and R²¹ is a nitro group or a cyano group, the ring A may further have substituent(s), and X² is a bivalent group in which the number of straight-chained carbon atom(s) is 1 to 5 which may have substituent(s); or a salt thereof, or its prodrug;
(19) An agent as defined in (1) above, wherein the compound is one selected from the group consisting of (1) ethyl
   1-benzyl-2,5-dimethyl-4-(4-nitrophenyl)-1H-pyrrole-3-carbox ylate or a salt thereof, (2) methyl
   1-benzyl-4-(3-cyanophenyl)-2,5-dimethyl-1H-pyrrole-3-carbox ylate or a salt thereof, (3) methyl
   1-benzyl-4-(4-formylphenyl)-2,5-dimethyl-1H-pyrrole-3-carbo xylate or a salt thereof, (4) methyl
   4-(4-cyanophenyl)-2,5-dimethyl-1-(3-pyridylmethyl)-1H-pyrro le-3-carboxylate or a salt thereof, (5)
   4-(4-cyanophenyl)-2,5-dimethyl-1-(3-pyridylmethyl)-1H-pyrro le-3-carbonitrile or a salt thereof, (6)
   4-(4-cyanophenyl)-2,5-dimethyl-1-((6-methyl-3-pyridyl)methy 1)-1H-pyrrole-3-carbonitrile or a salt thereof and (7)
   4-(4-cyanophenyl)-2,5-dimethyl-1-((6-chloro-3-pyridyl) methyl)-1H-pyrrole-3-carbonitrile or a salt thereof;
(20) An agent as defined in (1) above, wherein the androgen receptor is a normal androgen receptor and/or a mutant androgen receptor;
(21) An agent as defined in (1) above, which is a prophylactic or therapeutic agent for hormone-sensitive cancer in androgen-dependent stage and/or androgen-independent stage;
(22) An agent as defined in (1) above, which is a prophylactic or therapeutic agent for prostate cancer;
(23) A compound represented by the formula: 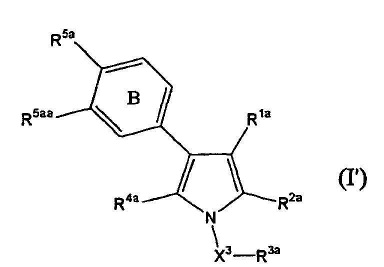 wherein R^{1a} is a hydrogen atom, a group binding through a carbon atom, a group binding through a nitrogen atom, a group binding through an oxygen atom or a group binding through a sulfur atom, R^{2a} is a hydrogen atom, a group binding through a carbon atom, a group binding through a nitrogen atom, a group binding through an oxygen atom or a group binding through a sulfur atom, R^{3a} is an aromatic ring group which may have substituent(s), R^{4a} is a hydrogen atom, a group binding through a carbon atom, a group binding through a nitrogen atom, a group binding through an oxygen atom or a group binding through a sulfur atom, at least one of R^{5a} and R^{5aa} is a nitro group, a cyano group or a formyl group, the ring B may further have substituent(s), and X³ is a bivalent group in which the number of straight-chained carbon atom (s) is 1 to 5, which may have substituent (s) ; (provided that (1) ethyl
   4-(3-cyanophenyl)-1-(2-naphthylmethyl)-1H-pyrrole-3-carboxy late, (2) ethyl
   4-(3-cyanophenyl)-1-(1-naphthylmethyl)-1H-pyrrole-3-carboxy late, (3) 1-benzyl-3-(3-nitrophenyl)-1H-pyrrole, (4)
   1-benzyl-3-methyl-4-(4-nitrophenyl)-1H-pyrrole, (5) tert-butyl
   4-ethyl-1-(4-nitrobenzyl)-3-(4-nitrophenyl)-1H-pyrrole-2-ca rboxylate, (6) tert-butyl
   4-ethyl-1-(4-methoxybenzyl)-3-(4-nitrophenyl)-1H-pyrrole-2-carboxylate, (7) tert-butyl
   1-benzyl-4-ethyl-3-(4-nitrophenyl)-1H-pyrrole-2-carboxylate, (8) tert-butyl
   1-benzyl-4-methyl-3-(4-nitrophenyl)-1H-pyrrole-2-carboxylat e, (9) methyl
   [4-(aminocarbonyl)-5-methyl-3-(4-nitrophenyl)-1-(2-pyridine - 2-ylethyl)-lH-pyrrole-2-yl]acetate, (10)
   1-benzyl-N-{2'-[(tert-butylamino)sulfonyl]-1,1'-biphenyl-4-yl}-4-(3-cyanophenyl)-1H-pyrrole-3-carboxamide, (11)
   3-{1-[(E)-1-cyano-2-(3,5-dichloro-1-methyl-1H-pyrazole-4-yl )-2-hydroxyethenyl]-1H-pyrrole-3-yl}benzonitrile, (12) methyl
   4-(4-amino-3-nitrophenyl)-1-benzyl-2,5-dimethyl-1H-pyrrole-3-carboxylate, (13) methyl
   1-benzoyl-4-(3-nitrophenyl)-1H-pyrrole-3-carboxylate, (14)
   4-[1-benzyl-2-(2-furanyl)-5-phenyl-1H-pyrrole-3-yl]-benzoni trile and (15)
   4-[1-benzyl-2,5-diphenyl-1H-pyrrole-3-yl]-benzonitrile are excluded) a salt thereof, or its prodrug;
(24) A compound as defined in (23) above, wherein X³ is a methylene group which may have substituent (s), R^{3a} is a C₆₋₁₄ aryl group which may have substituent(s), or a 5- to 14-membered aromatic heterocyclic group which may have substituent(s) or a salt thereof, or its prodrug;
(25) A compound as defined in (23) above, wherein X³ is a methylene group which may have substituent(s), and R^{3a} is a 5-to 14-membered aromatic heterocyclic group which may have substituent(s) or a salt thereof, or its prodrug;
(26) A compound represented by the formula: 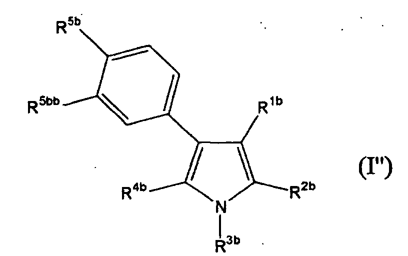 wherein R^{1b} is a cyano group or a group represented by the formula: -COOR^{6'1b} (wherein R^{6'1b} is a C₁₋₆ alkyl group), _{R}^{2b} is a hydrogen atom or a C₁₋₆ alkyl group, R^{3b} is a C₁₋₆ alkyl group which may have substituent (s), a C₇₋₁₅ aralkyl group which may have substituent (s) , a 5- to 14-membered aromatic heterocyclic group-C₁₋₆ alkyl group, a group represented by the formula: -CO-R^{9'3b} (wherein R^{9'3b} is a C₁₋₆ alkyl group which may have substituent (s), a C₆₋₁₄ aryl group which may have substituent (s) or a 5- to 14-membered aromatic heterocyclic group which may have substituent(s)) or a group represented by the formula: - SO₂-R^{12'3b} (wherein R^{12'3b} is a C₁₋₆ alkyl group which may have substituent (s), a C₆₋₁₄ aryl group which may have substituent (s) or a 5- to 14-membered aromatic heterocyclic group which may have substituent (s)), R^{4b} is a hydrogen atom or a C₁₋₆ alkyl group, at least one of R^{5b} and R^{5bb} is a nitro group, a cyano group or a formyl group; (provided (1) ethyl
   4-(3-cyanophenyl)-1-(2-naphthylmethyl)-1H-pyrrole-3-carboxy late, (2) ethyl
   4-(3-cyanophenyl)-1-(1-naphthylmethyl)-1H-pyrrole-3-carboxy late, (3) methyl
   4-(4-amino-3-nitrophenyl)-1-benzyl-2,5-dimethyl-1H-pyrrole-3-carboxylate, (4) methyl
   4-(4-amino-3-nitrophenyl)-1-butyl-2,5-dimethyl-1H-pyrrole-3 - carboxylate, (5) methyl
   4-(4-amino-3-nitrophenyl)-1,2,5-trimethyl-1H-pyrrole-3-carb oxylate, (6) methyl
   1-benzoyl-4-(3-nitrophenyl)-1H-pyrrole-3-carboxylate, (7) dimethyl 4-(3-nitrophenyl)-1H-pyrrole-1,3-dicarboxylate are excluded), its salt, or its prodrug;
(27) A compound as defined in (26) above, wherein R^{1b} is a cyano group or a group represented by the formula: -COOR^{6'1b} (wherein R^{6'1b} is a C₁₋₆ alkyl group), R^{2b} is a C₁₋₆ alkyl group, R^{3b} is a C₁₋₆ alkyl group which may have substituent (s) , a C₇₋₁₅ aralkyl group which may have substituent(s), a 5- to 14-membered aromatic heterocyclic group-C₁₋₆ alkyl group which may have substituent (s) , a group represented by the formula: -CO-R^{9'3b} (wherein R^{9'3b} is a C₁₋₆ alkyl group which may have substituent (s), a C₆₋₁₄ aryl group which may have substituent(s) or a 5- to 14-membered aromatic heterocyclic group which may have substituent(s)) or a group represented by the formula: -SO₂-R^{12'3b} (wherein R^{12'3b} is a C₁₋₆ alkyl group which may have substituent (s) , a C₆₋₁₄ aryl group which may have substituent (s) or a 5- to 14-membered aromatic heterocyclic group which may have substituent(s)), R^{4b} is a C₁₋₆ alkyl group, R^{5b} is a nitro group, a cyano group or a formyl group or a salt thereof, or its prodrug;
(28) A compound as defined in (26) above, wherein R^{3b} is a C₆₋₁₄ aryl-methyl group which may have substituent(s), or a 5- to 14-membered aromatic heterocyclic group-methyl group which may have substituent(s) or a salt thereof, or its prodrug;
(29) A compound as defined in (26) above, wherein R^{3b} is a 5-to 14-membered aromatic heterocyclic group-methyl group which may have substituent(s) a salt thereof, or its prodrug;
(30) A compound as defined in (26) above, wherein R^{1b} is a cyano group or a salt thereof, or its prodrug;
(31) A compound as defined in (26) above, wherein R^{3b} is a benzyl group which may have substituent(s) or a pyridylmethyl group which may have substituent (s) or a salt thereof, or its prodrug;
(32) A compound selected from the group consisting of (1) ethyl
   1-benzyl-2,5-dimethyl-4-(4-nitrophenyl)-1H-pyrrole-3-carbox ylate or its salt, (2) methyl
   1-benzyl-4-(3-cyanophenyl)-2,5-dimethyl-1H-pyrrole-3-carbox ylate or its salt, (3) methyl
   1-benzyl-4-(4-formylphenyl)-2,5-dimethyl-1H-pyrrole-3-carbo xylate or its salt, (4) methyl
   4-(4-cyanophenyl)-2,5-dimethyl-1-(3-pyridylmethyl)-1H-pyrro le-3-carboxylate or its salt, (5)
   4-(4-cyanophenyl)-2,5-dimethyl-1-(3-pyridylmethyl)-1H-pyrro le-3-carbonitrile or its salt, (6)
   4-(4-cyanophenyl)-2,5-dimethyl-1-((6-methyl-3-pyridyl)methy l)-1H-pyrrole-3-carbonitrile or its salt, and (7)
   4-(4-cyanophenyl)-2,5-dimethyl-1-((6-chloro-3-pyridyl)methy l)-1H-pyrrole-3-carbonitrile or its salt;
(33) A medicament containing a compound defined in (23) above or (26) above or a salt thereof, or its prodrug;
(34) A medicament which comprises a compound represented by the formula: 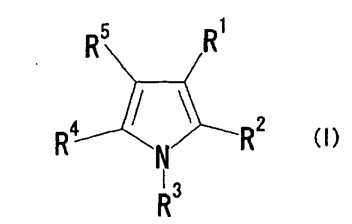 wherein R¹ is a hydrogen atom, a group binding through a carbon atom, a group binding through a nitrogen atom, a group binding through an oxygen atom or a group binding through a sulfur atom, _{R}² is a hydrogen atom, a group binding through a carbon atom, a group binding through a nitrogen atom, a group binding through an oxygen atom or a group binding through a sulfur atom, R³ is a hydrogen atom, a hydrocarbon group which may have substituent (s) , an acyl group or a heterocyclic group which may have substituent(s), R⁴ is a hydrogen atom, a group binding through a carbon atom, a group binding through a nitrogen atom, a group binding through a oxygen atom or a group binding through a sulfur atom, R⁵ is a cyclic group which may have substituent(s); or a salt thereof, or its prodrug and an anticancer drug in combination;
(35) A medicament as defined in (34) above, wherein the anticancer agent is an LH-RH derivative;
(36) A prophylactic or therapeutic agent for a cancer sensitive to a hormone in androgen-dependent stage and/or androgen-independent stage comprising a mutant androgen receptor antagonistic drug;
(37) A prophylactic or therapeutic agent for a cancer sensitive to a hormone in androgen-dependent stage and/or androgen-independent stage which comprises mutant androgen receptor antagonistic drug and an anticancer agent;
(38) An agent as defined in (37) above, wherein the anticancer agent is an LH-RH derivative;
(39) A prophylactic or therapeutic agent for a cancer sensitive to a hormone in androgen-dependent stage and/or androgen-independent stage containing a sensitivity-increased androgen receptor antagonistic drug;
(40) A prophylactic or therapeutic agent for a cancer sensitive to a hormone in androgen-dependent stage and/or androgen-independent stage which comprises a sensitivity-increased androgen receptor antagonistic drug and an anticancer agent;
(41) A prophylactic or therapeutic agent as defined in (40) above, wherein the anticancer agent is an LH-RH derivative;
(42) A method for antagonizing androgen receptor, which comprises administering an effective amount of a compound of the formula: 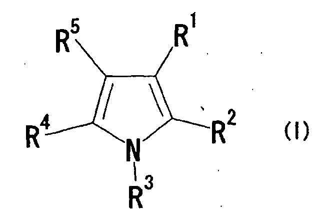 wherein R¹ is a hydrogen atom, a group binding through a carbon atom, a group binding through a nitrogen atom, a group binding through an oxygen atom or a group binding through a sulfur atom, R² is a hydrogen atom, a group binding through a carbon atom, a group binding through a nitrogen atom, a group binding through an oxygen atom or a group binding through a sulfur atom, R³ is. a hydrogen atom, a hydrocarbon group which may have substituent (s), an acyl group or a heterocyclic group which may have substituent(s), R⁴ is a hydrogen atom, a group binding through a carbon atom, a group binding through a nitrogen atom, a group binding through an oxygen atom or a group binding through a sulfur atom, R⁵ is a cyclic group which may have substituent (s); or a salt thereof, or its prodrug, to a mammal;
(43) A method for preventing or treating a cancer sensitive to a hormone in androgen-dependent stage and/or androgen-independent stage, which comprises administering an effective amount of a compound represented by the formula: 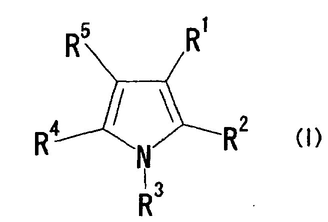 wherein R¹ is a hydrogen atom, a group binding through a carbon atom, a group binding through a nitrogen atom, a group binding through an oxygen atom or a group binding through a sulfur atom, R² is a hydrogen atom, a group binding through a carbon atom, a group binding through a nitrogen atom, a group binding through an oxygen atom or a group binding through a sulfur atom, R³ is a hydrogen atom, a hydrocarbon group which may have substituent (s), an acyl group or a heterocyclic group which may have substituent(s), R⁴ is a hydrogen atom, a group binding through a carbon atom, a group binding through a nitrogen atom, a group binding through an oxygen atom or a group binding through a sulfur atom, R⁵ is a cyclic group which may have substituent (s) ; or a salt thereof, or its prodrug, to a mammal;
(44) A method for preventing or treating prostate cancer, which comprises administering an effective amount of a compound represented by the formula: 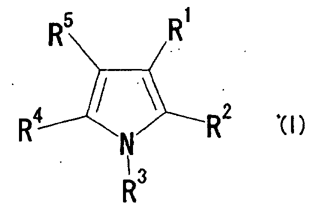 wherein R¹ is a hydrogen atom, a group binding through a carbon atom, a group binding through a nitrogen atom, a group binding through an oxygen atom or a group binding through a sulfur atom, R² is a hydrogen atom, a group binding through a carbon atom, a group binding through a nitrogen atom, a group binding through an oxygen atom or a group binding through a sulfur atom, R³ is a hydrogen atom, a hydrocarbon group which may have substituent (s) , an acyl group or a heterocyclic group which may have substituent(s), R⁴ is a hydrogen atom, a group binding through a carbon atom, a group binding through a nitrogen atom, a group binding through an oxygen atom or a group binding through a sulfur atom, R⁵ is a cyclic group which may have substituent (s) ; or a salt thereof, or its prodrug, to a mammal;
(45) A method as defined in (43) and (44) above in which an effective amount of an anticancer agent is further administered;
(46) A method as defined in (45) above, wherein the anticancer agent is an LH-RH derivative;
(47) A method for preventing or treating a cancer sensitive to a hormone in androgen-dependent stage and/or androgen-independent stage, which comprises administering an effective amount of mutant androgen receptor antagonistic drug to a mammal;
(48) A method as defined in (47) above, in which an effective amount of an anticancer agent is further administered;
(49) A method as defined in (48) above, wherein the anticancer agent is an LH-RH derivative;
(50) A method for preventing or treating a cancer sensitive to a hormone in androgen-dependent stage and/or androgen-independent stage, which comprises administering an effective amount of a sensitivity-increased androgen receptor antagonistic drug to a mammal;
(51) A method as defined in (50) above, in which an effective amount of an anticancer agent is further administered; and
(52) A method as defined in (51) above, wherein the anticancer agent is an LH-RH derivative.

### Best Mode for Carrying out the Invention

When the compound (I), (I') or (I") or a salt thereof has asymmetric carbon (s) , both of optical isomers and racemate are included in the scope of the present invention, and the compound or its salt may be either a hydrate or an anhydrate.

In the present specification, as the "hydrocarbon group" in the "hydrocarbon group which may have substituent (s)", there may be mentioned, for example, a chain- or a cyclic hydrocarbon group (e.g., an alkyl group, an alkenyl group, an alkynyl group, a cycloalkyl group, an aryl group, an aralkyl group, etc.). Among these, a chain- or a cyclic hydrocarbon group having 1 to 16 carbon atom(s), etc., are preferable.

As the "alkyl group", for example, a C₁₋₆ alkyl group (e.g., methyl, ethyl, propyl, isopropyl, butyl, isobutyl, sec-butyl, tert-butyl, pentyl, hexyl, etc.), etc., are preferable.

As the "alkenyl group", for example, a C₂₋₆ alkenyl group (e.g., vinyl, allyl, isopropenyl, 1-butenyl, 2-butenyl, 3-butenyl, 2-methyl-2-propenyl, 1-methyl-2-propenyl, 2-methyl-1-propenyl, etc.), etc., are preferable.

As the "alkynyl group", for example, a C₂₋₆ alkynyl group (e.g., ethynyl, propargyl, 1-butynyl, 2-butynyl, 3-butynyl, 1-hexynyl, etc.), etc., are preferable.

As the "cycloalkyl group", for example, a C₃₋₆ cycloalkyl group (e.g., cyclopropyl, cyclobutyl, cyclopentyl, cyclohexyl, etc.), etc., are preferable.

As the "aryl group", for example, a C₆₋₁₄ aryl group (e.g., phenyl, 1-naphthyl, 2-naphthyl, 2-biphenylyl, 3-biphenylyl, 4-biphenylyl, 2-anthryl, etc.), etc., are preferable, and a C₆₋₁₀ aryl group such as phenyl, etc., are more preferable.

As the "aralkyl group", for example, a C₇₋₁₆ aralkyl group (e.g., benzyl, phenethyl, diphenylmethyl, 1-naphthylmethyl, 2-naphthylmethyl, 2,2-diphenylethyl, 3-phenylpropyl, 4-phenylbutyl, 5-phenylpentyl, etc.), etc., are preferable, and a C₆₋₁₀ aryl-C₁₋₅ alkyl group such as benzyl, etc., are more preferable.

As the "substituent(s)" in the "hydrocarbon group which may have substituent (s) ", there may be mentioned, for example, oxo, a halogen atom (e.g., fluorine, chlorine, bromine, iodine, etc.), a C₁₋₃ alkylenedioxy (e.g., methylenedioxy, ethylenedioxy, etc.), nitro, cyano, a C₁₋₆ alkyl which may be halogenated, a C₂₋₆ alkenyl which may be halogenated, a carboxy-C₂₋₆ alkenyl (e.g., 2-carboxyethenyl, 2-carboxy-2-methyl ethenyl, etc.), a C₂₋₆ alkynyl which may be halogenated, a C₃₋₆ cycloalkyl which may be halogenated, a C₆₋₁₄ aryl (e.g., phenyl, 1-naphthyl, 2-naphthyl, 2-biphenylyl, 3-biphenylyl, 4-biphenylyl, 2-anthryl, etc.), a C₁₋₈ alkoxy which may be halogenated, a C₁₋₆ alkoxy-carbonyl-C₁₋₆ alkoxy (e.g., ethoxycarbonylmethyloxy, etc.), hydroxy, a C₆₋₁₄ aryloxy (e.g., phenyloxy, 1-naphthyloxy, 2-naphthyloxy, etc.), a C₇₋₁₆ aralkyloxy (e.g., benzyloxy, phenethyloxy, etc.), mercapto, a C₁₋₆ alkylthio which may be halogenated, a C₆₋₁₄ arylthio, (e.g., phenylthio, 1-naphthylthio, 2-naphthylthio, etc.), a C₇₋₁₆ aralkylthio (for example, benzylthio, phenethylthio, etc.), amino, a mono-C₁₋₆ alkylamino (e.g., methylamino, ethylamino, etc.), a mono-C₆₋₁₄ arylamino (e.g., phenylamino, 1-naphthylamino, 2-naphthylamino, etc.), a di-C₁₋₆ alkylamino (e.g., dimethylamino, diethylamino, ethylmethylamino, etc.), a di-C₆₋₁₄ arylamino (e.g., diphenylamino, etc.), formyl, carboxy, carboxy-C₂₋₆ alkenyl, carboxy-C₁₋₆ alkyl, a C₁₋₆ alkyl-carbonyl (e.g., acetyl, propionyl, etc.), a C₃₋₆ cycloalkyl-carbonyl (e.g., cyclopropylcarbonyl, cyclopentylcarbonyl, cyclohexylcarbonyl, etc.), a C₁₋₆ alkoxy-carbonyl (e.g., methoxycarbonyl, ethoxycarbonyl, propoxycarbonyl, tert-butoxycarbonyl, etc.), a C₆₋₁₄ aryl-carbonyl (e.g., benzoyl, 1-naphthoyl, 2-naphthoyl, etc.), a C₇₋₁₆ aralkyl-carbonyl (e.g., phenylacetyl, 3-phenylpropionyl, etc.), a C₆₋₁₄ aryloxy-carbonyl (e.g., phenoxycarbonyl, etc.), a C₇₋₁₆ aralkyloxy-carbonyl (e.g., benzyloxycarbonyl, phenethyloxycarbonyl, etc.), a 5- or 6-membered heterocyclic ring-carbonyl (e.g., nicotinoyl, isonicotinoyl, thenoyl, furoyl, morpholinocarbonyl, thiomorpholinocarbonyl, piperazin-1-ylcarbonyl, pyrrolidin-1-ylcarbonyl, etc.), carbamoyl, thiocarbamoyl, a mono-C₁₋₆ alkyl-carbamoyl (e.g., methylcarbamoyl, ethylcarbamoyl, etc.), di-C₁₋₆ alkyl-carbamoyl (e.g., dimethylcarbamoyl, diethylcarbamoyl, ethylmethylcarbamoyl, etc.), a mono- or a di-C₆₋₁₄ aryl-carbamoyl (e.g., phenylcarbamoyl, 1-naphthylcarbamoyl, 2-naphthylcarbamoyl, etc.), a mono- or a di- 5- or 6-membered heterocyclic group-carbamoyl (e.g., 2-pyridylcarbamoyl, 3-pyridylcarbamoyl, 4-pyridylcarbamoyl, 2-thienylcarbamoyl, 3-thienylcarbamoyl, etc.), a C₁₋₆ alkyl-sulfonyl (e.g., methylsulfonyl, ethylsulfonyl, etc.), a C₁₋₆ alkyl-sulfinyl (e.g., methylsulfinyl, ethylsulfinyl, etc.), a C₆₋₁₄ aryl-sulfonyl (e.g., phenylsulfonyl, 1-naphthylsulfonyl, 2-naphthylsulfonyl, etc.), a C₆₋₁₄ aryl-sulfinyl (e.g., phenylsulfinyl, 1-naphthylsulfinyl, 2-naphthylsulfinyl, etc.), formylamino, a C₁₋₆ alkyl-carbonylamino (e.g., acetylamino, etc.), a C₆₋₁₄ aryl-carbonylamino (e.g., benzoylamino, naphthoylamino, etc.), a C₁₋₆ alkoxy-carbonylamino (e.g., methoxycarbonylamino, ethoxycarbonylamino, propoxycarbonylamino, butoxycarbonylamino, etc.), a C₁₋₆ alkyl-sulfonylamino (e.g., methylsulfonylamino, ethylsulfonylamino, etc.), a C₆₋₁₄ aryl-sulfonylamino (e.g., phenylsulfonylamino, 2-naphthylsulfonylamino, 1-naphthylsulfonylamino, etc.), a C₁₋₆ alkyl-carbonyloxy (e.g., acetoxy, propionyloxy, etc.), a C₆₋₁₄ aryl-carbonyloxy (e.g., benzoyloxy, naphthylcarbonyloxy, etc.), a C₁₋₆ alkoxy-carbonyloxy (e.g., methoxycarbonyloxy, ethoxycarbonyloxy, propoxycarbonyloxy, butoxycarbonyloxy, etc.), a mono-C₁₋₆ alkyl-carbamoyloxy (e.g., methylcarbamoyloxy, ethylcarbamoyloxy, etc.), a di-C₁₋₆ alkyl-carbamoyloxy (e.g., dimethylcarbamoyloxy, diethylcarbamoyloxy, etc.), a C₆₋₁₄ aryl-carbamoyloxy (e.g., phenylcarbamoyloxy, naphthylcarbamoyloxy, etc.), nicotinoyloxy, a 5- to 7-membered saturated cyclic amino which may have substituent(s), a 5- to 10-membered aromatic heterocyclic group (e.g., 2-thienyl, 3-thienyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl, etc.), sulpho, etc.

The "hydrocarbon group", for example, may have one to five (preferably one to three) of the above substituent(s) at the substitutable position(s) and when the hydrocarbon group has two or more substituents, the respective substituents may be the same or different from each other.

As the "C₁₋₆ alkyl which may be halogenated", there may be mentioned, for example, a C₁₋₆ alkyl (e.g., methyl, ethyl, propyl, isopropyl, butyl, isobutyl, sec-butyl, tert-butyl, pentyl, hexyl, etc.) which may have one to five, preferably one to three halogen atom(s) (e.g., fluorine, chlorine, bromine, iodine, etc.), etc. Specifically, the examples include methyl, chloromethyl, difluoromethyl, trichloromethyl, trifluoromethyl, ethyl, 2-bromoethyl, 2,2,2-trifluoroethyl, pentafluoroethyl-, propyl, 3,3,3-trifluoropropyl, isopropyl, butyl, 4,4,4-trifluorobutyl, isobutyl, sec-butyl, tert-butyl, pentyl, isopentyl, neopentyl, 5,5,5-trifluoropentyl, hexyl, 6,6,6-trifluorohexyl, etc.

As the "C₂₋₆ alkenyl which may be halogenated", there may be mentioned a C₂₋₆ alkenyl (e.g., vinyl, propenyl, isopropenyl, 2-buten-1-yl, 4-penten-1-yl, 5-hexen-1-yl) which may have one to five, preferably one to three halogen atom(s) (e.g., fluorine, chlorine, bromine, iodine, etc.), etc.

As the "C₂₋₆ alkynyl which may be halogenated", there may be mentioned, for example, a C₂₋₆ alkynyl (e.g., 2-butyn-1-yl; 4-pentyn-1-yl, 5-hexyn-1-yl, etc.) which may have one to five, preferably one to three halogen atom(s) (e.g., fluorine, chlorine, bromine, iodine, etc.), etc.

As the "C₃₋₆ cycloalkyl which may be halogenated", there may be mentioned, for example, a C₃₋₆ cycloalkyl (e.g., cyclopropyl, cyclobutyl, cyclopentyl, cyclohexyl, etc.) which may have one to five, preferably one to three halogen atom(s) (e.g., fluorine, chlorine, bromine, iodine, etc.), etc. Specifically, examples include cyclopropyl, cyclobutyl, cyclopentyl, cyclohexyl, 4,4-dichlorocyclohexyl, 2,2,3,3-tetrafluorocyclopentyl, 4-chlorocyclohexyl, etc.

As the "C₁₋₈ alkoxy which may be halogenated", there may be mentioned, for example, a C₁₋₈ alkoxy (e.g., methoxy, ethoxy, propoxy, isopropoxy, butoxy, isobutoxy, sec-butoxy, pentyloxy, hexyloxy, etc.) which may have one to five, preferably one to three halogen atom(s) (e.g., fluorine, chlorine, bromine, iodine, etc.), etc. Specifically, examples include methoxy, difluoromethoxy, trifluoromethoxy, ethoxy, 2,2,2-trifluoroethoxy, propoxy, isopropoxy, butoxy, 4,4,4-trifluorobutoxy, isobutoxy, sec-butoxy, pentyloxy, hexyloxy, etc.

As the "C₁₋₆ alkylthio which may be halogenated", there may be mentioned, for example, a C₁₋₆ alkylthio (e.g., methylthio, ethylthio, propylthio, isopropylthio, butylthio, sec-butylthio, tert-butylthio, etc.) which may have one to five, preferably one to three halogen atom(s) (e.g., fluorine, chlorine, bromine, iodine, etc.), etc. Specifically, the examples include methylthio, difluoromethylthio, trifluoromethylthio, ethylthio, propylthio, isopropylthio, butylthio, 4,4,4-trifluorobutylthio, pentylthio, hexylthio, etc.

As the "5- to 7-membered saturated cyclic amino" in the "5- to 7-membered saturated cyclic amino which may have substituent(s)", there may be mentioned, for example, 5- to 7-membered saturated cyclic aminos which may contain one or two kind(s) of and one to four hetero atom(s) selected from a nitrogen atom, a sulfur atom and an oxygen atom apart from one nitrogen atom and carbon atom. Specifically, the examples include pyrrolidin-1-yl, piperidino, piperazin-1-yl, morpholino, thiomorpholino, hexahydroazepin-1-yl, etc.

As the substituent (s) in the "5- to 7-membered saturated cyclic amino which may have substituent(s)", there may be mentioned, for example, one to three of a C₁₋₆ alkyl (e.g., methyl, ethyl, propyl, isopropyl, butyl, isobutyl, sec-butyl, tert-butyl, pentyl, hexyl, etc.), a C₆₋₁₄ aryl (e.g., phenyl, 1-naphthyl, 2-naphthyl, 2-biphenylyl, 3-biphenylyl, 4-biphenylyl, 2-anthryl, etc.), a C₁₋₆ alkyl-carbonyl (e.g., acetyl, propionyl, etc.), a 5- to 10-membered aromatic heterocyclic group (e.g., 2-thienyl, 3-thienyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl, etc.), oxo, etc.

In the specification, as the "C₁₋₆ alkyl group", the "C₆₋₁₄ aryl group" and the "C₇₋₁₅ aralkyl group" in the "C₁₋₆ alkyl group which may have substituent(s)", the "C₆₋₁₄ aryl group which may have substituent (s)" and the "C₇₋₁₅ aralkyl group which may have substituent (s)", there may be mentioned those mentioned above, respectively.

In the specification, as the "substituent(s)" in the "C₁₋₆ alkyl group which may have substituent (s)", the "C₆₋₁₄ aryl group which may have substituent (s) " and the "C₇₋₁₅ aralkyl group which may have substituent(s)", there may be mentioned those as mentioned as the substituent (s) in the "hydrocarbon group which may have substituent(s)" mentioned above.

The "C₁₋₆ alkyl group", "C₆₋₁₄ aryl group" and "C₇₋₁₅ aralkyl group", for example, may have one to five (preferably one to three) of the above substituent(s) at the substitutable position(s) and when each of these groups has two or more substituents, the respective substituents may be the same or different from each other.

In the specification, as the "C₁₋₆ alkyl group" and the "heterocyclic group" in the "heterocyclic group-C₁₋₆ alkyl group which may have substituent(s)", the "heterocyclic group-sulfonyl group which may have substituent(s)" and the "heterocyclic group-carbonyl group which may have substituent (s)", there may be mentioned the "C₁₋₆ alkyl group" and the "heterocyclic group" as mentioned above, respectively.

As the "substituent(s)" in the "heterocyclic group-C₁₋₆ alkyl group which may have substituent(s)", "heterocyclic group-sulfonyl group which may have substituent(s)" and "heterocyclic group-carbonyl group which may have substituent(s)", there may be mentioned those as mentioned above as substituent (s) in the "hydrocarbon group which may have substituent(s)".

The "C₁₋₆ alkyl group" and the "heterocyclic group", for example, may have one to five (preferably one to three) of the above substituent (s) at the substitutable position (s) and when each of these groups has two or more substituents, the respective substituents may be the same or different from each other.

In the specification, as the "C₁₋₆ alkyl group" and the "C₆₋₁₄ aryl group" in the "C₁₋₆ alkyl group-sulfonyl group which may have substituent (s) ", the "C₆₋₁₄ aryl group-sulfonyl group which may have substituent (s) ", the "C₁₋₆ alkyl group-carbonyl group which may have substituent(s)" and the "C₆₋₁₄ aryl group-carbonyl group which may have substituent (s) ", there may be mentioned those as mentioned above for the "C₁₋₆ alkyl group" and the "C₆₋₁₄ aryl group", respectively.

As the "substituent (s) " in the "C₁₋₆ alkyl group-sulfonyl group which may have substituent(s)", the "C₆₋₁₄ aryl group-sulfonyl group which may have substituent (s)", the "C₁₋₆ alkyl group-carbonyl group which may have substituent (s) " and the "C₆₋₁₄ aryl group-carbonyl group which may have substituent(s)", there may be mentioned those as mentioned above for the substituent(s) in the "hydrocarbon group which may have substituent(s)".

The "C₁₋₆ alkyl group" and the "C₆₋₁₄ aryl group" may have, for example, one to five (preferably one to three) of the above substituent(s) at the substitutable position(s) and when each of these groups has two or more substituents, the respective substituents may be the same or different from each other.

In the present specification, as the "bivalent group in which the number of straight-chained carbon atom(s) is 1 to 5" in the "bivalent group in which the number of straight-chained carbon atom (s) is 1 to 5" which may have substituent (s) ", there may be mentioned, for example, a straight-chained aliphatic hydrocarbon group in which the number of straight-chained carbon atom(s) is 1 to 5 (e.g., a C₁₋₅ alkyl group such as methyl, ethyl, propyl, isopropyl, butyl, isobutyl, sec-butyl, tert-butyl, pentyl, etc. ; e.g., a C₁₋₅ alkenyl group such as vinyl, allyl, isopropenyl, 1-butenyl, 2-butenyl, 3-butenyl, 2-methyl-2-propenyl, 1-methyl-2-propenyl, 2-methyl-1-propenyl, etc. ; e.g., a C₁₋₅ alkynyl group such as ethynyl, propargyl, 1-butynyl, 2-butynyl, 3-butynyl, etc., etc.), etc. Among these, a C₁₋₅ alkyl group such as a methyl group, an ethyl group, an n-propyl group or the like is preferable.

As the "substituent(s)" in the "bivalent group in which the number of straight-chained carbon atom(s) is 1 to 5 which may have substituent(s)", there may be mentioned those as mentioned above for the substituent(s) in the "hydrocarbon group which may have substituent(s)".

The "bivalent group" may have one to five (preferably one to three) of the above substituent(s) at the substitutable position(s) and when the hydrocarbon group has two or more substituents, the respective substituents may be the same or different from each other.

In the present specification, as the "substituent(s)" in the "benzyl group which may have substituent(s)" and the "pyridylmethyl group which may have substituent (s)", there may be mentioned those as mentioned above for the substituent(s) in the "hydrocarbon group which may have substituent(s).

Each of the "benzyl group" and the "pyridylmethyl group" may have, for example, one to five (preferably one to three) of the above substituent at the substitutable position(s) and when each of these groups has two or more substituents, the respective substituents may be the same or different from each other.

In the specification, as the "heterocyclic group" in the "heterocyclic group which may have substituent (s)", there may be mentioned, for example, a monovalent group formed by removing a hydrogen atom from a 5- to 14-membered (monocyclic, bicyclic or tricyclic) heterocyclic ring containing one or two kind(s) and one to four (preferably one to three) hetero atom(s) optionally selected from a nitrogen atom, a sulfur atom and an oxygen atom apart from the carbon atom(s), preferably, (1) 5-to 14-membered (preferably 5- to 10-membered) aromatic heterocyclic ring, (2) 5- to 10-membered non-aromatic heterocyclic ring or (3) 7- to 10-membered bridged heterocyclic ring.

As the "5- to 14-membered (preferably 5- to 10-membered) aromatic heterocycle", there may be mentioned, for example, aromatic heterocycles such as thiophene, benzo[b]thiophene, benzo[b]furan, benzimidazole, benzoxazole, benzothiazole, benzisothiazole, naphto[2,3-b]thiophene, furan, pyrrole, imidazole, pyrazole, pyridine, pyrazine, pyrimidine, pyridazine, indol, isoindol, 1H-indazole, purine, 4H-quinolizine, isoquinoline, quinoline, phthalazine, naphthyridine, quinoxaline, quinzoline, cinnoline, carbazole, β-carboline, phenanthridine, acridine, phenazine, thiazole, isothiazole, phenothiazine, isoxazole, furazan, phenoxazine, etc. or rings formed by condensing the above heterocycle (preferably monocycle) with one or more (preferably one or two) aromatic ring(s) (e.g., a benzene ring, etc.), etc.

As the "5- to 10-membered non-aromatic heterocyclic ring", there may be mentioned, for example, pyrrolidine, imidazoline, pyrazolidine, pyrazoline, piperidine, piperazine, morpholine, thiomorpholine, dioxazole, oxadiazoline, thiadiazoline, triazoline, thiadiazole, dithiazole, etc.

As the "7- to 10-membered bridged heterocyclic ring", there may be mentioned, for example, quinuclidine, 7-azabicyclo[2.2.1]heptane, etc.

The "heterocyclic group" preferably is a 5- to 14-membered (preferably 5- to 10-membered) (monocyclic or bicyclic) heterocyclic group containing one or two kind(s) and preferably one to four hetero atom(s) selected from a nitrogen atom, a sulfur atom and an oxygen atom.
Specifically, examples include a aromatic heterocyclic group such as 2-thienyl, 3-thienyl, 2-furyl, 3-furyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, pyrazinyl, 2-pyrimidinyl, 4-pyrimidinyl, 3-pyrrolyl, 2-imidazolyl, 3-pyridazinyl, 3-isothiazolyl, 3-isoxazolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl, etc., a non-aromatic heterocyclic group such as 1-pyrrolidinyl, 2-pyrrolidinyl, 3-pyrrolidinyl, 2-imidazolinyl, 4-imidazolinyl, 2-pyrazolidinyl, 3-pyrazolidinyl, 4-pyrazolidinyl, piperidino, 2-piperidyl, 3-piperidyl, 4-piperidyl, 1-piperazinyl, 2-piperazinyl, morpholino, thiomorpholino, etc., and the like.

Among these, for example, a 5- or 6-membered heterocyclic group containing one to three hetero atom(s) selected from a nitrogen atom, a sulfur atom and an oxygen atom apart from the carbon atom(s), etc., are preferable.
Specifically, the examples include 2-thienyl, 3-thienyl, 2-pyridyl, 3-pyridyl, 4-pyridyl, 2-furyl, 3-furyl, pyrazinyl, 2-pyrimidinyl, 3-pyrrolyl, 3-pyridazinyl, 3-isothiazolyl, 3-isoxazolyl, 1-pyrrolidinyl, 2-pyrrolidinyl, 3-pyrrolidinyl, 2-imidazolinyl, 4-imidazolinyl, 2-pyrazolidinyl, 3-pyrazolidinyl, 4-pyrazolidinyl, piperidino, 2-piperidyl, 3-piperidyl, 4-piperidyl, 1-piperazinyl, 2-piperazinyl, morpholino, thiomorpholino, etc.

As the "substituent(s)" in the "heterocyclic group which may have substituent (s)", there may be mentioned those similar to the substituent (s) in the "hydrocarbon group which may have substituent(s)" mentioned above.

The "heterocyclic group" may have, for example, one to five (preferably one to three) of the above substituent(s) at the substitutable position(s) and when the hydrocarbon group has two or more substituents, each substituent may be the same or different from each other.

In the present specification, as an acyl group, there may be mentioned, for example, a group represented by the formula: - COOR⁶¹, -CONR⁷¹R⁸¹, -COR⁹¹, -COOR⁶², -CONR⁷²R⁸², -COR⁹², -COOR⁶⁴, -CONR⁷⁴R⁸⁴, -COR⁹⁴, -COOR^{6'1}, -CONR^{7'1}R^{8'1}, -COR^{9"1}, -COOR^{6'1'}, -CONR^{7'1'}R^{8'1'}, -COR^{9"1'}, -COOR^{6"1"}, -COOR^{6'1b}, -COR^{9'3b}, -(C=S)-NR¹⁰R¹¹, -SO₂-R¹²³ or -SO₂-R^{12'3b} (wherein R⁶¹, R⁷¹, R⁸¹, R⁹¹, R⁶², R⁷², R⁸², R⁹², R⁶⁴, R⁷⁴, R⁸⁴, R⁹⁴, R^{6'1}, R^{7'1}, R^{8'1}, R^{9''1}, R^{6'1'}, R^{7'1'}, R^{8'1'}, R^{9''1'}, R^{6''1''}, R^{6'1b}, R^{9'3b}, R¹²³ and R^{12'3b} have the meanings given above, R¹⁰ and R¹¹ are the same or different, and may be a hydrogen atom, a hydrocarbon group which may have the substituent(s) mentioned above or a heterocyclic group which may have the substituent(s) mentioned above, and R¹⁰ and R¹¹, taken together with a neighboring nitrogen atom, may form a cyclic group which may have substituent(s)). Specifically, examples include, a lower(C₁₋₆)alkanoyl group (e.g., formyl, acetyl, propionyl, butyryl, isobutyryl, valeryl, isovaleryl, pivaloyl, hexanoyl, etc.), a lower (C₃₋₇) alkenoyl group (e.g., acryloyl, methacryloyl, crotonoyl, isocrotonoyl, etc.), a C₄₋₇ cycloalkanecarbonyl group (e.g., cyclopropanecarbonyl group, cyclobutanecarbonyl group, cyclopentanecarbonyl group, cyclohexanecarbonyl group, etc.), a lower (C₁₋₄) alkane sulfonyl group (e.g., mesyl, ethanesulfonyl, propanesulfonyl, etc.), a C₇₋₁₄ aroyl group (e.g., benzoyl, p-toluoyl, 1-naphthoyl, 2-naphthoyl, etc.), a C₆₋₁₀ aryl lower (C₂₋₄)alkanoyl group (e.g., phenylacetyl, phenylpropionyl, hydroatropoyl, phenylbutyryl, etc.), a C₆₋₁₀ aryl lower (C₃₋₅) alkenoyl group (e.g., cinnamoyl, atropoyl, etc.), a C₆₋₁₀ arenesulfonyl group (e.g., benzenesulfonyl, p-toluenesulfonyl group, etc.), etc.

In the present specification, as the "cyclic group" in the "cyclic group which may have substituent(s)", there may be mentioned, for example, a cyclic hydrocarbon group or a heterocyclic group.

As the "cyclic hydrocarbon group", a cyclic hydrocarbon group having 1 to 16 carbon atom(s), etc., are preferable, and a cycloalkyl group and an aryl group, etc. may be used.

As the "cycloalkyl group", for example, a C₃₋₆ cycloalkyl group (for example, cyclopropyl, cyclobutyl, cyclopentyl, cyclohexyl, etc.), etc., are preferable.

As the "aryl group", for example, a C₆₋₁₄ aryl group (for example, phenyl, 1-naphthyl, 2-naphthyl, 2-biphenylyl, 3-biphenylyl, 4-biphenylyl, 2-anthryl, etc.), etc., are preferable.

As the heterocyclic group, those as mentioned above may be used.

As the "substituent(s)" in the "cyclic group which may have substituent (s) ", there maybe mentioned those as mentioned above for the substituent(s) in the "hydrocarbon group which may have substituent(s)".

The "cyclic group", for example, may have one to five (preferably one to three) of the above substituent(s) at the substitutable position(s) and when the hydrocarbon group has two or more substituents, the respective substituents may be the same or different from each other.

In the present specification, as the group that is bonded through the carbon atom, there may be used a cyano group, the hydrocarbon group which may have substituent(s) mentioned above, an acyl group mentioned above, a heterocyclic group which may have substituent (s), which is formed by removing a hydrogen atom substituted on a carbon atom from the heterocyclic ring which may have substituent(s) mentioned above; etc.

In the specification, as the "group that is bonded through the nitrogen atom", there may be used, for example, an amino group which may have substituent (s) , a heterocyclic group which may have substituent(s) formed by removing a hydrogen atom present on a nitrogen atom from the heterocyclic group which may have substituent(s) mentioned above.

As the "amino group which may have substituent(s)", (1) an amino group which may have one or two substituent (s) and (2) a cyclic amino group which may have substituent (s) may be used.

As the "substituent" in the "(1) amino group which may have one or two substituent (s) ", there may be used, for example, a hydrocarbon group which may have substituent(s) mentioned above, a heterocyclic group which may have substituent(s) mentioned above, an acyl group mentioned above, an alkylydene group which may have substituent(s); etc.

As the "alkylydene group" in the "alkylydene group which may have substituent (s) ", there may be used, for example, a C₁₋₆ alkylydene group (for example, methylidene, ethylidene, propylidene, etc.), etc.

As the "substituent(s)" in the "alkylidene group which may have substituent (s)", there may be mentioned those similar to the substituent (s) in the "hydrocarbon group which may have substituent(s)" mentioned above. The number of the substituent (s) is usually one to five, preferably one to three.

When the number of the "substituent(s)" in the "amino group which may have one or two substituent (s)" is two, the two substituents may be the same or different from each other.

As the "cyclic amino group" in the "cyclic amino group which may have substituent (s) " in the above (2) , there may be used, for example, a 5- to 7-membered non-aromatic cyclic amino group which may contain one or two kind (s) and one to four hetero atom(s) selected from a nitrogen atom, a sulfur atom and an oxygen atom apart from the nitrogen atom and carbon atom(s); etc., and specifically, pyrrolidin-1-yl, piperidino, piperazin-1-yl, morpholino, thiomorpholino, hexahydroazepin-1-yl, imidazolidin-1-yl, 2,3-dihydro-1H-imidazol-1-yl, tetrahydro-1(2H)-pyrimidinyl, 3,6-dihydro-1(2H)-pyrimidinyl, 3,4-dihydro-1(2H)-pyrimidinyl, etc. may be used.

As the "substituent (s) " in the "cyclic amino group which may have substituent(s)", there may be mentioned, for example, those similar to the substituent(s) in the "5- to 7-membered saturated cyclic amino group which may have substituent(s)" mentioned above as the "substituent(s)" in the "hydrocarbon group which may have substituent(s)". The number of the substituent is one to three.

As specific examples of the 5- to 7-membered non-aromatic cyclic amino group having an oxo group, there may be used 2-oxo imidazolidin-1-yl, 2-oxo-2,3-dihydro-1H-imidazol-1-yl, 2-oxotetrahydro-1(2H)-pyrimidinyl, 2-oxo-3,6-dihydro-1(2H)-pyrimidinyl, 2-oxo-3,4-dihydro-1(2H)-pyrimidinyl, 2-oxopyrrolidin-1-yl, 2-oxopiperidino, 2-oxopiperazin-1-yl, 3-oxopiperazin-1-yl, 2-oxo-2,3,4,5,6,7-hexahydroazepin-1-yl, etc.

In the present specification, as the "group that is bonded through the oxygen atom", there may be used, for example, a group represented by the formula: -OR¹³¹, -OR¹³² or -OR¹³⁴ (wherein R¹³¹, R¹³² and R¹³⁴ have the meanings given above), etc.

In the present specification, as the "group that is bonded through the sulfur atom", there may be used, for example, a group represented by the formula: -SR¹⁴ (wherein R¹⁴ is a hydrogen atom or a hydrocarbon group which may have substituent (s) mentioned above), a group represented by the formula: -SO-R¹⁵ (wherein _{R}¹⁵ is a hydrogen atom or a hydrocarbon group which may have substituent(s) mentioned above), a group represented by the formula: -SO₂-R¹²³ (wherein R¹²³ is a hydrogen atom or a hydrocarbon group which may have substituent(s) mentioned above), a group represented by the formula: -SO₂-R^{12'3b} (wherein R¹²'^{3b} has the meaning given above) and a sulfur-containing heterocyclic group which may have substituent(s) formed by removing a hydrogen atom substituted on a sulfur atom from a sulfur-containing heterocycle which may have substituent(s).

In the specification, as the "sulfur-containing heterocyclic group" in the "sulfur-containing heterocyclic group which may have substituent (s) ", there may be mentioned, for example, a monovalent group formed by removing an optional hydrogen atom from a 5- to 14-membered (monocyclic, bicyclic or tricyclic) sulfur-containing heterocycle containing one or two kind(s) of and one to four (preferably one to three) hetero atom(s) selected from a nitrogen atom, a sulfur atom and an oxygen atom apart from the carbon atom(s) and sulfur atom, preferably, (1) 5- to 14-membered (preferably 5- to 10-membered) aromatic heterocyclic group or (2) 5- to 10-membered non-aromatic sulfur-containing heterocyclic group.

As the "5- to 14-membered (preferably 5- to 10-membered) aromatic sulfur-containing heterocycle, there may be mentioned, for example, an aromatic sulfur-containing heterocycle such as thiophene, benzo[b]thiophene, naphto[2,3-b]thiophene, thiazole, isothiazole, etc., a ring formed by condensing the above ring (preferably monocycle) with one or more (preferably one or two) of an aromatic ring(s) (e.g., benzene ring, etc.); etc.

As the "5- to 10-membered aromatic sulfur-containing heterocyclic ring", there may be mentioned, for example, thiomorpholine, thiadiazole, dithiazole, etc.

As the "substituent(s)" in the "sulfur-containing heterocyclic group which may have substituent(s)", there may be mentioned those similar to the substituent(s) in the "hydrocarbon group which may have substituent (s) " as mentioned above.

The "sulfur-containing heterocyclic group", for example, may have one to five (preferably one to three) of the above substituent(s) at the substitutable position(s) and when the hydrocarbon group has two or more substituents, the respective substituents may be the same or different from each other.

In the present specification, as the "aromatic group" in the "aromatic group which may have substituent (s)", for example, an aromatic hydrocarbon group or an aromatic heterocyclic group may be used.

As the aromatic hydrocarbon group, an aromatic hydrocarbon group having 1 to 16 carbon atom(s), etc. is preferable, and specifically an aryl group may be used.

As the "aryl group", for example, a C₆₋₁₄ aryl group (for example, phenyl, 1-naphthyl, 2-naphthyl, 2-biphenylyl, 3-biphenylyl, 4-biphenylyl, 2-anthryl, etc.), etc., are preferable.

As the "aromatic heterocyclic group", there may be mentioned, for example, a monovalent group formed by removing an optional hydrogen atom from a 5- to 14-membered (monocyclic, bicyclic or tricyclic) aromatic heterocyclic ring containing one or two kind(s) and one to four (preferably one to three) hetero atom(s) selected from a nitrogen atom, a sulfur atom and an oxygen atom apart from the carbon atom(s), preferably, (1) 5- to 14-membered (preferably 5- to 10-membered) aromatic heterocyclic ring or (2) 7- to 10-membered heterocyclic bridged ring.

As the "5- to 14-membered (preferably 5- to 10-membered) aromatic heterocycle", there may be mentioned, for example, an aromatic heterocycle such as thiophene, benzo[b]thiophene, benzo[b]furan, benzimidazole, benzoxazole, benzothiazole, benzisothiazole, naphto[2,3-b]thiophene, furan, pyrrole, imidazole, pyrazole, pyridine, pyrazine, pyrimidine, pyridazine, indol, isoindol, 1H-indazole, purine, 4H-quinolizine, isoquinoline, quinoline, phthalazine, naphthyridine, quinoxaline, quinzoline, cinnoline, carbazole, β-carboline, phenanthridine, acridine, phenazine, thiazole, isothiazole, phenothiazine, isoxazole, furazan, phenoxazine, etc. or a ring formed by condensing the above ring (preferably monocycle) with one or more (preferably one or two) of an aromatic ring(s) (e.g., benzene ring, etc.); etc.

As the "7- to 10-membered heterocyclic bridged ring", there may be used, for example, quinuclidine, 7-azabicyclo [2. 2. 1]heptane, etc.

The "aromatic heterocyclic group" preferably is a 5- to 14-membered (preferably 5- to 10-membered) (monocyclic or bicyclic) aromatic heterocyclic group containing one or two kind(s) and preferably one to four hetero atom(s) selected from a nitrogen atom, a sulfur atom and an oxygen atom apart from the carbon atom(s). Specifically, there may be used, for example, 2-thienyl, 3-thienyl, 2-furyl, 3-furyl, 2-pyridyl, 3-pyridyl, 4-pyridyl, 2-quinolyl, 3-quinolyl, 4-quinolyl, 5-quinolyl, 8-quinolyl, 1-isoquinolyl, 3-isoquinolyl, 4-isoquinolyl, 5-isoquinolyl, pyrazinyl, 2-pyrimidinyl, 4-pyrimidinyl, 3-pyrrolyl, 2-imidazolyl, 3-pyridazinyl, 3-isothiazolyl, 3-isoxazolyl, 1-indolyl, 2-indolyl, 3-indolyl, 2-benzothiazolyl, 2-benzo[b]thienyl, 3-benzo[b]thienyl, 2-benzo[b]furanyl, 3-benzo[b]furanyl, etc.

Among these, for example, 5- or 6-membered aromatic heterocyclic group containing one to five hetero atom(s) selected from a nitrogen atom, a sulfur atom and an oxygen atom apart from the carbon atom(s), etc., are more preferable. Specifically, there may be mentioned 2-thienyl, 3-thienyl, 2-pyridyl, 3-pyridyl, 4-pyridyl, 2-furyl, 3-furyl, pyrazinyl, 2-pyrimidinyl, 3-pyrrolyl, 3-pyridazinyl, 3-isothiazolyl, 3-isoxazolyl, 1-pyrrolidinyl, 2-pyrrolidinyl, 3-pyrrolidinyl, 2-imidazolinyl, 4-imidazolinyl, 2-pyrazolidinyl, 3-pyrazolidinyl, 4-pyrazolidinyl, etc.

As the "substituent (s) " in the "heterocyclic group which may have substituent (s)", there may be mentioned those similar to the substituent (s) in the "hydrocarbon group which may have substituent(s)" mentioned above.

The "aromatic group" may have one to five (preferably one to three) of the above substituent(s) at the substitutable position(s) and when the hydrocarbon group has two or more substituents, the respective substituents may be the same or different from each other.

As the "substituent (s)" of the ring A and the ring B, there may be mentioned those similar to the substituent(s) in the "hydrocarbon group which may have substituent (s)" as mentioned above.

R¹ is a hydrogen atom, a group that is bonded through the carbon atom, a group that is bonded through the nitrogen atom, a group that is bonded through the oxygen atom or a group that is bonded through the sulfur atom.

As R¹, a hydrogen atom, a hydrocarbon group which may have substituent(s), an acyl group, a heterocyclic group which may have substituent (s) or a group that is bonded through the oxygen atom, etc., are preferable. Among these, a hydrogen atom, a hydrocarbon group which may have substituent(s), a group represented by the formula: -COOR⁶¹ (wherein R⁶¹ has the meaning given above), a group represented by the formula: -CONR⁷¹R⁸¹ (wherein R⁶¹ and R⁷¹ have the meanings given above), a group represented by the formula: -COR⁹¹ (wherein R⁹¹ has the meaning given above) or a group represented by the formula: -OR¹³¹ (wherein R¹³¹ has the meaning given above) , etc., are preferable. Particularly, as R¹, a group represented by the formula: -COOR⁶¹ (wherein R⁶¹ is a hydrocarbon group which may have substituent(s)) is preferable. As R⁶¹, a C₁₋₆ alkyl group such as methyl, ethyl, propyl, n-butyl, tert-butyl, etc. is preferable.

R² is a hydrogen atom, a group that is bonded through the carbon atom, a group that is bonded through the nitrogen atom, a group that is bonded through the oxygen atom or a group that is bonded through the sulfur atom.

As R², a hydrogen atom, a hydrocarbon group which may have substituent(s), a group represented by the formula: -COOR⁶² (wherein R⁶² is a hydrocarbon group which may have substituent (s) ) , a group represented by the formula: -CONR⁷²R⁸² (wherein R⁷² and R⁸² have the meanings given above), a group represented by the formula: -COR⁹², (wherein R⁹² has the meaning given above) and a group represented by the formula: -OR¹³² (wherein R¹³² has the meaning given above) are preferable. Among these, a hydrocarbon group which may have substituent (s) is more preferable, and in particular a C₁₋₆ alkyl group (e.g., methyl, ethyl or the like) is even more preferable.
R³ is a hydrogen atom, a hydrocarbon group which may have substituent(s), an acyl group, a heterocyclic group which may have substituent(s) or a group represented by the formula: -SO₂-R¹²³ (wherein R¹²³ has the meaning given above).

As R³, a C₁₋₆ alkyl group (e.g., methyl, ethyl, propyl, n-butyl, etc.) which may have substituent(s) (e.g., hydroxy), a C₇₋₁₅ aralkyl group (e.g., benzyl, phenylethyl, etc.) which may have substituent(s), a group represented by the formula: - COR^{9'3} (wherein R^{9'3} is a C₁₋₆ alkyl group which may have substituent(s) or a C₇₋₁₅ aralkyl group (e.g., benzyl, phenylethyl, etc.) which may have substituent(s)) and a group represented by the formula: -SO₂-R^{12'3} (wherein R^{12'3} is a C₁₋₆ alkyl group (e.g., methyl, ethyl, etc.) which may have substituent(s) or a C₇₋₁₅ aralkyl group (e.g., benzyl, phenylethyl, etc.) which may have substituent(s)); are preferable, particularly a C₁₋₆ alkyl group (e.g., methyl, ethyl, propyl, n-butyl, etc.) which may have substituent(s), a C₇₋₁₅ aralkyl group (e.g., benzyl, phenylethyl, etc.) which may have substituent(s), a C₁₋₆ alkyl-sulfonyl group (e.g., methylsulfonyl, etc.) which may have substituent (s) and a C₆₋₁₄ aryl-carbonyl group (e.g., benzoyl, etc.) are more preferable.

As _{R}³, a benzyl group which may have substituent(s) and a heterocyclic group-ethyl group which may have substituent (s) are still more preferable, and a heterocyclic ring ethyl group which may have substituent(s) is the most preferable.

R⁴ is a hydrogen atom, a group that is bonded through the carbon atom, a group that is bonded through the nitrogen atom, a group that is bonded through the oxygen atom or a group that is bonded through the sulfur atom.

As R⁴, a hydrogen atom, a hydrocarbon group which may have substituent(s), a group represented by the formula: -COOR⁶⁴ (wherein R⁶⁴ has the meaning given above), a group represented by the formula: -CONR⁷⁴R⁸⁴ (wherein R⁷⁴ and R⁸⁴ have the meanings given above), a group represented by the formula: -COR⁹⁴ (wherein R⁹⁴ has the meaning given above), and a group represented by the formula: -OR¹³⁴ (wherein R¹³⁴ has the meaning given above), etc., are preferable, and particularly a C₁₋₆ alkyl group (e.g., methyl, ethyl, or the like ) which may have substituent(s) is more preferable.

R⁵ is a cyclic group which may have substituent(s).

As R⁵, a cyclic hydrocarbon group which may have substituent(s) is preferable, and particularly an aryl group (e.g., phenyl and the like) such as a C₆₋₁₄ aryl group which may have substituent(s) (e.g., nitro, cyano, formyl, etc.), etc., are more preferable. Among the substitutable positions on phenyl group, para position is preferable while there are no restrictions.

As the compound (I) of the present invention, the following compounds (A) to (E) are preferably used.

### [Compound (A)]

Compound (I) wherein R¹ is a C₁₋₆ alkyl group which may have hydroxy, a group represented by the formula: -COOR^{6'1} (wherein R^{6'1} is a C₁₋₆ alkyl group), a group represented by the formula: -CONR^{7'1}R^{8'1} (wherein R^{7'1} and R^{8'1} are each a hydrogen atom, a C₁₋₆ alkyl group or a C₆₋₁₀ aryl group, and R^{7'1} and R^{8'1} are taken together to form a cyclic group which may have substituent(s)) or a group represented by the formula: -COR^{9''1} (wherein R^{9''1} is a C₁₋₆ alkyl group, a C₃₋₈ cycloalkyl group or a heterocyclic group which may have substituent(s)); R² and R⁴ are each a hydrogen atom or a C₁₋₆ alkyl group; R³ is a hydrogen atom or a hydrocarbon group which may have substituent(s) or a heterocyclic group which may have substituent (s) ; and R⁵ is an aryl group which may have substituent (s), a C₃₋₈ cycloalkyl group which may have substituent (s) or a heterocyclic group which may have substituent(s).

### [Compound (B)]

Compound (I) wherein R¹ is a group represented by, the formula: - COOR^{6'1} (wherein R^{6'1} is a C₁₋₆ alkyl group); R² is a C₁₋₆ alkyl group which may have substituent(s); R³ is a C₁₋₆ alkyl group which may have substituent (s), a C₇₋₁₅ aralkyl group which may have substituent (s) , a C₁₋₆ alkyl-sulfonyl group which may have substituent(s) or a C₆₋₁₄ aryl-carbonyl group which may have substituent(s); R⁴ is a C₁₋₆ alkyl group which may have substituent(s); and R⁵ a C₆₋₁₄ aryl group which may have substituent(s).

### [Compound (C)]

Compound (1) wherein R¹ is a group represented by the. formula: -COOR^{6'1} (wherein R^{6'1} is a C₁₋₆ alkyl); R² is a C₁₋₆ alkyl group; R³ is a C₁₋₆ alkyl group which may have hydroxy(s), a C₇₋₁₅ aralkyl group, a C₁₋₆ alkyl-sulfonyl group or a C₆₋₁₄ aryl-carbonyl group; R⁴ is a C₁₋₆ alkyl group; R⁵ is a C₆₋₁₄ aryl which may have nitro, cyano or a C₁₋₃ acyl group.

### [Compound (D)]

Compound represented by the formula: 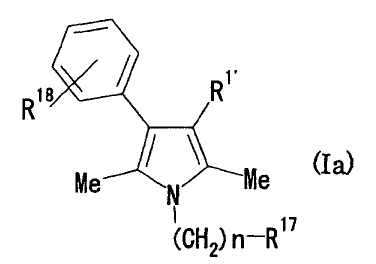 [wherein R^{1'} is a C₁₋₆ alkyl group, a group represented by the formula: -COOR^{6'1'} (wherein R^{6'1'} is a C₁₋₆ alkyl group), a group represented by the formula: -CONR^{7'1'}R^{8'1'} (wherein R^{7'1'} and R^{8'1'} are each a hydrogen atom, a C₁₋₆ alkyl group or a C₆₋₁₀ aryl group), a group represented by the formula: -C (OH) R^{161'}R^{16'1'} (wherein R^{161'} and R^{16'1'} are each a hydrogen atom or a C₁₋₆ alkyl group), a,group represented by the formula: -COR^{9''1'} (wherein R^{9''1'} is a C₁₋₆ alkyl group, a C₃₋₈ cycloalkyl group or a heterocyclic group which may have substituent(s)); R¹⁷ is a C₆₋₁₄ aryl group which may have substituent(s) or a heterocyclic group which may have substituent(s); R¹⁸ is a nitro group, a C₁₋₆ alkyl group which may have substituent(s), a halogen atom, a C₁₋₆ alkoxy group, a cyano group, a carboxyl group, a carboxylic acid ester group, a hydroxy group or an amido group; n is an integer from one to three].

### [Compound (E)]

Compound represented by the formula: 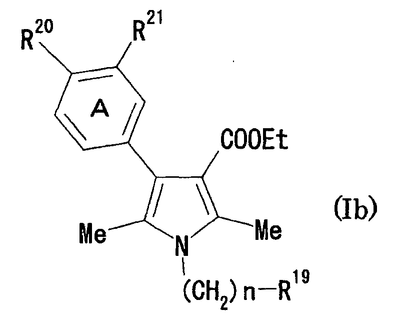 [wherein R¹⁹ is a C₆₋₁₄ aryl group (e.g., phenyl) which may have substituent(s) or a heterocyclic group which may have substituent (s) ; one of R²⁰ and R²¹ is a nitro group or a cyano group; ring A may further have substituent (s) (e.g., a C₁₋₆ alkyl, a halogen atom (e.g., chlorine), nitro, cyano, formyl); and n is an integer from one to three].

Specifically, as the compound (I) of the present invention, compounds described in Example 1 to 251 shown below are preferable. Among these, 1) ethyl
1-benzyl-2,5-dimethyl-4-(4-nitrophenyl)-1H-pyrrole-3-carbox ylate, 2) methyl
1-benzyl-4-(3-cyanophenyl)-2,5-dimethyl-1H-pyrrole-3-carbox ylate, 3) methyl
1-benzyl-4-(4-formylphenyl)-2,5-dimethyl-1H-pyrrole-3-carbo xylate, 4) methyl
4-(4-cyanophenyl)-2,5-dimethyl-1-(3-pyridylmethyl)-1H-pyrro le-3-carboxylate or salt thereof, 5)
4-(4-cyanophenyl)-2,5-dimethyl-1-(3-pyridylmethyl)-1H-pyrro le-3-carbonitrile or salt thereof, 6)
4-(4-cyanophenyl)-2,5-dimethyl-1-((6-methyl-3-pyridyl) methyl)-1H-pyrrole-3-carbonitrile or salt thereof, and 7)
4-(4-cyanophenyl)-2,5-dimethyl-1-((6-chloro-3-pyridyl) methyl)-1H-pyrrole-3-carbonitrile or salt thereof, etc., are more preferable.

Among the compound (I) of the present invention,
(1) a compound represented by the formula: 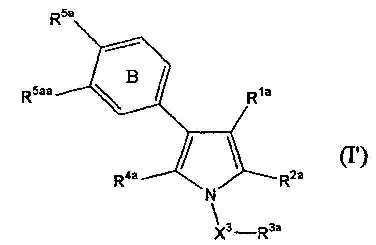 [wherein R^{1a} is a hydrogen atom, a group that is bonded through the carbon atom, a group that is bonded through the nitrogen atom, a group that is bonded through the oxygen atom or a group that is bonded through the sulfur atom; R^{2a} is a hydrogen atom, a group that is bonded through the carbon atom, a group that is bonded through the nitrogen atom, a group that is bonded through the oxygen atom or a group that is bonded through the sulfur atom; R^{3a} is an aromatic group which may have substituent(s); R^{4a} is a hydrogen atom, a group that is bonded through the carbon atom, a group that is bonded through the nitrogen atom, a group that is bonded through the oxygen atom or a group that is bonded through the sulfur atom; at least one of R^{5a} and R^{5aa} is a nitro group, a cyano group or a formyl group; ring B may further have substituent(s); and X³ is a bivalent group in which the number of straight-chained carbon atoms is 1 to 5 which may have substituent(s) ] (provided that (1) ethyl
   4-(3-cyanophenyl)-1-(2-naphthylmethyl)-1H-pyrrole-3-carboxy late, (2) ethyl
   4-(3-cyanophenyl)-1-(1-naphthylmethyl)-1H-pyrrole-3-carboxy late, (3) 1-benzyl-3-(3-nitrophenyl)-1H-pyrrole, (4)
   1-benzyl-3-methyl-4-(4-nitrophenyl)-1H-pyrrole, (5) tert-butyl
   4-ethyl-1-(4-nitrobenzyl)-3-(4-nitrophenyl)-1H-pyrrole-2-ca rboxylate, (6) tert-butyl
   4-ethyl-1-(4-methoxybenzyl)-3-(4-nitrophenyl)-1H-pyrrole-2-carboxylate, (7) tert-butyl
   1-benzyl-4-ethyl-3-(4-nitrophenyl)-1H-pyrrole-2-carboxylate, (8) tert-butyl
   1-benzyl-4-methyl-3-(4-nitrophenyl)-1H-pyrrole-2-carboxylat e, (9) methyl
   [4-(aminocarbonyl)-5-methyl-3-(4-nitrophenyl)-1-(2-pyridin-2-ylethyl)-1H-pyrrol-2-yl] acetate, (10)
   1-benzyl-N-{2'-[(tert-butylamino)sulfonyl]-1,1'-biphenyl-4-yl}-4-(3-cyanophenyl)-1H-pyrrole-3-carboxamide, (11)
   3-{1-[(E)-1-cyano-2-(3,5-dichloro-1-methyl-1H-pyrazol-4-yl) - 2-hydroxyethenyl]-1H-pyrrol-3-yl}benzonitrile, (12) methyl
   4-(4-amino-3-nitrophenyl)-1-benzyl-2,5-dimethyl-1H-pyrrole-3-carboxylate, (13) methyl
   1-benzoyl-4-(3-nitrophenyl)-1H-pyrrole-3-carboxylate, (14)
   4-[1-benzyl-2-(2-furanyl)-5-phenyl-1H-pyrrol-3-yl]-benzonit rile and (15)
   4-[1-benzyl-2,5-diphenyl-1H-pyrrol-3-yl]-benzonitrile are excluded), a salt thereof or a prodrug thereof ; or
(2) compound represented by the formula: 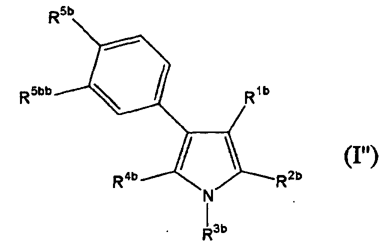 [wherein R^{1b} is a cyano group or a group represented by the formula: -COOR^{6'1b} (wherein R^{6'1b} is a C₁₋₆ alkyl group); R^{2b} is a hydrogen atom or a C₁₋₆ alkyl group; R^{3b} is a C₁₋₆ alkyl group which may have substituent (s) , a C₇₋₁₅ aralkyl group which may have substituent (s) , a 5- to 14-membered aromatic heterocyclic group-C₁₋₆ alkyl group which may have substituent (s), a group represented by the formula: -CO-R^{9'3b} (wherein R^{9'3b} is a C₁₋₆ alkyl group which may have substituent(s), a C₆₋₁₄ aryl group which may have substituent(s) or a 5- to 14-membered aromatic heterocyclic group which may have substituent(s)) or a group represented by the formula: -SO₂-R^{12'3b} (wherein R^{12'3b} is a C₁₋₆ alkyl group which may have substituent(s), a C₆₋₁₄ aryl group which may have substituent (s) or a 5- to 14-membered aromatic heterocyclic group which may have substituent(s)); R^{4b} is a hydrogen atom or a C₁₋₆ alkyl group; and at least one of R^{5b} and R^{5bb} is a nitro group, a cyano group or a formyl group] (provided that (1) ethyl
   4-(3-cyanophenyl)-1-(2-naphthylmethyl)-1H-pyrrole-3-carboxy late, (2) ethyl
   4-(3-cyanophenyl)-1-(1-naphthylmethyl)-1H-pyrrole-3-carboxy late, (3) methyl
   4-(4-amino-3-nitrophenyl)-1-benzyl-2,5-dimethyl-1H-pyrrole-3-carboxylate, (4) methyl
   4-(4-amino-3-nitrophenyl)-1-butyl-2,5-dimethyl-1H-pyrrole-3 - carboxylate, (5) methyl
   4-(4-amino-3-nitrophenyl)-1,2,5-trimethyl-1H-pyrrole-3-carb oxylate, (6) methyl
   1-benzoyl-4-(3-nitrophenyl)-1H-pyrrole-3-carboxylate and (7) dimethyl 4-(3-nitrophenyl)-1H-pyrrole-1,3-dicarboxylate are excluded), a salt thereof or prodrug thereof, are novel compounds.

In the above formula (I'), as R^{1a}, a group represented by the formula: -COOR^{6'1a} (wherein R^{6'1a} is a C₁₋₆ alkyl group) is preferable. As R^{6'1a}, methyl, ethyl, propyl, n-butyl, tert-butyl, etc., are preferable and particularly methyl and ethyl are more preferable.

As R^{2a}, a C₁₋₆ alkyl group such as methyl, ethyl, etc., is preferable and particularly methyl is more preferable.

As R^{3a}, a C₁₋₆ alkyl group (e.g., methyl, ethyl, propyl, butyl or the like) which may have substituent (s) such as hydroxy, a C₇₋₁₅ aralkyl group (e.g., benzyl, phenylethyl, etc.) which may have substituent(s), a group represented by the formula: - COR^{9'''3a} (wherein R^{9'''3a} is a C₁₋₆ alkyl group (e.g., methyl, ethyl or the like) which may have substituent (s), or a C₆₋₁₄ aryl group (e.g., phenylor the like) which may have substituent (s)), a group represented by the formula: -SO₂-R^{12'3a} (wherein R^{12'3a} is a C₁₋₆ alkyl group (e.g., methyl, ethyl or the like) which may have substituent(s) or a C₆₋₁₄ aryl group (e.g., phenyl or the like) which may have substituent (s)), etc., is preferable. Among these, methyl, ethyl, propyl, n-butyl, hydroxybutyl, methylsulfonyl, benzoyl, phenylsulfonyl, benzyl, phenylethyl, etc., are more preferable, and prticularly, benzyl is even more preferable.

As R^{4a}, a C₁₋₆ alkyl group such as methyl, ethyl, etc. is preferable, and methyl is more preferable.

It is preferable that one of R^{5a} and R^{5aa} is a nitro group, cyano group or a formyl group, and the other is a hydrogen atom, a C₁₋₆ alkyl group (e.g., methyl, ethyl, etc.), a halogen atom (e.g., fluorine, chlorine, bromine, iodine, etc., and more preferably chlorine), nitro group, a cyano group or a formyl group or the ring B has a hydrogen atom, a C₁₋₆ alkyl group (e.g., methyl, ethyl, etc.), a halogen atom (e.g., fluorine, chlorine, bromine, iodine, etc., and more preferably chlorine), nitro group, a cyano group or a formyl group at a substitutable position.

By m, an integer from one to five, preferably one or two, most preferably one, is denoted.

In the above formula (I''), as a C₁₋₆ alkyl group represented by R^{6'1b}, methyl, ethyl, propyl, n-butyl, tert-butyl, etc., are preferable, and particularly methyl and ethyl are more preferable.

As the C₁₋₆ alkyl group represented by R^{2b}, methyl and ethyl, etc., are preferable, and particularly methyl is more preferable.

As the cyclic group which may have substituent(s) represented by R^{3b}, a C₆₋₁₄ aryl group (e.g., phenyl, etc.) which may have substituent(s), etc., are preferable.

As a C₁₋₆ alkyl group represented by R^{4b}, methyl, ethyl, etc., are preferable, and particularly methyl is more preferable.
As R^{5b}, a nitro group is preferable.

Specifically, as the compound (I') or (I"), compounds produced in Examples 1 to 251 shown below, or salt thereof are preferable, and among these 1) ethyl
1-benzyl-2,5-dimethyl-4-(4-nitrophenyl)-1H-pyrrole-3-carbox ylate, 2) methyl
1-benzyl-4-(3-cyanophenyl)-2,5-dimethyl-1H-pyrrole-3-carbox ylate, 3) methyl
1-benzyl-4-(4-formylphenyl)-2,5-dimethyl-1H-pyrrole-3-carbo xylate, 4) methyl
4-(4-cyanophenyl)-2,5-dimethyl-1-(3-pyridylmethyl)-1H-pyrro le-3-carboxylate or salt thereof, 5)
4-(4-cyanophenyl)-2,5-dimethyl-1-(3-pyridylmethyl)-1H-pyrro le-3-carbonitrile or salt thereof, 6)
4-(4-cyanophenyl)-2,5-dimethyl-1-((6-methyl-3-pyridyl) methyl)-1H-pyrrole-3-carbonitrile or salt thereof and 7)
4-(4-cyanophenyl)-2,5-dimethyl-1-((6-chloro-3-pyridyl) methyl)-1H-pyrrole-3-carbonitrile or salt thereof, etc., are preferable.

As the salt of the compound (I) including compound (I') or (I'') of the present invention, there may be mentioned, for example, a metal salt, an ammonium salt, a salt with an organic base, a salt with an inorganic acid, a salt with an organic acid, a salt with a basic or acidic amino acid, etc. Preferable examples of the metal salt include an alkali metal salt such as sodium salt, potassium salt, etc.; an alkaline earth metal salt such as calcium salt, magnesium salt, barium salt, etc.; aluminum salt; etc. Preferable examples of the salt with an organic base include a salt with trimethylamine, triethylamine, pyridine, picoline, 2,6-lutidine, ethanolamine, diethanolamine, triethanolamine, cyclohexylamine, dicyclohexylamine, N,N'-dibenzylethylenediamine, etc.
Preferable examples of the salt with an inorganic acid include a salt with hydrochloric acid, hydrobromic acid, nitric acid, sulfuric acid, phosphoric acid, etc. Preferable examples of the salt with an organic acid include a salt with formic acid, acetic acid, trifluoroacetic acid, phthalic acid, fumaric acid,.oxalic acid, tartaric acid, maleic acid, citric acid, succinic acid, malic acid, methanesulfonic acid, benzenesulfonic acid, p-toluenesulfonic acid, etc.
Preferable examples of the salt with a basic amino acid include a salt with arginine, lysine, ornithine, etc. Preferable examples of the salt with an acidic amino acid include a salt with aspartic acid, glutamic acid, etc.

Among these, a pharmaceutically acceptable salt is preferable. For example, when compound has acidic functional group(s), an inorganic salt such as an alkali matal salt (e.g., sodium salt, potassium salt, etc.), an alkaline earth metal salt (e.g., calcium salt, magnesium salt, barium salt, etc.), an ammonium salt, etc., are preferable, and when compound has basic functional group(s), a salt with an inorganic acid such as hydrochloric acid, hydrobromic acid, nitric acid, sulfuric acid, phosphoric acid, etc. or a salt with an organic acid acetic acid, phthalic acid, fumaric acid, oxalic acid, tartaric acid, maleic acid, citric acid, succinic acid, methanesulfonic acid, p-toluenesulfonic acid, etc., preferable.

The compound (I) or salt thereof, including compounds (I') and (I''), of the present invention, can be obtained by the method shown in the reaction scheme 1 shown below or a method similar to the method or can also be obtained by a known method or a method similar thereto. Some compounds included in the formula (I) can be obtained by converting the functional groups of the other compounds (Compounds (I), (II), (III), etc.) obtained in accordance with the reaction shown in the following reaction scheme 1, by a per se known method or a similar method thereto (e.g., oxidation, reduction, hydrolysis, acylation, alkylation, amidation, amination, transfer reaction, etc.).

In the reaction scheme 1, while the respective symbols have the same meanings as those given above, the functional group(s) may be protected by a protective group and the like which is conventionally used in the field of organic synthesis, if necessary. X is a halogen atom. In the reaction scheme, each compound also includes a salt thereof, and as the salt, there may be mentioned the same salts as those of the compound (I).

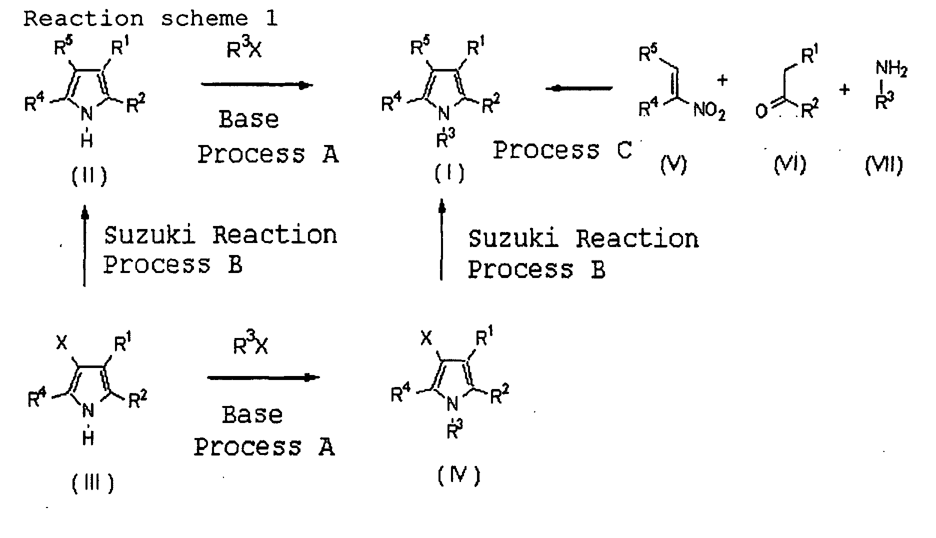

In the reaction scheme 1, as the compounds (II), (III), (IV), (V), (VI) and (VII), if these compounds are available in the market, those available in the market may be used as they are, while these compounds can be produced by per se known methods or by manners similar to known methods.

The compound (II) can be obtained, for example, by subjecting the compound (III) to Suzuki reaction or a reaction similar thereto. The compound (II) can also be obtained by a method described in literatures (Boberg et al., Liebigs. Ann. Chem. 1985, pp 239-250; Meyer et al., Liebigs. Ann. Chem. 1981, pp 1534-1544; Sanchez et al., J. Chem. Soc., Perkin I, Vol 2, 1982, p p 441-447; Barton et al., J. Chem. Soc., Chem. Commun. 1985, p p 1098-1100; Joule et al., Chapman & Hall, Heterocyclic Chemistry; Jones et al., John Wiley & Sons; Pyrroles: Part One: The Synthesis and the Physical and Chemical Aspects of the Pyrrole Ring (The Chemistry of Heterocyclic Compounds, Vol. 48, Part One) or by a method similar to these methods.
The compound (III) can be obtained, for example, by a method described in literatures (Baxter et al., J. Med. Chem. 1993, Vol. 36, pp 239-250; Petruso et al., J. Heterocyclic Chem. 1990, Vol. 27, pp 1209-1211) or by a method similar to these methods.

### [Process A]

The compound (I) or (IV) can be synthesized by reacting the compound (II) or (III) with an alkyl halide, an acyl halide, a sulfonyl halide, etc., in the presence of a base. The reaction can be carried out in a conventional manner.

As the halogen atom, there may be mentioned, for example, fluorine atom, chlorine atom, bromine atom, iodine atom, etc.

As the base, there may be mentioned, for example, an alkali metal hydride such as sodium hydride, potassium hydride, etc., alkali metal amide such as sodium amide, etc.

The amount of the base used is about 1.0 mol to about 10 mol (preferably about 1.0 mol to about 2.0 mol) per mol of the compound (II) or (III).

The amount of the alkyl halide, acyl halide, sulfonyl halide, etc., used is about 1.0 mol to about 10 mol (preferably about 1.0 mol to about 2.0 mol) per mol of the compound (II) or (III).

Reaction temperature is about -70 to about 100 °C, preferably about 0 to about 50 °C. Reaction time is about 5 minutes to about 20 hours.

The reaction of the present invention is usually carried out in a solvent which does not interfere with the reaction. As the solvent which does not interfere with the reaction, there may be used, for example, an ether such as diethylether, dioxane, tetrahydrofuran (THF), etc.; a saturated hydrocarbon such as hexane, pentane, etc.; a halogenated hydrocarbon such as dichloromethane, chloroform, etc.; an amide such as N,N-dimethylformamide, etc.; an aromatic hydrocarbon such as benzene, toluene, etc. Single solvent or a mixed solvent consisting of two or more of these solvents in a conventional ratio, may be used.

When the alkyl halide, acyl halide or sulfonyl halide used is protected by a protective group, the protective group can be removed by a known method or by a method similar to a known method to produce the compound (I). For example, when 2-(4-iodobutoxy)tetrahydropyran is used as an alkyl halide, tetrahydropyranyl group can be removed by treating under an acidic condition. As the acid, there may be used an inorganic acid such as hydrochloric acid, etc. The reaction can be carried out in a solvent such as an alcohol, etc., which does not interfere with the reaction. The reaction temperature normally is about 0 °C to about 100 °C.

### [Process B]

The compound (I) or (II) can be synthesized by subjecting the compound (IV) or (III), for example, to Suzuki reaction (cross condensation reaction by reacting an arylboric acid with an aryl halide or an aryloxytrifluoromethane sulfonate in the presence of palladium catalyst).

The reaction can be carried out by a method described in literature (Suzuki et al., Synth. Commum. 1981, Vol. 11, pp 513) or by a method similar thereto.

The reaction temperature is about 20 °C to about 150 °C, preferably about 60 °C to about 120 °C. The reaction time is about 1 hour to about 50 hours.

### [Process C]

The compound (I) can be synthesized by reacting the compound (V) with the compound (VI) and the compound (VII). The reaction can be carried out by a manner described in literatures (Bhaduri et al., J. Heterocyclic Chem. 1987, Vol. 24, pp 23-25) or in a manner similar to this method. The reaction temperature is about -70 °C to about 150 °C, preferably about 0 °C to about 100 °C. The reaction time is about 5 minutes to about 24 hours. The amount of the compound (VI) or (VII) used is about 1.0 mol to about 10 mol, preferably about 1.0 mol to about 3.0 mol per mol of the compound (V).

The reaction is usually carried out in an organic solvent which does not interfere with the reaction. As the solvent which does not interfere with the reaction, the may be used an alcohol such as methanol, ethanol, etc.; an ether such as diethylether, dioxane, tetrahydrofuran (THF), etc.; a saturated hydrocarbon such as hexane, pentane, etc.; a halogenated hydrocarbon such as dichloromethane, chloroform, etc.; an amide such as N,N-dimethylformamide, etc.; an aromatic hydrocarbon such as benzene, toluene, etc. Single solvent or a mixed solvent consisting of two or more of these solvents in a conventional ratio, may be used. The reaction may be carried out by the addition of an acid or a base, etc. When the compound (V), (VI) or (VII) is protected by a protective group, the protective group can be removed by a per se known method or by a method similar thereto to produce the compound (I).

A prodrug of the compound (I) means a compound which is converted to the compound (I) with a reaction due to an enzyme, a gastric acid, etc., under the physiological condition in vivo, that is, a compound which is converted to the compound (I) with oxidation, reduction, hydrolysis, etc. due to an enzyme; a compound which is converted to the compound (I) with hydrolysis due to gastric acid, etc. A prodrug for compound (I) may for example be a compound obtained by subjecting an amino group in the compound (I) to an acylation, alkylation or phosphorylation (e.g., a compound obtained by subjecting an amino group in compound (I) to an eicosanoylation, alanylation, pentylaminocarbonylation, (5-methyl-2-oxo-1,3-dioxolen-4-yl)methoxycarbonylation, tetrahydrofuranylation, pyrrolidylmethylation, pivaloyloxymethylation and tert-butylation, etc.); a compound obtained by subjecting a hydroxy group in compound (I) to an acylation, alkylation, phosphorylation or boration (e.g., a compound obtained by subjecting a hydroxy in compound (I) to an acetylation, palmitoylation, propanoylation, pivaloylation, succinylation, fumarylation, alanylation, dimethylaminomethylcarbonylation, etc.); a compound obtained by subjecting a carboxyl group in compound (I) to an esterification or amidation (e.g., a compound obtained by subjecting a carboxyl group in compound (I) to an ethylesterification, phenylesterification, carboxymethylesterification, dimethylaminomethylesterification, pivaloyloxymethylesterification, ethoxycarbonyloxyethylesterification, phthalidylesterification, (5-methyl-2-oxo-1,3-dioxolen-4-yl)methylesterification, cyclohexyloxycarbonylethylesterification, methylamidation, etc.) and the like. Any of these compounds can be produced from compound (I) by a per se known method.

A prodrug for compound (I) may be a compound which is converted into compound (I) under a physiological condition as described in "Pharmaceutical Research and Development", Vol. 7 (Design of molecules), pages 163-198 published in 1990 by Hirokawa Publishing Co. (Tokyo, Japan).

The androgen receptor antagonistic drug of the present invention, including the compound (I) or the salt thereof, (hereinafter, sometimes simply referred to as the androgen receptor antagonistic drug of the present invention) has a superior androgen receptor antagonistic action, etc., a low toxicity and a less side-effect, and therefore is useful as a safe pharmaceuticals and as an androgen receptor antagonists, etc.

The pharmaceutical composition of the present invention, which contains the androgen receptor antagonistic drug, shows superior androgen receptor antagonism and (or) prostate-specific antigen (PSA)-producing inhibitory effect to a mammal (for example, mice, rats, hamsters, rabbits, cats, dogs, bovines, sheep, monkeys, humans, etc.), superior oral absorptivity and superior metabolic stability, and can be used as prophylactic or therapeutic agent for androgen receptor-relating disease, for example, hormone sensitivity disease in androgen-dependent stage and (or) androgen-independent stage, especially hormone sensitivity cancer in androgen-dependent stage and (or) androgen-independent stage (e.g., prostate cancer, uterine cancer, breast cancer, hypophyseal tumor, hepatic cancer, etc.) , sex hormone sensitive diseases such as prostatic hypertrophy, endometriosis, uterine myoma, precocious puberty, menstrual pain, amenorrhea, premenstrual syndrome, multilocular ovary syndrome, etc. and agent for sterilization (or prophylaxis or treatment of sterility disease when rebound effect after drug withdrawal is applied), etc.

Especially, the compound (I) or salt thereof of the present invention shows antagonism to normal androgen receptor and (or) mutant receptor, and shows a superior prophylaxis or therapeutic effect for hormone sensitivity cancer in androgen-dependent stage and (or) androgen-independent stage.

Among the androgen receptor antagonistic drugs, an agent which shows antagonistic effect against a mutant androgen receptor or against androgen receptor having an increased sensitivity, is also useful as prophylactic or therapeutic agent for hormone sensitivity cancer in androgen-dependent stage and (or) androgen-independent stage.

The pharmaceutical composition containing the androgen receptor antagonistic drug can be safely administered orally or non-orally (e.g., topical, rectal, intravenous administration etc.) as a pharmaceutical composition in a mixture with a commonly known pharmaceutically acceptable carrier, etc., such as tablets (including sugar-coated tablets, film-coated tablets), powders, granules, capsules, (including soft capsules), liquid medicine, injections, suppositories, sustained release preparations, etc. Injectable preparations can be administered by an intravenous, intramuscular, subcutaneous or intra-tissue route or directly to the focuses.

As the pharmaceutically acceptable carrier which can be used for the production of the pharmaceutical composition of the present invention, there may be mentioned various organic or inorganic carriers which are commonly used as preparation material. For example, excipients, lubricants, binders and disintegrators for producing solid preparations, solvent, solubilizer, suspending agents, isotonizing agent, buffer agent and soothing agent for producing a liquid preparation, etc. may be mentioned. Further, a proper amount of additives such as preservatives, antioxidant, colorants, sweetening agents, adsorbing agent, wetting agents, etc. may be used, according to necessity.

As the excipients, there may be mentioned, for example, lactose, saccharose, D-mannitol, starch, corn starch, crystals cellulose, light anhydrous silicic acid, etc.

As the lubricants, there may be mentioned, for example, magnesium stearate, calcium stearate, talc, colloidal silica, etc.

As the binders, there may be mentioned, for example, crystals cellulose, saccharose, D-mannitol, dextrin, hydroxypropyl cellulose, hydroxypropylmethyl cellulose, polyvinyl pyrrolidone, starch, sucrose, gelatin, methyl cellulose, sodium carboxymethyl cellulose, etc.

As the disintegrators, there may be mentioned, for example, starch, carboxymethyl cellulose, carboxymethyl cellulose calcium, sodium carboxymethyl starch, L-hydroxypropyl cellulose, etc.

As the solvent, there may be mentioned, for example, water for injection, alcohol, propyleneglycol, macrogol, sesame oil, corn oil, olive oil, etc.

As the solubilizer, there may be mentioned, for example, polyethylene glycol, propyleneglycol, D-mannitol, benzyl benzoate, ethanol, trisaminomethane, cholesterol, triethanolamine, sodium carbonate, sodium citrate, etc.

As the suspending agents, there may be mentioned, for example, a surfactants such as stearyltriethanolamine, sodium laurylsulfate, laurylaminopropionic acid, lecithin, benzalkonium chloride, benzethonium chloride, glycerin monostearate, etc.; hydrophilic polymer such as polyvinyl alcohol, polyvinyl pyrolidone, sodium carboxymethyl cellulose, methyl cellulose, hydroxymethyl cellulose, hydroxyethyl cellulose, hydroxypropyl cellulose, etc.

As the isotonizing agent, there may be mentioned; for example, glucose, D-sorbitol, chloride sodium, glycerin, D-mannitol, etc.

As the buffer agent; there may be mentioned, for example, buffer solution such as a phosphate, an acetate, a carbonate, a citrate, , etc.

As the soothing agent, there may be mentioned, for example, benzyl alcohol, etc.

As the preservatives, there may be mentioned, for example; paraoxybenzoates, chlorobutanol, benzyl alcohol, phenethyl alcohol, dehydroacetic acid, sorbic acid, etc.

As the antioxidant, there may be mentioned, for example, a sulfite, ascorbic acid, α-tocopherol, etc.

The content of the androgen receptor antagonistic drug of the present invention in the pharmaceutical composition of the present invention can be appropriately selected depending on the administration subject, administration route, diseases and the like. For example, the content of the androgen receptor antagonistic drug is usually from about 0.01 % by weight to about 100 % by weight, preferably from about 0.1 % by weight to about 50 % by weight, more preferably from about 0.5 % by weight to about 20 % by weight, based on the preparation while it differs depending on the form of preparation.

The content of additives such as a carrier and the like in the pharmaceutical composition of the present invention differs depending on the form of preparation, and is usually from about 1 % by weight to about 99.99 % by weight, preferably from about 10 % by weight to about 90 % by weight, based on the preparation.

As a drug usable in combination the androgen receptor antagonistic drug of the present invention (hereinafter referred to as the other combined drug for combination therapy) , for example, a LH-RH derivative, etc. may be mentioned.

As the LH-RH derivative, there may be mentioned, an LH-RH derivative or salt thereof which is effective against hormone-dependent diseases, especially sex hormone-dependent diseases such as sex hormone-dependent cancers (e.g., prostate cancer, uterine cancer, breast cancer, hypophyseal tumor, hepatic cancer, etc.), prostatic hypertrophy, endometriosis, uterine myoma, precocious puberty, menstrual pain, amenorrhea, premenstrual syndrome, multilocular ovary syndrome, etc., and sterilization (or prophylaxis or treatment of sterility diseases when rebound effect after drug withdrawal is applied). Further, there may be mentioned an LH-RH derivative or salt thereof which is effective against benign tumor or malignant tumor which is sex hormone-independent and LH-RH sensitivity.

Specific examples of the LH-RH derivatives or salt thereof include peptides described in "Treatment with GnRH analogs: Controversies and perspectives" issued in 1996 by The Parthenon Publishing Group Ltd., WO 91-503165, JP 91-101695, JP 95-97334 and JP 94-259460, etc.

As the LH-RH derivative, there may be mentioned LH-RH agonists and LH-RH antagonists. As the LH-RH antagonist, there may be used, for example, a physiologically active peptide represented by the formula: X-D2Nal-D4ClPhe-D3Pal-Ser-A-B-Leu-C-Pro-DAlaNH₂ [wherein X is N(4H₂-furoyl)Gly or NAc; A is a residue selected from NMeTyr, Tyr, Aph(Atz) and NMeAph(Atz); B is a residue selected from DLys(Nic), DCit, DLys(AzaglyNic), DLys(AzaglyFur), DhArg(Et₂), DAph(Atz) and DhCi; and C is Lys(Nisp), Arg or hArg(Et₂)] or a salt thereof, etc.

As LH-RH agonist, there may be mentioned, for example, a physiologically active peptide represented by the formula: 5-oxo-Pro-His-Trp-Ser-Tyr-Y-Leu-Arg-Pro-Z [wherein Y is a residue selected from DLeu, DAla, DTrp, DSer (tBu), D2Nal and DHis (ImBzl) ; and Z is NH-C₂H₅ or Gly-NH₂] or salt thereof, etc.
Especially, a peptide wherein Y is DLeu and Z is NH-C₂H₅ (that is, peptide A represented by the formula: 5-oxo-Pro-His-Trp-Ser-Tyr-DLeu-Leu-Arg-Pro-NH-C2H5 ; leuprorelin) or a salt thereof (e.g., acetate) is preferable.

The drug in which an androgen receptor antagonistic drug of the present invention and other combined drug are combined, (hereinafter referred to as the combination agent of the present invention) has low toxicity, and for example, the androgen receptor antagonistic drug of the present invention or (and) the above-mentioned concomitant drug can be mixed, according to a known method per se, with a pharmacologically acceptable carrier to give pharmaceutical compositions, for example, tablets (including a sugar-coated tablet, film-coated tablet), powders, granules, capsules (including a soft capsule), solutions, injections, suppositories, sustained release agents and the like which can be safely administered orally or parenterally (e.g., local, rectum, vein, and the like). An injection can be administered by intravenous, intramuscular, subcutaneous or intra-tissue or directly to the lesion.

As the pharmaceutically acceptable carrier which may be used in production of the combination agent of the present invention, there may be mentioned various organic or inorganic carriers which are commonly used as preparation material. For example, excipients, lubricants, binders and disintegrators for producing a solid preparation, solvent, solubilizer, suspending agents, isotonizing agent, buffer agent and soothing agent for producing a liquid preparation, etc. may be mentioned. Further, a proper amount of additives such as preservatives, antioxidant, colorant, sweetening agents, adsorbing agent, wetting agents, etc. may be used, if necessary.

As the excipients, there may be mentioned, for example, lactose, saccharose, D-mannitol, starch, corn starch, crystalline cellulose, light anhydrous silicic acid, etc.

As the lubricants, there may be mentioned, for example, magnesium stearate, calcium stearate, talc, colloidal silica, etc.

As the binders there may be mentioned, for example, crystals cellulose, saccharose, D-mannnitol, dextrin, hydroxypropyl cellulose, hydroxypropylmethyl cellulose, polyvinyl pyrolidone, starch, sucrose, gelatin, methyl cellulose, sodium carboxymethyl cellulose, etc.

As the disintegrators, there may be mentioned, for example, starch, carboxymethyl cellulose, carboxymethyl cellulose calcium, sodium carboxymethyl starch, L-hydroxypropyl cellulose, etc.

As the solvent, there may be mentioned, for example; water for injection, alcohol, propyleneglycol, macrogol, sesame oil, corn oil, olive oil, etc.

As the solubilizer, there may be mentioned, for example, polyethylene glycol, propyleneglycol, D-mannnitol, benzyl benzoate, ethanol, trisaminomethane, cholesterol, triethanolamine, sodium carbonate, sodium citrate, etc.

As the suspending agents, there may be mentioned, for example, a surfactants such as stearyltriethanolamine, sodium laurylsulfate, laurylaminopropionic acid, lecithin, benzalkonium chloride, benzethonium chloride, glycerin monostearate, etc.; hydrophilic polymer such as polyvinyl alcohol, polyvinyl pyrolidone, sodium carboxymethyl cellulose, methyl cellulose, hydroxymethyl cellulose, hydroxyethyl cellulose, hydroxypropyl cellulose, etc.

As the isotonizing agent, there may be mentioned, for example, glucose, D-sorbitol, chloride sodium, glycerin, D-mannitol, etc.

As the buffer agent, there may be mentioned, for example, a buffer solution such as a phosphate, an acetate, a carbonate, a citrate, etc., etc.

As the soothing agent, there may be mentioned, for example, benzyl alcohol, etc.

As the preservatives, there may be mentioned, for example, paraoxybenzoic acid esters, chlorobutanol, benzyl alcohol, phenethyl alcohol, dehydroacetic acid, sorbic acid, etc.

As the antioxidant, there may be mentioned, for example, a sulfite salt, ascorbic acid, α-tocopherol, etc.

The compounding ratio of the androgen receptor antagonistic drug of the present invention to the other combined drug in the combination agent of the present invention can be appropriately selected depending on the administration subject, administration route, diseases and the like.

For example, the content of the androgen receptor antagonistic drug of the present invention in the combination agent of the present invention differs depending on the form of preparation, and is usually from about 0.01 % by weight to about 100 % by weight, preferably from about 0.1 % by weight to about 50 % by weight, more preferably from about 0.5 % by weight to about 20 % by weight, based on the preparation.

The content of the other combined drug in the combination agent of the present invention differs depending on the form of preparation, and is usually from about 0.01 % by weight to about 100 % by weight, preferably from about 0.1 % by weight to about 50 % by weight, more preferably from about 0.5 % by weight to about 20 % by weight, based on the preparation.

The content of additives such as a carrier and the like in the combination agent of the present invention differs depending on the form of preparation, and is usually from about 1 % by weight to about 99.99 % by weight, preferably from about 10 % by weight to about 90 % by weight, based on the preparation.

When the androgen receptor antagonistic drug of the present invention and the other combined drug for combination therapy are formulated separately, the same contents may be adopted.

These preparations can be produced by a per se known method commonly used in the pharmaceutical manufacturing process.

For example, the androgen receptor antagonistic drug of the present invention and the other combined drug for combination therapy can be made into an aqueous injection together with a dispersing agent (e.g., Tween 80 (manufactured by Atlas Powder, US), HCO 60 (manufactured by Nikko Chemicals), polyethylene glycol, carboxymethyl cellulose, sodium alginate, hydroxypropylmethyl cellulose, dextrin and the like), a stabilizer (e.g., ascorbic acid, sodium pyrosulfite, and the like), a surfactant (e.g., Polysorbate 80, macrogol and the like), a solubilizer (e.g., glycerin, ethanol and the like), a buffer (e.g., phosphoric acid and alkali metal salt thereof, citric acid and alkali metal salt thereof, and the like), an isotonizing agent (e.g., sodium chloride, potassium chloride, mannitol, sorbitol, glucose and the like), a pH regulator (e.g., hydrochloric acid, sodium hydroxide and the like), a preservative (e.g., ethyl p-oxybenzoate, benzoic acid, methylparaben, propylparaben, benzyl alcohol and the likey, a dissolving agent (e.g., cone. glycerin, meglumine and the like), a dissolution aid (e.g., propylene glycol, sucrose and the like) , a soothing agent (e.g., glucose, benzyl alcohol and the like), and the like, or can be dissolved, suspended or emulsified in a vegetable oil such as olive oil, sesame oil, cotton seed oil, corn oil and the like or a dissolution aid such as propylene glycol and molded into an oily injection.

In the case of a preparation for oral administration, an excipient (e.g., lactose, sucrose, starch and the like), a disintegrating agent (e.g., starch, calcium carbonate and the like), a binder (e.g., starch, gum Arabic, carboxymethyl cellulose, polyvinylpyrrolidone, hydroxypropyl cellulose and the like), a lubricant (e.g., talc, magnesium stearate, polyethylene glycol 6000 and the like) and the like, can be added to the androgen receptor antagonistic drug of the present invention or the other combined drug, according to a known method per se, and the mixture can be compression- molded, then if desirable, the molder product can be coated by a known method per se for the purpose of masking of taste, enteric property or durability, to obtain a preparation for oral administration. As this coating agent, for example, hydroxypropylmethyl cellulose, ethyl cellulose, hydroxymethyl cellulose, hydroxypropyl cellulose, polyoxyethylene glycol, Tween 80, Pluronic F68, cellulose acetate phthalate, hydroxypropylmethyl cellulose phthalate, hydroxymethyl cellulose acetate succinate, Eudragit (methacrylic acid/acrylic acid copolymer, manufactured by Rohm, DE) , pigment (e.g., iron oxide red, titanium dioxide, etc.) and the like, may be used. The preparation for oral administration may be either a quick release preparation or a sustained release preparation.

For example, in the case of a suppository, the androgen receptor antagonistic drug of the present invention and the other combined drug can be made into an oily or aqueous solid, semisolid or liquid suppository according to a known method per se. As the oily substrate used in the above-mentioned composition, for example, glycerides of higher fatty acids [e.g., cacao butter, Witebsols (manufactured by Dynamite Novel, DE), etc.], intermediate grade fatty acids [e.g., Myglyols (manufactured by Dynamite Novel, DE) , etc.], or vegetable oils (e.g., sesame oil, soy bean oil, cotton seed oil and the like) , and the like are listed. Further, as the aqueous substrate, for example, polyethylene glycols, propylene glycol are listed, and as the aqueous gel substrate, for example, natural gums, cellulose derivatives, vinyl polymers, acrylic acid polymers and the like are listed.

As the above-mentioned sustained release agent, sustained release microcapsules and the like are listed.
For obtaining a sustained release microcapsule, a known method per se can be adopted. It is preferable to mold into a sustained release preparation shown in [2] below.

A compound of the present invention is preferably molded into an oral administration preparation such as a solid preparation (e.g., powder, granule, tablet, capsule, etc.) and the like, or molded into a rectum administration preparation such as a suppository. Particularly, an oral administration preparation is preferable.

The other combined drug can be made into the above-mentioned drug form depending on the kind of drug.

[1] An injection of the androgen receptor antagonistic drug or the other combined drug and preparation method thereof, [2] a quick release preparation of sustained release preparation and the androgen receptor antagonistic drug or the other combined drug and preparation thereof and [3] a sublingual tablet, a buccal or an intraoral quick integrating agent of the androgen receptor antagonistic drug or the other combined drug or preparation thereof are specifically mentioned below.

### [1] Injectable preparation and its production

An injectable preparation prepared by dissolving the androgen receptor antagonistic drug of the present invention or the other combined drug for combination therapy in water is preferable. This injectable preparation may be allowed to contain a benzoate and/or a salicylate.

The injection is obtained by dissolving the androgen receptor antagonistic drug or the other combined drug of the present invention, and if desirable, a benzoate and/or a salicylate, into water.

As the above-mentioned salts of benzoic acid and salicylic acid, for example, salts of alkali metals such as sodium, potassium and the like, salts of alkaline earth metals such as calcium, magnesium and the like, ammonium salts, meglumine salts, organic acid salts such as tromethamol and the like, etc. are listed.

The concentration of the androgen receptor antagonistic drug or the other combined drug of the present invention in an injection is from 0.5 w/v % to 50 w/v %, preferably from about 3 w/v % to about 20 w/v %. The concentration of a salt of benzoic acid or/and a salt of salicylic acid is from 0.5 w/v % to 50 w/v %, preferably from 3 w/v % to 20 w/v %.

Conventional additives for use in an injection may be appropriately added in a preparation of the present invention. Examples of the additives include a stabilizer (e.g. ascorbic acid, sodium pyrosulfite, and the like), a surfactant (e.g., Polysorbate 80, macrogol and the like), a solubilizer (e.g., glycerin, ethanol and the like) , a buffer (e. g., phosphoric acid and alkali metal salt thereof, citric acid and alkali metal salt thereof, and the like), an isotonizing agent (e.g., sodium chloride, potassium chloride, and the like) , a dispersing agent (e.g., hydroxypropylmethyl cellulose, dextrin), a pH regulator (e.g., hydrochloric acid, sodium hydroxide and the like), a preservative (e.g., ethyl p-oxybenzoate, benzoic acid and the like), a dissolving agent (e.g., conc. glycerin, meglumine and the like), a dissolution aid (e.g., propylene glycol, sucrose and the like), a soothing agent (e.g., glucose, benzyl alcohol and the like), and the like. These additives are generally. blended in a usual proportion employed in an injection.

It is advantageous that the pH of the injection is controlled from 2 to 12, preferably from 2.5 to 8.0 by addition of a pH regulator.

An injectable preparation is obtained by dissolving the compound of the present invention or the other combined drug for combination therapy and if desirable, a salt of benzoic acid and/or a salt of salicylic acid, and if necessary, the above-mentioned additives into water. These may be dissolved in any order, and can be appropriately dissolved in the same manner as in a conventional method of producing an injection.

An aqueous solution for injection may be advantageously heated, alternatively, for example, filter sterilization, high pressure heat sterilization and the like can be conducted in the same manner as for a usual injection, to provide an injection.

It may be advantageous that an aqueous solution for injection is subjected to high pressure heat sterilization at 100 °C to 121 °C for 5 minutes to 30 minutes.

Further, a preparation endowed with the antibacterial property of a solution may also be produced so that it can be used as a preparation which is divided and administered multiple-times.

### [2] A sustained release preparation or a rapid release preparation, and its production

Preffered is a sustained release preparation which is obtained, by coating a core containing the androgen receptor antagonistic drug or the other combined drug of the present invention with a film agent such as a water-insoluble substance, swellable polymer and the like, if desirable. For example, a sustained release preparation for oral administration of once administration per day type is preferable.

As the water insoluble substance used in film forming agent, there may be mentioned, for example, a cellulose ether such as ethyl cellulose, butyl cellulose, etc.; a cellulose ester such as cellulose acetate, cellulose propionate, etc.; a polyvinyl ester such as polyvinyl acetate, polyvinyl butyrate, etc.; an acrylic acid polymer such as acrylic acid/methacrylic acid copolymer, methylmethacrylate copolymer, ethoxyethyl methacrylate/cinnamoethylmethacrylate/aminoalkyl methacrylate copolymer, polyacrylic acid, polymethacrylic acid, methacrylic acid alkyl amide copolymer, poly(methacrylic acid methyl), polymethacrylate, polymethacryl amide, amino alkyl methacrylate copolymer, poly(methacrylic acid anhydride), glycidyl methacrylate copolymer, specially an Eudragit (manufactured by Rohm Pharma) such as Eudragit RS-100, RL-100, RS-30D, RL-30D, RL-PO, RS-PO (copolymer of acrylic acid ethyl/methyl methacrylate/chlorotrimethylmethacrylate/ethyl ammonium), Eudragit NE-30D (copolymer of methyl methacrylate/ethyl acrylate), etc., a hydrogenated oil such as hardened caster oil (e.g., Lovely wax (Freunt), etc.), etc.; a wax such as carnauba wax, fatty acid glycerin ester, paraffin, etc.; polyglycerin fatty acid ester, etc.

As the swellable polymer, a polymer having acidic dissociating group and pH-dependent swelling property, is preferable, and a polymer having acidic dissociating group which swells little in a area such as stomach and swells in a neutral area such as the small intestine or the large intestine.

As the polymer having acidic dissociating group and pH-dependent swelling property, there may be mentioned, crosslinkable polyacrylic polymer such as Carbomer 934P, 940, 941, 974P, 980, 1342 and the like, polycarbophil, carcium polycarbophil (the last two are manufactured by BF Goodrich.), Hibiswako 103, 104, 105, 304 (all are manufactured by Wako Pure Chemical Co., Ltd.), and the like, are listed. The film forming agent used in a sustained release preparation may further contain a hydrophilic substance.

As the hydrophilic substance, there may be mentioned, for example, a polysaccharide such as pullulan, dextrin, arginic acid alkalimetal salt, etc.; a polysaccharide having a hydroxyalkyl group or a carboxyalkyl group such as hydroxypropyl cellulose, hydroxypropyl methyl cellulose, sodium carboxymethyl cellulose, etc.; methyl cellulose; polyvinyl pyrolidone; polyvinyl alcohol; polyethylene glycol; etc.

The content of water insoluble substance in the film forming agent of sustained release preparation is about 30 % (w/w) to about 90 % (w/w) , preferably about 35 % (w/w) to about 80 % (w/w) , and more preferably about 40 % (w/w) to about 75 % (w/w). The content of swellable polymer is about 3 % (w/w) to about 30 % (w/w), preferably about 3 % (w/w) to about 15 % (w/w). The film forming agent may further contain a hydrophilic substance, in this case, the content of the hydrophilic substance in the film forming agent is about 50 % (w/w) or less, preferably about 5 % (w/w) to about 40 % (w/w), and more preferably about 5 % (w/w) to about 35 % (w/w). This % (w/w) indicates % by weight based on a film agent composition which is obtained by removing a solvent (e.g., water, lower alcohols such as methanol, ethanol and the like) from a film agent solution.

The sustained release preparation is produced by preparing a core containing a drugs as exemplified below, then, coating the resulted core with a film agent liquid prepared by heating to melt a water-insoluble substance , swellable polymer and the like or by dissolving or dispersing it in a solvent.

### I. Preparing of cores containing an active ingredient

The form of core containing a drug to be coated with a film agent (hereinafter, sometimes simply referred to as core) is not particularly restricted, and preferably, the core is formed into particles such as a granule or fine particle.

When the core is composed of granules or fine particles, the average particle size thereof is preferably from about 150 to about 2, 000 µm, further preferably, from about 500 µm to 1,400 µm.

Preparation of the core can be effected by a usual production method. For example, a suitable excipient, binding agent, disintegrating agent, lubricant, stabilizer and the like are mixed into a drug, and the mixture is subjected to a wet extrusion granulating method, fluidized bed granulating method or the like, to prepare a core.

The content of drugs in a core is from about 0.5 % (w/w) to about 95 % (w/w), preferably from about 5.0 % (w/w) to about 80 % (w/w), further preferably from about 30 % (w/w) to about 70 % (w/w).

As the excipient contained in the core, for example, saccharides such as sucrose, lactose, mannitol, glucose and the like, starch, crystalline cellulose, calcium phosphate, corn starch and the like are used. Among them, crystalline cellulose, corn starch are preferable.

As the binders, there may be used, for example, polyvinyl alcohol, hydroxypropyl cellulose, polyethylene glycol, polyvinyl pyrolidone, Pulronick F68, arabic gum, gelatin, starch, etc. As the disintegrators, there may be used, for example, carboxymethyl cellulose calcium (ECG505), croscarmellose sodium (Ac-Di-Sol), crosslinkable polyvinyl pyrolidone (crospovidone), low substituted hydroxypropyl cellulose (L-HPC), etc. Among these, hydroxypropyl cellulose, polyvinyl pyrolidone, low substituted hydroxypropyl cellulose are preferable. As the lubricants or the aggregation inhibitor, there may be used, for example, talc, magnesium stearate and an inorganic salt thereof. As the lubricant, there may be used a polyethylene glycol, etc. As the stabilizing agent, there may be used an acid such as tartaric acid, citric acid, succinic acid, fumaric acid, maleic acid, etc.

A core can also be prepared by, in addition to the above-mentioned, for example, a rolling granulation method in which a drug or a mixture of a drug with an excipient, lubricant and the like is added portionwise onto an inert carrier particle which is the core of the core while spraying a binder dissolved in a suitable solvent such as water, lower alcohol (e.g., methanol, ethanol and the like) and the like, a pan coating method, a fluidized bed coating method or a melt granulating method. As the inert carrier particle, for example, those made of sucrose, lactose, starch, crystalline cellulose, waxes can be used, and the average particle size thereof is preferably from about 100 µm to about 1,500 µm.

For the purpose of separating the drug contained in the core from the film agent, the surface of the core may be coated with a protective agent. As the protective agent, for example, the above-mentioned hydrophilic substances, water-insoluble substances and the like are used. As the protective agent, preferably polyethylene glycol, and polysaccharides having a hydroxyalkyl group or carboxyalkyl group are used, more preferably, hydroxypropylmethyl cellulose and hydroxypropyl cellulose are used. The protective agent may contain, as stabilizer, acids such as tartaric acid, citric acid, succinic acid, fumaric acid, maleic acid and the like, and lubricants such as talc and the like. When the protective agent is used, the coating amount is from about 1 % (w/w) to about 15 % (w/w) , preferably from about 1 % (w/w) to about 10 % (w/w), further preferably from about 2 % (w/w) to about 8 % (w/w), based on the core.

The coating of the protective agent can be carried out by a usual coating method, and specifically, the coating can be carried out by spraying the protective agent by a fluidized bed coating method, pan coating method and the like.

### II. Coating of core with film agent

A core obtained in the above-mentioned step I is coated with a film agent solution obtained by heat-solving the above-mentioned water-insoluble substance and pH-dependent swellable polymer, and a hydrophilic substance, or by dissolving or dispersing them in a solvent, to give a sustained release preparation.

As the method for coating a core with a film agent solution, for example, a spray coating method and the like are listed.

The composition ratio of a water-insoluble substance, swellable polymer and hydrophilic substance in a film agent solution is appropriately selected so that the contents of these components in a coated film are the above-mentioned contents, respectively.

The coating amount of a film agent is from about 1 % (w/w) to about 90 % (w/w), preferably from about 5 % (w/w) to about 50 % (w/w), further preferably from about 5 % (w/w) to about 35 % (w/w), based on a core (not including coating amount of protective agent).

As the solvent in a film agent solution, water or an organic solvent can be used alone or in admixture thereof. In the case of use in admixture, the mixing ratio of water to an organic solvent (water/organic solvent: by weight) can be varied in the range from 1 to 100 %, and preferably from about 1 % to about 30 %. The organic solvent is not particularly restricted providing it dissolves a water-insoluble substance, and for example, lower alcohols such as methyl alcohol, ethyl alcohol, isopropyl alcohol, n-butyl alcohol and the like, lower alkanones such as acetone and the like, acetonitrile, chloroform, methylene chloride and the like are used. Among them, lower alcohols are preferable, and ethyl alcohol and isopropyl alcohol are particularly preferable. Water, and a mixture of water with an organic solvent are preferably used as a solvent for a film agent. In this case, if necessary, an acid such as tartaric acid, citric acid, succinic acid, fumaric acid, maleic acid and the like may also be added into a film agent solution for stabilizing the film agent solution.

An operation of coating by spray coating can be effected by a usual coating method, and specifically, it can be effected by spray-coating a film agent solution onto a core, for example, by a fluidized bed coating method, pan coating method and the like. In this case, if necessary, talc, titanium oxide, magnesium stearate, calcium stearate, light anhydrous silicic acid and the like may also be added as a lubricant, and glycerin fatty ester, hardened castor oil, triethyl citrate, cetyl alcohol, stearyl alcohol and the like may also be added as a plasticizer.

After coating with a film agent, if necessary, an antistatic agent such as talc and the like may be mixed.

The quick release preparation may be liquid (solution, suspension, emulsion and the like) or solid (particle, pill, tablet and the like). Oral agents and parenteral agents such as an injection and the like are used, and oral agents are preferable.

The quick release preparation, usually, may contain, in addition to an active component drug, also carriers, additives and excipients conventionally used in the production field (hereinafter, sometimes abbreviated as excipient). The preparation excipient used is not particularly restricted providing it is an excipient ordinarily used as a preparation excipient. For example, as the excipient for an oral solid preparation, lactose, starch, corn starch, crystalline cellulose (Avicel PH101, manufactured by Asahi Chemical Industry Co., Ltd., and the like), powder sugar, granulated sugar, mannitol, light anhydrous silicic acid, magnesium carbonate, calcium carbonate, L-cysteine and the like are listed, and preferably, corn starch and mannitol and the like are listed. These excipients can be used alone or in combination of two or more. The content of the excipient is, for example, from about 4.5 w/w % to about 99.4 w/w %, preferably from about 20 w/w % to about 98.5 w/w %, further preferably from about 30 w/w % to about 97 w/w %, based on the total amount of the quick release preparation.

The content of a drug in the quick release preparation can be appropriately selected in the range from about 0.5 % to about 95 %, preferably from about 1 % to about 60 % based on the total amount of the quick release preparation.

When the quick release preparation is an oral solid preparation, it usually contains, in addition to the above-mentioned components, also an integrating agent. As this integrating agent, there are used, for example, carboxymethyl cellulose calcium (ECG-505, manufactured by Gotoku Yakuhin), croscarmelose sodium (for example, Actisol, manufactured by Asahi Chemical Industry Co., Ltd.), crospovidone (for example, Colicone CL, manufactured by BASF), low-substituted hydroxypropyl cellulose (manufactured by Shin-Etsu Chemical Co., Ltd.), carboxymethylstarch (manufactured by Matsutani Kagaku K.K.), carboxymethylstarch sodium (Exprotab, manufactured by Kimura Sangyo), partially α-nized starch (PCS, manufactured by Asahi Chemical Industry Co., Ltd.), and the like are used, and for example, those which disintegrate a granule by adsorbing water in contact with water, causing swelling, or making a channel between an effective ingredient constituting the core and an excipient, can be used. These disintegrating agents can be used alone or in combination of two or more. The amount of the disintegrating agent used is appropriately selected depending on the kind and blending amount of a drug used, design of releasing property, and the like, and for example, from about 0.05 w/w % to about 30 w/w %, preferably from about 0.5 w/w % to about 15 w/w %, based on the total amount of the quick releasing agent.

When the quick release preparation is an oral solid preparation, it may further contain, in addition to the above-mentioned composition, if desired, additives conventional in solid preparations. As such an additive, there are used, for example, a binder (e.g., sucrose, gelatin, gum Arabic powder, methyl cellulose, hydroxypropyl cellulose, hydroxypropylmethyl cellulose, carboxylmethyl cellulose, polybinylpyrrolidone, pluran, dextrin and the like), a lubricant (e.g., polyethylene glycol, magnesium stearate, talc, light anhydrous silicic acid (for example, aerosil (Nippon Aerosil)), a surfactant (e.g., anionic surfactants such as sodium alkylsulfate and the like, nonionic surfactants such as polyoxyethylene fatty acid ester and polyoxyethylene sorbitan fatty acid ester, polyoxyethylene castor oil derivatives and the like), a coloring agent (e.g., tar coloring matter, caramel, iron oxide red, titanium oxide, riboflavins) , if necessary, an appetizing agent (e.g., sweetening agent, aroma chemical and the like), an adsorbent, preservative, wetting agent, antistatic agent, and the like. Further, as the stabilizer, an organic acid such as tartaric acid, citric acid, succinic acid, fumaric acid and the like may also be added.

As the above-mentioned binder, hydroxypropyl cellulose, polyethylene glycol and polyvinylpyrrolidone and the like are preferably used.

The quick releasing reparation can be prepared by, based on a usual technology of producing preparations, mixing the above-mentioned components, and if necessary, further kneading the mixture, and molding it. The above-mentioned mixing is conducted by generally used methods, for example, mixing, kneading and the like. Specifically, when a quick release preparation is formed, for example, into a particle, it can be prepared, according to the same means as in the above-mentioned method for preparing a core of a sustained release preparation, by mixing the components using a vertical granulator, universal kneader (manufactured by Hata Tekkosho), fluidized bed granulator FD-5S (manufactured by Pulek), and the like, then, subjecting the mixture to a wet extrusion granulation method, fluidized bed granulation method and the like.

Thus obtained quick releasing preparation and sustained releasing preparation may be themselves made into products or made into products appropriately together with preparation excipients and the like, separately, by an ordinary method, then, may be administered simultaneously or may be administered in combination at any administration interval, or they may be themselves made into one oral preparation (e.g., granule, fine particle, tablet, capsule and the like) or made into one oral preparation together with preparation excipients and the like. It may also be permissible that they are made into granules or fine particles, and filled in the same capsule to be used as a preparation for oral administration.

### [3] Sublinguial, buccal or intraoral quick disintegrating agent and preparation thereof

Sublinguial, buccal or intraoral quick disintegrating agents may be a solid preparation such as tablet and the like, or may be an oral mucosa membrane patch (film).

As the sublinguial, buccal or intraoral quick disintegrating agent, a preparation containing the androgen receptor antagonistic drug of the present invention or the combination drug and an excipient is preferable. It may contain also auxiliary agents such as a lubricant, isotonizing agent, hydrophilic carrier, water-dispersible polymer, stabilizer and the like. Further, for easy absorption and increase in in vivo use efficiency, β-cyclodextrin or β-cyclodextrin derivatives (e.g., hydroxypropyl-β-cyclodextrin and the like) and the like may also be contained.

As the above-mentioned excipient, lactose, sucrose, D-mannitol, starch, crystalline cellulose, light anhydrous silicic acid and the like are listed. As the lubricant, magnesium stearate, calcium stearate, talc, colloidal silica and the like are listed, and particularly, magnesium stearate and colloidal silica are preferable. As the isotonizing agent, sodium chloride, glucose, fructose, mannitol, sorbitol, lactose, saccharose, glycerin, urea and the like are listed, and particularly, mannitol is preferable. As the hydrophilic carrier, swellable hydrophilic carriers such as crystalline cellulose, ethyl cellulose, crosslinkable polyvinylpyrrolidone, light anhydrous silicic acid, silicic acid, dicalcium phosphate, calcium carbonate and the like are listed, and particularly, crystalline cellulose (e.g., fine crystalline cellulose and the like) is preferable. As the water-dispersible polymer, gums (e.g., gum tragacanth, acacia gum, cyamoposis gum), alginates (e.g., sodium alginate), cellulose derivatives (e.g., methyl cellulose, carboxymethyl cellulose, hydroxymethyl cellulose, hydroxypropyl cellulose, hydroxypropylmethyl cellulose) , gelatin, water-soluble starch, polyacrylic acids (e.g., Carbomer), polymethacrylic acid, polyvinyl alcohol, polyethylene glycol, polyvinylpyrrolidone, polycarbofil, ascorbate, palmitates and the like are listed, and hydroxypropylmethyl cellulose, polyacrylic acid, alginate, gelatin, carboxymethyl cellulose, polyvinylpyrrolidone, polyethylene glycol and the like are preferable. Particularly, hydroxypropylmethyl cellulose is preferable. As the stabilizer, cysteine, thiosorbitol, tartaric acid, citric acid, sodium carbonate, ascorbic acid, glycine, sodium sulfite and the like are listed, and particularly, citric acid and ascorbic acid are preferable.

The sublinguial, buccal or intraoral quick disintegrating agent can be produced by mixing the androgen receptor antagonistic drug of the present invention or the other combined drug and an excipient by a method known per se. Further, if desired, auxiliary agents such as a lubricant, isotonizing agent, hydrophilic carrier, water-dispersible polymer, stabilizer, coloring agent, sweetening agent, preservative and the like may be mixed. The sublingual, buccal or intraoral quick disintegrating agent is obtained by mixing the above-mentioned components simultaneously or at a time interval, then subjecting the mixture to tablet-making molding under pressure. For obtaining suitable hardness, it may also be permissible that the materials are moistened by using a solvent such as water, alcohol and the like if desired before and after the tablet making process, and after the molding, the materials are dried, to obtain a product.

In the case of molding into a mucosa membrane patch (film), the androgen receptor antagonistic drug of the present invention or the other combined drug and the above-mentioned water-dispersible polymer (preferably, hydroxypropyl cellulose, hydroxypropylmethyl cellulose), excipient and the like are dissolved in a solvent such as water and the like, and the resulted solution is cast to give a film. Further, additives such as a plasticizer, stabilizer, antioxidant, preservative, coloring agent, buffer, sweetening agent and the like may also be added. For imparting suitable elasticity to the film, glycols such as polyethylene glycol, propylene glycol and the like may be contained, or for enhancing adhesion of the film to an intraoral mucosa membrane lining, a bio-adhesive polymer (e.g., polycarbofil, carbopol) may also be contained. In the casting, a solution is poured on the non-adhesive surface, spread to uniform thickness (preferably, about 10 micron to about 1,000 micron) by an application tool such as a doctor blade and the like, then, the solution is dried to form a film. It may be advantageous that thus formed film is dried at room temperature or under heat, and cut into given area.

As the preferable intraoral quick disintegrating agent, there are listed solid quick scattering dose agents composed of a network body comprising the androgen receptor antagonistic drug of the present invention or the combination drug, and a water-soluble or water-diffusible carrier which is inert to the androgen receptor antagonistic drug of the present invention or the other combined drug, are listed. This network body is obtained by sublimating a solvent from the solid composition constituted of a solution prepared by dissolving the androgen receptor antagonistic drug of the present invention or the combination drug in a suitable solvent.

It is preferable that the composition of an intraoral quick disintegrating agent contains a matrix forming agent and a secondary component, in addition to the androgen receptor antagonistic drug of the present invention or the other combined drug.

Examples of the matrix forming agent include animal proteins or vegetable proteins such as gelatins, dextrins, soybean, wheat and psyllium seed protein and the like; rubber substances such as gum Arabic, guar gum, agar, xathane gum and the like; polysaccharides; alginic acids; carboxymethyl celluloses; caragenans; dextrans; pectines; synthetic polymers such as polyvinylpyrrolidone and the like; substances derived from a gelatin-gum Arabic complex, and the like. Further, saccharides such as mannitol, dextrose, lactose, galactose, trehalose and the like; cyclic saccharides such as cyclodextrin and the like; inorganic salts such as sodium phosphate, sodium chloride and aluminum silicate and the like; amino acids having 2 to 12 carbon atoms such as glycine, L-alanine, L-aspartic acid, L-glutamic acid, L-hydroxyproline, L-isoleucine, L-leucine, L-phenylalanine and the like, are contained.

One or more of the matrix forming agent(s) can be introduced in a solution or suspension before solidification. Such as matrix forming agent may be present in addition to a surfactant, or may be present while a surfactant being excluded. The matrix forming agents aid to maintain the androgen receptor antagonistic drug of the present invention or the other combined drug in the solution or suspension in diffused condition, in addition to formation of the matrix.

The composition may contain secondary components such as a preservative, antioxidant, surfactant, thickening agent, coloring agent, pH controlling agent, flavoring agent, sweetening agent, food taste masking agent and the like. As the suitable coloring agent, there are listed red, black and yellow iron oxides, and FD & C dyes such as FD & C Blue 2, FD & C Red 40 and the like manufactured by Elis and Eberald. Examples of the suitable flavoring agent include mint, raspberry, licorice, orange, lemon, grape fruit, caramel, vanilla, cherry, grape flavor and combinations thereof. Examples of the suitable pH controlling agent include citric acid, tartaric acid, phosphoric acid, hydrochloric acid and maleic acid. Examples of the suitable sweetening agent include aspartame, acesulfame K and thaumatin and the like. Examples of the suitable food taste masking agent include sodium bicarbonate, ion exchange resin, cyclodextrin-containing compounds, adsorbent substances and microcapsulated apomorphine.

The preparation contains the androgen receptor antagonistic drug of the present invention or the other combined drug in an amount usually from about 0.1 % by weight to about 50 % by weight, preferably from about 0.1 % by weight to about 30 % by weight, and preferable are preparations (such as the above-mentioned sublingual agent, buccal and the like) which can dissolve 90 % or more the androgen receptor antagonistic drug of the present invention or the other combined drug (into water) within the time range of about 1 minute to about 60 minutes, preferably of about 1 minute to 15 minutes, more preferably of about 2 minutes to 5 minutes, and intraoral quick disintegrating preparations which are disintegrated within the range of 1 second to 60 seconds, preferably of 1 to 30 seconds, further preferably of 1 to 10 seconds after place in an oral cavity.

The content of the above-mentioned excipient in the whole preparation is from about 10 % by weight to about 99 % by weight, preferably from about 30 % by weight to about 90 % by weight. The content of β-cyclodextrin or β-cyclodextrin derivative in the whole preparation is from 0 to about 30 % by weight. The content of the lubricant in the whole preparation is from about 0.01 % by weight to about 10 % by weight, preferably from about 1 % by weight to about 5 % by weight. The content of the isotonizing agent in the whole preparation is from about 0.1 % by weight to about 90 % by weight, preferably, from about 10 % by weight to about 70 % by weight. The content of the hydrophilic carrier agent in the whole preparation is from about 0.1 % by weight to about 50 % by weight, preferably, from about 10 % by weight to about 30 % by weight. The content of the water-dispersible polymer in the whole preparation is from about 0.1 to about 30 % by weight, preferably, from about 10 % by weight to about 25 % by weight. The content of the stabilizer in the whole preparation is from about 0.1 % by weight to about 10 % by weight, preferably, from about 1 % by weight to about 5 % by weight. The above-mentioned preparation may further contain additives such as a coloring agent, sweetening agent, preservative and the like, if necessary.

The dosage of a combination agent of the present invention differs depending on the kind of the compound (I) of the present invention, age, body weight, condition, drug form, administration method, administration period and the like, and for example, for one breast cancer patient (adult, body weight: about 60 kg), the combination agent is administered intravenously, at a dose of about 0.01 mg/kg/day to about 1, 000 mg/kg/day, preferably about 0.01 mg/kg/day to about 100 mg/kg/day, more preferably about 0.1 mg/kg/day to about 100 mg/kg/day, particularly about 0.1 mg/kg/day to about 50 mg/kg/day, especially about 1.5 mg/kg/day to about 30 mg/kg/day, in terms of the androgen receptor antagonistic drug of the present invention or the other combined drug, respectively, once or several time in division a day. Of course, since the dose as described above varies depending on various conditions, amounts smaller than the above-mentioned dosage may sometimes be sufficient, further, amounts over that range sometimes have to be administered.

The amount of the other combined drug can be set at any value unless side effects are problematical. The daily dosage in terms of the combination drug differs depending on the severity, age, sex, body weight, sensitivity difference of the subject, administration period, interval, and nature, pharmacy, kind of the pharmaceutical preparation, kind of effective ingredient, and the like, and not particularly restricted, and the amount of a drug is, in the case of oral administration for example, usually from about 0.001 mg to about 2,000 mg, preferably from about 0.01 mg to about 500 mg, further preferably from about 0.1 mg to about 100 mg, per 1 kg of a mammal and this is usually administered once to 4-times in division a day.

In administration of the combination agent of the present invention, the androgen receptor antagonistic drug of the present invention may be administered after administration of the other combined drug or the other combined drug may be administered after administration of the androgen receptor antagonistic drug of the present invention, though they may be. administered simultaneously. When administered at a time interval, the interval differs depending on the effective ingredient, drug form and administration method, and for example, when the other combined drug is administered first, a method in which the androgen receptor antagonistic drug of the present invention is administered within time range of from 1 minute to 3 days, preferably from 10 minutes to 1 day, more preferably from 15 minutes to 1 hour after administration of the combined drug is exemplified. When the androgen receptor antagonistic drug of the present invention is administered first, a method in which the combined drug is administered within time range of from 1 minute to 1 day, preferably from 10 minutes to 6 hours, more preferably from 15 minutes to 1 hour after administration of the androgen receptor antagonistic drug of the present invention is exemplified.

In a preferable administration method, for example, the other combined drug which has been formed into an oral administration preparation is administered orally at a daily dose of about 0.001 mg/kg to about 200 mg/kg, and 15 minuteslater, the androgen receptor antagonistic drug of the present invention which has been formed into an oral administration preparation is administered orally at a daily dose of about 0.005 mg/kg to about 100 mg/kg.

### Examples

Further, the present invention is specifically explained by the following Reference Examples and Examples, but these are merely practical examples and not limiting the present invention, and these Examples may be changed to such an extent that they do not deviate from the scope of the present invention.

In the following Reference Examples, "room temperature" means usually about 10 °C to about 35 °C, and "%" means weight percent unless otherwise specified. However, "yield" is indicated by mol/mol %.

Further, the other abbreviations used in the description have the meanings shown below.
- s:: singlet
- brs:: broad singlet
- d:: doublet
- t:: triplet
- q:: quartet
- dd:: double doublet
- ddd:: double double doublet
- dt:: double triplet
- m:: multiplet
- br :: broad
- J:: coupling constant
- Hz:: Hertz
- CDCl₃ :: Heavy chloroform
- ¹H-NMR :: Proton nuclear magnetic resonance
- Me:: methyl

### Reference Example 1

### Production of 1-nitro-4-[(1E)-2-nitro-1-propenyl]benzene

To a mixture of 4-nitrobenzaldehyde (23.9 g) and nitro ethane (30 g) was added n-butylamine (1.2 g) and the mixture was heated at 100 °C for 18 hours. The reaction mixture was concentrated. To the residue was added ethanol and the resulting crystals were collected by filtration. The crystals were further washed with ethanol and diisopropyl ether to obtain the titled compound (15.6 g) as yellow crystals.
¹H-NMR (CDCl₃) δ 2.46 (3H, s), 7.60 (2H, d, *J*= 8.7 Hz), 8.09 (1H, s), 8.33 (2H, d, *J*= 8.7 Hz).
IR (KBr): ν 3113, 3076, 3057, 2984, 2941, 2839, 1599, 1520 cm⁻¹.

### Reference Example 2

### Production of methyl

### 4-bromo-2,5-dimethyl-1H-pyrrole-3-carboxylate

To a solution of methyl 2,5-dimethyl-1H-pyrrole-3-carboxylate (6.85 g) and triethylamine (8.7 ml) in dichloromethane (270 ml) was added pyridine perbromohydrobromide (15.7 g) little by little at 0 °C. The reaction mixture was stirred at the same temperature for 2 hours and poured into saturated sodium chloride solution. The reaction mixture was extracted with ethyl acetate, and the ethyl acetate layer was dried over magnesium sulfate and concentrated. The residue was purified by column chromatography (carrier: silicagel, eluant: hexane-ethyl acetate) and recrystallized from ethyl acetate-hexane to obtain the titled compound (7.59 g) as yellow crystals.
¹H-NMR (CDCl₃) δ 2.19 (3H, s), 2.47 (3H, s) , 3.82 (3H, s), 8.20 (1H, s).

### Reference Example 3

### Production of ethyl 4-bromo-3,5-dimethyl-1H-pyrrole-2-carboxylate

By using ethyl 3,5-dimethyl-1H-pyrrole-2-carboxylate (5.15 g), the reaction and purification were carried out in the same manner as Reference Example 2 to obtain the titled compound (4.43 g) as colorless crystals.
¹H-NMR (CDCl₃) δ1.37 (3H, t, *J*=6.8 Hz), 2.26 (3H, s), 2.28 (3H, s), 4.32 (2H, q, *J*=6.8 Hz), 9.32 (1H, s).

### Reference Example 4

### Production of methyl 1-benzyl-4-bromo-2,5-dimethyl-1H-pyrrole-3-carboxylate

A suspension of sodium hydride (60% suspension of sodium hydride in oil, 0.26 g) in tetrahydrofuran (30 ml) was cooled on a ice bath, and to the suspension was added the compound (1.01 g) produced in Reference Example 2. The reaction mixture was heated at the same temperature for 30 minutes. To the reaction mixture was added benzyl bromide (0.57 ml), and the reaction mixture was stirred at room temperature for 4 hours. The reaction mixture was poured into saturated aqueous solution of sodium chloride and the reaction mixture was extracted with ethyl acetate. The ethyl acetate layer was dried over magnesium sulfate and concentrated. The residue was purified by column chromatography (carrier: silicagel, eluant: hexane-ethyl acetate), and recrystallized from ethyl acetate-hexane to obtain the titled compound (0.75 g) as colorless crystals.
¹H-NMR (CDCl₃) δ 2.15 (3H, s), 2.45 (3H, s), 3.84 (3H, s), 5.08 (2H, s), 6.8-7.0 (2H, m), 7.2-7.4 (3H, m).

### Reference Example 5

### Production of 1-benzyl-4-(methoxycarbonyl)-2,5-dimethyl-1H-pyrrol-3-ylbor onic acid

To a solution of the compound (550 mg) produced in Reference Example 4 in tetrahydrofuran (8.5 ml) was added dropwise a solution of butyllithium in hexane (1.6 mol/l, 1.1 ml) at -78 °C. The reaction mixture was stirred at the same temperature for 1 hour, and then a solution of trimethyl borate (1.94 ml) in tetrahydrofuran (34 ml) was added, and the reaction mixture was further stirred for 1 hour. To the reaction mixture was added a mixture of water (5 ml) and methanol (5ml), and the temperature of the reaction mixture was then elevated up to room temperature. The reaction mixture was poured into a saturated aqueous solution of sodium chloride and extracted with ethyl acetate. The ethyl acetate layer was dried over magnesium sulfate and concentrated. The residue was purified by column chromatography (carrier: silicagel, eluant: hexane-ethyl acetate) and recrystallized from ethyl acetate-hexane to obtain the titled compound (413 mg) as colorless crystals.
¹H-NMR (CDCl₃) δ 2.41 (3H, s), 2.46 (3H, s), 3.86 (3H, s), 6.8-7.0 (2H, m), 7.2-7.4 (3H, m).

### Reference Example 6

### Production of methyl 4-bromo-1-(4-fluorobenzyl)-2,5-dimethyl-1H-pyrrole-3-carbox ylate

By using the compound (3.71 g) produced in Reference Example 2 and 4-fluorobenzyl bromide (2.0 ml), the reaction and purification were carried out in the manner as Example 2 to obtain the titled compound (4.12 g) as colorless crystals.
¹H-NMR (CDCl₃) δ 2.14 (3H, s), 2.44 (3H, s), 3.85 (3H, s), 5.05 (2H, s), 6.8-7.0 (2H, m), 7.0-7.2 (2H, m).

### Reference Example 7

### Production of 1-(4-fluorobenzyl)-4-(methoxycarbonyl)-2,5-dimethyl-1H-pyrr ol-3-ylboronic acid

By using the compound (1.04 g) produced in Reference Example 6, the reaction and purification were carried out in the same. manner as Reference Example 5 to obtain the titled compound (510 mg) as colorless crystals.
¹H-NMR (CDCl₃) δ 2.40 (3H, s), 2.46 (3H, s), 3.87 (3H, s), 5.08 (2H, s), 6.7-6.9 (2H, m), 6.9-7.1 (2H, m), 7.58 (2H, brs).

### Reference Example 8

### Production of 4-bromo-2-(trifluoromethyl)benzonitrile

To a solution of 4-amino-2-(trifluoromethyl)benzonitrile (1.02 g) in acetonitrile (30 ml) was added dropwise *tert-butyl* nitrite (0.95 ml) at 0 °C. The reaction mixture was stirred at the same temperature for 0.5 hour. To the reaction mixture was added copper bromide (II) (1.39 g) little by little. The temperature of the reaction mixture was raised up to room temperature, and the reaction mixture was stirred further for 14 hours. The reaction mixture was poured into a saturated solution of sodium chloride, extracted with ethyl acetate, and the ethyl acetate layer was dried over magnesium sulfate and concentrated. The residue was purified by column chromatography (carrier: silicagel, eluant: hexane-ethyl acetate) and crystallized from ethyl acetate-hexane to obtain the titled compound (1.13 g) as pale yellow oily substance.
¹H-NMR (CDCl₃) δ 7.71 (1H, d, *J*=8.2 Hz), 7.8-7.9 (1H, m), 7.95 (1H, s).

### Reference Example 9

### Production of 4-bromophthalonitrile

By using 4-aminophthalonitrile(3.14 g), the reaction and purification were carried out in the same manner as Reference Example 8 to obtain the titled compound (729 mg) as colorless crystals.
¹H-NMR (CDCl₃) δ 7.68 (1H, d, *J*=8.4 Hz), 7.8-8.0 (2H, m).

### Reference Example 10

### Production of methyl 4-bromo-1-[tert-butyl(dimethyl)silyl]-2,5-dimethyl-1H-pyrro le-3-carboxylate

By using the compound (1.87 g) produced in Reference Example 2 and tert-butyl (dimethyl) silyl chloride (1.34 g), the reaction and purification were carried out in the same manner as Example 2 to obtain the titled compound (1.86 g) as colorless crystals.
¹H-NMR (CDCl₃) δ 0.57 (6H, s), 0.97 (9H, s), 2.30 (3H, s), 2.58 (3H, s), 3.83 (3H, s).

### Reference Example 11

### Production of 1-[tert-butyl(dimethyl)silyl]-4-(methoxycarbonyl)-2,5-dimet hyl-1H-pyrrol-3-ylboronic acid

By using the compound (0.95 g) produced in Reference Example 10, the reaction and purification were carried out in the same manner as Reference Example 5 to obtain the titled compound (640 mg) as colorless crystals.
¹H-NMR (CDCl₃) δ 0.60 (6H, s), 1.01 (9H, s), 2.55 (3H, s), 2.59 (3H, s), 3.86 (3H, s), 7.37 (2H, brs).

### Reference Example 12

### Production of 4-bromo-3,5-dimethyl-1H-pyrrole-2-carbonitrile

By using 3,5-dimethyl-1H-pyrrole-2-carbonitrile (1.82 g, produced by a method described in *Synthesis*, 1999, 46-48) , the reaction and purification were carried out in the same manner as Reference Example 2 to obtain the titled compound (2.74 g) as colorless crystals.
¹H-NMR (CDCl₃) δ 2.16 (3H, s), , 2.26 (3H, s), 8.73 (1H, s).

### Reference Example 13

### Production of 4-bromo-2,5-dimethyl-1H-pyrrole-3-carbonitrile

By using 2,5-dimethyl-1H-pyrrole-3-carbonitrile (750 mg, produced by a method described in *J. Org. Chem.,* 43, 4273-4276 (1978) ) , the reaction and purification were carried out in the same manner as Reference Example 2 to obtain the titled compound (867 mg) as colorless crystals.
¹H-NMR (CDCl₃) δ 2.19 (3H, s), 2.38 (3H, s), 8.45 (1H, s).

### Reference Example 14

### Production of 1-nitro-4-[(1E)-2-nitro-1-butenyl]benzene

By using 1-nitropropane (25 ml) , the reaction and purification were carried out in the same manner as Reference Example 1 to obtain the titled compound (9.48 g) as colorless crystals.
¹H-NMR (CDCl₃ δ 1.28 (3H, t, *J*=7.8 Hz), 2.83 (2H, q, *J*=7.8 Hz), 7.51 (2H, d, *J*=8.2 Hz), 7.76 (2H, d, *J*=8.2 Hz), 7.97 (1H, s).

### Reference Example 15

### Production of methyl 5-methylpyridine-2-carboxylate

Under carbon monoxide atmosphere, a solution of 2-bromo-5-methylpyridine (10.0 g), triethylamine (16.7 ml), methanol (2.4 ml), 1,1'-bis(diphenylphosphino)ferrocene (3.2 g) and palladium hydroxide (1.3 g) in N,N-dimethylformamide (150 ml) was stirred at 70 °C for 20 hours. The solution was allowed to stand until it cooled to room temperature. The reaction mixture was poured into a saturated aqueous solution of sodium chloride and extracted with ethyl acetate. The ethyl acetate layer was dried over magnesium sulfate, and concentrated. The residue was purified by column chromatography (carrier: silicagel, eluant: hexane-ethyl acetate) and recrystallized from ethyl acetate-hexane to obtain the titled compound (1.69 g) as yellow crystals.
¹H-NMR (CDCl₃) δ2.42 (3H, s), 4.00 (3H, s), 7.63 (1H, dd, *J*=1.5, 8.1 Hz), 8.03 (1H, d, *J*=8.1 Hz), 8.56 (1H, d, *J*=1.5 Hz).

### Reference Example 16

### Production of tert-butyl 5-methylpyridin-2-ylcarbamate

A solution of 2-amino-5-methylpyridine (5.01 g) and di-tert-butyl dicarbonate (11.7 ml) in 2-methyl-2-propanol (100 ml) was stirred at room temperature for 20 hours. The reaction mixture was poured into a saturated aqueous solution of sodium chloride, and extracted with ethyl acetate. The ethyl acetate layer was dried over magnesium sulfate, and concentrated. The residue was purified by column chromatography (carrier: silicagel, eluant: hexane-ethyl acetate) and recrystallized from ethyl acetate-hexane to obtain the titled compound (4.60 g) as yellow crystals.
¹H-NMR (CDCl₃) δ 1.49 (9H, s), 2.26 (3H, s), 7.4-7.6 (1H, m), 7.84 (1H, d, *J*=8.4 Hz), 8.0-8.2 (2H, m).

### Reference Example 17

### Production of methyl 4-bromo-1-(4-cyanobenzyl)-2,5-dimethyl-1H-pyrrole-3-carboxy late

By using the compound (1.88 g) produced in Reference Example 2 and 4-cyanobenzyl bromide (1.59 g), the reaction and purification were carried out in the same manner as Example 2 to obtain the titled compound (2.06 g) as colorless crystals.
1H-NMR (CDC13) δ 2.13 (3H, s), 2.42 (3H, s), 3.85 (3H, s), 5.13 (2H, s), 6.99 (2H, d, *J*=8.0 Hz), 7.63 (2H, d, *J*=8.0 Hz).

### Reference Example 18

### Production of (6-methylpyridin-3-yl)methanol

To a solution of methyl 6-methylnicotinate (2.45 g) in tetrahydrofuran (30 ml) was added slowly lithium aluminum hydride (0.62 g) at 0 °C. After the temperature of the reaction mixture was elevated to room temperature, the reaction mixture was stirred at the same temperature for 2 hours. The reaction mixture was poured into saturated brine, and extracted with ethyl acetate. The ethyl acetate layer was dried over magnesium sulfate and concentrated. The residue was purified by column chromatography (carrier: silicagel, eluant: hexane-ethyl acetate) to obtain the titled compound (1.49 g) as a yellow oily substance.
¹H-NMR (CDCl₃) δ 2.51 (3H, s), 3.86 (1H, brs), 4.66 (2H, s), 7.13 (1H, d, *J*=8.0 Hz), 7.61 (1H, dd, *J*=2.2, 8.0 Hz), 7.63 (1H, d, *J*=2.2 Hz) .

### Reference Example 19

### Production of 1,2-dichloro-4-((1E)-2-nitro-1-propenyl)benzene

By using 3,4-dichlorobenzaldehyde (91.44 g), the reaction and purification were carried out in the same manner as Reference Example 1 to obtain the titled compound (62.5 g) as yellow crystals.
¹H-NMR (CDCl₃) δ: 2.43 (3H, s), 7.23-7.27 (1H, m), 7.50-7.54 (2H, m), 7.95 (1H, s).

### Reference Example 20

### Production of 3-((1E)-2-nitro-1-propenyl)benzonitrile

By using 3-cyanobenzaldehyde (21.00 g), the reaction and purification were carried out in the same manner as Reference Example 1 to obtain the titled compound (16.51 g) as pale yellow crystals.
IR (KBr): ν 2232, 1520, 1327, 799, 681 cm⁻¹.
¹H-NMR (CDCl₃) δ: 2.44 (3H, s), 7.55-7.75 (4H, m), 8.03 (1H, s).

### Reference Example 21

### Production of ethyl 4-bromo-1-(4-fluorobenzyl)-3,5-dimethyl-1H-pyrrole-2-carbox ylate

By using (3.69 g) produced in Reference Example 3 and 4-fluorobenzyl bromide (1.9 ml), the reaction and purification were carried out in the same manner as Example 2 to obtain the titled compound (4.11 g) as colorless crystals.
IR (KBr): ν 1694, 1512, 1271, 1138 cm⁻¹.
¹H-NMR (CDCl₃ δ: 1.29 (3H, t, *J*=7.2 Hz), 2.19 (3H, s) , 2.34 (3H, s), 4.23 (2H, q, *J*=7.2 Hz), 5.55 (2H, s), 6.88-7.00 (4H, m).

### Reference Example 22

### Production of 4-bromo-3,5-dimethyl-1H-pyrrole-2-carboxylic acid

A mixture of the compound (7.00 g) produced in Reference Example 3, 6N sodium hydroxide (100 ml) and ethanol (100 ml) was heated for 3 hours under reflux and concentrated. The residue was cooled on a ice bath, adjusted to pH 1 by adding hydrogen chloride little by little, and extracted with ethyl acetate. The ethyl acetate layer was dried over anhydrous magnesium sulfate and concentrated to obtain the titled compound (6.20 g) as pale brown crystals.
IR (KBr): ν 3212, 1686, 1240 cm⁻¹.
¹H-NMR (CDCl₃) δ: 2.28 (3H, s), 2.32 (3H, s), 8.91 (1H, br).

### Reference Example 23

### Production of benzyl 4-bromo-3,5-dimethyl-1H-pyrrole-2-carboxylate

To a mixture of the compound (6.20 g) produced in Reference Example 22, potassium hydrogencarbonate (8.5 g) and dimethylformamide (50 ml) was added dropwise benzyl bromide (3.45 ml) . The reaction mixture was stirred at room temperature for 14 hours, diluted with water and extracted with ethyl acetate. The ethyl acetate layer was washed with 1N hydrochloric acid, water and saturated sodium bicabonate water, successively, dried over magnesium sulfate and concentrated. The residue was purified by column chromatography (carrier: silicagel, eluant: ethyl acetate) to obtain the titled compound (8.46 g) as colorless crystals.
IR (KBr): ν 3283, 1672, 1288 cm⁻¹.
¹H-NMR (CDCl₃) δ: 2.25 (3H, s) , 2.30 (3H, s), 5.30 (2H, s), 7.32-7.41 (5H, m), 8.79 (1H, br).

### Reference Example 24

### Production of benzyl 4-bromo-1-(4-cyanobenzyl)-3,5-dimethyl-1H-pyrrole-2-carboxy late

A suspension of sodium hydride (60% oil suspension, 0.58 g) in dimethylformamide (10 ml) was cooled on ice bath, and to the suspension was added dropwise a solution of the compound (4.46 g) produced in Reference Example 23 in dimethylformamide (15 ml) . The reaction mixture was stirred at the same temperature for 10 minutes. To the reaction mixture was added 4-cyanobenzyl bromide (2.84 g) and the reaction mixture was further stirred for 3 hours. The reaction mixture was poured into water and extracted with ethyl acetate. The ethyl acetate layer was washed with water, water and saturated brine, successively, dried over magnesium sulfate and concentrated. The residue was purified by column chromatography (carrier: silicagel, eluant: hexane-ethyl acetate), and recrystallized from ethyl acetate-hexane to obtain the titled compound (5.73 g) as colorless crystals.
IR (KBr): ν 2228, 1694, 1424, 1271, 1134, 743 cm⁻¹.
¹H-NMR (CDCl₃) δ: 2.18 (3H, s), 2.34 (3H, s), 5.20 (2H, s), 5.62 (2H, s), 6.96 (2H, d, *J*=8.8 Hz), 7.30-7.33 (5H, m), 7.55 (2H, d, *J*=8.8 Hz).

### Reference Example 25

### Production of 4-((1E)-2-nitro-1-propenyl)benzonitrile

By using 4-cyanobenzaldehyde (75.9 g), the reaction and purification were carried out in the same manner as Reference Example 1 to obtain the titled compound (55.1 g) as pale yellow crystals.

### Reference Example 26

### Production of 1-bromo-4-((1E)-2-nitro-1-propenyl)benzene

By using 4-bromobenzaldehyde (50.4 g), the reaction and purification were carried out in the same manner as Reference Example 1 to obtain the titled compound (33.2 g) as pale brown crystals.

### Reference Example 27

### Production of 4-((1E)-2-nitro-1-ethenyl)benzonitrile

To a mixture of 4-cyanobenzaldehyde (50.7 g) and nitro methane (100 g) was added *n*-butylamine (2.4 ml), and the reaction mixture was heated for 18 hours under reflux. The reaction mixture was cooled to room temperature. The resulting precipitates were collected to obtain the titled compound (21.7 g) as a brown powder.
¹H-NMR (CDCl₃) δ: 7.61 (1H, d, *J*=13.6 Hz), 7.66 (2H, d, *J*=8.4 Hz), 7.76 (2H, d, *J*=8.4 Hz), 7.99 (1H, d, *J*=13.6 Hz).

### Reference Example 28

### Production of 2-iodomethyl-1-trityl-1H-imidazole

To a mixture of 2-hydroxymethyl-1-trityl-1H-imidazole (5.13 g) , triethylamine (2.5 ml) and dichloromethane (100 ml) was added methanesulfonyl chloride (1.3 ml), and the reaction mixture was stirred at room temperature for 18 hours and poured into water. The dichloromethane layer was separated, washed with saturated brine, dried over magnesium sulfate and concentrated. The residue was dissolved in acetone (100 ml), and to the reaction mixture was added sodium iodide (6.75 g) . The reaction mixture was heated for 1 hour under reflux, concentrated and dissolved in a solution of ethyl acetate and 5% sodium thiosulfate. The ethyl acetate layer was separated and washed with saturated brine, dried over magnesium sulfate and concentrated to obtain the titled compound (2.53 g) as colorless crystals.
IR (KBr): ν 1491, 1447, 752, 700 cm⁻¹.
¹H-NMR (CDCl₃) δ: 3.76 (2H, s), 6.69 (1H, d, J=1.6Hz), 7.08-7.41 (16H, m).

### Reference Example 29

### Production of 4-bromomethyl-1-trityl-1H-imidazole

A mixture was 4-hydroxymethyl-1-trityl-1H-imidazole (3.40 g) , triphenylphosphine (3.14 g) and dichloromethane (50 ml) was cooled on ice bath, and to the mixture was added N-bromosuccinimide (2.14 g) . The reaction mixture was stirred at 0 °C for 2 hours. To the reaction mixture was added saturated sodium bicarbonate solution, and the dichloromethane layer was separated. The dichloromethane layer was purified by column chromatography (carrier: silicagel, eluant: hexane-ethyl acetate) to obtain the titled compound (2.00 g) as colorless solids. The compound was unstable and thus it was used for the following reaction at once.

### Reference Example 30

### Production of 4-iodomethyl-1-trityl-1H-1,2,3-triazole

By using 4-hydroxymethyl-1-trityl-1H-1,2,3-triazole (9.30 g), the reaction and purification were carried out in the same manner as Reference Example 28 to obtain the titled compound (9.93 g) as pale brown crystals.

### Reference Example 31

### Production of 4-bromo-1-(4-fluorobenzyl)-3,5-dimethyl-1H-pyrrole-2-carbox ylic acid

By using the compound (2.00 g) produced in Reference Example 21, the reaction and purification were carried out in the same manner as Reference Example 22 to obtain the titled compound (1.85 g) as colorless crystals.
IR (KBr): ν 1655, 1508, 1447, 1292, 1217, 1148 cm⁻¹.
¹H-NMR (CDCl₃) δ: 2.19 (3H, s), 2.37 (3H, s), 5.56 (2H, s), 6.87-6.99 (4H, m).

### Reference Example 32

### Production of tert-butyl 4-bromo-1-(4-fluorobenzyl)-3,5-dimethyl--1H-pyrrol-2-ylcarba mate

Amixture of the compound (1.11 g) produced in Reference Example 31, diphenylphosphoryl azide (1.17 g), triethylamine(0.71 ml) and tert-butyl alcohol (15 ml) was heated for 16 hours under reflux and concentrated. The residue was dissolved in tetrahydrofuran and saturated sodium bicarbonate water. The tetrahydrofuran layer was separated, washed with saturated brine, dried over magnesium sulfate and concentrated. The residue was purified by column chromatography (carrier: silicagel, eluant: hexane-ethyl acetate) to obtain the titled compound (0.84 g).
IR (KBr): ν 3295, 1701, 1510, 1159 cm⁻¹.
¹H-NMR (CDCl₃) δ: 1.42 (9H, s), 1.95 (3H, s), 2.07 (3H, s), 4.92 (2H, s), 5.50-5.70 (1H, br), 6.90-7.00 (4H, m).

### Reference Example 33

### Production of 3-(1-bromoethyl)pyridine

A mixture of 3-ethylpyridine (0.86 g), 2,2'-azobis(isobutyronitrile) (0.13 g), N-bromosuccinimide (1.57 g) and carbon tetrachloride (16 ml) was heated in a 90 °C oil bath for 1 hour. The reaction mixture was cooled to room temperature and insoluble matter was removed by filtration. The filtrate was washed with water, saturated brine, successively, dried over magnesium sulfate and concentrated to obtain the titled crude compound (0.84 g) as a colorless oily substance. The obtained crude compound was used for the next reaction.
¹H-NMR (CDCl₃) δ: 2.06 (3H, d, *J*=7.0 Hz), 5.19 (1H, q, *J*=7.0 Hz), 7.30 (1H, dd, *J*=8.0, 4.8 Hz), 7.79 (1H, dt, *J*=8.0, 2.0 Hz), 8.53 (1H, dd, *J*=4.8, 2.0 Hz), 8.65-8.67 (1H, m).

### Example 1

### Production of ethyl 2,5-dimethyl-4-(4-nitrophenyl)-1H-pyrrole-3-carboxylate

A suspension of powdery potassium hydroxide (0.15 g) in tetrahydrofuran (5 ml) was cooled in an ice bath, and to the suspension was added dropwise ethyl acetoacetate (1.0 ml) . The mixture was stirred at the same temperature for 15 minutes, and to the mixture was added the compound (0.56 g) produced in Reference Example 1. The reaction mixture was stirred at room temperature for 3 hours, poured into water and extracted with ethyl acetate. The ethyl acetate layer was washed with saturated brine, dried over magnesium sulfate and concentrated. To the residue were added methanol (16 ml), water (1.2 ml) and concentrated hydrochloric acid (0.2 ml) and,the reaction mixture was heated for 2 hours under reflux. The reaction mixture was concentrated, diluted with saturated sodium bicarbonate water and extracted with ethyl acetate. The ethyl acetate layer was washed with saturated brine, dried over magnesium sulfate and concentrated. The residue was purified by column chromatography (carrier: silicagel, eluant: hexane-ethyl acetate) to obtain the titled compound (0.32 g) as yellow crystals.
¹H-NMR (CDCl₃) δ: 1.11 (3H, t, *J*=7.2 Hz), 2.14 (3H, s), 2.52 (3H, s), 4.12 (2H, q, *J*=7.2 Hz), 7.41 (2H, d, *J*=8.7 Hz), 8.20 (2H, d, *J*=8.7 Hz), 8.23 (1H, brs).
IR (KBr): ν 2976, 2930, 1684, 1595, 1516 cm⁻¹.

### Example 2

### Production of ethyl 1,2,5-trimethyl-4-(4-nitrophenyl)-1H-pyrrole-3-carboxylate

A suspension of sodium hydride (60% oil suspension, 32 mg) in tetrahydrofuran (1 ml) was cooled in an ice bath, and to the suspension was added dropwise a solution of the compound (230 mg) produced in Example 1 in tetrahydrofuran (1 ml). The reaction mixture was stirred at the same temperature for 1 hour. To the reaction mixture was added methyl iodide (0.05 ml) and the reaction mixture was further stirred for 3 hours. The reaction mixture was poured into saturated brine and extracted with ethyl acetate. The ethyl acetate layer was dried over magnesium sulfate and concentrated. The residue was purified by column chromatography (carrier: silicagel, eluant: hexane-ethyl acetate) and recrystallized from ethyl acetate-hexane to obtain the titled compound (0.09 g) as yellow crystals.
¹H-NMR (CDCl₃) δ 1.04 (3H, t, *J*=7.2 Hz), 2.11 (3H, s), 2.56 (3H, s), 3.49 (3H, s), 4.07 (2H, q, *J*=7.2 Hz), 7.37 (2H, d, *J*=8.7 Hz), 8.19 (2H, d, *J*=8.7 Hz).
IR (KBr): ν 2978, 2934, 2905, 2693, 1597, 1537, 1512 cm⁻¹.

### Example 3

### Production of ethyl 1-ethyl-2,5-dimethyl-4-(4-nitrophenyl)-1H-pyrrole-3-carboxy late

By using the compound (0.29 g) produced in Example 1 and ethyl iodide (0.09 ml), the reaction and purification were carried out in the same manner as Example 2 to obtain the titled compound (180 mg) as yellow crystals.
¹H-NMR (CDCl₃) δ 1.03 (3H, t, *J*=7.0 Hz), 1.32 (3H, t, *J*=7.0 Hz), 2.13 (3H, s), 2.58 (3H, s), 3.92 (2H, q, *J*=7.0 Hz), 4.07 (2H, q, *J*=7.0 Hz), 7.3-7.5 (2H, m), 8.1-8.3 (2H, m).
IR (KBr): ν2982, 2936, 1694, 1597, 1514, 1345 cm⁻¹.

### Example 4

### Production of ethyl 1-benzyl-2,5-dimethyl-4-(4-nitrophenyl)-1H-pyrrole-3-carbox ylate

By using the compound (4.87 g) produced in Example 1 and benzyl bromide (2.0 ml), the reaction and purification were carried out in the same manner as Example 2 to obtain the titled compound (5.3 g) as yellow crystals.
¹H-NMR (CDCl₃) δ 1.03 (3H, t, *J*=7.2 Hz), 2.05 (3H, s), 2.51 (3H, s), 4.10 (2H, q, *J*=7.2 Hz), 5.13 (2H, s), 6.9-7.1 (2H, m), 7.2-7.4 (3H, m), 7.42 (2H, d, *J*=8.8 Hz), 8.21 (2H, d, *J*=8.8 Hz).
IR (KBr): ν 2978, 2921, 1694, 1597, 1514, 1345 cm⁻¹.

### Example 5

### Production of ethyl 1-butyl-2,5-dimethyl-4-(4-nitrophenyl)-1H-pyrrole-3-carboxy late

By using the compound (0.29 g) produced in Example 1 and butyl iodide (0.13 ml), the reaction and purification were carried out in the same manner as Example 2 to obtain the titled compound (132 mg) as yellow crystals.
¹H-NMR (CDCl₃) δ 0.99 (3H, t, *J*=7.2 Hz) , 1.03 (3H, t, *J*=7.4 Hz), 1.3-1.5 (2H, m), 1.6-1.8 (2H, m), 2.12 (3H, s), 2.56 (3H, s), 3.83 (2H, t, *J*=7.8 Hz), 4.07 (2H, q, *J*=7.4 Hz), 7.37 (2H, d, *J*=8.8 Hz), 8.19 (2H, d, *J*=8.8 Hz).
IR (KBr): ν 2961, 2934, 1696, 1597, 1514, 1343 cm⁻¹.

### Example 6

### Production of ethyl 1-(4-hydroxybutyl)-2,5-dimethyl-4-(4-nitrophenyl)-1H-pyrrol e-3-carboxylate

By using the compound (0.32 g) produced in Example 1 and 2-(4-iodobutoxy)tetrahydropyran (0.32 g), the reaction and purification were carried out in the same manner as Example 2 to obtain a crude product (0.16 g) containing ethyl 2,5-dimethyl-4-(4-nitrophenyl)-1-[4-(tetrahydro-2H-pyran-2-yloxy)butyl]-1H-pyrrole-3-carboxylate. To the crude product were added methanol (3 ml) and 1 N hydrochloric acid (1 ml), and the mixture was stirred at room temperature for 2 hours. The reaction mixture was neutralized by the addition of saturated sodium bicarbonate and extracted with ethyl acetate. The ethyl acetate layer was washed with saturated brine, dried over magnesium sulfated and concentrated. The residue was purified by recrystallization (ethyl acetate-hexane) to obtain the titled compound (62 mg) as brown crystals.
¹H-NMR (CDCl₃) δ 1.03 (3H, t, *J*=7.4 Hz), 1.5-1.9 (4H, m), 2.13 (3H, s), 2.57 (3H, s), 3.6-3.8 (2H, m), 3.8-4.0 (2H, m), 4.07 (2H, q, *J*=7.0 Hz), 7.37 (2H, d, *J*=8.8 Hz), 8.19 (2H, d, *J*=8.8 Hz).
IR (KBr) : ν 3480, 2980, 2938, 2872, 1694, 1597, 1514, 1343 cm⁻¹.

### Example 7

### Production of ethyl 2,5-dimethyl-1-(methylsulfonyl)-4-(4-nitrophenyl)-1H-pyrrol e-3-carboxylate

By using the compound (0.14 g) produced in Example 1 and methanesulfonyl chloride (0.05 ml), the reaction and purification were carried out in the same manner as Example 2 to obtain the titled compound (115 mg) as yellow crystals.
¹H-NMR (CDCl₃) δ 0.97 (3H, t, *J*=7.2 Hz), 2.28 (3H, s), 2.80 (3H, s), 3.26 (3H, s), 4.05 (2H, q, *J*=7.2 Hz), 7.3-7.5 (2H, m), 8.2-8.3 (2H, m).
IR (KBr): ν 2982, 2934, 1709, 1599, 1373, 1348 cm⁻¹.

### Example 8

### Production of ethyl 1-benzoyl-2,5-dimethyl-4-(4-nitrophenyl)-1H-pyrrole-3-carbo xylate

By using the compound (0.14 g) produced in Example 1 and benzoyl chloride (0.06 ml), the reaction and purification were carried out in the same manner as Example 2 to obtain the titled compound (129 mg) as yellow crystals.
¹H-NMR (CDCl₃) δ 1.06 (3H, t, *J*=7.2 Hz), 1.98 (3H, s), 2.39 (3H, s), 4.11 (2H, q, *J*=7.2 Hz), 7.4-7.5 (2H, m), 7.5-7.6 (2H, m), 7.6-7.9 (3H, m), 8.1-8.3 (2H, m).
IR (KBr): ν 2982, 1705, 1599, 1516, 1346, 1329 cm⁻¹.

### Example 9

### Production of ethyl 2,5-dimethyl-4-(4-nitrophenyl)-1-(phenylsulfonyl)-1H-pyrrol. e-3-carboxylate

By using the compound (0.14 g) produced in Example 1 and benzenesulfonyl chloride, the reaction and purification were carried out in the same manner as Example 2 to obtain the titled compound (111 mg) as yellow crystals.
¹H-NMR (CDCl₃) δ 0.94 (3H, t, *J*=7.2 Hz), 2.29 (3H, s), 2.77 (3H, s), 4.02 (2H, q, *J*=7.2 Hz), 7.2-7.4 (2H, m), 7.5-7.8 (3H, m), 7.8-7.9 (2H, m), 8.1-8.3 (2H, m).
IR (KBr): ν 2982, 2938, 1709, 1599, 1518, 1375, 1346 cm⁻¹.

### Example 10

### Production of ethyl 2,5-dimethyl-4-(4-nitrophenyl)-1-(2-phenylethyl)-1H-pyrrole - 3-carboxylate

By using the compound (0.15 g) produced in Example 1 and phenethyl iodide (0.12 ml), the reaction and purification were carried out in the same manner as Example 2 to obtain the titled compound (16 mg) as yellow crystals.
¹H-NMR (CDCl₃) δ 1.04 (3H, t, *J*=7.2 Hz), 1.92 (3H, s), 2.51 (3H, s), 2.95 (2H, t, *J*=7.4 Hz), 4.0-4.2 (4H, m), 7.0-7.2 (2H, m), 7.2-7.4 (5H, m), 8.19 (2H, d, *J*=8.8 Hz).
IR (KBr): ν 3027, 2980, 2928, 1696, 1597, 1518, 1375, 1346 cm⁻¹.

### Example 11

### Production of methyl 4-(3-cyanophenyl)-2,5-dimethyl-1H-pyrrole-3-carboxylate

By using the compound (0.23 g) produced in Reference Example 2 and 3-cyanophenylboronic acid (0.16 g), the reaction and purification were carried out in the same manner as Example 14 to obtain the titled compound (138 mg) as pale yellow crystals.
¹H-NMR (CDCl₃) δ 2.11 (3H, s), 2.52 (3H, s), 3.63 (3H, s), 7.4-7.6 (4H, m), 8.04 (1H, s).
IR (KBr): ν 2951, 2226, 1674, 1606, 1446 cm⁻¹.

### Example 12

### Production of methyl 1-benzyl-4-(3-cyanophenyl)-2,5-dimethyl-1H-pyrrole-3-carbox ylate

By using the compound (84 mg) produced in Example 11 and benzyl bromide (0.05 ml), the reaction and purification were carried out in the same manner as Example 2 to obtain the titled compound (51 mg) as colorless crystals.
¹H-NMR (CDCl₃) δ 2.01 (3H, s), 2.50 (3H, s), 3.60 (3H, s), 5. 12 (2H, s), 6.9-7.0 (2H, m), 7.2-7.6 (7H, m).
IR (KBr): ν 3065, 3032, 2988, 2948, 2228, 1699, 1534 cm⁻¹.

### Example 13

### Production of 2,5-dimethyl-4-(4-nitrophenyl)-1H-pyrrole-3-carboxylic acid

The compound (390 mg) produced in Example 1 was added to concentrated sulfuric acid (1.0 ml) little by little, and the mixture was stirred at room temperature for 0.5 hour. The reaction mixture was added dropwise to ice water and extracted with ethyl acetate. The ethyl acetate layer was dried over magnesium sulfate and concentrated. The residue was recrystallized from ethyl acetate to obtain the titled compound (202 mg) as yellow crystals.
¹H-NMR (DMSO-d₆) δ2.09 (3H, s), 2.41 (3H, s), 7.44 (2H, d, *J*=7.6 Hz), 8.15 (2H, d, *J*=7.6 Hz), 11.27 (1H, s).
IR (KBr): ν 3368,3325,3304,1667 cm⁻¹.

### Example 14

### Production of methyl 4-(4-fluorophenyl)-2,5-dimethyl-1H-pyrrole-3-carboxylate

To a mixture of the compound (0.23 g) produced in Reference Example 2, 4-fluorophenylboronic acid (0.15 g), tetrakis(triphenylphosphine)palladium (0.06 g) and anhydrous sodium carbonate (0.32 g) were added dimethylformamide (8 ml) and water (2 ml) and the mixture was heated at 130 °C for 20 hours and cooled to room temperature. To the resulting reaction mixture was added water and extracted with ethyl acetate. The ethyl acetate layer was washed with saturated brine, dried over anhydrous magnesium sulfate and concentrated. The resulting crude product was purified by column chromatography (carrier: silicagel, eluant: hexane-ethyl acetate) to obtain the titled compound (138 mg) as pale yellow crystals.
¹H-NMR (CDCl₃) δ 2.09 (3H, s), 2.51 (3H, s), 3.62 (3H, s), 6.9-7.1 (2H, m), 7.1-7.3 (2H, m), 7.99 (1H, s).
IR (KBr): ν 3287, 3096, 2994, 2949, 1667 cm⁻¹.

### Example 15

### Production methyl of 4-(4-acetylphenyl)-2,5-dimethyl-1H-pyrrole-3-carboxylate

By using the compound (0.23 g) produced in Reference Example 2 and 4-acetylphenylboronic acid (0.18 g), the reaction and purification were carried out in the same manner as Example 14 to obtain the titled compound (146 mg) as pale yellow crystals.
¹H-NMR (CDCl₃) δ 2.13 (3H, s), 2.52 (3H, s), 2.62 (3H, s), 3.63 (3H, s), 7.34 (2H, d, *J*=8.4 Hz), 7.94 (2H, d, *J*=8.4 Hz), 8.23 (1H, s).
IR (KBr): ν 3306, 3119, 2949, 1682 cm⁻¹.

### Example 16

### Production of methyl 4-(4-(methoxycarbonyl)phenyl)-2,5-dimethyl-1H-pyrrole-3-car boxylate

By using the compound (0.23 g) produced in Reference Example 2 and 4-(methoxycarbonyl)phenylboronic acid (0.20 g), the reaction and purification were carried out in the same manner as Example 14 to obtain the titled compound (77 mg) as pale yellow crystals.
¹H-NMR (CDCl₃) δ 2.13 (3H, s), 2.52 (3H, s), 3.61 (3H, s), 3.92 (3H, s), 7.35 (2H, d, *J*=8.0 Hz), 7.59 (2H, d, *J*=8.4 Hz), 8.01 (1H, s).
IR (KBr): ν 3320, 2951, 1716, 1696 cm⁻¹.

### Example 17

### Production of methyl 4-(4-cyanophenyl)-2,5-dimethyl-1H-pyrrole-3-carboxylate

By using the compound (0.23 g) produced in Reference Example 2 and 4-cyanophenylboronic acid (0.16 g), the reaction and purification were carried out in the same manner as Example 14 to obtain the titled compound (82 mg) as pale yellow crystals.
¹H-NMR (CDCl₃) δ 2.13 (3H, s), 2.52 (3H, s), 3.63 (3H, s), 7.35 (2H, d, *J*=8.0 Hz), 7.62 (2H, d, *J*=8.4 Hz), 8.14 (1H, s).
IR (KBr): ν 3296, 2990, 2951, 2226, 1674 cm⁻¹.

### Example 18

### Production of methyl 2,5-dimethyl-4-(4-(trifluoromethyl)phenyl)-1H-pyrrole-3-car boxylate

By using the compound (0.23 g) produced in Reference Example 2 and 4-(trifluoromethyl)phenylboronic acid (0.21 g), the reaction and purification were carried out in the same manner as Example 14 to obtain the titled compound (108 mg) as pale yellow crystals.
¹H-NMR (CDCl₃) δ 2.12 (3H, s), 2.52 (3H, s), 3.63 (3H, s), 7.35 (2H, d, *J*=8.0 Hz), 7.59 (2H, d, *J*=8.0 Hz), 8.01 (1H, s).
IR (KBr): ν 3287, 2949, 2862, 2714, 1669 cm⁻¹.

### Example 19

### Production of methyl 4-(4-formylphenyl)-2,5-dimethyl-1H-pyrrole-3-carboxylate

By using the compound (0.23 g) produced in Reference Example 2 and 4-formylphenylboronic acid (0.21 g), the reaction and purification were carried out in the same manner as Example 14 to obtain the titled compound (125 mg) as pale yellow crystals.
¹H-NMR (CDCl₃) δ 2.15 (3H, s), 2.53 (3H, s), 3.63 (3H, s), 7.41 (2H, dd, *J*= 6.6, 1.8 Hz), 7.86 (2H, dd, *J*= 6.6, 1.8 Hz), 8.11 (1H, s), 10.02 (1H, s).
IR (KBr): ν 2948, 2841, 1695, 1682, 1447 cm⁻¹.

### Example 20

### Production of methyl 1-benzyl-4-(4-formylphenyl)-2,5-dimethyl-1H-pyrrole-3-carbo xylate

By using the compound (86 mg) produced in Example 19 and benzyl bromide (0.05 ml), the reaction and purification were carried out in the same manner as Example 2 to obtain the titled compound (46 mg) as colorless crystals.
¹H-NMR (CDCl₃) δ 2.06 (3H, s), 2.50 (3H, s), 3.60 (3H, s), 5.13 (2H, s), 6.9-7.0 (2H, m), 7.2-7.6 (5H, m), 7.87 (2H, d, *J*=8.8 Hz), 10.03 (1H, s).
IR (KBr): ν 3030, 2948, 1696, 1605, 1537, 1439 cm⁻¹.

### Example 21

### Production of methyl 2,5-dimethyl-4-(4-(methylsulfonyl)phenyl)-1H-pyrrole-3-carb oxylate

By using the compound (0.23 g) produced in Reference Example 2 and 4-(methylsulfonyl)phenylboronic acid (0.22 g), the reaction and purification were carried out in the same manner as Example 14 to obtain the titled compound (198 mg) as pale yellow crystals.
¹H-NMR (CDCl₃) δ 2.13 (3H, s), 2.52 (3H, s), 3.10 (3H, s), 3.65 (3H, s), 7.44 (2H, d, *J*=8.0 Hz), 7.89 (2H, d, *J*=8.0 Hz), 8.16 (1H, s).
IR (KBr): ν 3287, 2949, 2862, 2714, 1669 cm⁻¹.

### Example 22

### Production of 4-(4-(methoxycarbonyl)-2,5-dimethyl-1H-pyrrol-3-yl)benzoic acid

By using the compound (0.23 g) produced in Reference Example 2 and 4- (dihydroxyboryl) benzoic acid ( 0 .18 g) , the reaction and purification were carried out in the same manner as Example 14 to obtain the titled compound (118 mg) as pale yellow crystals.
¹H-NMR (DMSO-d₆) δ 2.06 (3H, s), 2.40 (3H, s), 3.51 (3H, s), 7.26 (2H, d, *J*=8.0 Hz), 7.87 (2H, d, *J*=8.0 Hz), 11.25 (1H, s).
IR (KBr): ν 3296, 3252, 1682, 1669 cm⁻¹.

### Example 23

### Production of methyl 4-(4-(aminocarbonyl)phenyl)-2,5-dimethyl-1H-pyrrole-3-carbo xylate

By using the compound (0.23 g) produced in Reference Example 2 and 4-(aminocarbonyl)phenylboronic acid (0.17 g), the reaction and purification were carried out in the same manner as Example 14 to obtain the titled compound (150 mg) as pale yellow crystals.
¹H-NMR (CDCl₃) δ 2.12 (3H, s), 2.49 (3H, s), 3.60 (3H, s), 6.18 (1H, brs), 7.04 (1H, brs), 7.30 (2H, d, *J*=8.0 Hz), 7.83 (2H, d, *J*=8.0 Hz), 10.28 (1H, s).
IR (KBr): ν 3103, 2955, 1669, 1609 cm⁻¹.

### Example 24

### Production of methyl 4-(3-nitrophenyl)-2,5-dimethyl-1H-pyrrole-3-carboxylate

By using the compound (0.46 g) produced in Reference Example. 2 and 3-nitrophenylboronic acid (0.34 g), the reaction and purification were carried out in the same manner as Example 14 to obtain the titled compound (240 mg) as yellow crystals.
¹H-NMR (CDCl₃) δ2.14 (3H, s), 2.54 (3H, s), 3.63 (3H, s), 7.5-7.7 (2H, m), 8.03 (1H, s), 8.1-8.2 (2H, m).

### Example 25

### Production of methyl 4-(3-aminophenyl)-2,5-dimethyl-1H-pyrrole-3-carboxylate

By using the compound (0.23 g) produced in Reference Example 2 and 3-aminophenylboronic acid (0.16 g), the reaction and purification were carried out in the same manner as Example 14 to obtain the titled compound (77 mg) as pale yellow crystals.
¹H-NMR (CDCl₃) δ 2.11 (3H, s) , 2.50 (3H, s), 3.63 (3H, s) , 6.5-6.7 (3H, m), 7.0-7.2 (1H, m), 7.94 (1H, s).
IR (KBr): ν 3306, 3020, 2948, 1682 cm⁻¹.

### Example 26

### Production of methyl 2,5-dimethyl-4-(4-(methylthio)phenyl)-1H-pyrrole-3-carboxyl ate

By using the compound (0.23 g) produced in Reference Example 2 and 4-(methylthio)phenylboronic acid (0.17 g) , the reaction and purification were carried out in the same manner as Example 14 to obtain the titled compound (172 mg) as pale yellow crystals.
¹H-NMR (CDCl₃) δ 2.11 (3H, s), 2.50 (6H, s), 3.63 (3H, s), 7.1-7.4 (4H, m), 7.96 (1H, s).
IR (KBr): ν 3310, 2988, 2946, 2920, 1669 cm⁻¹.

### Example 27

### Production of methyl 4-(1-benzofuran-2-yl)-2,5-dimethyl-1H-pyrrole-3-carboxylate

By using the compound (0.23 g) produced in Reference Example 2 and 1-benzofuran-2-ylboronic acid (0.17 g), the reaction and purification were carried out in the same manner as Example 14 to obtain the titled compound (78 mg) as pale yellow crystals.
¹H-NMR (CDCl₃) δ 2.33 (3H, s), 2.51 (3H, s), 3.71 (3H, s), 6.76 (1H, s), 7.2-7.3 (2H, m), 7.4-7.6 (2H, m), 8.06 (1H, s).
IR (KBr): ν 3310, 2949, 2924, 1682 cm⁻¹.

### Example 28

### Production of methyl 2,5-dimethyl-4-(2-naphthyl)-1H-pyrrole-3-carboxylate

By using the compound (0.23 g) produced in Reference Example 2 and 2-naphthylboronic acid (0.18 g), the reaction and purification were carried out in the same manner as Example 14 to obtain the titled compound (91 mg) as pale yellow crystals.
¹H-NMR (CDCl₃) δ 2.16 (3H, s), 2.54 (3H, s), 3.58 (3H, s), 7.1-7.3 (3H, m), 7.66 (1H, s), 7.7-7.9 (3H, m), 7.98 (1H, s).
IR (KBr): ν 303, 3052, 2947, 1682 cm⁻¹.

### Example 29

### Production of methyl 2,5-dimethyl-4-(3-pyridinyl)-1H-pyrrole-3-carboxylat

By using the compound (0.23 g) produced in Reference Example 2 and 3-pyridinyldiethylborane (0.15 g), the reaction and purification were carried out in the same manner as Example 14 to obtain the titled compound (99 mg) as pale yellow crystals.
¹H-NMR (CDCl₃) δ2.13 (3H, s), 2.53 (3H, s), 3.62 (3H, s), 7.2-7.4 (1H, m), 7.5-7.7 (1H, m), 8.4-8.5 (2H, m), 8.57 (1H, s).
IR (KBr): ν 3287, 2948, 2743, 1693 cm⁻¹.

### Example 30

### Production of methyl 4-(3-furyl)-2,5-dimethyl-1H-pyrrole-3-carboxylate

By using the compound (0.23 g) produced in Reference Example 2 and 3-furylboronic acid (0.12 g), the reaction and purification were carried out in the same manner as Example 14 to obtain the titled compound (112 mg) as pale yellow crystals.
¹H-NMR (CDCl₃) δ 2.18 (3H, s), 2.50 (3H, s), 3.71 (3H, s), 6.47 (1H, dd, *J*=0.8, 1.8 Hz), 7.38 (1H, d, *J*=0.8 Hz), 7.42 (1H, d, *J*=1.8 Hz), 7.94 (1H, s).
IR (KBr): ν 3294, 2947, 2714, 1669 cm⁻¹.

### Example 31

### Production of methyl 2,5-dimethyl-4-(3-thienyl)-1H-pyrrole-3-carboxylate

By using the compound (0.23 g) produced in Reference Example 2 and 3-thienylboronic acid (0.13 g), the reaction and purification were carried out in the same manner as Example 14 to obtain the titled compound (109 mg) as pale yellow crystals.
¹H-NMR (CDCl₃) δ 2.17 (3H, s), 2.51 (3H, s), 3.67 (3H, s), 7.06 (2H, d, *J*=4.4 Hz), 7.2-7.3 (1H, m), 7.93 (1H, s).
IR (KBr): ν 3306, 2947, 2922, 1682 cm⁻¹.

### Example 32

### Production of methyl 4-(2,4-dimethoxy-5-pyrimidinyl)-2,5-dimethyl-1H-pyrrole-3-c arboxylate

By using the compound (0.23 g) produced in Reference Example 2 and 2,4-dimethoxy-5-pyrimidinylboronic acid (0.19 g), the reaction and purification were carried out in the same manner as Example 14 to obtain the titled compound (24 mg) as pale yellow crystals.
¹H-NMR (CDCl₃) δ 2.10 (3H, s), 2.52 (3H, s), 3.61 (3H, s), 3.94 (3H, s), 4.03 (3H, s), 8.02 (1H, s), 8.10 (1H, s)
IR (KBr): ν 3277, 2951, 1682, 1591 cm⁻¹.

### Example 33

### Production of methyl 4-(2-furyl)-2,5-dimethyl-1H-pyrrole-3-carboxylate

By using the compound (0.23 g) produced in Reference Example 2 and 2-furylboronic acid (0.19 g), the reaction and purification were carried out in the same manner as Example 14 to obtain the titled compound (24 mg) as pale yellow crystals.
¹H-NMR (CDCl₃) δ 2.24 (3H, s), 2.48 (3H, s), 3.72 (3H, s), 6.3-6.4 (1H, m), 6.4-6.5 (1H, m), 7.4-7.5 (1H, m), 8.00 (1H, s).
IR (KBr): ν 3303, 3160, 2949, 1682 cm⁻¹.

### Example 34

### Production of methyl 4-(4-hydroxyphenyl)-2,5-dimethyl-1H-pyrrole-3-carboxylate

By using the compound (0.23 g) produced in Reference Example 2 and 4-hydroxyphenylboronic acid (0.14 g), the reaction and purification were carried out in the same manner as Example 14 to obtain the titled compound (151 mg) as pale yellow crystals.
¹H-NMR (CDCl₃) δ 2.11 (3H, s), 2.51 (3H, s), 3.63 (3H, s), 4.93 (1H, s), 6.80 (2H, d, *J*= 8.8 Hz), 7.11 (2H, d, *J=* 8.8 Hz), 7.87 (1H, s).
IR (KBr): ν 3285, 2949, 1667, 1448 cm⁻¹.

### Example 35

### Production of methyl 4-(4-(hydroxymethyl)phenyl)-2,5-dimethyl-1H-pyrrole-3-carbo xylate

By using the compound (0.23 g) produced in Reference Example 2 and 4-hydroxyphenylboronic acid (0.15 g), the reaction and purification were carried out in the same manner as Example 14 to obtain the titled compound (89 mg) as pale yellow crystals.
¹H-NMR (CDCl₃) δ 2.12 (3H, s), 2.51 (3H, s), 3.62 (3H, s), 4.6-4.8 (3H, m), 7.25 (2H, d, *J*= 8. 0 Hz), 7. 35 (2H, d, *J*= 8. 0 Hz) , 7.97 (1H, s).
IR (KBr): ν 3248, 2920, 2860, 1684, 1539 cm⁻¹.

### Example 36

### Production of methyl 4-(3,5-bis(trifluoromethyl)phenyl)-2,5-dimethyl-1H-pyrrole-3-carboxylate

By using the compound (0.23 g) produced in Reference Example 2 and 3, 5-bis (trifluoromethyl)phenylboronic acid (0.26 g) , the reaction and purification were carried out in the same manner as Example 14 to obtain the titled compound (222 mg) as pale yellow crystals.
¹H-NMR (CDCl₃) δ 2.15 (3H, s) , 2.54 (3H, s) , 3.60 (3H, s)., 7.70 (2H, s), 7.75 (1H, s), 8.07 (1H, s).
IR (KBr): ν 3366, 2953, 1680, 1539 cm⁻¹.

### Example 37

### Production of methyl 4-(3,5-dichlorophenyl)-2,5-dimethyl-1H-pyrrole-3-carboxylat e

By using the compound (0.23 g) produced in Reference Example 2 and 3, 5-dichlorophenylboronic acid (0.19 g) , the reaction and purification were carried out in the same manner as Example 14 to obtain the titled compound (262 mg) as pale yellow crystals.
¹H-NMR (CDCl₃) δ 2.12 (3H, s), 2.51 (3H, s), 3.64 (3H, s), 7.1-7.2 (2H, m), 7.2-7.3 (1H, m), 7.99 (1H, s).
IR (KBr): ν 3299, 2948, 1682, 1588 cm⁻¹.

### Example 38

### Production of methyl 4-(3,4-dichlorophenyl)-2,5-dimethyl-1H-pyrrole-3-carboxylat e

By using the compound (0.55 g) produced in Reference Example 2 and 3, 4-dichlorophenylboronic acid (0. 38 g), the reaction and purification were carried out in the same manner as Example 14 to obtain the titled compound (393 mg) as pale yellow crystals.
¹H-NMR (CDCl₃) δ 2.11 (3H, s), 2.50 (3H, s), 3.64 (3H, s), 7.08 (1H, dd, *J*= 1.8, 8.0 Hz), 7.33 (1H, d, *J=* 1.8 Hz), 7.39 (1H, d, *J*= 8.0 Hz), 8.04 (1H, s).
IR (KBr): ν 3303, 2949, 2922, 1674 cm⁻¹.

### Example 39

### Production of methyl 1-benzyl-2,5-dimethyl-4-(3-nitrophenyl)-1H-pyrrole-3-carbox ylate

By using the compound (152 mg) produced in Example 24 and benzyl bromide (0.14 ml), the reaction and purification were carried out in the same manner as Example 2 to obtain the titled compound (126 mg) as yellow crystals.
¹H-NMR (CDCl₃) δ 2.04 (3H, s), 2.51 (3H, s), 3.60 (3H, s), 5.14 (2H, s), 6.95 (2H, d, *J*= 4.6 Hz), 7.2-7.4 (3H, m) , 7.4-7.6 (2H, m), 8.1-8.2 (2H, m).
IR (KBr): ν 3063, 2948, 1699, 1526 cm⁻¹.

### Example 40

### Production of methyl 1-benzyl-4-(4-fluorophenyl)-2,5-dimethyl-1H-pyrrole-3-carbo xylate

By using the compound (66 mg) produced in Example 14 and benzyl bromide (0.05 ml), the reaction and purification were carried out in the same manner as Example 2 to obtain the titled compound (23 mg) as colorless crystals.
¹H-NMR (CDCl₃) δ 2.01 (3H, s), 2.49 (3H, s), 3.60 (3H, s), 5.11 (2H, s), 6.9-7.0 (2H, m), 7.0-7.1 (2H, m), 7.2-7.4 (5H, m).
IR (kBr): ν 3031, 2946, 1696, 1537 cm⁻¹.

### Example 41

### Production of methyl 1-benzyl-2,5-dimethyl-4-(4-(trifluoromethyl)phenyl)-1H-pyrr. ole-3-carboxylate

By using the compound (66 mg) produced in Example 18 and benzyl bromide (0.05 ml), the reaction and purification were carried out in the same manner as Example 2 to obtain the titled compound (15 mg) as colorless oily substance.
¹H-NMR (CDCl₃) δ 2.03 (3H, s), 2.50 (3H, s), 3.60 (3H, s), 5.12 (2H, s), 6.9-7.0 (2H, m), 7.2-7.5 (5H, m), 7.5-7.7 (2H, m).

### Example 42

### Production of methyl 1-benzyl-4-(4-cyanophenyl)-2,5-dimethyl-1H-pyrrole-3-carbox ylate

By using the compound (176 mg) produced in Example 17 and benzyl bromide (0.14 ml), the reaction and purification were carried. out in the same manner as Example 2 to obtain the titled compound (141 mg) as colorless crystals.
¹H-NMR (CDCl₃) δ 2.03 (3H, s), 2.50 (3H, s), 3.61 (3H, s), 5.13 (2H, s), 6.94 (2H, d, *J*= 4.4 Hz), 7.2-7.4 (5H, m), 7.63 (2H, d, *J*= 4.4 Hz).
IR (KBr): ν 3031, 2948, 2224, 1699 cm⁻¹.

### Example 43

### Production of methyl 1-benzyl-4-(3,5-dichlorophenyl)-2,5-dimethyl-1H-pyrrole-3-c arboxylate

By using the compound (150 mg) produced in Example 37 and benzyl bromide (0.10 ml), the reaction and purification were carried out in the same manner as Example 2 to obtain the titled compound (170 mg) as colorless crystals.
¹H-NMR (CDCl₃) δ 2.02 (3H, s), 2.48 (3H, s), 3.63 (3H, s), 5.11 (2H, s), 6.8-7.0 (2H, m), 7.2-7.4 (4H, m), 8.2-8.4 (2H, m).
IR (KBr): ν 3065, 3031, 2946, 1700 cm⁻¹.

### Example 44

### Production of (2,5-dimethyl-4-(4-nitrophenyl)-1H-pyrrol-3-yl)methanol

The solution of the compound (290 mg) produced in Example 1 in toluene (8 ml) was cooled on an ice bath and to the solution was added dropwise a solution of 1 N diisobutyl aluminum hydride in toluene solution (2.0 ml) . This mixture was stirred at room temperature for 1 hour, and the reaction mixture was then poured into water and extracted with ethyl acetate. The ethyl acetate layer was washed with saturated brine, dried over magnesium sulfate and concentrated. The residue was purified by column chromatography (carrier: silicagel, eluant: hexane-ethyl acetate) to obtain the titled compound (0.12 g) as red crystals.
¹H-NMR (CDCl₃) δ 2.30 (3H, s), 2.32 (3H, s), 4.48 (2H, d, *J*= 3.6 Hz), 7.5-7.7 (2H, m), 7.86 (1H, s), 8.2-8.3 (2H, m).
IR (KBr): ν 3240, 2868, 1593, 1507 cm⁻¹.

### Example 45

### Production of (1-benzyl-2,5-dimethyl-4-(4-nitrophenyl)-1H-pyrrol-3-yl)met hanol

By using the compound (300 mg) produced in Example 4 and a solution of 1N diisobutyl aluminum hydride in toluene (1. 6 ml), the reaction and purification were carried out in the same manner as Example 44 to obtain the titled compound (136 mg) as yellow crystals.
¹H-NMR (CDCl₃) δ 2.21 (3H, s), 2.25 (3H, s), 4.52 (2H, d, *J*= 4.8 Hz), 5.11 (2H, s), 6.9-7.0 (2H, m), 7.2-7.4 (3H, m), 7.60 (2H, d, *J*= 8.8 Hz), 8.25 (2H, d, *J=*8.8 Hz).
IR (KBr): ν 3261, 2920, 2867, 1593 cm⁻¹.

### Example 46

### Production of 1-benzyl-3-(methoxymethyl)-2,5-dimethyl-4-(4-nitrophenyl)-1 H-pyrrole

To a solution of the compound (230 mg) produced in Example 45 in tetrahydrofuran (1 ml) was added sodium hydride (60% oil suspension, 20 mg) at room temperature. The reaction mixture was stirred at room temperature for 1 hour. To the reaction mixture was added methyl iodide (0.032 ml) and the reaction mixture was further stirred at 50 °C for 3 hours. The reaction mixture was poured into saturated brine and extracted with ethyl acetate. The ethyl acetate layer was dried over magnesium sulfate and concentrated. The residue was purified by column chromatography (carrier: silicagel, eluant: hexane-ethyl acetate) and recrystallized from ethyl acetate-hexane to obtain the titled compound (78 mg) as yellow crystals.
¹H-NMR (CDCl₃) δ 2.20 (3H, s), 2.23 (3H, s), 3.37 (3H, s), 4.21 (2H, s), 5.10 (2H, s), 6.9-7.0 (2H, m), 7.2-7.4 (3H, m), 7.5-7.6 (2H, m), 8.2-8.3 (2H, m).
IR (KBr): ν 3069, 2984, 5806, 1593 cm⁻¹.

### Example 47

### Production of 1-benzyl-2,5-dimethyl-4-(4-nitrophenyl)-1H-pyrrole-3-carbox. ylic acid

By using the compound (86 mg) produced in Example 4 and concentrated sulfuric acid (1.0 ml), the reaction and purification were carried out in the same manner as Example 13 to obtain the titled compound (202 mg) as yellow crystals.
¹H-NMR (CDCl₃) δ 2.04 (3H, s), 2.51 (3H, s), 5.13 (2H, s), 6.95 (2H, d, *J*= 8.0 Hz), 7.2-7.5 (5H, m), 8.19 (2H, d, *J*= 8.8 Hz),
IR (KBr): ν 2851, 2585, 1661, 1599 cm⁻¹.

### Example 48

### Production of ethyl 1-(cyclohexylmethyl)-2,5-dimethyl-4-(4-nitrophenyl)-1H-pyrr ole-3-carboxylate

By using the compound (170 mg) produced in Example 1 and cyclohexyl bromide (0.14 ml), the reaction and purification were carried out in the same manner as Example to obtain the titled compound (146 mg) as yellow crystals.
¹H-NMR (CDCl₃) δ 0.9-1.4 (5H, m), 1.02 (3H, t, *J*= 7.0 Hz), 1.6-1.9 (6H, m), 2.10 (3H, s), 2.54 (3H, s), 3.66 (2H, d, *J*= 7.0 Hz), 4.06 (2H, q, *J=* 7. 0 Hz), 7. 37 (2H, d, *J=* 8.8 Hz), 8.19 (2H, d, *J*= 8.8 Hz).
IR (KBr): ν 2978, 2928, 2851, 1696 cm⁻¹.

### Example 49

### Production of ethyl 2,5-dimethyl-1-(2-methylbenzyl)-4-(4-nitrophenyl)-1H-pyrrol e-3-carboxylate

By using the compound (100 mg) produced in Example 1 and 2-methylbenzyl bromide (0.067 ml), the reaction and purification were carried out in the same manner as Example 2 to obtain the titled compound (59 mg) as yellow crystals.
¹H-NMR (CDCl₃) δ 1.07 (3H, t, *J*= 7.0 Hz), 2.01 (3H, s), 2.40 (3H, s), 2.49 (3H, s), 4.11 (2H, q, *J*= 7.0 Hz), 5.03 (2H, s), 6.33 (1H, d, *J*= 6.6 Hz), 7.0-7.3 (3H, m), 7.42 (2H, d, *J*= 8.4 Hz), 8.21 (2H, d, *J*= 8.4 Hz).
IR (KBr): ν 2984, 2943, 1696, 1597 cm⁻¹.

### Example 50

### Production of ethyl 2,5-dimethyl-1-(3-methylbenzyl)-4-(4-nitrophenyl)-1H-pyrrol e-3-carboxylate

By using the compound (100 mg) produced in Example 1 and 3-methylbenzyl bromide (0.068 ml), the reaction and purification were carried out in the same manner as Example 2 to obtain the titled compound (65 mg) as yellow crystals.
¹H-NMR (CDCl₃) δ 1.06 (3H, t, *J=* 7.0 Hz), 2.04 (3H, s), 2.34 (3H, s), 2.51 (3H, s), 4.09 (2H, q, *J*= 7.0 Hz) , 5.08 (2H, s) , 6.72 (1H, d, *J=* 8.4 Hz), 6.80 (1H, s), 7.09 (1H, d, *J*= 7.6 Hz), 7.11 (1H, d, *J=* 7.8 Hz), 7.42 (2H, d, *J*= 8.8 Hz), 8.20 (2H, d, *J*= 8.8 Hz).
IR (KBr): ν 2982, 2905, 1696, 1597 cm⁻¹.

### Example 51

### Production of ethyl 2,5-dimethyl-1-(-4-methylbenzyl)-4-(4-nitrophenyl)-1H-pyrrol e-3-carboxylate

By using the compound (100 mg) produced in Example 1 and 4-methylbenzyl bromide (0.092 ml), the reaction and purification were carried out in the same manner as Example 2 to obtain the titled compound (67 mg) as yellow crystals.
¹H-NMR (CDCl₃) δ 1.05 (3H, t, *J*= 7.4 Hz), 2.04 (3H, s), 2.34 (3H, s), 2.51 (3H, s), 4.09 (2H, q, *J*= 7.4 Hz), 5.08 (2H, s), 6.72 (2H, d, *J*= 8.0 Hz), 7.15 (2H, d, *J*= 8.0 Hz), 7.41 (2H, d, *J*= 8.8 Hz), 8.20 (2H, d, *J*= 8.8 Hz).
IR (KBr): ν 2980, 2926, 2868, 1696 cm⁻¹.

### Example 52

### Production of ethyl 1-(2-fluorobenzyl)-2,5-dimethyl-4-(4-nitrophenyl)-1H-pyrrol e-3-carboxylate

By using the compound (100 mg) produced in Example 1 and 2-fluorobenzyl bromide (0.063 ml), the reaction and purification were carried out in the same manner as Example 2 to obtain the titled compound (47 mg) as yellow crystals.
¹H-NMR (CDCl₃) δ 1.06 (3H, t, *J*= 7.0 Hz), 2.05 (3H, s), 2.51 (3H, s), 4.10 (2H, q, *J*= 7. 0 Hz) , 5.16 (2H, s), 6.4-6.6 (1H, m), 7.0-7.2 (2H, m), 7.2-7.4 (1H, m), 7.3-7.5 (2H, m), 8.1-8.3 (2H, m).
IR (KBr): ν 3065, 2982, 2936, 1699 cm⁻¹.

### Example 53

### Production of ethyl 1-(4-methoxybenzyl)-2,5-dimethyl-4-(4-nitrophenyl)-1H-pyrro le-3-carboxylate

By using the compound (100 mg) produced in Example 1 and 4-methoxybenzyl chloride (0.068 ml), the reaction and purification were carried out in the same manner as Example 2 to obtain the titled compound (14 mg) as yellow crystals.
¹H-NMR (CDCl₃) δ 1.05 (3H, t, *J*= 7.0 Hz), 2.05 (3H, s), 2.52 (3H, s), 3.80 (3H, s), 4.09 (2H, q, *J*= 7.0 Hz), 5.06 (2H, s), 6.88 (4H, s), 7.41 (2H, d, *J*= 8.8 Hz), 8.20 (2H, d, *J*= 8.8 Hz).
IR (KBr): ν 2948, 1696, 1595, 1514 cm⁻¹.

### Example 54

### Production of ethyl 2,5-dimethyl-4-(4-nitrophenyl)-1-(4-(trifluoromethyl)benzyl )-1H-pyrrole-3-carboxylate

By using the compound (100 mg) produced in Example 1 and 4-(trifluoromethyl)benzyl bromide (0.12 g), the reaction and purification were carried out in the same manner as Example 2 to obtain the titled compound (80 mg) as yellow crystals.
¹H-NMR (CDCl₃) δ 1.06 (3H, t, *J*= 7.0 Hz), 2.03 (3H, s), 2.50 (3H, s), 4.10 (2H, q, *J*= 7.0 Hz), 5.18 (2H, s), 7.07 (2H, d, *J*= 8. 0 Hz), 7.41 (2H, d, *J*= 8.8 Hz), 7.62 (2H, d, *J*= 8. 0 Hz), 8.21 (2H, d, *J*= 8.8 Hz).
IR (KBr): ν 2984, 2944, 1696, 1597 cm⁻¹.

### Example 55

### Production of ethyl 1-(4-cyanobenzyl)-2,5-dimethyl-4-(4-nitrophenyl)-1H-pyrrole - 3-carboxylate

By using the compound (100 mg) produced in Example 1 and 4-cyanobenzyl bromide (0.10 g), the reaction and purification were carried out in the same manner as Example 2 to obtain the titled compound (94 mg) as yellow crystals.
¹H-NMR (CDCl₃) δ 1.06 (3H, t, *J*= 7.0 Hz), 2.02 (3H, s), 2.49 (3H, s), 4.10 (2H, q, *J*= 7.0 Hz), 5.18 (2H, s), 7.06 (2H, d, *J*= 8.8 Hz), 7.41 (2H, d, *J*= 8.8 Hz), 7.66 (2H, d, *J*= 8.8 Hz), 8.22 (2H, d, *J*= 8.8 Hz).
IR (KBr): ν 2984, 2493,2230, 1696 cm⁻¹.

### Example 56

### Production of ethyl 1-(4-chlorobenzyl)-2,5-dimethyl-4-(4-nitrophenyl)-1H-pyrrol e-3-carboxylate

By using the compound (100 mg) produced in Example 1 and 4-chlorobenzyl bromide (0.11 g), the reaction and purification were carried out in the same manner as Example 2 to obtain the titled compound (94 mg) as yellow crystals.
¹H-NMR (CDCl₃) δ 1.06 (3H, t, *J*= 7.0 Hz), 2.02 (3H, s), 2.50 (3H, s), 4.10 (2H, q, *J*= 7.0 Hz), 5.09 (2H, s), 6.89 (2H, d, *J*= 8.8 Hz), 7.32 (2H, d, *J=* 8.8 Hz), 7.41 (2H, d, *J=* 8.8 Hz), 8.21 (2H, d, *J=* 8.8 Hz).
IR (KBr): ν 2978, 2945, 1696, 1597 cm⁻¹.

### Example 57

### Production of ethyl 2,5-dimethyl-1-(4-(methylsulfonyl)benzyl)-4-(4-nitrophenyl} - 1H-pyrrole-3-carboxylat

By using the compound (100 mg) produced in Example 1 and 4-(methylsulfonyl)benzyl bromide (0.17 g), the reaction and purification were carried out in the same manner as Example 2 to obtain the titled compound (16 mg) as yellow crystals.
¹H-NMR (CDCl₃) δ 1.06 (3H, t, *J*= 7.0 Hz), 2.02 (3H, s), 2.50 (3H, s), 3.08 (3H, s), 4.10 (2H, q, *J*= 7.0 Hz), 5.22 (2H, s), 7.16 (2H, d, *J*= 8.8 Hz), 7.42 (2H, d, *J*= 8.8 Hz), 7.73 (2H, d, *J*= 8.8 Hz), 8.22 (2H, d, *J*= 8.8 Hz).
IR (KBr): ν 2990, 1696, 1597, 1516 cm⁻¹.

### Example 58

### Production of ethyl 1-(1,1'-biphenyl-4-ylmethyl)-2,5-dimethyl-4-(4-nitrophenyl) - lH-pyrrole-3-carboxylate

By using the compound (100 mg) produced in Example 1 and 4-(bromomethyl)-1,1'-biphenyl (0.17 g), the reaction and purification were carried out in the same manner as Example 2 to obtain the titled compound (79 mg) as yellow crystals.
¹H-NMR (CDCl₃) δ 1.06 (3H, t, *J*= 7.0 Hz), 2.08 (3H, s), 2.55 (3H, s), 4.10 (2H, q, *J*= 7.0 Hz), 5.17 (2H, s) , 7.03 (2H, d, *J*= 8.4 Hz), 7.3-7.5 (5H, m), 7.5-7.7 (4H, m), 8.21 (2H, d, *J*= 8.4 Hz).
IR (KBr): ν 2984, 2943, 1696, 1597 cm⁻¹.

### Example 59

### Production of ethyl 3,5-dimethyl-4-(4-nitrophenyl)-1H-pyrrole-2-carboXylate

By using the compound (0.98 g) produced in Reference Example 3 and 4-nitrophenylboronic acid (0.67 g), the reaction and purification were carried out in the same manner as Example 14 to obtain the titled compound (506 mg) as yellow crystals.
¹H-NMR (CDCl₃) δ 1.39 (3H, t, *J*= 7.0 Hz), 2.31 (3H, s), 2.32 (3H, s), 4.35 (2H, q, *J*= 7.0 Hz), 7.40 (2H, d, *J*= 8.8 Hz), 8.27 (2H, d, *J*= 8.8 Hz), 8.96 (1H, s).
IR (KBr): ν 3285, 2992, 1651, 1595 cm⁻¹.

### Example 60

### Production of ethyl 1-benzyl-3,5-dimethyl-4-(4-nitrophenyl)-1H-pyrrole-2-carbox ylate

By using the compound (141 mg) produced in Example 59 and benzyl bromide (0.10 ml), the reaction and purification were carried out in the same manner as Example 2 to obtain the titled compound (103 mg) as yellow crystals.
¹H-NMR (CDCl₃) δ 1.29 (3H, t, *J*= 7.0 Hz), 2.15 (3H, s), 2.31 (3H, s), 4.26 (2H, q, *J*= 7.0 Hz), 5.65 (2H, s), 6.9-7.1 (2H, m), 7.2-7.5 (5H, m), 8.2-8.4 (2H, m).
IR (KBr): ν 3065, 2984, 2932, 1690 cm⁻¹.

### Example 61

### Production of methyl 1-benzyl-4-(3,4-dichlorophenyl)-2,5-dimethyl-1H-pyrrole-3-c arboxylate

By using the compound (208 mg) produced in Example 38 and benzyl bromide (0.13 ml), the reaction and purification were carried out in the same manner as Example 2 to obtain the titled compound (156 mg) as colorless crystals.
¹H-NMR (CDCl₃) δ 2.02 (3H, s), 2.49 (3H, s), 3.62 (3H, s), 5.11 (2H, s), 6.8-7.0 (2H, m), 7.0-7.2 (1H, m), 7.2-7.5 (5H, m).
IR (KBr): ν 3031, 2986, 2911, 1699 cm⁻¹.

### Example 62

### Production of methyl 4-(3,4-dichlorophenyl)-1-((2,4-difluorophenyl)sulfonyl)-2,5 - dimethyl-1H-pyrrole-3-carboxylate

By using the compound (100 mg) produced in Example 38 and 2,4-difluorobenzenesulfonyl chloride (72 mg), the reaction and purification were carried out in the same manner as Example 2 to obtain the titled compound (15 mg) as colorless crystals.
¹H-NMR (CDCl₃) δ 2.22 (3H, s), 2.66 (3H, s), 3.57 (3H, s), 6.9-7.1 (4H, m), 7.2-7.5 (1H, m), 8.0-8.2 (1H, m).
IR (KBr): ν 2947, 1713, 1603, 1435 cm⁻¹.

### Example 63

### Production of methyl 4-(3,4-dichlorophenyl)-2,5-dimethyl-1-(methylsulfonyl)-1H-p yrrole-3-carboxylate

By using the compound (130 mg) produced in Example 38 and methanesulfonyl chloride (0.041 ml), the reaction and purification were carried out in the same manner as Example 2 to obtain the titled compound (114 mg) as colorless crystals.
¹H-NMR (CDCl₃) δ 2.27 (3H, s), 2.77 (3H, s), 3.23 (3H, s), 3.60 (3H, s), 7.01 (1H, dd, *J*= 1.8, 8.4 Hz), 7.27 (1H, d, *J*= 1.8 Hz), 7.44 (1H, d, *J*= 8.4 Hz),
IR (KBr): ν 3011, 2934, 1713, 1549 cm⁻¹.

### Example 64

### Production of ethyl 1-((3,5-dimethyl-4-isoxazolyl)methyl)-2,5-dimethyl-4-(4-nit rophenyl)-1H-pyrrole-3-carboxylate

By using the compound (100 mg) produced in Example 1 and 4-(bromomethyl)-3,5-dimethylisoxazole (95 mg), the reaction and purification were carried out in the same manner as Example 2 to obtain the titled compound (101 mg) as yellow crystals.
¹H-NMR (CDCl₃) δ 1.03 (3H, t, *J*= 7.2 Hz), 2.01 (3H, s), 2.04 (3H, s), 2.12 (3H, s), 2.53 (3H, s), 4.07 (2H, q, *J*= 7.2 Hz), 4.85 (2H, s), 7.36 (2H, d, *J*= 8.4 Hz), 8.21 (2H, d, *J*= 8.4 Hz).
IR (KBr): ν 2984, 2932, 1696, 1597 cm⁻¹.

### Example 65

### Production of ethyl 1-(4-fluorobenzyl)-2,5-dimethyl-4-(4-nitrophenyl)-1H-pyrrol e-3-carboxylate

By using the compound (120 mg) produced in Example 1 and 4-fluorobenzyl bromide (0.063 ml), the reaction and purification were carried out in the same manner as Example 2 to obtain the titled compound (132 mg) as yellow crystals.
¹H-NMR (CDCl₃) δ 1.06 (3H, t, *J*= 7.2 Hz), 2.04 (3H, s), 2.51 (3H, s), 4.09 (2H, q, *J*= 7.2 Hz), 5.10 (2H, s), 6.8-7.0 (2H, m), 7.0-7.2 (2H, m), 7.41 (2H, d, *J*= 8.7 Hz), 8.21 (2H, d, *J*= 8.7 Hz).
IR (KBr): ν 2980, 2942, 1696, 1512 cm⁻¹.

### Example 66

### Production of ethyl 2,5-dimethyl-4-(4-nitrophenyl)-1-(4-pyridinylmethyl)-1H-pyr role-3-carboxylate

To a solution of 4-(chloromethyl)pyridine hydrochloride (0.35 g) in N,N-dimethylformamide (3 ml) was added sodium hydride (60% oil suspension, 20 mg) at room temperature. The reaction mixture was stirred at room temperature for 1 hour. To the reaction mixture was added the compound (230 mg) produced in Example 1 and the reaction mixture was further stirred at room temperature for 14 hours.

The reaction mixture was poured into saturated brine and extracted with ethyl acetate. The ethyl acetate layer was dried over magnesium sulfate and concentrated. The residue was purified by column chromatography (carrier: silicagel, eluant: hexane-ethyl acetate), and recrystallized from ethyl acetate-hexane to obtain the titled compound (80 mg) as yellow crystals.
¹H-NMR (CDCl₃) δ 1.07 (3H, t, *J*= 6.8 Hz), 2.04 (3H, s), 2.51 (3H, s), 4.09 (2H, q, *J*= 6.8 Hz), 5.10 (2H, s), 6.8-7.0 (2H, m), 7.0-7.2 (2H, m), 7.4-7.5 (2H, m), 8.1-8.3 (2H, m).
IR (KBr): ν 2980, 2909, 1696, 1599 cm⁻¹.

### Example 67

### Production of ethyl 1-(4-(methoxycarbonyl)benzyl)-2,5-dimethyl-4-(4-nitrophenyl )-1H-pyrrole-3-carboxylate

By using the compound (100 mg) produced in Example 1 and 4-(methoxycarbonyl)benzyl bromide (0.12 g), the reaction and purification were carried out in the same manner as Example 2 to obtain the titled compound (26 mg) as yellow crystals.
¹H-NMR (CDCl₃) δ 1.06 (3H, t, *J*= 7.0 Hz), 2.04 (3H, s), 2.50 (3H, s), 3.92 (3H, s), 4.10 (2H, q, *J*= 7.0 Hz), 5.18 (2H, s), 7.03 (2H, d, *J*= 8.4 Hz), 7.42 (2H, d, *J*= 8.8 Hz), 8.02 (2H, d, *J*= 8.4 Hz), 8.22 (2H, d, *J*= 8.8 Hz).
IR (KBr): ν 2984, 2951, 1723, 1699 cm⁻¹.

### Example 68

### Production of ethyl 2,5-dimethyl-1-(1-naphthyl methyl)-4-(4-nitrophenyl)-1H-pyrrole-3-carboxylate

By using the compound (100 mg) produced in Example 1 and 1-(chloromethyl)naphthalene (0.10 ml), the reaction and purification were carried out in the same manner as Example 2 to obtain the titled compound (70 mg) as yellow crystals.
¹H-NMR (CDCl₃) δ 1.08 (3H, t, *J*= 6.8 Hz), 2.04 (3H, s), 2.49 (3H, s), 4.13 (2H, q, *J*= 6.8 Hz), 5.57 (2H, s), 6.48 (1H, d, *J*= 4.8 Hz), 7.38 (1H, t, *J*= 5.0 Hz), 7.4-7.7 (4H, m), 7.81 (1H, d, *J*= 5.4 Hz), 7.95 (1H, d, *J=* 5.2 Hz), 8. 03 (1H, d, *J=* 5.4 Hz), 8.2-8.3 (2H, m).
IR (KBr): ν 3061, 2980, 2942, 1696 cm⁻¹.

### Example 69

### Production of ethyl 1-(2,4-difluorobenzyl)-2,5-dimethyl-4-(4-nitrophenyl)-1H-py rrole-3-carboxylate

By using the compound (100 mg) produced in Example 1 and 2,4-difluorobenzyl bromide (0.064 ml), the reaction and purification were carried out in the same manner as Example 2 to obtain the titled compound (109 mg) as yellow crystals.
¹H-NMR (CDCl₃) δ 1.06 (3H, t, *J*= 7.0 Hz), 2.04 (3H, s), 2.50 (3H, s), 4. 10 (2H, q, *J*= 7.0 Hz), 5.11 (2H, s), 6.4-6.6 (1H, m), 6.8-7.0 (2H, m), 7.4-7.5 (2H, m), 8.2-8.3 (2H, m).
IR (KBr): ν 3077, 2982, 2942, 1696 cm⁻¹.

### Example 70

### Production of ethyl 1-((5-(ethoxycarbonyl)-2-furyl)methyl)-2,5-dimethyl-4-(4-ni trophenyl)-1H-pyrrole-3-carboxylate

By using the compound (100 mg) produced in Example 1 and ethyl 5-(chloromethyl)furan-2-carboxylate(0.080 ml), the reaction and purification were carried out in the same manner as Example 2 to obtain the titled compound (45 mg) as yellow crystals.
¹H-NMR (CDCl₃) δ 1.04 (3H, t, *J*= 6.8 Hz), 1.38 (3H, t, *J*= 6.8 Hz), 2.15 (3H, s), 2.60 (3H, s), 4.08 (2H, q, *J*= 6.8 Hz), 4.36 (2H, q, *J*= 6.8 Hz), 5.10 (2H, s), 6.11 (1H, d, *J*= 3.6 Hz), 7.10 (1H, d, *J*= 3.6 Hz), 7.38 (2H, d, *J*= 8.4 Hz), 8.21 (2H, d, *J*= 8.4 Hz).
IR (KBr): ν 3123, 2982, 2938, 1726, 1694 cm⁻¹.

### Example 71

### Production of methyl 4-(3,4-dichlorophenyl)-1-(2,4-difluorobenzyl)-2,5-dimethyl-1H-pyrrole-3-carboxylate

By using the compound (1.0 g) produced in Example 38 and 2,4-difluorobenzyl bromide (0.51 ml), the reaction and purification were carried out in the same manner as Example 2 to obtain the titled compound (1.04 g) as colorless crystals.
¹H-NMR (CDCl₃) δ 2.02 (3H, s), 2.48 (3H, s), 3.63 (3H, s), 5.09 (2H, s), 6.46 (1H, q, *J*= 8.4 Hz), 6.8-7.0 (2H, m), 7.09 (1H, dd, *J*= 1.8, 8.2 Hz), 7.34 (1H, d, *J*= 1.8 Hz), 7.41 (1H, d, *J*= 8.2 Hz).
IR (KBr): ν 3074, 2948, 2922, 1699 cm⁻¹.

### Example 72

### Production of methyl 4-(3,4-dichlorophenyl)-2,5-dimethyl-1-(1-phenylethyl)-1H-py rrole-3-carboxylate

By using the compound (140 mg) produced in Example 38 and 1-phenylethyl bromide (0.077ml), the reaction and purification were carried out in the same manner as Example 2 to obtain the titled compound (49 mg) as colorless crystals.
¹H-NMR (CDCl₃) δ 1.91 (3H, d, *J=* 7.6 Hz), 1.91 (3H, s), 2.43 (3H, s), 3.59 (3H, s), 5.64 (1H, q, *J*= 7.6 Hz), 7.0-7.2 (3H, m), 7.2-7.4 (5H, m).
IR (KBr): ν 3438, 2986, 2946, 1699 cm⁻¹.

### Example 73

### Production of methyl 4-(3,4-dichlorophenyl)-2,5-dimethyl-1-(phenylsulfonyl)-1H-p yrrole-3-carboxylate

By using the compound (120 mg) produced in Example 38 and benzenesulfonyl chloride (0.056 ml), the reaction and purification were carried out in the same manner as Example 2 to obtain the titled compound (130 mg) as colorless crystals.
¹H-NMR (CDCl₃) δ 2.27 (3H, s), 2.74 (3H, s), 3.57 (3H, s), 6.98 (1H, dd, *J*= 1.8, 8.0 Hz), 7.23 (1H, d, *J*= 1.8 Hz) , 7.41 (1H, d, *J*= 8.0 Hz), 7.4-7.8 (5H, m).
IR (KBr): ν 3069, 2949, 1713, 1547 cm⁻¹.

### Example 74

### Production of ethyl 1-((5-chloro-2-thienyl)methyl)-2,5-dimethyl-4-(4-nitropheny 1)-1H-pyrrole-3-carboxylate

By using the compound (100 mg) produced in Example 1 and 2-chloro-5- (chloromethyl) thiophene (0. 08 g) , the reaction and purification were carried out in the same manner as Example 2 to obtain the titled compound (19 mg) as yellow crystals.
¹H-NMR (CDCl₃) δ 1.04 (3H, t, *J*= 7.0 Hz), 2.12 (3H, s), 2.59 (3H, s), 4.08 (2H, q, *J*= 7.0 Hz), 5.13 (2H, s), 6.56 (1H, d, *J*= 3.6 Hz), 6.77 (1H, d, *J*= 3.6 Hz), 7.39 (2H, d, *J*= 8.8 Hz), 8.20 (2H, d, *J*= 8.8 Hz).
IR (KBr): ν 2982, 2941, 1696, 1597 cm⁻¹.

### Example 75

### Production of methyl 1-benzyl-4-(4-cyano-3-(trifluoromethyl)phenyl)-2,5-dimethyl - 1H-pyrrole-3-carboxylate

By using the compound (150 mg) produced in Reference Example 5 and the compound (130 mg) produced in Reference Example 8, the reaction and purification were carried out in the same manner as Example 14 to obtain the titled compound (138 mg) as colorless crystals.
¹H-NMR (CDCl₃ δ 2.05 (3H, s), 2.51 (3H, s), 3.62 (3H, s), 5.14 (2H, s), 6.9-7.0 (2H, m), 7.2-7.4 (3H, m), 7.5-7.6 (1H, m), 7.68 (1H, s), 7.80 (1H, d, *J*= 8.0 Hz).
IR (KBr): ν 2980, 2922, 2224, 1699 cm⁻¹.

### Example 76

### Production of methyl 1-benzyl-4-(3-chloro-4-cyanophenyl)-2,5-dimethyl-1H-pyrrole - 3-carboxylate

By using the compound (150 mg) produced in Reference Example 5 and 4-bromo-2-chlorobenzonitrile (110 mg), the reaction and purification were carried out in the same manner as Example 14 to obtain the titled compound (111 mg) as colorless crystals.
¹H-NMR (CDCl₃) δ 2.04 (3H, s), 2.49 (3H, s), 3.63 (3H, s), 5.12 (2H, s), 6.8-7.0 (2H, m), 7.2-7.5 (5H, m), 7.6-7.7 (1H, m).
IR (KBr): ν 2948, 2922, 2228, 1701 cm⁻¹.

### Example 77

### Production of methyl 1-benzyl-4-(3,4-dicyanophenyl)-2,5-dimethyl-1H-pyrrole-3-ca rboxylate

By using the compound (81 mg) produced in Reference Example 5 and the compound (60 mg) produced in Reference Example 9, the reaction and purification were carried out in the same manner as Example 14 to obtain the titled compound (37 mg) as colorless crystals.
¹H-NMR (CDCl₃) δ 2.04 (3H, s), 2.51 (3H, s), 3.64 (3H, s), 5.13 (2H, s), 6.8-7.0 (2H, m), 7.2-7.4 (3H, m), 7.6-7.8 8 (3H, m).
IR (KBr): ν 2947, 2232, 1699, 1599 cm⁻¹.

### Example 78

### Production of methyl 4-(4-cyanophenyl)-1-(4-fluorobenzyl)-2,5-dimethyl-1H-pyrrol e-3-carboxylate

By using the compound (1.5 g) produced in Reference Example 7 and 4-cyanophenylboronic acid (0.65 g), the reaction and purification were carried out in the same manner as Example 14 to obtain the titled compound (1.0 g) as colorless crystals.
¹H-NMR (CDCl₃) δ 2.02 (3H, s), 2.49 (3H, s), 3.61 (3H, s), 5.09 (2H, s), 6.8-7.0 (2H, m), 7.0-7.2 (2H, m), 7.35 (2H, d, *J*= 8.2 Hz), 7.64 (2H, d, *J*= 8.2 Hz).
IR (KBr): ν 3048, 2948, 2922, 2226, 1699 cm⁻¹.

### Example 79

### Production of methyl 1-(4-cyanobenzyl)-4-(3-cyanophenyl)-2,5-dimethyl-1H-pyrrole -3-carboxylate

By using the compound (0.70 g) produced in Reference Example 17 and 3-cyanophenylboronic acid (0.30 g), the reaction and purification were carried out in the same manner as Example 14 to obtain the titled compound (0.30 g) as colorless crystals.
¹H-NMR (CDCl₃) δ 1.99 (3H, s), 2.47 (3H, s), 3.61 (3H, s), 5.18 (2H, s), 7.05 (2H, d, *J=* 8.4 Hz), 7.4-7.6 6 (4H, m), 7.66 (2H, d, *J=* 8.4 Hz).
IR (KBr): ν 3067, 2947, 2253, 2228, 1699 cm⁻¹.

### Example 80

### Production of methyl 1-(4-cyanobenzyl)-4-(4-cyanophenyl)-2,5-dimethyl-1H-pyrrole - 3-carboxylate

By using the compound (0.70 g) produced in Reference Example 17 and 4-cyanophenylboronic acid (0.30 g), the reaction and purification were carried out in the same manner as Example 14 to obtain the titled compound (0.43 g) as colorless crystals.
¹H-NMR (CDCl₃) δ 2.00 (3H, s), 2.47 (3H, s), 3.61 (3H, s), 5.17 (2H, s), 7.05 (2H, d, *J*=8.4 Hz), 7.35 (2H, d, *J*=8.4 Hz), 7.64 (2H, d, *J*=8.4 Hz), 7.65 (2H, d, *J*=8.4 Hz).
IR (KBr): ν 2948, 2922, 2253, 2226, 1699 cm⁻¹.

### Example 81

### Production of methyl 4-(4-cyano-3-(trifluoromethyl)phenyl)-1-(4-fluorobenzyl)-2, 5-dimethyl-1H-pyrrole-3-carboxylate

By using the compound (479 mg) produced in Reference Example 7 and the compound (392 mg) produced in Reference Example 8, the reaction and purification were carried out in the same manner as Example 14 to obtain the titled compound (496 mg) as colorless crystals.
¹H-NMR (CDCl₃) δ 2.04 (3H, s), 2.51 (3H, s), 3.61 (3H, s), 5.10 (2H, s), 6.8-7.0 (2H, m), 7.0-7.2 (2H, m), 7.54 (1H, d, *J*=8.0 Hz), 7.67 (1H, s), 7.80 (1H, d, *J*=8.0 Hz).

### Example 82

### Production of methyl 4-(4-cyanophenyl)-1-((5-cyano-2-pyridinyl)methyl)-2,5-dimet hyl-1H-pyrrole-3-carboxylate

By using the compound (180 mg) produced in Example 17 and 6-(bromomethyl)nicotinonitrile (0.23 g, produced by a method described in *J. Org. Chem*., 65, 7718-7722 (2000)), the reaction and purification were carried out in the same manner as Example 2 to obtain the titled compound (46 mg) as colorless crystals.
¹H-NMR (CDCl₃) δ 2.03 (3H, s), 2.49 (3H, s), 3.61 (3H, s), 5.28 (2H, s), 6.79 (1H, d, *J*=8.0 Hz), 7.35 (2H, d, *J*=8.4 Hz), 7.65 (2H, d, *J*=8.4 Hz), 7.95 (1H, dd, *J*=1.8, 8.0 Hz), 8.89 (1H, d, *J*=1.8 Hz)).
IR (KBr): ν 3059, 2986, 2948, 2226, 1699 cm⁻¹.

### Example 83

### Production of methyl 4-(4-cyanophenyl)-1-((4-cyano-1,3-thiazol-2-yl)methyl)-2,5-dimethyl-1H-pyrrole-3-carboxylate

By using the compound (86 mg) produced in Example 17 and 2-(bromomethyl)-1,3-thiazole-4-carbonitrile (0.13 g, produced by a method described in *J. Org. Chem*., **65,** 7718-7722 (2000)), the reaction and purification were carried out in the same manner as Example 2 to obtain the titled compound (46 mg) as colorless crystals.
¹H-NMR (CDCl₃) δ 2.12 (3H, s), 2.58 (3H, s), 3.61 (3H, s), 5.38 (2H, s), 7.33 (2H, d, *J*=8.0 Hz), 7.65 (2H, d, *J*=8.0 Hz), 7.99 (1H, s).
IR (KBr): ν 3107, 2948, 2226, 1696 cm⁻¹.

### Example 84

### Production of methyl 4-(4-cyanophenyl)-2,5-dimethyl-1-(3-pyridinylmethyl)-1H-pyr role-3-carboxylate

By using the compound (0.18 g) produced in Example 17 and 3- (chloromethyl)pyridine hydrochloride (0.14 g) , the reaction and purification were carried out in the same manner as Example 66 to obtain the titled compound (79 mg) as colorless crystals.
¹H-NMR (CDCl₃) δ 2.04 (3H, s), 2.51 (3H, s), 3.61 (3H, s), 5.15 (2H, s), 7.2-7.4 (3H, m), 7.35 (1H, d, *J*=8.4 Hz), 7.64 (2H, d, *J*=8.4 Hz), 8.37 (1H, s), 8.56 (1H, d, *J*=3.2 Hz).
IR (KBr): ν 2988, 2948, 2224, 1699 cm⁻¹.

### Example 85

### Production of methyl 4-(4-cyanophenyl)-2,5-dimethyl-1-(2-pyridinylmethyl)-1H-pyr role-3-carboxylate

By using the compound (0.18 g) produced in Example 17 and 2- (chloromethyl)pyridine hydrochloride (0.14 g) , the reaction and purification were carried out in the same manner as Example 66 to obtain the titled compound (69 mg) as colorless crystals.
¹H-NMR (CDCl₃) δ 2.05 (3H, s), 2.51 (3H, s), 3.61 (3H, s), 5.22 (2H, s), 6.63 (1H, d, *J*=8.4 Hz), 7.1-7.3 (1H, m), 7.36 (2H, d, *J*=8.0 Hz), 7.64 (2H, d, *J*=8.0 Hz), 7.6-7.8 (1H, m), 8.61 (1H, d, *J*=4.8 Hz).
IR (KBr): ν 2986, 2922, 2224, 1699 cm⁻¹.

### Example 86

### Production of 4-((3-(4-cyanophenyl)-4-(methoxycarbonyl)-2,S-dimethyl-1H-p yrrol-1-yl)methyl)benzoic acid

By using the compound (0.18 g) produced in Example 17 and 4-bromomethylbenzoic acid (0.18 g), the reaction and purification were carried out in the same manner as Example 66 to obtain the titled compound (78 mg) as colorless crystals.
¹H-NMR (DMSO-d₆) δ 2.02 (3H, s), 2.49 (3H, s), 3.62 (3H, s), 5.19 (2H, s), 7.05 (2H, d, *J*=8.4 Hz), 7.36 (2H, d, *J*=8.4 Hz), 7.64 (2H, d, *J*=8.4 Hz), 8.09 (2H, d, *J*=8.4 Hz).
IR (KBr): ν 2498, 2922, 2226, 1699 cm⁻¹.

### Example 87

### Production of methyl 4-(4-cyanophenyl)-1-(4-methoxybenzyl)-2,5-dimethyl-1H-pyrro le-3-carboxylate

By using the compound (420 mg) produced in Example 17 and 4-methoxybenzyl chloride (0.22 ml), the reaction and purification were carried out in the same manner as Example 2 to obtain the titled compound (382 mg) as colorless crystals.
¹H-NMR (CDCl₃) δ 2.03 (3H, s), 2.50 (3H, s), 3.61 (3H, s), 3.80 (3H, s), 5.06 (2H, s), 6.87 (4H, s), 7.35 (2H, d, *J*=8.6 Hz), 7.64 (2H, d, *J*=8.6 Hz).
IR (KBr): ν 3060, 2948, 2837, 2224, 1699 cm⁻¹.

### Example 88

### Production of methyl 4-(4-cyanophenyl)-1-(4-hydroxybenzyl)-2,5-dimethyl-1H-pyrro le-3-carboxylate and 4-(4-cyanophenyl)-1-(4-hydroxybenzyl)-2,5-dimethyl-1H-pyrro le-3-carboxylic acid

The solution of the compound (370 mg) produced in Example 87 in chloride methylene (2 ml) was cooled on an ice bath. To the solution was added dropwise a solution of 1 N boron tribromide in dichloromethane (4 ml). The reaction mixture was stirred at the same temperature for 1 hour, poured into saturated brine and extracted with ethyl acetate. The ethyl acetate layer was dried over magnesium sulfate and concentrated. The residue was separated by column chromatography (carrier: silicagel, eluant: hexane-ethyl acetate) and recrystallized from ethyl acetate-hexane to obtain methyl 4-(4-cyanophenyl)-1-(4-hydroxybenzyl)-2,5-dimethyl-1H-pyrro le-3-carboxylate (32 mg) as colorless crystals and 4-(4-cyanophenyl)-1-(4-hydroxybenzyl)-2,5-dimethyl-1H-pyrro le-3-carboxylic acid (141 mg) and as colorless crystals. Methyl 4-(4-cyanophenyl)-1-(4-hydroxybenzyl)-2,5-dimethyl-1H-pyrro le-3-carboxylate
¹H-NMR (CDCl₃) δ 2.03 (3H, s), 2.50 (3H, s), 3.61 (3H, s), 4.98 (1H, s), 5.04 (2H, s), 6.81 (4H, s), 7.35 (2H, d, *J*=8.4 Hz), 7.63 (2H, d, *J*=8.4 Hz).
IR (KBr): ν 2982, 2949, 2228, 1930, 1714 cm⁻¹.
4-(4-cyanophenyl)-1-(4-hydroxybenzyl)-2,5-dimethyl-1H-pyrro le-3-carboxylic acid
¹H-NMR (DMSO-*d*₆) δ 2.04 (3H, s), 2.43 (3H, s), 5.07 (2H, s), 6.73 (2H, d, *J*=8.6 Hz), 6.83 (2H, d, *J*=8.6 Hz), 7.37 (2H, d, *J*=8.4 Hz), 7.75 (2H, d, *J*=8.4 Hz), 9.42 (1H, s).
IR (KBr): ν 3387, 2232, 1661, 1609 cm⁻¹.

### Example 89

### Production of methyl 1-(2-bromo-4-cyanobenzyl)-4-(4-cyanophenyl)-2,5-dimethyl-1H - pyrrole-3-carboxylate

By using the compound (1.34 g) produced in Example 17 and 2-bromo-4-cyanobenzyl bromide (1.45 g, produced by a manner described in *J*. *Org*. *Chem*., 65, 7718-7722 (2000)), the reaction and purification were carried out in the same manner as Example 2 to obtain the titled compound (990 mg) as colorless crystals.
¹H-NMR (CDCl₃) δ 1.99 (3H, s), 2.44 (3H, s), 3.63 (3H, s), 5.13 (2H, s), 6.44 (1H, d, *J*=8.0 Hz) , 7.36 (2H, d, *J*=8.6 Hz), 7.5-7.6 (1H, m), 7.66 (2H, d, *J*=8.6 Hz), 7.93 (1H, d, *J*=1.4 Hz).
IR (KBr): ν 2948, 2922, 2226, 1699 cm⁻¹.

### Example 90

### Production of methyl 4-(4-cyanophenyl)-1-(4-cyano-2-(1-pyrrolidinyl)benzyl)-2,5-dimethyl-1H-pyrrole-3-carboxylate

A mixture of the compound (230 mg) produced in Example 89, pyrrolidine (0.21 ml), sodium tert-butoxide (100 mg), 2,2'-bis(diphenylphosphino)-1,1'-binaphthyl (15 mg) and tris(dibenzylideneacetone)dipalladium (20 mg) in tetrahydrofuran (2 ml) was stirred for 1 hour under reflux. The reaction mixture was poured into saturated brine and extracted with ethyl acetate. The ethyl acetate layer was dried over magnesium sulfate and concentrated. The residue was purified by column chromatography (carrier: silicagel, eluant: hexane-ethyl acetate) and recrystallized from ethyl acetate-hexane to obtain the titled compound (107 mg) as colorless crystals.
¹H-NMR (CDCl₃) δ 1.95 (3H, s), 2.0-2.2 (4H, m), 2.42 (3H, s), 3.2-3.4 (4H, m), 3.61 (3H, s), 5.05 (2H, s) , 6.35 (1H, d, *J*=8.0 Hz), 7.10 (1H, dd, *J*=1.4, 8.0 Hz), 7.22 (1H, d, *J*=1.4 Hz), 7.35 (2H, d, *J*=8.0 Hz), 7.64 (2H, d, *J*=8.0 Hz).
IR (KBr): ν 2949, 2880, 2226, 1699 cm⁻¹.

### Example 91

### Production of methyl 1-(4-cyano-2-(4-morpholinyl)benzyl)-4-(4-cyanophenyl)-2,5-d imethyl-1H-pyrrole-3-carboxylate

By using the compound (230 mg) produced in Example 89 and morpholine (0.14 ml), the reaction and purification were carried out in the same manner as Example 90 to obtain the titled compound (46 mg) as colorless crystals.
¹H-NMR (CDCl₃) δ 1.95 (3H, s), 2.0-2.1 (4H, m), 2.42 (3H, s), 3.2-3.4 (4H, m), 5.05 (2H, s), 6.35 (1H, d, *J*=8.0 Hz), 7.11 (1H, d, *J*=8.8 Hz), 7.2-7.3 (1H, m), 7.35 (2H, d, *J*=8.0 Hz), 7.64 (2H, d, *J*=8.0 Hz).
IR (KBr): ν 2953, 2855, 2226, 1699 cm⁻¹.

### Example 92

### Production of methyl 1-(4-(aminocarbonyl)benzyl)-4-(4-cyanophenyl)-2,5-dimethyl-1H-pyrrole-3-carboxylate

To a solution of the compound (150 mg) produced in Example 86 in tetrahydrofuran (1 ml) was added dropwise thionyl chloride (0.08 ml) . The reaction mixture was stirred at room temperature for 1 hour, and the solvent was distilled off under reduced pressure. The residue was dissolved in tetrahydrofuran(1 ml) and added dropwise to 25% aqueous ammonia (2 ml) . The reaction mixture was stirred at room temperature for 2 hours, poured into saturated brine and extracted with ethyl acetate. The ethyl acetate layer was dried over magnesium sulfate and concentrated. The residue was purified by column chromatography (carrier: silicagel, eluant: hexane-ethyl acetate) and recrystallized from ethyl acetate-hexane to obtain the titled compound (71 mg) as colorless crystals.
¹H-NMR (CDCl₃) δ 2.02 (3H, s), 2.49 (3H, s), 3.62 (3H, s), 5.17 (2H, brs), 5.75 (1H, brs), 6.01 (1H, brs), 7.03 (2H, d, *J*=8.4 Hz), 7.36 (2H, d, *J*=8.8 Hz), 7.64 (2H, d, *J*=8.4 Hz), 7.80 (2H, d, *J*=8.8 Hz).
IR (KBr): ν 3355, 3183, 2949, 2226, 1682 cm⁻¹.

### Example 93

### Production of methyl 4-(4-cyanophenyl)-2,5-dimethyl-1-(4-((methylamino)carbonyl) benzyl)-1H-pyrrole-3-carboxylate

By using the compound (150 mg) produced in Example 86 and an aqueous solution of methylamine (2 ml), the reaction and purification were carried out in the same manner as Example 92 to obtain the titled compound (46 mg) as colorless crystals. ¹H-NMR (CDCl₃) δ 2.01 (3H, s), 2.48 (3H, s), 3.02 (3H, d, *J*=5.2 Hz), 3.61 (3H, s), 5.16 (2H, s), 6.13 (1H, s), 7.00 (2H, d, *J*=8.4 Hz), 7.29 (2H, d, *J*=8.4 Hz), 7.64 (2H, d, *J*=8.4 Hz), 7.74 (2H, d, *J*=8.4 Hz).
IR (KBr): ν 3329, 2948, 2226, 1699, 1653 cm⁻¹.

### Example 94

### Production of methyl 4-(4-cyanophenyl)-1-(4-((dimethylamino)carbonyl)benzyl)-2,5 - dimethyl-1H-pyrrole-3-carboxylate

A solution of the compound (120 mg) produce in Example 86, 2N dimethylamine tetrahydrofuran solution (0.5 ml), 1-hydroxybenzotriazole monohydrate (52 mg) and 1-ethyl-3-(3-dimethylaminopropyl)carbodiimide hydrochloride (65 mg) in N,N-dimethylformamide (6 ml) was stirred at room temperature for 6 hours. The reaction mixture was poured into saturated brine and extracted with ethyl acetate. The ethyl acetate layer was dried over magnesium sulfate and concentrated. The residue was purified by column chromatography (carrier: silicagel, eluant: hexane-ethyl acetate) and recrystallized from ethyl acetate-hexane to obtain the titled compound (58 mg) as colorless crystals.
¹H-NMR (CDCl₃) δ 2.02 (3H, s), 2.49 (3H, s), 2.98 (3H, s), 3.11 (3H, s), 3.61 (3H, s), 5.14 (2H, s), 6.97 (2H, d, *J*=8.4 Hz), 7.36 (2H, d, *J*=8.4 Hz), 7.41 (2H, d, *J*=8.4 Hz), 7.64 (2H, d, *J*=8.4 Hz).
IR (KBr): ν 2948, 2224, 1699, 1634 cm⁻¹.

### Example 95

### Production of methyl 4-(4-cyanophenyl)-2,5-dimethyl-1-(methylsulfonyl)-1H-pyrrol e-3-carboxylate

By using the compound (0.20 g) produced in Example 17 and methanesulfonyl chloride (0.074 ml), the reaction and purification were carried out in the same manner as Example 66 to obtain the titled compound (191 mg) as colorless crystals.
¹H-NMR (CDCl₃) δ 2.27 (3H, s), 2.79 (3H, s), 3.25 (3H, s), 3.57 (3H, s), 7.28 (2H, d, *J*=8.0 Hz), 7.67 (2H, d, *J*=8.0 Hz).
IR (KBr): ν 3009, 2934, 2228, 1929, 1713 cm⁻¹.

### Example 96

### Production of methyl 4-(4-cyanophenyl)-1-(ethylsulfonyl)-2,5-dimethyl-1H-pyrrole -3-carboxylate

By using the compound (0.20 g) produced in Example 17 and ethanesulfonyl chloride (0.095 ml), the reaction and purification were carried out in the same manner as Example 66 to obtain the titled compound (99 mg) as colorless crystals.
¹H-NMR (CDCl₃) δ 1.40 (3H, t, *J*=7.2 Hz), 2.26 (3H, s), 2.78 (3H, s), 3.35 (2H, q, *J*=7.2 Hz), 3.57 (3H, s), 7.28 (2H, d, *J*=7.8 Hz), 7.66 (2H, d, *J*=7.8 Hz).
IR (KBr): ν 2982, 2949, 2228, 1930, 1714 cm⁻¹.

### Example 97

### Production of methyl 4-(4-cyanophenyl)-2,5-dimethyl-1-(propylsulfonyl)-1H-pyrrol e-3-carboxylate

By using the compound (0.20 g) produced in Example 17 and 1-propanesulfonyl chloride (0.11 ml), the reaction and purification were carried out in the same manner as Example 66 to obtain the titled compound (170 mg) as colorless crystals.
¹H-NMR (CDCl₃) δ 1.09 (3H, t, *J*=7.0 Hz) , 1.86 (2H, sextet, *J*=7.0 Hz), 2.26 (3H, s), 2.77 (3H, s), 3.2-3.4 (2H, m), 3.57 (3H, s), 7.38 (2H, d, *J*=8.4 Hz), 7.66 (2H, d, *J*=8.4 Hz)..
IR (KBr): ν 2972, 2938, 2228, 1713 cm⁻¹.

### Example 98

### Production of methyl 4-(4-cyanophenyl)-1-(isopropylsulfonyl)-2,5-dimethyl-1H-pyr role-3-carboxylate

By using the compound (0.20 g) produced in Example 17 and isopropylsulfonyl chloride (0.05 ml), the reaction and purification were carried out in the same manner as Example 2 to obtain the titled compound (195 mg) as colorless crystals.
¹H-NMR (CDCl₃) δ 1.41 (6H, d, *J*=7.0 Hz), 2.25 (3H, s), 2.77 (3H, s), 3.46 (1H, septet, *J*=7.0 Hz), 3.57 (3H, s), 7.28 (2H, d, *J*=8.4 Hz), 7.66 (2H, d, *J*=8.4 Hz).
IR (KBr): ν 2986, 2949, 2228, 1929, 1713 cm⁻¹.

### Example 99

### Production of methyl 4-(4-cyanophenyl)-2,5-dimethyl-1-(4-pyridinylmethyl)-1H-pyr role-3-carboxylate

By using the compound (0.49 g) produced in Example 17 and 4- (chloromethyl)pyridine hydrochloride (0.41 g) , the reaction and purification were carried out in the same manner as Example 66 to obtain the titled compound (403 mg) as colorless crystals.
¹H-NMR (CDCl₃) δ 2.02 (3H, s), 2.48 (3H, s), 3.62 (3H, s), 5.13 (2H, s), 6.88 (2H, d, *J*=5.8 Hz), 7.36 (2H, d, *J*=8.2 Hz), 7.65 (2H, d, *J*=8.2 Hz), 8.59 (2H, d, *J*=5.8 Hz).
IR (KBr): ν 2947, 2224, 1699, 1603 cm⁻¹.

### Example 100

### Production of methyl 4-(4-cyano-3-(trifluoromethyl)phenyl)-2,5-dimethyl-1H-pyrro le-3-carboxylate

By using the compound (630 mg) produced in Reference Example 11 and the compound (530 mg) produced in Reference Example 8, the reaction and purification were carried out in the same manner as Example 14 to obtain the titled compound (393 mg) as colorless crystals.
¹H-NMR (CDCl₃) δ 2.16 (3H, s) , 2.53 (3H, s) , 3. 64 (3H, s) , 7.55 (1H, d, *J*=8.0 Hz), 7.67 (1H, s), 7.80 (1H, d, *J*=8.0 Hz), 8.15 (1H, s).
IR (KBr): ν 3320, 2951, 2230, 1674 cm⁻¹.

### Example 101

### Production of methyl 4-(4-cyano-3-(trifluoromethyl)phenyl)-1,2,5-trimethyl-1H-py rrole-3-carboxylate

By using the compound (78 mg) produced in Example 100 and methyl iodide (0.031 ml), the reaction and purification were carried out in the same manner as Example 2 to obtain the titled compound (62 mg) as yellow crystals.
¹H-NMR (CDCl₃) δ 2.12 (3H, s), 2.56 (3H, s), 3.49 (3H, s), 3.59 (3H, s), 7.50 (1H, d, *J*=8.4 Hz), 7.63 (1H, s) , 7.79 (1H, d, *J*=8.4 Hz).
IR (KBr): ν 2249, 2230, 1699, 1178 cm⁻¹.

### Example 102

### Production of methyl 1-(4-cyanobenzyl)-4-(4-cyano-3-(trifluoromethyl)phenyl)-2,5 - dimethyl-1H-pyrrole-3-carboxylate

By using the compound (120 mg) produced in Example 100 and 4-cyanobenzyl bromide (63 mg), the reaction and purification were carried out in the same manner as Example 2 to obtain the titled compound (116 mg) as yellow crystals.
¹H-NMR (CDCl₃) δ 2.03 (3H, s), 2.48 (3H, s), 3.62 (3H, s), 5.19 (2H, s), 7.05 (2H, d, *J*=8.1 Hz), 7.54 (1H, d, *J*=7.5 Hz), 7.6-7.7 (3H, m), 7.80 (1H, d, *J*=8.1 Hz).
IR (KBr): ν 2990, 2949, 2230, 1699 cm⁻¹.

### Example 103

### Production of 4-(4-cyanophenyl)-2,5-dimethyl-1H-pyrrole-3-carboxylic acid

By using the compound (1.01 g) produced in Example 17, the reaction and purification were carried out in the same manner as Example 88 to obtain the titled compound (0.68 g) as yellow crystals.
¹H-NMR (DMSO-d₆) δ 2.05 (3H, s), 2.39 (3H, s), 7.35 (2H, d, *J*=8.4 Hz), 7.71 (2H, d, *J*=8.4 Hz).
IR (KBr): ν 3565, 3215, 2253, 1651 cm⁻¹.

### Example 104

### Production of 4-(4-cyanophenyl)-2,5-dimethyl-1H-pyrrole-3-carboxamide

By using the compound (500 mg) produced in Example 103, the reaction and purification were carried out in the same manner as Example 92 to obtain the titled compound (265 mg) as colorless. crystals.
¹H-NMR (CDCl₃) δ 2.15 (3H, s), 2.52 (3H, s), 4.99 (1H, brs), 5.25 (1H, brs), 7.43 (2H, d, *J*=8.0 Hz), 7.68 (2H, d, *J*=8.0 Hz), 8.27 (1H, s).
IR (KBr): ν 3320, 2951, 2230, 1674 cm⁻¹.

### Example 105

### Production of 4-(4-cyanophenyl)-2,5-dimethyl-1H-pyrrole-3-carbonitrile

To a solution of the compound (250 mg) produced in Example 104 and pyridine (0.13ml) in *N,N*-dimethylformamide (5 ml) was added dropwise oxalyl chloride (0.11 ml) under ice cooling, and the mixture was stirred at the same temperature for 0.5 hour. The reaction mixture was poured into saturated brine and extracted with ethyl acetate. The ethyl acetate layer was dried over magnesium sulfate and concentrated. The residue was purified by column chromatography (carrier: silicagel, eluant: hexane-ethyl acetate) and recrystallized from ethyl acetate-hexane to obtain the titled compound (190 mg) as colorless crystals.
¹H-NMR (CDCl₃) δ 2.32 (3H, s), 2.44 (3H, s), 7.52 (2H, d, J=8.4 Hz), 7.70 (2H, d, *J*=8.4 Hz), 8.26 (1H, s).
IR (KBr): ν 3272, 3185, 2216, 1606 cm⁻¹.

### Example 106

### Production of 1-(4-cyanobenzyl)-4-(4-cyanophenyl)-2,5-dimethyl-1H-pyrrole - 3-carbonitrile

By using the compound (80 mg) produced in Example 105 and 4-cyanobenzyl bromide (63 mg), the reaction and purification were carried out in the same manner as Example 2 to obtain the titled compound (89 mg) as colorless crystals.
¹H-NMR (CDCl₃) δ 2.18 (3H, s), 2.36 (3H, s), 5.17 (2H, s), 7.04 (2H, d, *J*=8.1 Hz), 7.52 (2H, d, *J*=8.4 Hz), 7.67 (2H, d, *J*=8.4 Hz), 7.71 (2H, d, *J*=8.1 Hz).
IR (KBr): ν 3058, 2951, 2920, 2216 cm⁻¹.

### Example 107

### Production of 4-(4-cyanophenyl)-2,5-dimethyl-1-(3-pyridinylmethyl)-1H-pyr role-3-carbonitrile.

By using the compound (80 mg) produced in Example 105 and 3-(chloromethyl)pyridine hydrochloride (71 mg), the reaction and purification were carried out in the same manner as Example 66 to obtain the titled compound (61 mg) as colorless crystals.
¹H-NMR (CDCl₃) δ 2.21 (3H, s), 2.39 (3H, s), 5.14 (2H, s), 7.18 (1H, d, *J*=7.8 Hz), 7.3-7.4 (1H, m), 7.51 (2H, d, *J*=8.4 Hz), 7.71 (2H, d, *J*=8.4 Hz), 8.36 (1H, s), 8.59 (1H, s).
IR (KBr): ν 3038, 2953, 2922, 2216 cm⁻¹.

### Example 108

### Production of methyl 4-(4-cyano-3-(trifluoromethyl)phenyl)-2,5-dimethyl-1-(3-pyr idinylmethyl)-1H-pyrrole-3-carboxylate

By using the compound (120 mg) produced in Example 100 and 3-(chloromethyl)pyridine hydrochloride (71 mg), the reaction and purification were carried out in the same manner as Example 66 to obtain the titled compound (55 mg) as colorless crystals.
¹H-NMR (CDCl₃) δ 2.06 (3H, s), 2.52 (3H, s), 3.61 (3H, s), 5.16 (2H, s), 7.1-7.3 (2H, m), 7.53 (1H, d, *J*=8.4 Hz), 7.66 (1H, s), 7.80 (1H, d, *J*=8.4 Hz), 8.35 (1H, s), 8.56 (1H, s).
IR (KBr): ν 2948, 2224, 1699, 1634 cm⁻¹.

### Example 109

### Production of methyl 1-((6-chloro-3-pyridinyl)methyl)-4-(4-cyanophenyl)-2,5-dime thyl-1H-pyrrole-3-carboxylate

By using the compound (1.08 g) produced in Example 17 and 2-chloro-5-(chloromethyl)pyridine (0.13 g), the reaction and purification were carried out in the same manner as Example 2 to obtain the titled compound (767 mg) as colorless crystals.
¹H-NMR (CDCl₃) δ 2.03 (3H, s) , 2.50 (3H, s) , 3.60 (3H, s) , 5.12 (2H, s), 7.14 (1H, dd, *J*=8.4, 2.4 Hz), 7.2-7.4 (3H, m), 7.63 (2H, d, *J*=8.4 Hz), 8.12 (1H, d, *J*=2.4 Hz).
IR (KBr): ν 2988, 2947, 2224, 1696 cm⁻¹.

### Example 110

### Production of methyl 4-(4-cyanophenyl)-1-((6-fluoro-3-pyridinyl)methyl)-2,5-dime. thyl-1H-pyrrole-3-carboxylate

By using the compound (550 mg) produced in Example 17 and 5- (chloromethyl) -2-fluoropyridine (0.13 g, produce by a method described in *J*. *Org. Chem*., 65, 7718-7722 (2000)), the reaction and purification were carried out in the same manner as Example 2 to obtain the titled compound (398 mg) as colorless crystals.
¹H-NMR (CDCl₃) δ 2.04 (3H, s), 2.51 (3H, s), 3.61 (3H, s), 5.13 (2H, s), 6.93 (1H, dd, *J*=2.7, 7.5 Hz), 7.2-7.4 (1H, m), 7.33 (2H, d, *J*=7.5 Hz), 7.63 (2H, d, *J*=7.5 Hz), 7.93 (1H, s).
IR (KBr): ν 2990, 2949, 2224, 1699 cm⁻¹.

### Example 111

### Production of methyl 4-(4-cyanophenyl)-1-((6-methoxy-3-pyridinyl)methyl)-2,5-dim ethyl-1H-pyrrole-3-carboxylate

A solution of the compound (160 mg) produced in Example 110 and sodium methoxide (34 mg) in N,N-dimethylformamide (3 ml) was stirred at 120 °C for 24 hours under reflux. The reaction mixture was poured into saturated brine and extracted with ethyl acetate. The ethyl acetate layer was dried over magnesium sulfate and concentrated. The residue was purified by column chromatography (carrier: silicagel, eluant: hexane-ethyl acetate) and recrystallized from ethyl acetate-hexane to obtain the titled compound (21 mg) as colorless crystals.
¹H-NMR (CDCl₃) δ 2.05 (3H, s), 2.52 (3H, s), 3.60 (3H, s), 3.92 (3H, s), 5.05 (2H, s), 6.73 (1H, d, *J*=8.8 Hz), 7.18 (1H, dd, *J*=2.6, 8.8 Hz), 7.33 (2H, d, *J*=8.4 Hz) , 7. 63 (2H, d, *J*=8.4 Hz) , 7.79 (1H, d, *J*=2.6 Hz).
IR (KBr): ν 2988, 2947, 2226, 1699 cm⁻¹.

### Example 112

### Production of methyl 1-(4-cyano-2-fluorobenzyl)-4-(4-cyanophenyl)-2,5-dimethyl-1 H-pyrrole-3-carboxylate

By using the compound (600 mg) produced in Example 17 and 4-cyano-2-fluorobenzyl bromide (510 mg, produce by a method described in *J*. *Org*. *Chem*., 65, 7718-7722 (2000)), the reaction and purification were carried out in the same manner as Example 2 to obtain the titled compound (156 mg) as colorless crystals.
¹H-NMR (CDCl₃) δ 2.01 (3H, s), 2.47 (3H, s), 3.62 (3H, s), 5.20 (2H, s), 6.5-6.7 (1H, m), 7.35 (2H, d, *J*=8.6Hz), 7.4-7.5 (2H, m), 7.65 (2H, d, *J*=8.6 Hz).
IR (KBr): ν 3069, 2949, 2226, 1699 cm⁻¹.

### Example 113

### Production of methyl 4-(4-cyanophenyl)-2,5-dimethyl-1-((6-(trifluoromethyl)-3-py ridinyl)methyl)-1H-pyrrole-3-carboxylate

By using the compound (550 mg) produced in Example 17 and 5-(chloromethyl)-2-(trifluoromethyl)pyridine (0.46 g), the reaction and purification were carried out in the same manner as Example 2 to obtain the titled compound (399 mg) as colorless crystals.
¹H-NMR (CDCl₃) δ 2.03 (3H, s), 2.50 (3H, s), 3.61 (3H, s), 5.23 (2H, s), 7.2-7.4 (1H, m), 7.34 (2H, d, *J*=8.4 Hz), 7.4-7.6 (1H, m), 7.64 (2H, d, *J*=8.4 Hz), 8.47 (1H, s).
IR (KBr): ν 2990, 2949, 2226, 1699 cm⁻¹.

### Example 114

### Production of methyl 4-(4-cyanophenyl)-1-((2-cyano-4-pyridinyl)methyl)-2,5-dimet hyl-1H-pyrrole-3-carboxylate

By using the compound (600 mg) produced in Example 17 and 4-(chloromethyl)pyridine-2-carbonitrile (330 mg, produced by a method described in *J*. *Org*. *Chem*., 65, 7718-7722 (2000)), the reaction and purification were carried out in the same manner as Example 2 to obtain the titled compound (134 mg) as colorless crystals.
¹H-NMR (CDCl₃) δ 2.01 (3H, s), 2.47 (3H, s), 3.63 (3H, s), 5.18 (2H, s), 7.08 (1H, d, *J*=4.4 Hz), 7.28 (1H, d, *J*=5.0 Hz), 7.36 (2H, d, *J*=8.4 Hz), 7.66 (2H, d, *J*=8.4 Hz), 8.71 (1H, d, *J*=5.0 Hz).
IR (KBr): ν 3058, 2988, 2224, 1693 cm⁻¹.

### Example 115

### Production of 1-((6-chloro-3-pyridinyl)methyl)-4-(4-cyanophenyl)-2,5-dime thyl-1H-pyrrole-3-carbonitrile

By using the compound (150 mg) produced in Example 105 and 6-chloro-3-(chloromethyl)pyridine (130 mg), the reaction and purification were carried out in the same manner as Example 2 to obtain the titled compound (133 mg) as colorless crystals.
¹H-NMR (CDCl₃) δ 2.01 (3H, s), 2.40 (3H, s), 5.13 (2H, s), 7.16 (1H, dd, *J*=2.2, 8.0 Hz), 7.35 (1H, d, *J*=8.0 Hz), 7.51 (2H, d, *J*=8.4 Hz), 7.72 (2H, d, *J*=8.4 Hz), 8.13 (1H, d, *J*=2.2 Hz).
IR (KBr): ν 3051, 2986, 2314, 1607 cm⁻¹.

### Example 116

### Production of methyl 4-(3,4-dichlorophenyl)-2,5-dimethyl-1-(3-pyridinylmethyl)-1 H-pyrrole-3-carboxylate

By using the compound (200 mg) produced in Example 38 and 3- (chloromethyl)pyridine hydrochloride (130 mg) , the reaction and purification were carried out in the same manner as Example 66 to obtain the titled compound (103 mg) as colorless crystals.
¹H-NMR (CDCl₃) δ 2.00 (3H, s), 2.46 (3H, s), 3.63 (3H, s), 5.16 (2H, s), 7.0-7.2 (2H, m), 7.2-7.4 (2H, m), 7.41 (1H, d, *J*=8.2 Hz), 8.37 (1H, m), 8.55 (1H, d, *J*=4.4 Hz).
IR (KBr): ν 2990, 2948, 1699, 1533 cm⁻¹.

### Example 117

### Production of methyl 1-((6-chloro-3-pyridinyl)methyl)-4-(4-cyano-3-(trifluoromet hyl)phenyl)-2,5-dimethyl-1H-pyrrole-3-carboxylate

By using the compound (150 mg) produced in Example 100 and 2-chloro-5-(chloromethyl)pyridine (90 mg), the reaction and purification were carried out in the same manner as Example 2 to obtain the titled compound (91 mg) as colorless crystals.
¹H-NMR (CDCl₃) δ 2.05 (3H, s), 2.52 (3H, s), 3.61 (3H, s), 5.14 (2H, s), 7.16 (1H, dd, *J*=2.4, 8.6 Hz), 7.34 (1H, d, *J*=8.4 Hz), 7.52 (1H, d, *J*=8.8 Hz), 7.66 (1H, s), 7.81 (1H, d, *J*=8.8 Hz), 7.52 (1H, d, *J*=2.4 Hz).
IR (KBr): ν 2996, 2949, 2230, 1699 cm⁻¹.

### Example 118

### Production of methyl 4-(4-cyanophenyl)-1-((2-cyano-3-pyridinyl)methyl)-2,5-dimet hyl-1H-pyrrole-3-carboxylate

By using the compound (450 mg) produced in Example 17 and 3- (bromomethyl) pyridine-2-carbonitrile (500 mg, produced by a method described in *J*. *Org*. *Chem*., **65**, 7718-7722 (2000)), the reaction and purification were carried out in the same manner as Example 2 to obtain the titled compound (61 mg) as colorless crystals.
¹H-NMR (CDCl₃) δ 2.02 (3H, s), 2.48 (3H, s), 3.61 (3H, s), 5.35 (2H, s), 6.93 (1H, d, *J*=8.1 Hz), 7.34 (2H, d, *J*=8.7 Hz), 7.49 (4H, dd, *J*=4.8, 6.8 Hz), 7.64 (2H, d, *J*=8.7 Hz), 8.66 (1H, d, *J*=4.8 Hz).
IR (KBr): ν 3054, 2992, 2949, 2253, 2226 cm⁻¹.

### Example 119

### Production of 3-((3-cyano-4-(4-cyanophenyl)-2,5-dimethyl-1H-pyrrol-1-yl)m ethyl)-2-pyridine carbonitrile

By using the compound (180 mg) produced in Example 105 and 3-(bromomethyl)pyridine-2-carbonitrile (500 mg, produced by a method described in *J. Org. Chem.,* 65, 7718-7722 (2000) ) , the reaction and purification were carried out in the same manner as Example 2 to obtain the titled compound (91 mg) as colorless crystals.
¹H-NMR (CDCl₃) δ 2.20 (3H, s), 2.38 (3H, s), 5.35 (2H, s), 6.94 (1H, d, *J*=8.0 Hz), 7.52 (2H, d, *J*=8.0 Hz), 7.2-7.4 (1H, m), 7.73 (2H, d, *J*=8.0 Hz), 8.71 (1H, d, *J*=4.4 Hz).
IR (KBr): ν 3069, 2949, 2226, 1699 cm⁻¹.

### Example 120

### Production of 4-(4-cyanophenyl)-2,5-dimethyl-1-(2-pyrazinyl methyl)-1H-pyrrole-3-carbonitrile

By using the compound (230 mg) produced in Example 105 and 2- (bromomethyl) pyrazine (200 mg, produced by a method described in *J. Org*. *Chem*., 65, 7718-7722 (2000)), the reaction and purification were carried out in the same manner as Example 2 to obtain the titled compound (198 mg) as colorless crystals.
¹H-NMR (CDCl₃) δ 2.26 (3H, s), 2.45 (3H, s), 5.23 (2H, s), 7.52 (2H, d, *J*=8.8 Hz), 7.71 (2H, d, *J*=8.8 Hz), 8.26 (1H, s), 8.58 (2H, s).
IR (KBr): ν 3048, 2988, 2951, 2215, 1607 cm⁻¹.

### Example 121

### Production of 5-((3-cyano-4-(4-cyanophenyl)-2,5-dimethyl-1H-pyrrol-1-yl)m ethyl)nicotinonitrile

By using the compound (540 mg) produced in Example 105 and 5- (chloromethyl) nicotinonitrile (600 mg, produced by a method described in *J. Org. Chem.,* 65, 7718-7722 (2000)), the reaction and purification were carried out in the same manner as Example 2 to obtain the titled compound (99 mg) as colorless crystals.
¹H-NMR (CDCl₃) δ 2.21 (3H, s), 2.39 (3H, s), 5.20 (2H, s), 7.46 (1H, s), 7.52 (2H, d, *J*=8.4 Hz), 7.72 (2H, d, *J*=8.4 Hz), 8.52 (1H, d, *J*=2.1 Hz), 8.86 (1H, s).
IR (KBr): ν 3057, 2990, 2216, 1609 cm⁻¹.

### Example 122

### Production of 4-(4-cyanophenyl)-3,5-dimethyl-1H-pyrrole-2-carbonitrile

By using the compound (1.99 g) produced in Reference Example 12 and 4-cyanophenylboronic acid (1.62 g), the reaction and purification were carried out in the same manner as Example 14 to obtain the titled compound (1.09 g) as colorless crystals.
¹H-NMR (CDCl₃) δ 2.20 (3H, s), 2.29 (3H, s), 7.34 (2H, d, *J*=8.4 Hz), 7.70 (2H, d, *J*=8.4 Hz), 8.71 (1H, s).
IR (KBr): ν 3277, 3071, 2215, 1674, 1609 cm⁻¹.

### Example 123

### Production of 4-(4-cyanophenyl)-3,5-dimethyl-1-(3-pyridinylmethyl)-1H-pyr role-2-carbonitrile

By using the compound (620 mg) produced in Example 122 and 3- (chloromethyl) pyridine hydrochloride (550 mg) , the reaction and purification were carried out in the same manner as Example 66 to obtain the titled compound (467 mg) as colorless crystals.
¹H-NMR (CDCl₃) δ 2.18 (3H, s), 2.21 (3H, s), 5.24 (2H, s), 7.2-7.4 (3H, m), 7.4-7.5 (1H, m), 7.6-7.8 (2H, m), 8.42 (1H, s), 8.59 (1H, d, *J*=4.6 Hz).
IR (KBr): ν 3026, 2990, 2226, 2207, 1607 cm⁻¹.

### Example 124

### Production of methyl 4-(4-cyanophenyl)-2-ethyl-5-methyl-1H-pyrrole-3-carboxylate

By using the compound (6.01 g) produced in Reference Example 1 and methyl 3-oxopentanoate (12 ml), the reaction and purification were carried out in the same manner as Example 1 to obtain the titled compound (5.8 g) as colorless crystals.
¹H-NMR (CDCl₃) δ 1.28 (3H, t, *J*=7.2 Hz), 2.14 (3H, s), 2.97 (2H, q, *J*=7.2 Hz), 3.62 (3H, s), 7.35 (2H, d, *J*=8.4 Hz), 7.62 (2H, d, *J*=8.4 Hz), 8. 07 (1H, s).
IR (KBr): ν 3318, 2975, 2949, 2226, 1682 cm⁻¹.

### Example 125

### Production of 4-(4-cyanophenyl)-2-ethyl-5-methyl-1H-pyrrole-3-carboxylic acid

By using the compound (86 mg) produced in Example 124, the reaction and purification were carried out in the same manner as Example 88 to obtain the titled compound (46 mg) as colorless crystals.
¹H-NMR (DMSO-d₆) δ 1.11 (3H, t, *J*=7.4 Hz), 2.00 (3H, s), 2.79 (2H, q, *J*=7.4 Hz), 7.31 (2H, d, *J*=8.4 Hz), 7.67 (2H, d, *J*=8.4 Hz), 11.11 (1H, s), 11.36 (1H, s).
IR (KBr): ν 3300, 2982, 2226, 1651 cm⁻¹.

### Example 126

### Production of 4-(4-cyanophenyl)-2-ethyl-5-methyl-1H-pyrrole-3-carboxamide

By using the compound (1.29 g) produced in Example 125, the reaction and purification were carried out in the same manner as Example 92 to obtain the titled compound (1.09 g) as colorless crystals.
¹H-NMR (DMSO-d₆) δ 1.16 (3H, t, *J*=7.2 Hz), 2.15 (3H, s), 2.70 (2H, q, *J*=7.2 Hz), 6.48 (1H, brs), 6.80 (1H, brs), 7.37 (2H, d, *J*=8.4 Hz), 7.73 (2H, d, *J*=8.4 Hz), 10.88 (1H, s).
IR (KBr): ν 3321, 2974, 2224, 1653 cm⁻¹.

### Example 127

### Production of 4-(4-cyanophenyl)-2-ethyl-5-methyl-1H-pyrrole-3-carbonitril e

By using the compound (950 mg) produced in Example 126, the reaction and purification were carried out in the same manner as Example 105 to obtain the titled compound (694 mg) as colorless crystals.
¹H-NMR (CDCl₃) δ 1.33 (3H, t, *J*=7.5 Hz), 2.32 (3H, s), 2.81 (2H, q, *J*=7.5 Hz), 7.52 (2H, d, *J*=8.7 Hz), 7.69 (2H, d, *J*=8.4 Hz), 8.35 (1H, s).
IR (KBr): ν 3277, 3046, 2976, 2215, 1607 cm⁻¹.

### Example 128

### Production of 4-(4-cyanophenyl)-2-ethyl-5-methyl-1-(3-pyridinylmethyl)-1H - pyrrole-3-carbonitrile

By using the compound (580 mg) produced in Example 127 and 3- (chloromethyl)pyridine hydrochloride (530 mg), the reaction and purification were carried out in the same manner as Example 66 to obtain the titled compound (421 mg) as colorless crystals.
¹H-NMR (CDCl₃) δ 1.23 (3H, t, *J*=7.8 Hz) , 2.18 (3H, s) , 2.79 (2H, q, *J*=7.8 Hz), 5.17 (2H, s), 7.1-7.2 (1H, m), 7.2-7.4 (1H, m), 7.52 (2H, d, *J*=8.8 Hz), 7.71 (2H, d, *J*=8.8 Hz), 8.35 (1H, d, *J*=1.8 Hz), 8.58 (1H, d, *J*=4.4 Hz).
IR (KBr): ν 3034, 2976, 2938, 2215, 1607 cm⁻¹.

### Example 129

### Production of methyl 4-(4-cyanophenyl)-2-isopropyl-5-methyl-1H-pyrrole-3-carboxy late

By using the compound (6.00 g) produced in Reference Example 1 and methyl 4-methyl-3-oxopentanoate (13.8 ml), the reaction and purification were carried out in the same manner as Example 1 to obtain the titled compound (5.09 g) as colorless crystals.
¹H-NMR (CDCl₃) δ 1.30 (6H, d, *J*=6.6Hz), 2.14 (3H, s), 3.60 (3H, s), 3.79 (1H, septet, *J*=6.6 Hz), 7.34 (2H, d, *J*=8.0 Hz), 7. 62 (2H, d, *J*=8.0 Hz), 8.13 (1H, s).
IR (KBr): ν 3320, 2970, 2870, 2226, 1682 cm⁻¹.

### Example 130

### Production of 4-(4-cyanophenyl)-2-isopropyl-5-methyl-1H-pyrrole-3-carboxy lic acid

By using the compound (4.2 g) produced in Example 129, the reaction and purification were carried out in the same manner as Example 88 to obtain the titled compound (1.41 g) as colorless crystals.
¹H-NMR (DMSO-d₆) δ 1.16 (6H, d, *J*=7.2 Hz), 2.02 (3H, s), 3.69 (1H, septet, *J*=6.6 Hz), 7.30 (2H, d, *J*=8.0 Hz), 7.66 (2H, d, *J*=8.0 Hz), 10.96 (1H, s), 11.37 (1H, s).
IR (KBr): ν 3337, 2967, 2226, 1661 cm⁻¹.

### Example 131

### Production of 4-(4-cyanophenyl)-2-isopropyl-5-methyl-1H-pyrrole-3-carboxa mide

By using the compound (1.34 g) produced in Example 130, the reaction and purification were carried out in the same manner as Example 92 to obtain the titled compound (0.53 g) as colorless crystals.
¹H-NMR (DMSO-d₆) δ 1.21 (6H, d, *J*=7.0 Hz), 2.17 (3H, s), 3.35 (1H, septet, *J*=7.0 Hz), 6.50 (1H, brs), 6.83 (1H, brs), 7.38 (2H, d, *J*=8.0 Hz), 7.73 (2H, d, *J*=8.0 Hz), 10.76 (1H, s).
IR (KBr): ν 3345, 2962, 2224, 1609 cm⁻¹.

### Example 132

### Production of 4-(4-cyanophenyl)-2-isopropyl-5-methyl-1H-pyrrole-3-carboni trile

By using the compound (950 mg) produced in Example 131, the reaction and purification were carried out in the same manner as Example 105 to obtain the titled compound (358 mg) as colorless crystals.
¹H-NMR (CDCl₃) δ 1.37 (6H, d, *J*=7.0 Hz), 2.32 (3H, s), 3.24 (1H, septet, *J*=7.0 Hz), 7.53 (2H, d, *J*=8.0 Hz), 7.70 (2H, d, *J*=8.0 Hz), 8.24 (1H, s).
IR (KBr): ν 3289, 2971, 2213, 1609 cm⁻¹.

### Example 133

### Production of 4-(4-cyanophenyl)-2-isopropyl-5-methyl-1-(3-pyridinylmethyl )-1H-pyrrole-3-carbonitrile

By using the compound (320 mg) produced in Example 132 and 3- (chloromethyl)pyridine hydrochloride (250 mg) , the reaction and purification were carried out in the same manner as Example 66 to obtain the titled compound (132 mg) as colorless crystals.
¹H-NMR (CDCl₃) δ 1.39 (6H, d, *J*=7.0 Hz), 2.18 (3H, s), 3.02 (1H, septet, *J*=7.0 Hz), 5.18 (2H, s), 7.1-7.2 (1H, m), 7.2-7.4 (1H, m), 7.52 (2H, d, *J*=8.6 Hz), 7.72 (2H, d, *J*=8.6 Hz), 8.34 (1H, s), 8.58 (2H, d, *J*=4.8 Hz).
IR (KBr): ν 2973, 2932, 2215, 1607 cm⁻¹.

### Example 134

### Production of methyl 4-(4-cyanophenyl)-2,5-diethyl-1H-pyrrole-3-carboxylate

By using the compound (3.54 g) produced in Reference Example 14 and methyl 3-oxopentanoate (6 ml), the reaction and purification were carried out in the same manner as Example 1 to obtain the titled compound (3.7 g) as colorless crystals.
¹H-NMR (CDCl₃) δ 1.10 (3H, t, *J*=7.4 Hz), 1.29 (3H, t, *J*=7.4 Hz), 2.49 (2H, q, *J*=7.4 Hz), 2.98 (2H, q, *J*=7.4 Hz), 3.61 (3H, s), 7.34 (2H, d, *J*=8.4 Hz), 7.62 (2H, d, *J*=8.4 Hz), 8.08 (1H, s).
IR (KBr): ν 3326, 2973, 2878, 2226, 1747, 1682 cm⁻¹.

### Example 135

### Production of 4-(4-cyanophenyl)-2,5-diethyl-1H-pyrrole-3-carboxylic acid

By using the compound (2.87 g) produced in Example 134, the reaction and purification were carried out in the same manner as Example 88 to obtain the titled compound (1.91 g) as colorless crystals.
¹H-NMR (DMSO-d₆) δ 1.0-1.2 (6H, m), 2.2-2.4 (2H, m), 2.7-2.9 (2H, m), 7.2-7.4 (2H, m), 7.6-7.8 (2H, m), 11.08 (1H, s), 11.18 (1H, s).
IR (KBr): ν 3310, 2973, 2936, 2230, 1699 cm⁻¹.

### Example 136

### Production of 4-(4-cyanophenyl)-2,5-diethyl-1H-pyrrole-3-carboxamide

By using the compound (1.80 g) produced in Example 135, the reaction and purification were carried out in the same manner as Example 92 to obtain the titled compound (0.78 g) as colorless crystals.
¹H-NMR (CDCl₃) δ 1.15 (3H, t, *J*=7.6 Hz), 1.29 (3H, t, *J*=7.6 Hz), 2.50 (2H, q, *J*=7.6 Hz), 3.01 (2H, q, *J*=7.4 Hz), 5.07 (2H, brs) , 7.44 (2H, d, *J*=8.4 Hz), 7.68 (2H, d, *J*=8.4 Hz), 8.24 (1H, s).
IR (KBr): ν 3337, 2973, 2226, 1680, 1643 cm⁻¹.

### Example 137

### Production of 4-(4-cyanophenyl)-2,5-diethyl-1H-pyrrole-3-carbonitrile

By using the compound (700 mg) produced in Example 136, the reaction and purification were carried out in the same manner as Example 105 to obtain the titled compound (366 mg) as colorless crystals.
¹H-NMR (CDCl₃) δ 1.24 (3H, t, *J*=7.6 Hz), 1.34 (3H, t, *J*=7.6 Hz), 2.68 (2H, q, *J*=7.6 Hz) , 2.82 (2H, q, *J*=7.6 Hz), 7.51 (2H, d, *J*=8.4 Hz), 7.69 (2H, d, *J*=8.4 Hz), 8.32 (1H, s).
IR (KBr): ν 3326, 2973, 2878, 2226, 1747, 1682 cm⁻¹.

### Example 138

### Production of 4-(4-cyanophenyl)-2,5-diethyl-1-(3-pyridinylmethyl)-1H-pyrr ole-3-carbonitrile

By using the compound (320 mg) produced in Example 137 and 3- (chloromethyl)pyridine hydrochloride (250 mg), the reaction and purification were carried out in the same manner as Example 66 to obtain the titled compound (132 mg) as colorless crystals.
¹H-NMR (CDCl₃) δ 1.11 (3H, t, *J*=7.6 Hz), 1.21 (3H, t, *J*=7.6 Hz), 2.55 (2H, q, *J*=7.6 Hz), 2.72 (2H, q, *J*=7.6 Hz), 5.19 (2H, s), 7.13 (1H, d, *J*=7.8 Hz), 7.2-7.4 (1H, m), 7.53 (2H, d, *J*=8.4 Hz), 7.72 (2H, d, *J*=8.4 Hz), 8.33 (1H, d, *J*=1.4 Hz), 8.5-8.6 (1H, m).
IR (KBr): ν 3040, 2973, 2940, 2215 cm⁻¹.

### Example 139

### Production of 4-(4-cyanophenyl)-2,5-dimethyl-1-(4-pyrimidinylmethyl)-1H-p yrrole-3-carbonitrile

By using the compound (620 mg) produced in Example 105 and 4-(chloromethyl)pyrimidine (540 mg, Produced by a method described in *J. Org. Chem.,* 65, 7718-7722 (2000)), the reaction and purification were carried out in the same manner as Example 2 to obtain the titled compound (19 mg) as colorless crystals.
¹H-NMR (CDCl₃) δ 2.21 (3H, s), 2.40 (3H, s), 5.17 (2H, s), 6.76 (1H, d, *J*=5.0 Hz), 7.53 (2H, d, *J*=8.6 Hz), 7.72 (2H, d, *J*=8.6 Hz), 8.75 (1H, d, *J*=5.0 Hz), 9.22 (1H, s).
IR (KBr): ν 3042, 2972, 2222, 1582 cm⁻¹.

### Example 140

### Production of 4-(4-cyanophenyl)-1-((4-methoxy-3,5-dimethyl-2-pyridinyl)me thyl)-2,5-dimethyl-1H-pyrrole-3-carbonitrile

By using the compound (600 mg) produced in Example 105 and 2-(chloromethyl)-4-methoxy-3,5-dimethylpyridine (780 mg), the reaction and purification were carried out in the same manner as Example 2 to obtain the titled compound (665 mg) as colorless crystals.
¹H-NMR (CDCl₃) δ 2.16 (3H, s), 2.24 (3H, s), 2.29 (3H, s), 2.34 (3H, s), 3.79 (3H, s), 5.07 (2H, s), 7.53 (2H, d, *J*=8.0 Hz), 7.68 (2H, d, *J*=8.0 Hz), 8.16 (1H, s).
IR (KBr): ν 2947, 2926, 2224, 2213, 1608 cm⁻¹.

### Example 141

### Production of 4-(4-cyanophenyl)-1-((4-hydroxy-3,5-dimethyl-2-pyridinyl)me thyl)-2,5-dimethyl-1H-pyrrole-3-carbonitrile

By using the compound (180 mg) produced in Example 140, the reaction and purification were carried out in the same manner as Example 88 to obtain the titled compound (104 mg) as colorless crystals.
¹H-NMR (CDCl₃) δ 2.00 (3H, s), 2.14 (3H, s), 2. 16 (3H, s), 2.37 (3H, s), 5.08 (2H, s), 7.3-7.5 (3H, m), 7.67 (2H, d, *J*=8.1 Hz). IR (KBr): ν 2955, 2890, 2215, 1633 cm⁻¹.

### Example 142

### Production of 5-((3-cyano-4-(4-cyanophenyl)-2,5-dimethyl-1H-pyrrol-1-yl)m ethyl)-2,4-dimethyl-3-pyridinyl benzenesulfonate

By using the compound (120 mg) produced in Example 105 and benzenesulfonic acid 5- (bromomethyl) -2, 4-dimethyl-3-pyridinyl (200 mg, produced by a method described in *J*. *Org*. *Chem*., 65, 7718-7722 (2000) and *J. Org*. *Chem*., 61, 813-815 (1996)), the reaction and purification were carried out in the same manner as Example 2 to obtain the titled compound (131 mg) as colorless crystals.
¹H-NMR (CDCl₃) δ 2.18 (6H, s), 2.33 (3H, s), 2.37 (3H, s), 5.03 (2H, s), 7.33 (1H, s), 7.51 (2H, d, *J*=8.0Hz), 7.6-7.8 (5H, m), 8.00 (2H, d, *J*=8.0 Hz).
IR (KBr): ν 3072, 2921, 2216, 1609 cm⁻¹.

### Example 143

### Production of 4-(4-cyanophenyl)-1-((5-hydroxy-4,6-dimethyl-3-pyridinyl)me thyl)-2,5-dimethyl-1H-pyrrole-3-carbonitrile

A solution of the compound (95 mg) produced in Example 142 and anhydrous potassium carbonate (100 mg) in methanol (3 ml) was stirred for 10 minutes under reflux. The reaction mixture was poured into saturated brine and extracted with ethyl acetate. The ethyl acetate layer was dried over magnesium sulfate and concentrated. The residue was purified by column chromatography (carrier: silicagel, eluant: hexane-ethyl acetate) and recrystallized from ethyl acetate-hexane to obtain the titled compound (58 mg) as colorless crystals.
¹H-NMR (CDCl₃) δ 2.17 (3H, s), 2.29 (3H, s), 2.34 (3H, s), 2.48 (3H, s), 5.02 (2H, s), 7.03 (1H, s), 7.51 (2H, d, *J*=8.4 Hz), 7.71 (2H, d, *J*=8.4 Hz).
IR (KBr): ν 2928, 2867, 2215, 1609 cm⁻¹.

### Example 144

### Production of 4-(4-cyanophenyl)-1-((5-hydroxy-2-pyridinyl)methyl)-2,5-dim ethyl-1H-pyrrole-3-carbonitrile

By using the compound (140 mg) produced in Example 105 and 6-(bromomethyl)-3-pyridinyl benzenesulfonate (201 mg, produced by a method described in *J*. *Org. Chem*., 65, 7718-7722 (2000) and *J*. *Org. Chem*., 61, 813-815 (1996)), the reaction and purification were carried out in the same manner as Example 2 to obtain the titled compound (51 mg) as colorless crystals.
¹H-NMR (CDCl₃) δ 2.23 (3H, s), 2.40 (3H, s) , 5.13 (2H, s) , 5. 95 (1H, brs), 6.66 (1H, d, *J*=8.4 Hz), 7.16 (1H, dd, *J*=3.0, 8.8 Hz), 7.51 (2H, d, *J*=8.4 Hz), 7.70 (2H, d, *J*=8.4 Hz), 8.24 (1H, d, *J*=3.0 Hz).
IR (KBr): ν 3339, 2982, 2215, 1607 cm⁻¹.

### Example 145

### Production of methyl 5-((3-cyano-4-(4-cyanophenyl)-2,5-dimethyl-1H-pyrrol-1-yl)m ethyl)nicotinate

By using the compound (600 mg) produced in Example 105 and methyl 5-(chloromethyl)nicotinate (750 mg, produced by a method described in *J*. *Org*. *Chem*., 65, 7718-7722 (2000)), the reaction and purification were carried out in the same manner as Example 2 to obtain the titled compound (205 mg) as colorless crystals.
¹H-NMR (CDCl₃) δ 2.22 (3H, s), 2.40 (3H, s), 3.97 (3H, s), 5.19 (2H, s), 7.52 (2H, d, *J*=8.0 Hz), 7.72 (2H, d, *J*=8.0 Hz), 7.89 (1H, s), 8.44 (1H, d, *J*=2.2 Hz), 9.18 (1H, d, *J*=1.4 Hz).
IR (KBr): ν 3042, 2972, 2222, 1582 cm⁻¹.

### Example 146

### Production of 4-(4-cyanophenyl)-1-((5-(1-hydroxy-1-methyl ethyl)-3-pyridinyl)methyl)-2,5-dimethyl-1H-pyrrole-3-carbon itrile

To a solution of the compound (80 mg) produced in Example 145 in tetrahydrofuran (2 ml) was added dropwise a 3N solution of methylmagnesium bromide in tetrahydrofuran (0.37 ml) under ice cooling. The reaction mixture was stirred at the same temperature for 1 hour. The reaction mixture was poured into saturated brine and extracted with ethyl acetate. The ethyl acetate layer was dried over magnesium sulfate and concentrated. The residue was purified by column chromatography (carrier: silicagel, eluant: hexane-ethyl acetate) and recrystallized from ethyl acetate-hexane to obtain the titled compound (64 mg) as colorless crystals.
¹H-NMR (CDCl₃) δ 1.60 (6H, s), 2.22 (3H, s), 2.40 (3H, s), 5.14 (2H, s), 7.4-7.6 (1H; m), 7.52 (2H, d, *J*=8.4 Hz), 7.72 (2H, d, *J*=8.4 Hz), 8.08 (1H, s), 8.65 (1H, s).
IR (KBr): ν 3042, 2972, 2222, 1582 cm⁻¹.

### Example 147

### Production of ethyl 4-(4-cyanophenyl)-5-methyl-2-phenyl-1H-pyrrole-3-carboxylat e

By using the compound (4.96 g) produced in Reference Example 1 and ethyl 3-oxo-3-phenylpropanoate (15.2 g), the reaction and purification were carried out in the same manner as Example 1 to obtain the titled compound (155 mg) as colorless crystals.
¹H-NMR (CDCl₃) δ 0.93 (3H, t, *J*=7.4 Hz), 2.22 (3H, s) , 4.00 (2H, q, *J*=7.4 Hz), 7.3-7.5 (5H, m), 7.54 (2H, d, *J*=8.4 Hz), 7.66 (2H, d, *J*=8.4 Hz), 8.24 (1H, s).
IR (KBr): ν 3203, 2982, 2226, 1682 cm⁻¹.

### Example 148

### Production of 4-(4-cyanophenyl)-1-((4,5-dimethoxy-3-pyridinyl)methyl)-2,5 - dimethyl-1H-pyrrole-3-carbonitrile

By using the compound (300 mg) produced in Example 105 and 3- (chloromethyl) -4, 5-dimethoxypyridine (430 mg) , the reaction and purification were carried out in the same manner as Example 2 to obtain the titled compound (224 mg) as colorless crystals.
¹H-NMR (CDCl₃) δ 2.24 (3H, s), 2.43 (3H, s), 3.79 (3H, s), 3.94 (3H, s), 5.18 (2H, s), 6.83 (1H, d, *J*=5.4 Hz), 7.51 (2H, d, J=8.4 Hz), 7.68 (2H, d, *J*=8.4 Hz), 8.19 (1H, d, *J*=5.4 Hz).
IR (KBr): ν 2982, 2948, 2213, 1607 cm⁻¹.

### Example 149

### Production of ethyl 4-(4-cyanophenyl)-5-methyl-2-phenyl-1-(3-pyridinylmethyl)-1 H-pyrrole-3-carboxylate

By using the compound (120 mg) produced in Example 147 and 3-(chloromethyl)pyridine hydrochloride (94 mg), the reaction and purification were carried out in the same manner as Example 66 to obtain the titled compound (152 mg) as colorless crystals.
¹H-NMR (CDCl₃) δ 1.26 (3H, t, *J*=6.8 Hz), 2.04 (3H, s), 4.12 (2H, q, *J*=6.8 Hz), 5.00 (2H, s), 7.1-7.4 (7H, m) , 7.48 (2H, d, *J*=8.4 Hz), 7.66 (2H, d, *J*=8.4 Hz), 8.21 (1H, s), 8.51 (1H, d, *J*=5.0 Hz).
IR (KBr): ν 3203, 2982, 2226, 1682 cm⁻¹.

### Example 150

### Production of 4-(4-cyanophenyl)-1-((6-fluoro-3-pyridinyl)methyl)-2,5-dime thyl-1H-pyrrole-3-carbonitrile

By using the compound (600 mg) produced in Example 105 and 5-(chloromethyl)-2-fluoropyridine (400 mg, produced by a method described in *J*. *Org*. *Chem.,* 65, 7718-7722 (2000)), the reaction and purification were carried out in the same manner as Example 2 to obtain the titled compound (432 mg) as colorless crystals.
¹H-NMR (CDCl₃) δ 2.22 (3H, s), 2.40 (3H, s), 5.13 (2H, s), 6.97 (1H, dd, *J*=3.0, 8.4 Hz), 7.2-7.4 (1H, m), 7.51 (2H, d, *J*=8.4 Hz), 7.72 (2H, d, *J*=8.4 Hz), 7.94 (1H, s).
IR (KBr): ν 3056, 2922, 2216, 1607 cm⁻¹.

### Example 151

### Production of benzenesulfonic acid 5-((3-cyano-4-(4-cyanophenyl)-2,5-dimethyl-1H-pyrrol-1-yl)m ethyl)-2-pyridinyl

By using the compound (400 mg) produced in Example 105 and 5-(bromomethyl)-2-pyridinyl benzenesulfonate (830 mg, produced by a method described in *J*. *Org. Chem.,* 61, 813-815 (1996)), the reaction and purification were carried out in the same manner as Example 2 to obtain the titled compound (498 mg) as colorless crystals.
¹H-NMR (CDCl₃) δ 2.17 (3H, s), 2.33 (3H, s), 5.11 (2H, s), 6.64 (1H, s), 6.78 (1H, d, *J*=5.1 Hz), 7.4-7.6 (4H, m), 7.6-7.8 (3H, m), 8.01 (2H, d, *J*=7.2 Hz), 8.25 (1H, d, *J*=5.1 Hz).
IR (KBr): ν 3065, 2988, 2216, 1607 cm⁻¹.

### Example 152

### Production of 4-(4-cyanophenyl)-2,5-dimethyl-1-((6-oxo-1,6-dihydro-3-pyri dinyl)methyl)-1H-pyrrole-3-carbonitrile

By using the compound (310 mg) produced in Example 151, the reaction and purification were carried out in the same manner as Example 143 to obtain the titled compound (158 mg) as colorless crystals.
¹H-NMR (CDCl₃) δ 2.20 (3H, s), 2.37 (3H, s), 4.94 (2H, s), 5.8-6.0 (1H, m), 6.0-6.1 (1H, m), 7.3-7.4 (1H, m), 7.51 (2H, d, *J*=8.4 Hz), 7.72 (2H, d, *J*=8.4 Hz).
IR (KBr): ν 3078, 2951, 2840, 2216, 1661, 1609 cm⁻¹.

### Example 153

### Production of 4- (4-cyanophenyl) -2-ethyl-1- ((5-hydroxy-2-pyridinyl) methyl) - 5-methyl-1H-pyrrole-3-carbonitrile

By using the compound (70 mg) produced in Example 127 and 6-(bromomethyl)-3-pyridinyl benzenesulfonate (120 mg, produced by a method described in *J. Org. Chem.,* 61, 813-815 (1996)), the reaction was carried out in the same manner as Example 2, whereby an alkylation reaction and a de-benzensulphonylation reaction proceeded simultaneously. Purification was carried out in the same manner as Example 2 to obtain the titled compound (47 mg) as colorless crystals.
¹H-NMR (CDCl₃) δ 1.20 (3H, t, *J*=7.4 Hz), 2.19 (3H, s), 2.79 (2H, q, *J*=7.4 Hz), 5.16 (2H, s), 6.62 (1H, d, *J*=8.4 Hz), 6.69 (1H, s), 7.17 (1H, dd, *J*=3.0, 8.4 Hz), 7.51 (2H, d, *J*=8.4 Hz), 7.70 (2H, d, *J*=8.4 Hz), 8.24 (1H, d, *J*=2.6 Hz).
IR (KBr): ν 3380, 2980, 2215, 1607 cm⁻¹.

### Example 154

### Production of 4-(4-cyanophenyl)-1-((5-hydroxy-2-pyridinyl)methyl)-2-isopr opyl-5-methyl-1H-pyrrole-3-carbonitrile

By using the compound (180 mg) produced in Example 132 and 6-(bromomethyl)-3-pyridinyl benzenesulfonate (260 mg, produced by a method described in *J. Org. Chem.,* 61, 813-815 (1996)), the reaction was carried out in the same manner as Example 2, whereby an alkylation reaction and a de-benzensulphonylation reaction proceeded simultaneously. Purification was carried out in the same manner as Example 2 to obtain the titled compound (130 mg) as colorless crystals.
¹H-NMR (CDCl₃) δ 1.37 (6H, d, *J*=7.0 Hz), 2.20 (3H, s), 3.08 (1H, septet, *J*=7.0 Hz), 5.17 (2H, s), 5.97 (1H, s), 6.60 (1H, d, *J*=8.4 Hz) , 7.16 (1H, dd, *J*=3.0, 8.4 Hz), 7.52 (2H, d, *J*=8.4 Hz), 7.71 (2H, d, *J*=8.4 Hz), 8.24 (1H, d, *J*=2.6 Hz).
IR (KBr): ν 3429, 2976, 2213, 1607 cm⁻¹.

### Example 155

### Production of 4-(3-cyanophenyl)-2,5-dimethyl-1H-pyrrole-3-carbonitrile

By using the compound (400 mg) produced in Reference Example 13 and 3-cyanophenylboronic acid (0.31 g), the reaction and purification were carried out in the same manner as Example 14 to obtain the titled compound (213 mg) as colorless crystals.
¹H-NMR (CDCl₃) δ 2.29 (3H, s), 2.44 (3H, s), 7.4-7.8 (4H, m), 8.24 (1H, s).
IR (KBr): ν 3287, 2928, 2215, 1593 cm⁻¹.

### Example 156

### Production of 4-(3-cyanophenyl)-2,5-dimethyl-1-(3-pyridinylmethyl)-1H-pyr role-3-carbonitrile

By using the compound (80 mg) produced in Example 155 and 3- (chloromethyl) pyridine hydrochloride (140 mg) , the reaction and purification were carried out in the same manner as Example 66 to obtain the titled compound (76 mg) as colorless crystals.
¹H-NMR (CDCl₃) δ 2.19 (3H, s), 2.39 (3H, s), 5.15 (2H, s), 7.1-7.4 (2H, m), 7.4-7.8 (4H, m), 8.37 (1H, s), 8.59 (1H, d, *J*=3.8 Hz).
IR (KBr): ν 3034, 2922, 2215, 1603 cm⁻¹.

### Example 157

### Production of 1-((6-chloro-3-pyridinyl)methyl)-4-(3-cyanophenyl)-2,5-dime thyl-1H-pyrrole-3-carbonitrile

By using the compound (80 mg) produced in Example 155 and 2-chloro-5-(chloromethyl)pyridine (90 mg), the reaction and purification were carried out in the same manner as Example 2 to obtain the titled compound (23 mg) as colorless crystals.
¹H-NMR (CDCl₃) δ 2.19 (3H, s), 2.39 (3H, s), 5.12 (2H, s), 7.15 (1H, dd, *J*=2.6, 8.4 Hz), 7.35 (1H, d, *J*=8.0 Hz), 7.5-7.8 (4H, m), 8.14 (1H, d, *J*=2.6 Hz).
IR (KBr): ν 3053, 2919, 2215, 1462 cm⁻¹.

### Example 158

### Production of 4-(4-formyl-1-naphthyl)-2,5-dimethyl-1H-pyrrole-3-carbonitr ile

By using the compound (410 mg) produced in Reference Example 13 and 4-formyl-1-naphthylboronic acid (450 mg), the reaction and purification were carried out in the same manner as Example 14 to obtain the titled compound (285 mg) as colorless crystals.
¹H-NMR (CDCl₃) δ 2.06 (3H, s), 2.50 (3H, s), 7.5-7.7 (2H, m), 7.6-7.8 (1H, m), 7.88 (1H, d, *J*=7.8 Hz), 8.03 (1H, d, *J*=7.8 Hz), 8.31 (1H, s), 9.34 (1H, d, *J*=8.4 Hz), 10.42 (1H, s).
IR (KBr): ν 3264, 2921, 2214, 1682, 1574 cm⁻¹.

### Example 159

### Production of 4-(4-cyano-1-naphthyl)-2,5-dimethyl-1H-pyrrole-3-carbonitri le

A solution of the compound (241 mg) obtained in Example 158, hydroxylamine hydrochloride (73 mg) in 1-methylpyrrolidine-2-one (1.5 ml) was heated at 110 °C for 2 hours with stirring. The reaction mixture was poured into saturated brine and extracted with ethyl acetate. The ethyl acetate layer was dried over magnesium sulfate and concentrated. The residue was purified by column chromatography (carrier: silicagel, eluant: hexane-ethyl acetate) and recrystallized from ethyl acetate-hexane to obtain the titled compound (188 mg) as colorless crystals.
¹H-NMR (CDCl₃) δ 2.05 (3H, s), 2.48 (3H, s), 7.4-8.0 (5H, m), 8.30 (1H, d, *J*=8.0 Hz), 8.37 (1H, s).
IR. (KBr): ν 3287, 3056, 2921, 2218, 1580 cm⁻¹.

### Example 160

### Production of 4-(4-cyano-1-naphthyl)-2,5-dimethyl-1-(3-pyridinylmethyl)-1 H-pyrrole-3-carbonitrile

By using the compound (140 mg) produced in Example 159 and 3- (chloromethyl)pyridine hydrochloride (180 mg) , the reaction and purification were carried out in the same manner as Example 66 to obtain the titled compound (97 mg) as colorless crystals.
¹H-NMR (CDCl₃) δ 1.98 (3H, s), 2.44 (3H, s), 5.20 (2H, s), 7.2-7.3 (1H, m), 7.3-7.4 (1H, m), 7.48 (1H, d *J*=7.2 Hz), 7.60 (1H, t, *J*=7.2 Hz), 7.71 (1H, t, *J*=7.2 Hz), 7.82 (1H, d, *J*=8.4 Hz), 7.96 (1H, d, *J*=7.5 Hz) 8.31 (1H, d, *J*=8.1 Hz), 8.39 (1H, d, *J*=1.5 Hz), 8. 61 (1H, dd, *J*=1.5, 3.0 Hz),.
IR (KBr): ν 3036, 2951, 2922, 2218, 1578 cm⁻¹.

### Example 161

### Production of methyl 1-(4-cyanobenzyl)-4-(3,4-dichlorophenyl)-2,5-dimethyl-1H-py rrole-3-carboxylate

By using the compound (200 mg) produced in Example 38 and 4-cyanobenzyl bromide (120 mg), the reaction and purification were carried out in the same manner as Example 2 to obtain the titled compound (95 mg) as colorless crystals.
¹H-NMR (CDCl₃) δ 2.00 (3H, s), 2.46 (3H, s), 3.63 (3H, s), 5.16 (2H, s), 7.0-7.2 (3H, m), 7.33 (1H, d, *J*=1.8 Hz), 7.41 (1H, d, *J*=8.4 Hz), 7.65 (2H, d, *J*=8.4 Hz).
IR (KBr): ν 2990, 2948, 2230, 1699 cm⁻¹.

### Example 162

### Production of 4-(4-cyanophenyl)-2,5-dimethyl-1-((6-methyl-3-pyridinyl)met hyl)-1H-pyrrole-3-carbonitrile

A solution of the compound (420 mg) produced in Example 105 the compound (470 mg) produced in Reference Example 18 and (tributylphosphoranylidene)acetonitrile (920 mg) in toluene (10 ml) was stirred for 20 hours under reflux. The reaction mixture was poured into saturated brine and extracted with ethyl acetate. The ethyl acetate layer was dried over magnesium sulfate and concentrated. The residue was purified by column chromatography (carrier: silicagel, eluant: hexane-ethyl acetate) and recrystallized from ethyl acetate-hexane to obtain the titled compound (266 mg) as colorless crystals.
¹H-NMR (CDCl₃) δ 2.21 (3H, s), 2.39 (3H, s), 2.56 (3H, s), 5. 10 (2H, s), 7.07 (1H, dd, *J*=2.6, 8.0 Hz), 7.16 (1H, d, *J*=8.0 Hz), 7.51 (2H, d, *J*=8.4 Hz), 7.71 (2H, d, *J*=8.4 Hz), 8.23 (1H, d, *J*=2.6 Hz).
IR (KBr): ν 3052, 2922, 2215, 1607 cm⁻¹.

### Example 163

### Production of methyl 5-((3-cyano-4-(4-cyanophenyl)-2,5-dimethyl-1H-pyrrol-1-yl)m ethyl)-2-pyridine carboxylate

By using the compound (1.62 g) produced in Example 105 and methyl 5-(bromomethyl)pyridine-2-carboxylate (1.68 g, produced from the compound of Reference Example 15 by a method described in *J*. *Org*. *Chem*., *65, 7718-7722 (2000)*), the reaction and purification were carried out in the same manner as Example 2 to obtain the titled compound (721 mg) as colorless crystals.
¹H-NMR (CDCl₃) δ 2.20 (3H, s), 2.39 (3H, s), 4.02 (3H, s), 5.22 (2H, s), 7.29 (1H, dd, *J*=2.1, 7.8 Hz), 7.50 (2H, d, *J*=7.8 Hz), 7.71 (2H, d, *J*=7.8 Hz), 8.13 (1H, d, *J*=7.8 Hz), 7.49 (2H, d, *J*=1.5 Hz).
IR (KBr): ν 3054, 2951, 2216, 1726, 1609 cm⁻¹.

### Example 164

### Production of 4-(4-cyanophenyl)-1-((6-(1-hydroxy-1-methyl ethyl)-3-pyridinyl)methyl)-2,5-dimethyl-1H-pyrrole-3-carbon itrile

By using the compound (130 mg) produced in Example 163, the reaction and purification were carried out in the same manner as Example 146 to obtain the titled compound (54 mg) as colorless crystals.
¹H-NMR (CDCl₃) δ 1.54 (6H, s), 2.22 (3H, s), 2.40 (3H, s), 4.52 (1H, s), 5.14 (2H, s), 7.22 (1H, dd, *J*=1.8, 8.4 Hz), 7.40 (1H, d, *J*=8.4 Hz), 7.51 (2H, d, *J*=8.1 Hz), 7.71 (2H, d, *J*=8.1 Hz), 8.21 (1H, d, *J*=8.1 Hz).
IR (KBr): ν 3364, 2975, 2924, 2216, 1607 cm⁻¹.

### Example 165

### Production of tert-butyl 5-((3-cyano-4-(4-cyanophenyl)-2,5-dimethyl-1H-pyrrol-1-yl)m ethyl)-2-pyridinylcarbamate

By using the compound (350 mg) produced in Example 105 and tert-butyl 5-(bromomethyl)pyridin-2-ylcarbamate (820 mg, produced from the compound of Reference Example 16 by a method described in *J. Org. Chem., 65, 7718-7722 (2000)),* the reaction and purification were carried out in the same manner as Example 2 to obtain the titled compound (223 mg) as colorless crystals.
¹H-NMR (CDCl₃) δ 1.52 (9H, s), 2.21 (3H, s), 2.40 (3H, s), 5.05 (2H, s), 7.2-7.3 (1H, m), 7.50 (2H, d, *J*=8.4 Hz), 7.70 (2H, d, *J*=8.4 Hz), 7.89 (1H, d, *J*=1.8 Hz), 7.94 (1H, d, *J*=8.7 Hz).
IR (KBr): ν 3171, 2980, 2216, 1728, 1607 cm⁻¹.

### Example 166

### Production of 4-(4-cyanophenyl)-2,5-dimethyl-1-((1-oxide-3-pyridinyl)meth yl)-1H-pyrrole-3-carbonitrile

A mixture of the compound (103 mg) produced in Example 107, m-chloroperbenzoic acid (140 mg) and dichloromethane (3 ml) was stirred at room temperature for 14 hours. The reaction mixture was poured into saturated brine and extracted with ethyl acetate. The ethyl acetate layer was dried over magnesium sulfate and concentrated. The residue was purified by column chromatography (carrier: silicagel, eluant: hexane-ethyl acetate) and recrystallized from ethyl acetate-hexane to obtain the titled compound (30 mg) as colorless crystals.
¹H-NMR (CDCl₃) δ 2.22 (3H, s), 2.40 (3H, s), 5.09 (2H, s), 6.86 (1H, d, *J*=7.8 Hz), 7.2-7.4 (1H, m), 7.51 (2H, d, *J*=8.4 Hz), 7.73 (2H, d, *J*=8.4 Hz), 7.84 (1H, s), 8.17 (1H, d, *J*=5.7 Hz).
IR (KBr): ν 3040, 2953, 2216, 1609, 1541 cm⁻¹.

### Example 167

### Production of 5-((3-cyano-4-(4-cyanophenyl)-2,5-dimethyl-1H-pyrrol-1-yl)m ethyl)-2-pyridine carboxylic acid

A solution of the compound (544 mg) produced in Example 163 and 1N sodium hydroxide solution (3 ml) in ethanol (3 ml) was stirred at room temperature for 2 hours. The reaction mixture was poured into saturated brine, neutralized with hydrochloric acid and extracted with ethyl acetate. The ethyl acetate layer was dried over magnesium sulfate and concentrated. The residue was purified by column chromatography (carrier: silicagel, eluant: hexane-ethyl acetate) and recrystallized from ethyl acetate-hexane to obtain the titled compound (467 mg) as colorless crystals.
¹H-NMR (CDCl₃) δ 2.22 (3H, s), 2.40 (3H, s), 5.26 (2H, s), 7.4-7.6 (3H, m), 7.73 (2H, d, *J*=8.1 Hz), 8.26 (1H, d, *J*=8.1 Hz), 8.33 (1H, s).
IR (KBr): ν 3400, 3067, 2924, 2222, 1728, 1609 cm⁻¹.

### Example 168

### Production of 1-((6-amino-3-pyridinyl)methyl)-4-(4-cyanophenyl)-2,5-dimet hyl-1H-pyrrole-3-carbonitrile

A solution of the compound (192 mg) produced in Example 165 in trifluoroacetic acid (1 ml) was stirred at room temperature for 0.5 hour and concentrated. The residue was poured into saturated brine, neutralized with aqueous solution of sodium hydrogencarbonate and extracted with ethyl acetate. The ethyl acetate layer was dried over magnesium sulfate and concentrated. The residue was purified by column chromatography (carrier: silicagel, eluant: hexane-ethyl acetate) and recrystallized from ethyl acetate-hexane to obtain the titled compound (125 mg) as colorless crystals.
¹H-NMR (CDCl₃) δ 2.23 (3H, s), 2.41 (3H, s), 4.51 (2H, brs), 4.98 (2H, s), 6.49 (2H, d, *J*=8.4 Hz), 7.50 (2H, d, *J*=8.4 Hz), 7.70 (2H, d, *J*=8.4 Hz), 7.80 (1H, s).
IR (KBr): ν 3366, 2922, 2214, 1609, 1505 cm⁻¹.

### Example 169

### Production of 5-((3-cyano-4-(4-cyanophenyl)-2,5-dimethyl-1H-pyrrol-1-yl)m ethyl)-2-pyridine carboxamide

By using the compound (107 mg) produced in Example 167 and a 25% aqueous solution of ammonia (2 ml), the reaction and purification were carried out in the same manner as Example 92 to obtain the titled compound (114 mg) as colorless crystals.
¹H-NMR (CDCl₃) δ 2.21 (3H, s), 2.40 (3H, s), 5.21 (2H, s), 5.59 (1H, brs), 7.42 (1H, d, *J*=8.4 Hz), 7.52 (2H, d, *J*=8.0Hz), 7.6-7.8 (3H, m), 8.2-8.3 (2H, m).
IR (KBr): ν 3166, 2953, 2216, 1682, 1609 cm⁻¹.

### Example 170

### Production of 5-((3-cyano-4-(4-cyanophenyl)-2,5-dimethyl-1H-pyrrol-1-yl)m ethyl)-N-methyl-2-pyridine carboxamide

By using the compound (107 mg) produced in Example 167 and an aqueous solution of methylamine (2 ml), the reaction and purification were carried out in the same manner as Example 92 to obtain the titled compound (68 mg) as colorless crystals.
¹H-NMR (CDCl₃) δ 2.21 (3H, s), 2.40 (3H, s), 3.04 (3H, d, *J*=5.2 Hz), 5.20 (2H, s), 7.41 (1H, d, *J*=8.4 Hz), 7.52 (2H, d, *J*=8.8 Hz), 7.72 (2H, d, *J*=8.8 Hz), 7.94 (1H, s), 8.2-8.3 (2H, m).
IR (KBr): ν 3389, 3050, 2953, 2216, 1674 cm⁻¹.

### Example 171

### Production of 5-((3-cyano-4-(4-cyanophenyl)-2,5-dimethyl-1H-pyrrol-1-yl)m ethyl)-N,N-dimethyl-2-pyridine carboxamide

By using the compound (107 mg) produced in Example 167 and an aqueous solution of dimethylamine (2 ml), the reaction and purification were carried out in the same manner as Example 92 to obtain the titled compound (69 mg) as colorless crystals.
¹H-NMR (CDCl₃) δ 2.21 (3H, s), 2.40 (3H, s), 3.11 (3H, s), 3.15 (3H, s), 5.18 (2H, s), 7.2-7.4 (1H, m), 7.52 (2H, d, *J*=8.6 Hz), 7.6-7.8 (3H, m), 8.29 (1H, d, *J*=1.6 Hz).
IR (KBr): ν 2930, 2216, 1634, 1609, 1541 cm⁻¹.

### Example 172

### Production of N-(5-((3-cyano-4-(4-cyanophenyl)-2,5-dimethyl-1H-pyrrol-1-y l)methyl)-2-pyridinyl)acetamide

To a mixture of the compound (81 mg) produced in Example 168, triethylamine(0.083 ml) and tetrahydrofuran (1 ml) was added acetyl chloride (0.021 ml) at room temperature. The reaction mixture was stirred at room temperature for 0.5 hour. The residue was poured into saturated brine, neutralized with aqueous solution of sodium bicarbonate and extracted with ethyl acetate. The ethyl acetate layer was dried over magnesium sulfate and concentrated. The residue was purified by column chromatography (carrier: silicagel, eluant: hexane-ethyl acetate) and recrystallized from ethyl acetate-hexane to obtain the titled compound (57 mg) as colorless crystals.
¹H-NMR (CDCl₃) δ 2.22 (6H, s), 2.40 (3H, s), 5.09 (2H, s), 7.2-7.4 (1H, m), 7.51 (2H, d, *J*=8.4 Hz), 7.71 (2H, d, *J*=8.4 Hz), 7.91 (1H, d, *J*=2.0 Hz), 7.99 (1H, s), 8.21 (1H, d, *J*=8.4 Hz).
IR (KBr): ν 3327, 3100, 2986, 2214, 1689 cm⁻¹.

### Example 173

### Production of 5-((3-cyano-4-(4-cyanophenyl)-2,5-dimethyl-1H-pyrrol-1-yl)m ethyl)-2-pyridine carbonitrile

By using the compound (81 mg) produced in Example 169, the reaction and purification were carried out in the same manner as Example 105 to obtain the titled compound (54 mg) as colorless crystals.
¹H-NMR (CDCl₃) δ 2.20 (3H, s), 2.38 (3H, s), 5.22 (2H, s), 7.29 (1H, dd, *J*=2.1, 8.1 Hz), 7.50 (2H, d, *J*=8.7 Hz), 7.6-7.8 (3H, m), 8.47 (1H, d, *J*=2.1 Hz).
IR (KBr): ν 3065, 2988, 2216, 1609 cm⁻¹.

### Example 174

### Production of 1-((6-bromo-3-pyridinyl)methyl)-4-(4-cyanophenyl)-2,5-dimet hyl-1H-pyrrole-3-carbonitrile

By using the compound (2.11 g) produced in Example 105 and 2-bromo-5- (bromomethyl) pyridine (2.38 g, produced by a method described in *J*. *Org*. *Chem*., 61, 813-815 (1996)), the reaction and purification were carried out in the same manner as Example 2 to obtain the titled compound (2.54 g) as colorless crystals.
¹H-NMR (CDCl₃) δ 2.20 (3H, s), 2.39 (3H, s) , 5.09 (2H, s) , 7.04 (1H, dd, *J*=2.7, 8.4 Hz), 7.4-7.6 (3H, m), 7.71 (2H, d, *J*=8.1 Hz), 8.10 (1H, d, *J*=2.7 Hz).
IR (KBr): ν 3053, 2949, 2920, 2215, 1609 cm⁻¹.

### Example 175

### Production of 4-{[3-cyano-4-(4-cyanophenyl)-2,5-dimethyl-1H-pyrrol-1-yl]m ethyl}benzoic acid

By using the compound (880 mg) produced in Example 105 and 4-(bromomethyl)benzoic acid (920 mg), the reaction and purification were carried out in the same manner as Example 2 to obtain the titled compound (720 mg) as colorless crystals.
¹H-NMR (DMSO-d₆) δ 2.15 (3H, s), 2.31 (3H, s), 5.34 (2H, s), 7.10 (2H, d, *J*=7.8 Hz), 7.56 (2H, d, *J*=7.8 Hz), 7.8-8.0 (4H, m).

### Example 176

### Production of 4-((3-cyano-4-(4-cyanophenyl)-2,5-dimethyl-1H-pyrrol-1-yl)m ethyl)-N,N-dimethylbenzamide

By using the compound (100 mg) produced in Example 175 and 50% aqueous solution of dimethylamine (2 ml), the reaction and purification were carried out in the same manner as Example 92 to obtain the titled compound (43 mg) as colorless crystals.
¹H-NMR (CDCl₃) δ 2.20 (3H, s), 2.38 (3H, s), 2.98 (3H, s), 3.11 (3H, s), 5.14 (2H, s), 6.96 (2H, d, *J*=8.0 Hz), 7.43 (2H, d, *J*=8.0 Hz), 7.53 (2H, d, *J*=8.4 Hz), 7.72 (2H, d, *J*=8.4 Hz).
IR (KBr): ν 2932, 2214, 1633, 1608, 1398 cm⁻¹.

### Example 177

### Production of 4-(4-cyanophenyl)-2,5-dimethyl-1-(4-(1-pyrrolidinylcarbonyl )benzyl)-1H-pyrrole-3-carbonitrile

By using the compound (100 mg) produced in Example 175 and pyrrolidine (0.3 ml), the reaction and purification were carried out in the same manner as Example 92 to obtain the titled compound (34 mg) as colorless crystals.
¹H-NMR (CDCl₃) δ 1.8-2.1 (4H, m), 2.19 (3H, s), 2.38 (3H, s), 3.3-3.5 (2H, m), 3.6-3.8 (2H, m), 5.13 (2H, s), 6.96 (2H, d, *J*=8.6 Hz), 7.53 (4H, d, *J*=8.4 Hz), 7.71 (2H, d, *J*=8.6 Hz).
IR (KBr): ν 2978, 2926, 2880, 2224, 1607, 1427 cm⁻¹.

### Example 178

### Production of methyl 4-(1,1'-biphenyl-4-yl)-2,5-dimethyl-1H-pyrrole-3-carboxylat e

By using the compound (0.23 g) produced in Reference Example 2 and 1,1' -biphenyl-4-ylboronic acid (0.20 g), the reaction and purification were carried out in the same manner as Example 14 to obtain the titled compound (186 mg) as pale yellow crystals.
¹H-NMR (CDCl₃) δ 2.16 (3H, s), 2.52 (3H, s), 3.64 (3H, s), 7.2-7.4 (3H, m), 7.4-7.5 (2H, m), 7.5-7.7 (4H, m), 8.04 (1H, s).
IR (KBr): ν 3303, 3027, 2948, 1682 cm⁻¹.

### Example 179

### Production of tert-butyl 2,5-dimethyl-4-(4-nitrophenyl)-1H-pyrrole-3-carboxylate

By using the compound (8.00 g) produced in Reference Example 1 and tert-butyl acetoacetate (14.7 ml), the reaction and purification were carried out in the same manner as Example 1 to obtain the titled compound (1.26 g) as yellow crystals.
IR (KBr): ν 3285, 1659, 1514, 1343, 1159, 1101, 854 cm⁻¹.
¹H-NMR (CDCl₃) δ: 1.32 (9H, s), 2.13 (3H, s), 2.51 (3H, s), 7.39 (2H, d, *J*=8.7 Hz), 7.99 (1H, br), 8.21 (2H, d, *J*=8.7 Hz).

### Example 180

### Production of tert-butyl 1-benzyl-2,5-dimethyl-4-(4-nitrophenyl)-1H-pyrrole-3-carbox ylate

By using the compound (0.19 g) produced in Example 179 and benzyl bromide (0.09 ml), the reaction and purification were carried out in the same manner as Example 2 to obtain the titled compound (0.16 g) as yellow crystals.
IR (KBr): ν 2976, 1694, 1597, 1514, 1343, 1152, 855, 731 cm⁻¹.
¹H-NMR (CDCl₃) δ: 1.28 (9H, s) , 2.03 (3H, s), 2.49 (3H, s) , 5. 11 (2H, s), 6.95-6.98 (2H, m) , 7.26-7. 44 (5H, m) , 8.20-8.24 (2H, m).

### Example 181

### Production of 2,5-dimethyl-3-(4-nitrophenyl)-1H-pyrrole

The compound (0.80 g) produced in Example 179 was dissolved in TFA (7 ml), and the solution was stirred at room temperature for 7 hours and concentrated. The residue was purified by column chromatography (carrier: silicagel, eluant: hexane-ethyl acetate) to obtain the titled compound (0.49 g) as a pale brown powder.
IR (KBr): ν 3358, 1593, 1499, 1323, 1113, 853, 775, 758 cm⁻¹.
¹H-NMR (CDCl₃) δ: 2.27 (3H, s), 2.43 (3H, s), 6.07-6.08 (1H, m), 7.46-7.53 (2H, m), 7.87 (1H, br), 8.16-8.23 (2H, m).

### Example 182

### Production of 1-benzyl-2,5-dimethyl-3-(4-nitrophenyl)-1H-pyrrole and 3-benzyl-2,5-dimethyl-4-(4-nitrophenyl)-1H-pyrrole

By using the compound (0.39 g) produced in Example 181 and benzyl bromide (0.26 ml), the reaction and purification were carried out in the same manner as Example 2 to obtain 1-benzyl-2,5-dimethyl-3-(4-nitrophenyl)-1H-pyrrole (0.10 g) as yellow crystals and 3-benzyl-2,5-dimethyl-4-(4-nitrophenyl)-1H-pyrrole (0.01 g) as yellow crystals. 1-benzyl-2,5-dimethyl-3-(4-nitrophenyl)-1H-pyrrole
IR (KBr): ν 1593, 1507, 1343, 1327 cm⁻¹.
¹H-NMR (CDCl₃) δ: 2.21 (3H, s), 2.31 (3H, s), 5.09 (2H, s), 6. 17 (1H, s), 6.94 (2H, d, J=7.2 Hz), 7.24-7.36 (3H, m), 7.49-7.54 (2H, m), 8.19-8.23 (2H, m). 3-benzyl-2,5-dimethyl-4-(4-nitrophenyl)-1H-pyrrole
IR (KBr): ν 3407, 1593, 1507, 1345, 856 cm⁻¹.
¹H-NMR (CDCl₃) δ: 2.18 (3H, s) , 2.28 (3H, s), 3.79 (2H, s), 7.03-7.27 (7H, m), 7.77 (1H, br), 8.10-8.15 (2H, m).

### Example 183

### Production of methyl 2,5-dimethyl-4-(4-nitrophenyl)-1-phenyl-1H-pyrrole-3-carbox ylate

Amixture of the compound (0.40 g) produced in Reference Example 1, methyl acetoacetate (0.43 ml) , aniline (0.20 ml) andmethanol (7 ml) was stirred at room temperature for 18 hours and at 70 °C for 5 hours and concentrated. The residue was purified by column chromatography (carrier: silicagel, eluant: hexane-ethyl acetate) to obtain the titled compound (0.05 g) as yellow crystals.
¹H-NMR (CDCl₃) δ: 1.90 (3H, s), 2.32 (3H, s), 3.65 (3H, s), 7.23-7.26 (2H, m), 7.43-7.58 (5H, m), 8.23 (2H, d, *J*=8.7 Hz).

### Example 184

### Production of methyl 1-benzyl-2,5-dimethyl-4-(4-nitrophenyl)-1H-pyrrole-3-carbox ylate

By using the compound (4.64 g) produced in Reference Example 1, methyl acetoacetate (5.18 g), benzyl amine (2.63 g) and methanol (75 ml), the reaction and purification were carried out in the same manner as Example 183 to obtain the titled compound (1.40 g) as yellow crystals.
IR (KBr): ν 1699, 1514, 1341 cm⁻¹.
¹H-NMR (CDCl₃) δ: 2.05 (3H, s), 2.51 (3H, s), 3.62 (3H, s), 5.13 (2H, s), 6.94-6.96 (2H, m) , 7.29-7.43 (5H, m) , 8.22 (2H, d, *J*=8.7 Hz).

### Example 185

### Production of 1-(1-benzyl-2,5-dimethyl-4-(4-nitrophenyl)-1H-pyrrol-3-yl)e thanone

A mixture of the compound (0.42 g) obtained in Reference Example 1, 2,4-pentandione (0.41 ml), benzylamine (0.24 ml) and dimethylformamide (10 ml) was stirred at room temperature for 18 hours and at 80 °C for 24 hours, poured into water and extracted with ethyl acetate. The ethyl acetate layer was washed with water and saturated brine, successively, dried over magnesium sulfate and concentrated. The residue was purified by column chromatography (carrier: silicagel, eluant: hexane-ethyl acetate) to obtain the titled compound (0.12 g) as yellow crystals.
IR (KBr): ν 1651, 1597, 1516, 1348 cm⁻¹.
¹H-NMR (CDCl₃) δ: 1.98 (3H, s), 2.04 (3H, s), 2.46 (3H, s), 5.13 (2H, s), 6.97 (2H, d, *J*=7.2 Hz), 7.30-7.45 (5H, m), 8.26 (2H, d, *J*=9.0 Hz).

### Example 186

### Production of 1-(1-benzyl-2,5-dimethyl-4-(4-nitrophenyl)-1H-pyrrol-3-yl)e thanol

The compound (0.14 g) produced in Example 185 was dissolved in methanol (3 ml) and tetrahydrofuran (1 ml) and to the solution was added sodium boron hydride (0.28 g). The reaction mixture was stirred at room temperature for 16 hours and concentrated. The residue was diluted with water and extracted with ethyl acetate. The ethyl acetate layer was dried over magnesium sulfate and concentrated. The residue was purified by column chromatography (carrier: silicagel, eluant: hexane-ethyl acetate) and recrystallized from ethyl acetate-hexane to obtain the titled compound (0.02 g) as pale brown solids.
IR (KBr): ν 1595, 1514, 1345, 733 cm⁻¹.
¹H-NMR (CDCl₃) δ: 1.42 (3H, d, *J*=7.0 Hz), 2.09 (3H, s), 2.30 (3H, s), 4.96 (1H, q, *J*=7.0 Hz), 5.08 (2H, s), 6.95 (2H, d, *J*=7.4 Hz), 7.26-7.53 (5H, m), 8.23 (2H, d, *J*=8.8 Hz).

### Example 187

### Production of N,1-dibenzyl-2,5-dimethyl-4-(4-nitrophenyl)-1H-pyrrole-3-ca rboxamide

To a solution of the compound (45 mg) produced in Example 47, benzyl amine (0.028 ml) and triethylamine (0.028 ml) in dimethylformamide (0.5 ml) was added cyanide diethylphosphate (0.024 ml) and the reaction mixture was stirred at room temperature for 4 hours. The reaction mixture was poured into water and extracted with ethyl acetate. The ethyl acetate layer was washed with 1N hydrochloric acid, water and saturated sodium bicarbonate water, successively, dried over magnesium sulfate, and concentrated. The residue was purified by column chromatography (carrier: silicagel, eluant: hexane-ethyl. acetate) to obtain the titled compound (31 mg) as yellow crystals.
¹H-NMR (CDCl₃) δ: 2.07 (3H, s), 2.48 (3H, s), 4.38 (2H, d, *J*=5.7 Hz), 5.11 (2H, s), 5.18 (1H, brm), 6.96 (2H, d, *J*=6.6 Hz), 7.02-7.05 (2H, m), 7.24-7.37 (8H, m), 8.05 (2H, d, *J*=9.0 Hz).

### Example 188

### Production of 1-benzyl-N,2,5-trimethyl-4-(4-nitrophenyl)-1H-pyrrole-3-car boxamide

By using the compound (105 mg) produced in Example 47 as a starting material and a 2M solution of methylamine in tetrahydrofuran (0.5 ml) as an amine component, the reaction and purification were carried out in the same manner as Example 187 to obtain the titled compound (9 mg) as yellow crystals.
IR (KBr): ν 1595, 1508, 1339 cm⁻¹.
¹H-NMR (CDCl₃) δ: 2.11 (3H, s), 2.45 (3H, s), 2.75 (3H, d, *J*=5.1 Hz), 4.99 (1H, br), 5.11 (2H, s), 6.96 (2H, d, J=6.6 Hz), 7.26-7.48 (5H, m), 8.26 (2H, d, J=9.0 Hz).

### Example 189

### Production of 1-benzyl-N,N,2,5-tetramethyl-4-(4-nitrophenyl)-1H-pyrrole-3 - carboxamide

By using the compound (105 mg) produced in Example 47 as a starting material and a 2M solution of dimethylamine in tetrahydrofuran (0.5 ml) as an amine component, the reaction and purification were carried out in the same manner as Example 187 to obtain the titled compound (71 mg) as yellow crystals.
IR (KBr): ν 1620, 1595, 1510, 1339 cm⁻¹.
¹H-NMR (CDCl₃) δ: 2.22 (6H, s), 2.61 (3H, s), 2.95 (3H, s), 5.09 (2H, s), 6.97 (2H, d, *J*=6.6 Hz), 7.25-7.42 (5H, m), 8.19 (2H, d, *J*=9.0 Hz).

### Example 190

### Production of 1-benzyl-N-(2-(4-hydroxyphenyl)ethyl)-2,5-dimethyl-4-(4-nit rophenyl)-1H-pyrrole-3-carboxamide

By using the compound (105 mg) produced in Example 47 as a starting material and 2- (4-hydroxyphenyl) ethylamine (82 mg) as an amine component, the reaction and purification were carried out in the same manner as Example 187 to obtain the titled compound (69 mg) as yellow crystals.
IR (KBr): ν 1595, 1514, 1339 cm⁻¹.
¹H-NMR (CDCl₃) δ: 2.04 (3H, s), 2.43 (3H, s), 2.58 (2H, t, *J*=6.6 Hz), 3.49 (2H, q, *J*=6.6 Hz), 4.97-5.01 (2H, m), 5.08 (2H, s), 6.63 (2H, d, *J*=8.4 Hz), 6.78 (2H, d, *J*=8.4 Hz), 6.94 (2H, d, *J*=7.2 Hz), 7.26-7.35 (5H, m), 8.10 (2H, d, *J*=9.0 Hz).

### Example 191

### Production of 1-benzyl-2,5-dimethyl-4-(4-nitrophenyl)-1H-pyrrole-3-carbox amide

By using the compound (105 mg) produced in Example 47, ammonium chloride (54 mg), triethylamine (0.21 ml) and cyanide diethylphosphate (0.055 ml), the reaction and purification were carried out in the same manner as Example 187 to obtain the titled compound (36 mg) as yellow crystals.
IR (KBr): ν 1653, 1595, 1508, 1339 cm⁻¹.
¹H-NMR (CDCl₃) δ: 2.08 (3H, s), 2.49 (3H, s), 4.85-5.25 (2H, br), 5.12 (2H, s), 6.96 (2H, d, *J*=6.9 Hz), 7.27-7.37 (3H, m), 7.50 (2H, d, *J*=8.7 Hz), 8.25 (2H, d, J=8.7 Hz).

### Example 192

### Production of 1-benzyl-N-butyl-2,5-dimethyl-4-(4-nitrophenyl)-1H-pyrrole-3-carboxamide

By using the compound (105 mg) produced in Example 47 as a starting material and n-butylamine (0.060 ml) as an amine component, the reaction and purification were carried out in the same manner as Example 187 to obtain the titled compound (55 mg) as yellow crystals.
IR (KBr): ν 1638, 1595, 1508, 1339 cm⁻¹.
¹H-NMR (CDCl₃) δ: 0.81 (3H, t, *J*=6.6 Hz), 1. 09 (2H, sextet, *J*=6.6 Hz), 1.27 (2H, quint, *J*=6.6 Hz), 2.10 (3H, s), 2.45 (3H, s), 3.21 (2H, q, *J*=6.6 Hz), 5.00 (1H, brm), 5.11 (2H, s), 6.97 (2H, d, *J*=6.6 Hz), 7.26-7.50 (5H, m), 8.25 (2H, d, *J*=9.0 Hz).

### Example 193

### Production of 1-benzyl-2,5-dimethyl-4-(4-nitrophenyl)-N-undecyl-1H-pyrrol e-3-carboxamide

By using the compound (105 mg) produced in Example 47 as a starting material and n-undecyl amine (0.13 ml) as an amine component, the reaction and purification were carried out in the same manner as Example 187 to obtain the titled compound (83 mg) as yellow crystals.
IR (KBr): ν 1638, 1595, 1514, 1339 cm⁻¹.
¹H-NMR (CDCl₃) δ: 0.88 (3H, t, *J*=6.6 Hz), 1.04-1.32 (18H, m), 2.10 (3H, s), 2.45 (3H, s), 3.20 (2H, q, *J*=6.6 Hz), 5.00 (1H, brm), 5.11 (2H, s), 6.97 (2H, d, *J*=6.6 Hz), 7.26-7.50 (5H, m), 8.25 (2H, d, *J*=9.0 Hz).

### Example 194

### Production of tert-butyl 6-(((1-benzyl-2,5-dimethyl-4-(4-nitrophenyl)-1H-pyrrol-3-yl )carbonyl)amino)hexylcarbamate

By using the compound (105 mg) produced in Example 47 as a starting material and tert-butyl N-(6-aminohexyl)carbamate (130 mg) as an amine component, the reaction and purification were carried out in the same manner as Example 187 to obtain the titled compound (77 mg) as yellow crystals.
IR (KBr): ν 1696, 1638, 1595, 1514, 1339 cm⁻¹.
¹H-NMR (CDCl₃) δ: 1.04-1.43 (8H, m), 1.44 (9H, s), 2.10 (3H, s), 2.45 (3H, s), 3.05 (2H, q, *J*=6.6 Hz), 3.20 (2H, q, *J*=6.6 Hz), 4.55 (1H, br), 5.03 (1H, br), 5.11 (2H, s), 6.97 (2H, d, *J*=7.2 Hz), 7.25-7.50 (5H, m), 8.25 (2H, d, *J*=9.0 Hz).

### Example 195

### Production of 4-((1-benzyl-2,5-dimethyl-4-(4-nitrophenyl)-1H-pyrrol-3-yl) carbonyl)morpholine

By using the compound (105 mg) produced in Example 47 as a starting material and morpholine (0.052 ml) as an amine component, the reaction and purification were carried out in the same manner as Example 187 to obtain the titled compound (59 mg) as yellow crystals.
IR (KBr): ν 1622, 1595, 1512, 1339, 1113, 733 cm⁻¹.
¹H-NMR (CDCl₃) δ: 2:21 (3H, s), 2.24 (3H, s), 2.60-2.80 (8H, brm), 5.09 (2H, s), 6.93 (2H, d, J=6.6 Hz), 7.25-7.43 (5H, m), 8.22 (2H, d, J=9.0 Hz).

### Example 196

### Production of 1-benzyl-2,5-dimethyl-4-(4-nitrophenyl)-N-(2-(1-piperidinyl )ethyl)-1H-pyrrole-3-carboxamide

To a solution of the compound (105 mg) produced in Example 47, 1-(2-aminoethyl)piperidine (0.086 ml) and triethylamine (0.063 ml) in dimethylformamide (1 ml) was added cyanide diethylphosphate (0.055 ml) and the reaction mixture was stirred at room temperature for 16 hours. The reaction mixture was poured into saturated sodium bicarbonate water and extracted with ethyl acetate. The ethyl acetate layer was dried over magnesium sulfate and concentrated. The residue was purified by column chromatography (carrier: Chromatorex NH-silica gel, produced by Fuji Silysia Chemical Lyd., eluant: hexane-ethyl acetate) to obtain the titled compound (67 mg) as yellow crystals.
IR (KBr): ν 1638, 1595, 1541, 1512, 1339 cm⁻¹.
¹H-NMR (CDCl₃ δ: 1.26-1.29 (6H, br), 2.04-2.07 (4H, br), 2.11 (3H, s), 2.19 (3H, t, *J*=6.0 Hz), 2.46 (3H, s), 3.31 (2H, q, *J*=6.0 Hz), 5.11 (2H, s), 5.74 (1H, brm), 6.99 (2H, d, *J*=7.2 Hz), 7.26-7.50 (5H, m), 8.24 (2H, d, J=9.0 Hz).

### Example 197

### Production of tert-butyl 1-(2,4-difluorobenzyl)-2,5-dimethyl-4-(4-nitrophenyl)-1H-py rrole-3-carboxylate

By using the compound (4.16 g) produced in Reference Example 1, tert-butyl acetoacetate (6.33 g) and 2,4-difluorobenzyl amine (3.15 g) , the reaction and purification were carried out in the same manner as Example 185 to obtain the titled compound (1.33 g) as yellow crystals.
IR (KBr): ν 1694, 1514, 1436, 1152 cm⁻¹.
¹H-NMR (CDCl₃) δ: 1.28 (9H, s), 2.02 (3H, s), 2.48 (3H, s), 5.09 (2H, s), 6.46-6.58 (1H, m), 6.78-6.93 (2H, m) , 7.40 (2H, d, *J*=9.0 Hz), 8.22 (2H, d, *J*=9.0 Hz).

### Example 198

### Production of 1-(2,4-difluorobenzyl)-2,5-dimethyl-3-(4-nitrophewyl)-1H-py rrole

By using the compound (0.44 g) produced in Example 197, the reaction and purification were carried out in the same manner as Example 181 to obtain the titled compound (0.24 g) as yellow crystals.
IR (KBr): ν 1593, 1507, 1341 cm⁻¹.
¹H-NMR (CDCl₃) δ: 2.20 (3H, s), 2.30 (3H, s), 5.08 (2H, s), 6.17 (1H, s), 6.39-6.50 (1H, m), 6.75-6.92 (2H, m), 7.48-7.54 (2H, m), 8.18-8.25 (2H, m).

### Example 199

### Production of tert-butyl 4-(3,4-dichlorophenyl)-1-(2,4-difluorobenzyl)-2,5-dimethyl-1H-pyrrole-3-carboxylate

By using the compound (1.16 g) produced in Reference Example 19, tert-butyl acetoacetate (1.66 ml) and 2,4-difluorobenzyl amine (0. 65 ml), the reaction and purification were carried out in the same manner as Example 185 to obtain the titled compound (0.16 g) as colorless crystals.
IR (KBr): ν 1690, 1155 cm⁻¹.
¹H-NMR (CDCl₃) δ: 1.30 (9H, s), 2.00 (3H, s), 2.46 (3H, s), 5.07 (2H, s), 6.45-6.56 (1H, m), 6.77-6.92 (2H, m), 7.0.7 (1H, dd, *J*=8.2, 2.0 Hz), 7.34 (1H, d, *J*=2.0 Hz), 7.41 (1H, d, *J*=8.2 Hz).

### Example 200

### Production of ethyl 4-(3-cyanophenyl)-1-(2,4-difluorobenzyl)-2,5-dimethyl-1H-py rrole-3-carboxylate

By using the compound (4.14 g) produced in Reference Example 20, ethyl acetoacetate (5.72 g) and 2,4-difluorobenzyl amine (3.43 g) , the reaction and purification were carried out in the same manner as Example 185 to obtain the titled compound (2.18 g) as colorless crystals.
IR (KBr): ν 2228, 1694, 1505, 1416, 1296, 1202, 1142, 1092, 966 cm⁻¹.
¹H-NMR (CDCl₃) δ: 1.03 (3H, t, *J*=7.0 Hz), 2.01 (3H, s), 2.50 (3H, s), 4.07 (2H, q, *J*=7.0 Hz), 5.10 (2H, s), 6.42-6.54 (1H, m), 6.79-6.93 (2H, m), 7.39-7.58 (4H, m).

### Example 201

### Production of ethyl 4-(3-cyanophenyl)-1-(4-fluorobenzyl)-5-methyl-2-(trifluorom ethyl)-1H-pyrrole-3-carboxylate and ethyl 4-(3-cyanophenyl)-5-methyl-2-(trifluoromethyl)-1H-pyrrole-3 - carboxylate

By using the compound (1.13 g) produced in Reference Example 20, ethyl 4,4,4-trifluoroethyl acetoacetate (2.21 g) and 4-fluorobenzyl amine (0.75 ml), the reaction and purification were carried out in the same manner as Example 185 to obtain ethyl 4-(3-cyanophenyl)-1-(4-fluorobenzyl)-5-methyl-2-(trifluorom ethyl)-1H-pyrrole-3-carboxylate (0.31 g) and ethyl
4-(3-cyanophenyl)-5-methyl-2-(trifluoromethyl)-1H-pyrrole-3 - carboxylate (0.04 g) as colorless crystals. ethyl 4-(3-cyanophenyl)-1-(4-fluorobenzyl)-5-methyl-2-(trifluorom ethyl)-1H-pyrrole-3-carboxylate
IR (KBr): ν 2230, 1725, 1512, 1264, 1202, 1159, 1119 cm⁻¹.
¹H-NMR (CDCl₃) δ: 1.15 (3H, t, *J*=7.2 Hz), 2.04 (3H, s), 4.19 (2H, q, *J*=7.2 Hz), 5.25 (2H, s), 6.94-7.08 (4H, m), 7.45-7.62 (4H, m). ethyl
4-(3-cyanophenyl)-5-methyl-2-(trifluoromethyl)-1H-pyrrole-3 -carboxylate
IR (KBr): ν 3277, 2232, 1696, 1304, 1194, 1146, 1038 cm⁻¹.
¹H-NMR (CDCl₃) δ: 1.16 (3H, t, *J*=7.0 Hz), 2.17 (3H, s), 4.17 (2H, q, *J*=7.0 Hz), 7.47-7.67 (4H, m), 9.36 (1H, br).

### Example 202

### Production of methyl 4-(3-cyanophenyl)-1-(4-fluorobenzyl)-2-(methoxymethyl)-5-me thyl-1H-pyrrole-3-carboxylate

By using the compound (1.13 g) produced in Reference Example 20, methyl 4-methoxyacetoacetate (1.75 g) and 4-fluorobenzyl amine (0.75ml), the reaction and purification were carried out in the same manner as Example 185 to obtain the titled compound (0.55 g) as an oily substance.
IR (KBr): ν 2230, 1701, 1508, 1209, 1088 cm⁻¹.
¹H-NMR (CDCl₃) δ: 1.97 (3H, s), 3.37 (3H, s), 3.62 (3H, s), 4.75 (2H, s), 5.23 (2H, s), 6.91-7.06 (4H, m), 7.42-7.60 (4H, m).

### Example 203

### Production of ethyl 4-(4-cyanophenyl)-1-(4-fluorobenzyl)-3,5-dimethyl-1H-pyrrol e-2-carboxylate

A mixture of the compound (1.00 g) produced in Reference Example 21, 4-cyanophenylboronic acid (0.46 g), tetrakis(triphenyl phosphine) palladium (0.32 g), a 2M aqueous solution of sodium carbonate (8 ml) and toluene (20 ml) was hated at 90 °C for 16 hours, and cooled to room temperature. To the reaction mixture were added water and ethyl acetate, and insoluble matter was filtered off. The ethyl acetate layer was separated, washed with saturated brine, dried over anhydrous magnesium sulfate and concentrated. The resulting crude product was purified by column chromatography (carrier: silicagel, eluant: hexane-ethyl acetate) to obtain the titled compound (0.45 g) as colorless crystals.
IR (KBr): ν 2228, 1688, 1510, 1132 cm⁻¹.
¹H-NMR (CDCl₃) δ: 1.30 (3H, t, *J*=7.2 Hz), 2.12 (3H, s), 2.28 (3H, s), 4.26 (2H, q, *J*=7.2 Hz), 5.60 (2H, s), 6.95-7.00 (4H, m), 7.33 (2H, d, *J*=8.7 Hz), 7.69 (2H, d, *J*=8.7 Hz).

### Example 204

### Production of 4-(4-cyanophenyl)-1-(4-fluorobenzyl)-3,5-dimethyl-1H-pyrrol e-2-carboxylic acid and ethyl 4-(4-(aminocarbonyl)phenyl)-1-(4-fluorobenzyl)-3,5-dimethyl - lH-pyrrole-2-carboxylate(0.03 g)

The compound (0.10 g) produced in Example 203 was dissolved in ethanol (0.5 ml) and tetrahydrofuran (0.3 ml), and to the solution was added 2N sodium hydroxide (0.5 ml) . The reaction mixture was stirred at room temperature for 5 hours, and at 50 °C for 10 hours, poured into 1N hydrochloric acid, and extracted with ethyl acetate. The ethyl acetate layer was dried over anhydrous magnesium sulfate and concentrated. The resulting crude product was purified by column chromatography (carrier: silicagel, eluant: hexane-ethyl acetate) to obtain 4-(4-cyanophenyl)-1-(4-fluorobenzyl)-3,5-dimethyl-1H-pyrrol e-2-carboxylic acid (0.02 g) as colorless crystals and ethyl 4-(4-(aminocarbonyl)phenyl)-1-(4-fluorobenzyl)-3,5-dimethyl - 1H-pyrrole-2-carboxylate (0.03 g) as colorless crystals. 4-(4-cyanophenyl)-1-(4-fluorobenzyl)-3,5-dimethyl-1H-pyrrol e-2-carboxylic acid
IR (KBr): ν 2228, 1655, 1510, 1146 cm⁻¹.
¹H-NMR (CDCl₃) δ: 2.12 (3H, s), 2.31 (3H, s), 5.61 (2H, s), 6.95-7.02 (4H, m), 7.32 (2H, d, *J*=8.1 Hz), 7.69 (2H, d, *J*=8.1 Hz). ethyl 4-(4-(aminocarbonyl)phenyl)-1-(4-fluorobenzyl)-3,5-dimethyl - 1H-pyrrole-2-carboxylate (0.03 g)
IR (KBr): ν 1686, 1671, 1132 cm⁻¹.
¹H-NMR (CDCl₃) δ: 1.30 (3H, t, *J*=7.2 Hz), 2.12 (3H, s), 2.28 (3H, s), 4.25 (2H, q, *J*=7.2 Hz), 5.59 (2H, s), 5.50-6.20 (2H, br), 6.94-7.01 (4H, m), 7.31 (2H, d, *J*=8.1 Hz), 7.84 (2H, d, J=8.1 Hz).

### Example 205

### Production of benzyl 1-(4-cyanobenzyl)-4-(4-cyanophenyl)-3,5-dimethyl-1H-pyrrole - 2-carboxylate

By using the compound (4.23 g) produced in Reference Example 24 and 4-cyanophenylboronic acid (1.61 g), the reaction and purification were carried out in the same manner as Example 203 to obtain the titled compound (2.63 g) as colorless crystals.
IR (KBr): ν 2228, 1688, 1607, 1416, 1287, 1128 cm⁻¹.
¹H-NMR (CDCl₃) δ: 2.11 (3H, s), 2.28 (3H, s), 5.23 (2H, s), 5.66 (2H, s), 7.02 (2H, d, *J*=8.4 Hz), 7.27-7.35 (7H, m), 7.57 (2H, d, *J*=8.0 Hz), 7.70 (2H, d, *J*=8.4 Hz).

### Example 206

### Production of 1-(4-cyanobenzyl)-4-(4-cyanophenyl)-3,5-.dimethyl-1H-pyrrole - 2-carboxylic acid

The compound (2.27 g) produced in Example 205 was dissolved in ethanol (40 ml) and tetrahydrofuran (20 ml), and to the solution was added 10% palladium-carbon (1.0 g) . Under hydrogen atmosphere, the reaction mixture was stirred at room temperature for 30 minutes and insoluble matter was filtered off. The filtrate was concentrated to obtain the titled compound (2.00 g) as colorless crystals.
IR (KBr): ν 2228, 1651, 1607, 1441, 1146 cm⁻¹.
¹H-NMR (CDCl₃) δ: 2.11 (3H, s), 2.31 (3H, s), 5.69 (2H, s), 7.07 (2H, d, *J*=8.0 Hz), 7.34 (2H, d, *J*=8.0 Hz), 7.60 (2H, d, *J*=8.0 Hz), 7.71 (2H, d, *J*=8.0 Hz).

### Example 207

### Production of 1-(4-cyanobenzyl)-4-(4-cyanophenyl)-3,5-dimethyl-1H-pyrrole -2-carboxamide

To a solution of the compound (89 mg) produced in Example 206 in tetrahydrofuran (1 ml) was added thionyl chloride (0.06 ml). The reaction mixture was stirred at room temperature for 30 minutes and concentrated. The residue was dissolved in tetrahydrofuran (1 ml) , added to a mixed solution of 25% aqueous ammonia (1 ml) and tetrahydrofuran(0.5 ml). The reaction mixture was stirred at room temperature for 2 hours, poured into saturated sodium bicarbonate water, and extracted with ethyl acetate. The ethyl acetate layer was dried over anhydrous magnesium sulfate and concentrated. The resulting crude product was purified by column chromatography (carrier: silicagel, eluant: hexane-ethyl acetate) to obtain the titled compound (46 mg) as colorless crystals.
IR (KBr): ν 2228, 1653, 1605, 1437 cm⁻¹.
¹H-NMR (CDCl₃) δ: 2.10 (3H, s), 2.29 (3H, s), 5.52 (2H, br), 5.68 (2H, s), 7.09 (2H, d, *J*=8.4 Hz), 7.33 (2H, d, *J*=8.4 Hz), 7.60 (2H, d, *J*=8.4 Hz), 7.71 (2H, d, *J*=8.4 Hz).

### Example 208

### Production of 1-(4-cyanobenzyl)-4-(4-cyanophenyl)-N,3,5-trimethyl-1H-pyrr ole-2-carboxamide

By using the compound (80 mg) produced in Example 206 as a starting material and 33% methylamine ethanol solution (1 ml) as an amine component, the reaction and purification were carried out in the same manner as Example 207 to obtain the titled compound (52 m g) as colorless crystals.
IR (KBr): ν 2228, 1636, 1607, 1530, 729 cm⁻¹.
¹H-NMR (CDCl₃) δ: 2.07 (3H, s), 2.22 (3H, s), 2.93 (3H, d, *J*=4.8 Hz), 5.59 (2H, s) , 5.65 (1H, br), 7.09 (2H, d, *J*=8.7 Hz), 7.31 (2H, d, *J*=8.1 Hz), 7.58 (2H, d, *J*=Hz), 7.69 (2H, d, *J*=8.7 Hz).

### Example 209

### Production of 1-(4-cyanobenzyl)-4-(4-cyanophenyl)-N,N,3,5-tetramethyl-1H-pyrrole-2-carboxamide

By using the compound (80 mg) produced in Example 2.06 as a starting material and 50% dimethylamine solution (1 ml) as an amine component, the reaction and purification were carried out in the same manner as Example 207 to obtain the titled compound (35 m g) as colorless crystals.
IR (KBr): ν 2226, 1607, 1697, 1111, 731 cm⁻¹.
¹H-NMR (CDCl₃) δ: 2.01 (3H, s), 2.14 (3H, s), 2.96 (6H, s), 5.00-5.50 (2H, br), 7.13 (2H, d, *J*=8.4 Hz), 7.36 (2H, d, *J*=8.4 Hz), 7.60 (2H, d, *J*=8.4 Hz), 7.67 (2H, d, *J*=8.4 Hz).

### Example 210

### Production of 4-(1-(4-cyanobenzyl)-2,4-dimethyl-5-(4-morpholinylcarbonyl) - 1H-pyrrol-3-yl)benzonitrile

To a solution of the compound (80 mg) produced in Example 206 in tetrahydrofuran (1 ml) was added thionyl chloride (0.06 ml). The reaction mixture was stirred at room temperature for 30 minutes and concentrated. The residue was dissolved in tetrahydrofuran (1 ml) and added to a solution of morpholine (44 mg) and triethylamine (0.083 ml) in tetrahydrofuran (0.5 ml). The reaction mixture was stirred at room temperature for 16 hours, poured into water and extracted with ethyl acetate. The ethyl acetate layer was dried over anhydrous magnesium sulfate and concentrated. The resulting crude product was purified by column chromatography (carrier: silicagel, eluant: hexane-ethyl acetate) to obtain the titled compound (81 mg) as colorless crystals.
IR (KBr): ν 2226, 1607, 1433, 1115, 731 cm⁻¹.
¹H-NMR (CDCl₃) δ: 2.02 (3H, s), 2.18 (3H, s), 3.00-3.75 (8H, m), 5.20-5.45 (2H, br), 7.12 (2H, d, *J*=8.7 Hz), 7.34 (2H, d, J=8.7 Hz), 7.62 (2H, d, J=8.7 Hz), 7.68 (2H, d, J=8.7 Hz).

### Example 211

### Production of 1-(4-cyanobenzyl)-4-(4-cyanophenyl)-3,5-dimethyl-N-(3-pyrid inyl)-1H-pyrrole-2-carboxamide

To a solution of the compound (80 mg) produced in Example 206, 3-aminopyridine (47 mg) and triethylamine (0.047 ml) in dimethylformamide (1 ml) was added cyanide diethylphosphate (0.041 ml) and the reaction mixture was stirred at room temperature for 18 hours and at 70 °C for 6 hours. The reaction mixture was poured into water, and extracted with ethyl acetate. The ethyl acetate layer was washed with saturated brine, dried over magnesium sulfate and concentrated.
The residue was purified by column chromatography (carrier: silicagel, eluant: hexane-ethyl acetate) to obtain the titled compound (29 mg) as colorless crystals.
IR (KBr): ν 2222, 1655, 1603, 1541, 1424, 845 cm⁻¹.
¹H-NMR (CDCl₃) δ: 2.11 (3H, s), 2.37 (3H, s), 5.63 (2H, s), 7.13 (2H, d, *J*=7.8 Hz), 7.25-7.29 (1H, m), 7.35 (2H, d, *J*=8.4 Hz), 7.48 (1H, s), 7.60 (2H, d, *J*=7.8 Hz), 7.72 (2H, d, *J*=8.4 Hz), 8.12-8.15 (1H, m), 8.34 (1H, d, *J*=4.5 Hz), 8.55-8.56 (1H, m).

### Example 212

### Production of benzyl 4-(4-cyanophenyl)-3,5-dimethyl-1H-pyrrole-2-carboxylate

By using the compound (3.38 g) produced in Reference Example 23 and 4-cyanophenylboronic acid (1.76 g), the reaction and purification were carried out in the same manner as Example 203 to obtain the titled compound (1.14 g) as colorless crystals.
IR (KBr): ν 3301, 2226, 1665, 1609, 1433, 1269, 1096 cm⁻¹.
¹H-NMR (CDCl₃) δ: 2.26 (3H, s), 2.31 (3H, s), 5.33 (2H, s), 7.31-7.44 (7H, m), 7.68 (2H, d, *J*=8.7 Hz), 8.80 (1H, br).

### Example 213

Production of 4-(4-cyanophenyl)-3,5-dimethyl-1H-pyrrole-2-carboxylic acid By using the compound (1.12 g) produced in Example 212, the reaction and purification were carried out in the same manner as Example 206 to obtain the titled compound (0.62 g) as colorless crystals.
IR (KBr): ν 2919, 2226, 1651, 1252 cm⁻¹.
¹H-NMR (CDCl₃+CD₃OD) δ: 2.27 (3H, s), 2.31 (3H, s), 7.36 (2H, d, *J*=8.4 Hz), 7.68 (2H, d, *J*=8.4 Hz).

### Example 214

### Production of 4-(4-cyanophenyl)-3,5-dimethyl-N-(3-pyridinyl)-1H-pyrrole-2 - carboxamide

The compound (72 mg) produced in Example 213, 3-aminopyridine (57 mg) and triethylamine (0.056 ml) were dissolved in dimethylformamide (1 ml) and to the solution were added 1-hydroxybenzotriazole (55 mg) and 1-ethyl-3-(3-dimethylaminopropyl)carbodiimide hydrochloride (69 mg). The reaction mixture was stirred at room temperature for 18 hours and at 60 °C for 72 hours. The reaction mixture was poured into water and extracted with ethyl acetate. The ethyl acetate layer was washed with saturated brine, dried over magnesium sulfate and concentrated. The residue was purified by column chromatography (carrier: silicagel, eluant: hexane-ethyl acetate) to obtain the titled compound (9 mg) as a colorless powder.
IR (KBr) : ν 2226, 1638, 1605, 1483, 1445, 1426, 1381, 1325 cm⁻¹.
¹H-NMR (CDCl₃ + CD₃OD) δ: 2.31 (3H, s), 2.40 (3H, s), 7.32-7.40 (3H, m), 7.72 (2H, d, *J*=8.4 Hz), 8.25-8.52 (3H, m).

### Example 215

### Production of ethyl 4-(4-cyanophenyl)-2-(4-fluorobenzyl)-5-methyl-1H-pyrrole-3-carboxylate

By using the compound (1.88 g) produced in Reference Example 25 and ethyl 4-(4-fluorophenyl)-3-oxobutanoate (4.48 g), the reaction and purification were carried out in the same manner as Example 1 to obtain the titled compound (0.21 g) as pale yellow solids.
IR (KBr): ν 2226, 1694, 1607, 1508, 1225, 1159, 841 cm⁻¹.
¹H-NMR (CDCl₃) δ: 1.06 (3H, t, *J*=6.9 Hz), 2.06 (3H, s), 4.09 (2H, q, *J*=6.9 Hz), 4.29 (2H, s), 6.98-7.04 (2H, m), 7.20-7.26 (2H, m), 7.35 (2H, d, J=8.4 Hz), 7.60 (2H, d, J=8.4 Hz), 8.13 (1H, br).

### Example 216

### Production of 4-(1-(4-cyanobenzyl)-5-(hydroxymethyl)-2,4-dimethyl-1H-pyrr ol-3-yl)benzonitrile

The compound (0.22 g) produced in Example 206 and N-ethyl diisopropylamine (0.13 ml) was dissolved in tetrahydrofuran (5 ml), and to the solution was added benzotriazol-1-yloxy tris(dimethylamino)phosphoniumhexafluorophosphate (0.31 g). The reaction mixture was stirred at room temperature for 30 minutes and to the reaction mixture was added sodium boron hydride (55 mg). The reaction mixture was further stirred at room temperature for 18 hours, poured into water and extracted with ethyl.acetate. The ethyl acetate layer was washed with saturated brine, dried over magnesium sulfate and concentrated. The residue was purified by column chromatography (carrier: silicagel, eluant: hexane-ethyl acetate) to obtain the titled compound (0.03 g) as colorless crystals.
IR (KBr): ν 3495, 2226, 1605, 733, 550 cm⁻¹.
¹H-NMR (CDCl₃) δ: 1.49 (1H, br), 2.10 (6H, s), 4.53 (2H, s), 5.30 (2H, s), 7.04 (2H, d, *J*=8.4 Hz), 7.35 (2H, d, *J*=8.4 Hz), 7.61 (2H, d, *J*=8.4 Hz), 7.66 (2H, d, *J*=8.4 Hz).

### Example 217

### Production of tert-butyl 3-(4-cyanophenyl)-4-methyl-1H-pyrrole-2-carboxylate

To a mixture of the compound (1.88 g) produced in Reference Example 25, tert-butyl isocyanoacetate (1.55 g), tetrahydrofuran (10 ml) and isopropyl alcohol (10 ml) was added N,N,N',N'-tetramethyl guanidine (1.84 g) . The reaction mixture was stirred at 60 °C for 2 hours, poured into water and extracted with ethyl acetate. The ethyl acetate layer was washed with saturated brine, dried over magnesium sulfate and concentrated. The residue was washed with diisoprpyl ether to obtain the titled compound (2.25 g) as pale brown crystals.
IR (KBr): ν 3287, 2226, 1667, 1393, 1291, 1155 cm⁻¹.
¹H-NMR (CDCl₃) δ: 1.34 (9H, s), 1.96 (3H, s), 6.75-6.77 (1H, m), 7.42 (2H, d, *J*=8.4 Hz), 7.65 (2H, d, *J*=8.4 Hz), 8.99 (1H, br).

### Example 218

### Production of tert-butyl 3-(4-cyanophenyl)-4-methyl-1-(3-pyridinylmethyl)-1H-pyrrole - 2-carboxylate

A suspension of sodium hydride (60% oil suspension, 0.64 g) in dimethylformamide (20 ml) was cooled in an ice bath, and to the suspension was added 3-chloromethylpyridine hydrochloride (0.95 g). The reaction mixture was stirred at the same temperature for 10 minutes. To the reaction mixture was added the compound (1.50 g) produced in Example 217, and the reaction mixture was stirred at room temperature for 2 hours and at 60 °C for 18 hours. The reaction mixture was poured into water and extracted with ethyl acetate. The ethyl acetate layer was washed with water and saturated brine, successively; dried over magnesium sulfate and concentrated. The residue was purified by column chromatography (carrier: silicagel, eluant: hexane-ethyl acetate) to obtain the titled compound (1.12 g) as colorless crystals.
IR (KBr): ν 2226, 1692, 1368, 1304, 1161, 1088 cm⁻¹.
¹H-NMR (CDCl₃) δ: 1.14 (9H, s), 1.87 (3H, s), 5.52 (2H, s), 6.70 (1H, s), 7.23-7.35 (3H, m), 7.48-7.51 (1H, m), 7.63-7.67 (2H, m), 8.45 (1H, dd, *J*=2.1, 0.6 Hz), 8.52 (1H, dd, *J*=4.8, 1.5 Hz).

### Example 219

### Production of 4-(4-methyl-1-(3-pyridinylmethyl)-1H-pyrrol-3-yl)benzonitri le

The compound (61 mg) produced in Example 218 was dissolved in TFA (0.5 ml) and the solution was stirred at room temperature for 16 hours. The reaction mixture was concentrated, diluted with saturated sodium bicarbonate water, and extracted with ethyl acetate. The ethyl acetate layer was dried over magnesium sulfate and concentrated. The resulting residue was purified by column chromatography (carrier: silicagel, eluant: hexane-ethyl acetate) to obtain the titled compound (15 mg) as colorless crystals.
IR (KBr): ν 2222, 1605, 1541, 1427, 1412, 1152, 843, 787, 712 cm⁻¹.
¹H-NMR (CDCl₃) δ: 2.22 (3H, s), 5.04 (2H, s), 6.53-6.54 (1H, m), 6.85 (1H, d, *J*=2.4 Hz), 7.25-7.28 (1H, m), 7.44-7.62 (5H, m), 8.50 (1H, s), 8.56 (1H, d, *J*=3.6 Hz).

### Example 220

### Production of ethyl 4-(4-cyanophenyl)-2,5-dimethyl-1H-pyrrole-3-carboxylate

To solution of the compound (0.89 g) produced in Example 103 in tetrahydrofuran (10 ml) were added thionyl chloride (0.80 ml) and dimethylformamide (0.025 ml). The mixture was stirred at room temperature for 2 hours and concentrated. The residue was dissolved in tetrahydrofuran (5 ml), and to the solution was added ethanol (16 ml). The reaction mixture was stirred at room temperature for 2 hours and concentrated. The residue was dissolved in saturated sodium bicarbonate water and ethyl acetate. The ethyl acetate layer was separated, dried over anhydrous magnesium sulfate and concentrated to obtain the titled compound (0.97 g) as colorless crystals.
IR (KBr) : ν 3301, 2226, 1671, 1607, 1435, 1294, 1175, 1094 cm⁻¹.
¹H-NMR (CDCl₃) δ: 1.09 (3H, t, *J*=7.0 Hz), 2.13 (3H, s), 2.53 (3H, s), 4.10 (2H, q, *J*=2.0 Hz), 7.35 (2H, d, *J*=8.0 Hz), 7.62 (2H, d, *J*=8.0 Hz), 7.98 (1H, br).

### Example 221

### Production of ethyl 4-(4-cyanophenyl)-2,5-dimethyl-1-(3-pyridinylmethyl)-1H-pyr role-3-carboxylate

A suspension of sodium hydride (60% oil suspension, 0.35 g) in dimethylformamide (8 ml) was cooled in an ice bath, and to the suspension was added 3-chloromethylpyridine hydrochloride (0.53 g), and the reaction mixture was stirred at the same temperature for 10 minutes. To the reaction mixture was added a solution of the compound (0.77 g) produced in Example 220 in dimethylformamide(6 ml). The reaction mixture was stirred at room temperature for 4 hours, poured into water and extracted with ethyl acetate. The ethyl acetate layer was washed with water, saturated brine, successively, dried over magnesium sulfate and concentrated. The residue was purified by column chromatography (carrier: silicagel, eluant: hexane-ethyl acetate) to obtain the titled compound (0.94 g) as colorless crystals.
IR (KBr): ν 2224, 1694, 1292, 1157 cm⁻¹.
¹H-NMR (CDCl₃) δ: 1.04 (3H, t, *J*=7.0 Hz), 2.03 (3H, s), 2.51 (3H, s), 4.08 (2H, q, *J*=7.0 Hz), 5.15 (2H, s), 7.17-7.37 (4H, m), 7.63 (2H, d, *J*=8.0 Hz), 8.37 (1H, d, *J*=1.4 Hz), 8.56 (1H, dd, *J*=4.4, 1.4 Hz).

### Example 222

### Production of isopropyl 4-(4-cyanophenyl)-2,5-dimethyl-1H-pyrrole-3-carboxylate

By using the compound (1.03 g) produced in Example 103 as a starting material and isopropyl alcohol (16 ml) as an alcohol component, the reaction and purification were carried out in the same manner as Example 220 to obtain the titled compound (0.74 g) as colorless crystals.
IR (KBr): ν 3303, 2226, 1669, 1607, 1435, 1292, 1171, 1092 cm⁻¹.
¹H-NMR (CDCl₃) δ: 1.09 (6H, d, *J*=6.2 Hz), 2.12 (3H, s), 2.53 (3H, s), 5.10 (1H, septet, *J*=6.2 Hz), 7.35 (2H, d, *J*=8.4 Hz), 7.62 (2H, d, *J*=8.4 Hz), 7.97 (1H, br).

### Example 223

### Production of isopropyl 4-(4-cyanophenyl)-2,5-dimethyl-1-(3-pyridinylmethyl)-1H-pyr role-3-carboxylate

By using the compound (0.64 g) produced in Example 222, the reaction and purification were carried out in the same manner as Example 221 to obtain the titled compound (0.64 g) as colorless crystals.
IR (KBr): ν 2224, 1694, 1291, 1165, 1109 cm⁻¹.
¹H-NMR (CDCl₃) δ: 1.04 (6H, d, *J*=6.2 Hz), 2.02 (3H, s), 2.51 (3H, s), 5.00 (1H, septet, *J*=6.2 Hz), 5.14 (2H, s), 7.19-7.37 (4H, m), 7.63 (2H, d, *J*=8.0 Hz), 8.37 (1H, d, *J*=1.4 Hz), 8.56 (1H, dd, *J*=4.4, 1.4 Hz).

### Example 224

### Production of ethyl 4-(4-bromophenyl)-2,5-dimethyl-1H-pyrrole-3-carboxylate

By using the compound (20.00 g) produced in Reference Example 26, the reaction and purification were carried out in the same manner as Example 1 to obtain the titled compound (16.46 g) as colorless crystals.
IR (KBr): ν 3304, 1674, 1433, 1291, 1173, 1094 cm⁻¹.
¹H-NMR (CDCl₃) δ: 1.10 (3H, t, *J*=7.2 Hz), 2.10 (3H, s), 2.51 (3H, s), 4.09 (2H, q, *J*=7.2 Hz), 7.11 (2H, d, *J*=8.1 Hz), 7.44 (2H, d, *J*=8.1 Hz), 7.92 (1H, br).

### Example 225

### Production of 5-(4-(4-bromophenyl)-2,5-dimethyl-1H-pyrrol-3-yl)-3-methyl-1,2,4-oxadiazole

A suspension of sodium hydride (60% oil suspension, 1.20 g) in tetrahydrofuran (50 ml) was heated to 70 °C, and to the suspension was added acetamide oxime (2.22 g). The reaction mixture was stirred at the same temperature for 1 hour. To the reaction mixture was added the compound (4.51 g). produced in Example 224, and the reaction mixture was heated for 20 hours under reflux. The reaction mixture was cooled to room temperature, and insoluble matter was filtered off. The filtrate of the tetrahydrofuran layer was washed with saturated brine, dried over magnesium sulfate and concentrated. The residue was recrystallized from ethyl acetate to obtain the titled compound (3.00 g) as colorless crystals.
IR (KBr): ν 3256, 1576, 1399, 1345 cm⁻¹.
¹H-NMR (CDCl₃) δ: 2.15 (3H, s), 2.34 (3H, s), 2.58 (3H, s), 7.12 (2H, d, *J*=8.8 Hz), 7.47 (2H, d, *J*=8.8 Hz), 8.22 (1H, br).

### Example 226

### Production of 4-(2,5-dimethyl-4-(3-methyl-1,2,4-oxadiazol-5-yl)-1H-pyrrol - 3-yl)benzonitrile

Under nitrogen atmosphere, a mixture of the compound (2.80 g) produced in Example 225, zinc cyanide (2.00 g), tris(dibenzylideneacetone)dipalladium (0.73 g), 1,1'-bis(diphenylphosphino)ferrocene (0.44 g) and dimethylformamide (30 ml) was stirred at 80 °C for 18 hours and at 120 °C for 24 hours. The reaction mixture was cooled to room temperature and diluted with ethyl acetate and water. Insoluble matter was filtered off, and the ethyl acetate layer, which was separated from the filtrate, was washed with water and saturated brine, successively, dried over magnesium sulfate and concentrated. The residue was purified by column chromatography (carrier: silicagel, eluant: hexane-ethyl acetate) to obtain the titled compound (0.22 g) as colorless crystals.
IR (KBr): ν 3301, 2228, 1609, 1584, 1402, 1346 cm⁻¹.
¹H-NMR (CDCl₃) δ: 2.20 (3H, s), 2.33 (3H, s), 2.61 (3H, s), 7.35 (2H, d, *J*=8.4 Hz), 7.63 (2H, d, *J*=8.4 Hz), 8.11 (1H, br).

### Example 227

### Production of 4-(2,5-dimethyl-4-(3-methyl-1,2,4-oxadiazol-5-yl)-1-(3-pyri dinylmethyl)-1H-pyrrol-3-yl)benzonitrile

By using the compound (0.19 g) produced in Example 221, the reaction and purification were carried out in the same manner as Example 225 to obtain the titled compound (0.10 g) as colorless crystals.
IR (KBr): ν 2226, 1588, 1410, 1337, 735 cm⁻¹.
¹H-NMR (CDCl₃) δ: 2.12 (3H, s), 2.34 (3H, s), 2.60 (3H, s), 5.21 (2H, s), 7.21-7.38 (4H, m), 7.65 (2H, d, J=8.4 Hz), 8.40 (1H, d, J=1.8 Hz), 8.57 (1H, dd, J=4.6, 1.8 Hz).

### Example 228

### Production of 4-(4-cyanophenyl)-2,5-dimethyl-1-((1-trityl-1H-imidazol-2-y l)methyl)-1H-pyrrole-3-carbonitrile

By using the compound (0.30 g) produced in Example 105 and the compound (0.68 g) produced in Reference Example 28, the reaction and purification were carried out in the same manner as Reference Example 24 to obtain the titled compound (0.37 g) as colorless crystals.
IR (KBr): ν 2215, 1609, 1447, 1426, 733, 704 cm⁻¹.
¹H-NMR (CDCl₃) δ: 1.88 (3H, s), 2.04 (3H, s), 3.96 (2H, s), 6.96 (2H, s), 7.21-7.48 (17H, m), 7.64 (2H, d, *J*=8.4 Hz).

### Example 229

### Production of 4-(4-cyanophenyl)-1-(1H-imidazol-2-ylmethyl)-2,5-dimethyl-1 H-pyrrole-3-carbonitrile

A mixture of the compound (0.36 g) produced in Example 228, pyridine hydrochloride (0.12 g) and methanol (3 ml) was heated at 70 °C for 1 hour and concentrated. The residue was dissolved in ethyl acetate and 1N hydrochloric acid, and the aqueous layer was separated. The aqueous layer was neutralized with saturated sodium bicarbonate water and extracted with ethyl acetate. The obtained ethyl acetate layer was dried over magnesium sulfate and concentrated. The residue was recrystallized form ethyl acetate to obtain the titled compound (0.16 g) as colorless crystals.
IR (KBr): ν 2222, 1607 cm⁻¹.
¹H-NMR (CDCl₃) δ: 2.26 (3H, s), 2.43 (3H, s), 5.20 (2H, s), 7.00 (1H, s), 7.10 (1H, s), 7.47 (2H, d, *J*=8.4 Hz), 7.68 (2H, d, *J*=8.4 Hz), 8.83 (1H, br).

### Example 230

### Production of 4-(4-cyanophenyl)-2,5-dimethyl-1-((1-methyl-1H-imidazol-2-y 1)methyl)-1H-pyrrole-3-carbonitrile

To a mixture of the compound (60 mg) produced in Example 229, potassium carbonate (55 mg) and dimethylformamide (2 ml) was added methyl iodide (0.013 ml). The reaction mixture was stirred at room temperature for 14 hours, diluted with water and extracted with ethyl acetate. The ethyl acetate layer was washed with saturated brine, dried over magnesium sulfate and concentrated. The residue was purified by column chromatography (carrier: silicagel, eluant: ethyl acetate-methanol) to obtain the titled compound (36 mg) as colorless crystals.
IR (KBr): ν 2213, 1607, 1427, 735 cm⁻¹.
¹H-NMR (CDCl₃) δ: 2.23 (3H, s), 2.41 (3H, s), 3.60 (3H, s), 5.12 (2H, s), 6.89 (1H, d, *J*=1.2 Hz), 6.99 (1H, d, *J*=1.2 Hz), 7.54 (2H, d, *J*=8.4 Hz), 7.68 (2H, d, *J*=8.4 Hz).

### Example 231

### Production of 4-(4-cyanophenyl)-2,5-dimethyl-1-((1-trityl-1H-imidazol-4-y l)methyl)-1H-pyrrole-3-carbonitrile

By using the compound (0.30 g) produced in Example 105 and the compound (1.10 g) produced in Reference Example 29, reaction and purification were carried out in the same manner as Reference Example 24 to obtain the titled compound (0.31 g) as colorless crystals.
IR (KBr): ν 2213, 1607, 1445, 743, 702 cm⁻¹.
¹H-NMR (CDCl₃) δ: 2.28 (3H, s), 2.43 (3H, s), 4.94 (2H, s), 6.55 (1H, s), 7.08-7.15 (6H, m), 7.30-7.37 (9H, m), 7.42-7.47 (3H, m) , 7.67 (2H, d, *J*=8.8 Hz).

### Example 232

### Production of 4-(4-cyanophenyl)-1-(1H-imidazol-4-ylmethyl)-2,5-dimethyl-1 H-pyrrole-3-carbonitrile

By using the compound (0.29 g) produced in Example 231, the reaction and purification were carried out in the same manner as Example 229 to obtain the titled compound (0.15 g) as colorless crystals.
IR (KBr): ν 2226, 2213, 1607, 855 cm⁻¹.
¹H-NMR (CDCl₃+ DMSO-d₆) δ: 2.34 (3H, s), 2.49 (3H, s), 5.03 (2H, s), 6.68 (1H, s), 7.48-7.56 (3H, s), 7.68 (2H, d, *J*=8.4 Hz).

### Example 233

### Production of 4-(4-cyanophenyl)-2,5-dimethyl-1-((1-methyl-1H-imidazol-4-y l)methyl)-1H-pyrrole-3-carbonitrile and 4-(4-cyanophenyl)-2,5-dimethyl-1-((1-methyl-1H-imidazol-5-y l)methyl)-1H-pyrrole-3-carbonitrile

By using the compound (80 mg) produced in Example 232, the reaction and purification were carried out in the same manner as Example 230 to obtain 4-(4-cyanophenyl)-2,5-dimethyl-1-((1-methyl-1H-imidazol-4-y 1)methyl)-1H-pyrrole-3-carbonitrile (63 mg) and 4-(4-cyanophenyl)-2,5-dimethyl-1-((1-methyl-1H-imidazol-5-y 1)methyl)-1H-pyrrole-3-carbonitrile (3 mg) as colorless crystals.
4-(4-cyanophenyl)-2,5-dimethyl-1-((1-methyl-1H-imidazol-4-y l)methyl)-1H-pyrrole-3-carbanitrile
IR (KBr): ν 2213, 1607, 1541, 1507, 1427, 831, 735 cm⁻¹.
¹H-NMR (CDCl₃) δ: 2.33 (3H, s), 2.48 (3H, s), 3.65 (3H, s), 4.99 (2H, s), 6.56 (1H, s), 7.39 (1H, s), 7.50 (2H, d, *J*=8.4 Hz), 7.68 (2H, d, *J*=8.4 Hz).
4-(4-cyanophenyl)-2,5-dimethyl-1-((1-methyl-1H-imidazol-5-y l)methyl)-1H-pyrrole-3-carbonitrile
IR (KBr): ν 2215, 1607, 1539, 1507, 1427, 833, 737 cm⁻¹.
¹H-NMR (CDCl₃) δ: 2.23 (3H, s), 2.42 (3H, s), 3.61 (3H, s), 5.04 (2H, s), 6.57 (1H, s), 7.47-7.52 (3H, m), 7.71 (2H, d, *J*=8.4 Hz).

### Example 234

### Production of 4-(4-cyanophenyl)-2,5-dimethyl-1-((1-trityl-1H-1,2,3-triazo 1-4-yl)methyl)-1H-pyrrole-3-carbonitrile

By using the compound (0.13 g) produced in Example 105 and the compound (0.27 g) produced in Reference Example 30, the reaction and purification were carried out in the same manner as Reference Example 24 to obtain the titled compound (0.26 g) as colorless crystals.
IR (KBr): ν 2215, 1609, 1445, 745, 700 cm⁻¹.
¹H-NMR (CDCl₃) δ: 2.24 (3H, s), 2.40 (3H, s), 5.12 (2H, s), 7.05-7.35 (15H, m), 7.42-7.47 (3H, m), 7.69 (2H, d, *J*=8.4 Hz).

### Example 235

### Production of 4-(4-cyanophenyl)-2,5-dimethyl-1-(1H-1,2,3-triazol-4-ylmeth yl)-1H-pyrrole-3-carbonitrile

To a mixture of the compound (0.25 g) produced in Example 234, anisole (0.2 ml) and dichloromethane (4 ml) was added TFA (2 ml), and the reaction mixture was stirred at room temperature for 10 minutes. The reaction mixture was concentrated, and the residue was dissolved in ethyl acetate and saturated sodium bicabonate water. The ethyl acetate layer was dried over magnesium sulfate and concentrated. The resulting residue was purified by column chromatography (carrier: silicagel, eluant: hexane-ethyl acetate) and recrystallized from ethyl acetate-hexane to obtain the titled compound (0.03 g) as colorless crystals.
IR (KBr): ν 3196, 2220, 1607, 1541, 1427, 735 cm⁻¹.
¹H-NMR (CDCl₃) δ: 2.32 (3H, s), 2.49 (3H, s), 5.21 (2H, s), 7.46-7.52 (3H, m), 7.68 (2H, d, *J*=8.4 Hz).

### Example 236

### Production of ethyl (3-cyano-4-(4-cyanophenyl)-2,5-dimethyl-1H-pyrrol-1-yl)acet ate

By using the compound (0.33 g) produced in Example 105 and bromoethyl acetate (0.20 ml) , the reaction and purification were carried out in the same manner as Reference Example 24 to obtain the titled compound (0.16 g) as colorless crystals.
IR (KBr): ν 2216, 1748, 1609, 1427, 1372, 1208, 1024, 833 cm⁻¹.
¹H-NMR (CDCl₃) δ: 1.32 (3H, t, *J*=7.0 Hz), 2.23 (3H, s), 2.39 (3H, s) , 4.28 (2H, q, *J*=7.0 Hz), 4.59 (2H, s), 7.50 (2H, d, *J*=8.4 Hz), 7.70 (2H, d, *J*=8.4 Hz).

### Example 237

### Production of 4-(4-cyanophenyl)-2,5-dimethyl-1-((3-methyl-1,2,4-oxadiazol - 5-yl)methyl)-1H-pyrrole-3-carbonitrile

By using the compound (0.12 g) produced in Example 236, the reaction and purification were carried out in the same manner as Example 225 to obtain the titled compound (0.06 g) as colorless crystals.
IR (KBr): ν 2218, 1609, 1426, 1327, 735 cm⁻¹.
¹H-NMR (CDCl₃) δ: 2.34 (3H, s), 2.42 (3H, s), 2.51 (3H, s), 5.25 (2H, s), 7.49 (2H, d, *J*=8.4 Hz), 7.70 (2H, d, *J*=8.4 Hz).

### Example 238

### Production of tert-butyl 4-(4-cyanophenyl)-1-(4-fluorobenzyl)-3,5-dimethyl-1H-pyrrol - 2-ylcarbamate

By using the compound (0.82 g) produced in Reference Example 32, the reaction and purification were carried out in the same manner as Example 203 to obtain the titled compound (0.53 g) as colorless crystals.
IR (KBr): ν 3266, 2224, 1694, 1510, 1281, 1159, 829 cm⁻¹.
¹H-NMR (CDCl₃) δ: 1.45 (9H, s), 1.99 (3H, s), 2.11 (3H, s) , 4.99 (2H, s), 5.72 (1H, br), 6.98-7.00 (4H, m), 7.35 (2H, d, *J*=8.1 Hz), 7.64 (2H, d, *J*=8.1 H).

### Example 239

### Production of 4-(4-cyanophenyl)-2,5-dimethyl-1-(1-(3-pyridinyl)ethyl)-1H-pyrrole-3-carbonitrile

By using the compound (0.34 g) produced in Example 105 and the compound (0.50 g) produced, in Reference Example 33, the reaction and purification were carried out in the same manner as Reference Example 24 to obtain the titled compound (0.22 g) as colorless crystals.
IR (KBr): ν 2216, 1607, 1424, 729 cm⁻¹.
¹H-NMR (CDCl₃) δ: 1.99 (3H, d, *J*=7.2 Hz), 2.15 (3H, s), 2.31 (3H, s), 5.67 (1H, q, *J*=7.2 Hz), 7.30-7.43 (2H, m), 7.50 (2H, d, *J*=8.4 Hz), 7.69 (2H, d, *J*=8.4 Hz), 8.44 (1H, d, *J*=1.8 Hz), 8.59 (1H, dd, *J*=4.4, 1.8 Hz).

### Example 240

### Production of ethyl (3-cyano-4-(4-cyanophenyl)-2,5-dimethyl-1H-pyrrol-1-yl)(3-p yridinyl)acetate

By using the compound (0. 66 g) produced in Example 105 and ethyl bromo-(3-pyridyl)acetate (1.20 g), the reaction and purification were carried out in the same manner as Reference Example 24 to obtain the titled compound (0.16 g) as colorless crystals.
IR (KBr): ν 2220, 1748, 1609, 1424, 1200, 1022, 733 cm⁻¹.
¹H-NMR (CDCl₃) δ: 1.37 (3H, t, *J*=7.0 Hz), 2.15 (3H, s), 2.30 (3H, s), 4.41 (2H, q, *J*=7.0 Hz), 6.19 (1H, s), 7.30-7.54 (4H, m), 7.71 (2H, d, *J*=8.4 Hz), 8.43 (1H, d, *J*=2.6 Hz), 8.64 (1H, dd, *J*=4.8, 1.4 Hz).

### Example 241

### Production of 4-(4-cyanophenyl)-2,5-dimethyl-1-(4-pyridinylmethyl)-1H-pyr role-3-carbonitrile

A mixture of the compound (89 mg) produced in Example 105, 4-pyridyl methanol (87 mg), (tributylphosphoranylidene)acetonitrile (0.19 g) and toluene (3 ml) was heated at 110 °C for 14 hours and concentrated. The residue was purified by column chromatography (carrier: silicagel, eluant: ethyl acetate-methanol) and recrystallized from ethyl acetate to obtain the titled compound (4 mg) as colorless crystals.
IR (KBr): ν 2215, 1607, 1416 cm⁻¹.
¹H-NMR (CDCl₃) δ: 2.19 (3H, s), 2.36 (3H, s), 5.11 (2H, s), 6.85 (2H, d, *J*=6.0 Hz), 7.52 (2H, d, *J*=8.4 Hz), 7.71 (2H, d, *J*=8.4 Hz), 8.61 (2H, d, *J*=6.0 Hz).

### Example 242

### Production of methyl 4-(4-cyanophenyl)-2-methyl-1H-pyrrole-3-carboxylate

To a suspension of powdery potassium hydroxide (3.16 g) in tetrahydrofuran (120 ml) was added dropwise methyl acetoacetate (17.8 ml). The reaction mixture was stirred at the same temperature for 30 minutes and cooled to 0 °C. To the mixture was added the compound (9.56 g) produced in Reference Example 27, and the reaction mixture was stirred at 0 °C for 30 minutes. The reaction mixture was poured into water, extracted with ethyl acetate. The ethyl acetate layer was washed with saturated brine, dried over magnesium sulfate and concentrated. To the residue were added methanol (80 ml), water (16 ml) and concentrated hydrochloric acid (1.2 ml), and the reaction mixture was heated for 2 hours under reflux. The reaction mixture was concentrated, diluted with saturated sodium bicarbonate water and extracted with ethyl acetate. The ethyl acetate layer was washed with saturated brine, dried over magnesium sulfate and concentrated. The residue was dissolved in 20 % solution of ammonia in methanol. The solution was stirred at room temperature for 40 hours and concentrated. The residue was purified by column chromatography (carrier: silicagel, eluant: hexane-ethyl acetate) to obtain the titled compound (2.00 g) as yellow crystals.
IR (KBr): ν 3304, 2226, 1682, 1607, 1447, 1296, 1130, 845, 791, 731 cm⁻¹.
¹H-NMR (CDCl₃) δ: 2.55 (3H, s), 3.70 (3H, s), 6.64 (1H, d, *J*=2.4 Hz), 7.44-7.49 (2H, m), 7.57-7.62 (2H, m), 8.29 (1H, br).

### Example 243

### Production of benzyl 4-(4-cyanophenyl)-2-methyl-1H-pyrrole-3-carboxylate

To a mixture of the compound (26.00 g) produced in Reference Example 27, benzyl acetoacetate (29.40 g) and methanol (35 ml) was added sodium methoxide (2.03 g), and the reaction mixture was stirred at room temperature for 1 hour. To the reaction mixture was added 20% solution of ammonia in methanol, and the reaction mixture was further stirred at room temperature for 14 hours and concentrated. To the residue were added saturated brine and ethyl acetate, and the ethyl acetate layer was separated. The ethyl acetate layer was dried over magnesium sulfate and concentrated. The residue was purified by column chromatography (carrier: silicagel, eluant: hexane-ethyl acetate) to obtain the titled compound (15.20 g) as yellow crystals.
IR (KBr): ν 2226, 1680, 1607, 1439, 1292, 1113 cm⁻¹.
¹H-NMR (CDCl₃) δ: 2.56 (3H, s), 5.16 (2H, s), 6.62 (1H, d, *J*=2. 6 Hz), 7.14-7.50 (9H, m), 8.29 (1H, br).

### Example 244

### Production of 4-(4-cyanophenyl)-2-methyl-1H-pyrrole-3-carboxylic acid

The compound (15.10 g) produced in Example 243 was dissolved in methanol (200 ml) and tetrahydrofuran (100 ml), and to the solution was added 10% palladium-carbon (3.0 g) . Under hydrogen atmosphere, the reaction mixture was stirred at room temperature for 3 hours. Insoluble matter was filtered off, and the filtrate was concentrated to obtain the titled compound (10.50 g) as colorless crystals.
IR (KBr): ν 3295, 2234, 1661, 1607, 1464, 1310, 1146, 843 cm⁻¹.
¹H-NMR (200 MHz, DMSO-d₆) δ: 2.36 (3H, s), 6.81 (1H, d, *J*=2.2 Hz), 7.48 (2H, d, *J*=8.4 Hz), 7.66 (2H, d, *J*=8.4 Hz), 11.39 (1H, br).

### Example 245

### Production of 4-(4-cyanophenyl)-2-methyl-1H-pyrrole-3-carboxamide

By using the compound (10.40 g) produced in Example 244, the reaction and purification were carried out in the same manner as Example 92 to obtain the titled compound (7.01 g) as colorless crystals.
IR (KBr): ν 3333, 2224, 1638, 1605, 839 cm⁻¹.
¹H-NMR (CDCl₃) δ: 2.55 (3H, s), 5.10-5.40 (2H, m), 6.68 (1H, d, *J*=2.6 Hz), 7.53 (2H, d, *J*=8.8 Hz), 7.65 (2H, d, *J*=8.8 Hz), 8.28 (1H, br).

### Example 246

### Production of 4-(4-cyanophenyl)-2-methyl-1H-pyrrole-3-carbonitrile

By using the compound (7.00 g) produced in Example 245, the reaction and purification were carried out in the same manner as Example 105 to obtain the titled compound (2.97 g) as colorless crystals.
IR (KBr): ν 3301, 2222, 1607 cm⁻¹.
¹H-NMR (CDCl₃) δ: 2.50 (3H, s), 6.94 (1H, d, *J*=2.4 Hz), 7.66 (2H, d, *J*=8.8 Hz), 7.74 (2H, d, *J*=8.8 Hz), 8.46 (1H, br).

### Example 247

### Production of 4-(4-cyanophenyl)-2-methyl-1-(3-pyridinylmethyl)-1H-pyrrole - 3-carbonitrile

By using the compound (1.82 g) produced in Example 246, the reaction and purification were carried out in the same manner as Example 221 to obtain the titled compound (2.11 g) as colorless crystals.
IR (KBr): ν 2926, 2220, 1609, 1427 cm⁻¹.
¹H-NMR (CDCl₃) δ: 2.40 (3H, s), 5.12 (2H, s), 6.89 (1H, s), 7.33-7.35 (2H, m), 7.65 (2H, d, *J*=8.4 Hz), 7.72 (2H, d, *J*=8.4 Hz), 8.48 (1H, s), 8.60-8.64 (1H, m).

### Example 248

### Production of 4-(4-cyanophenyl)-1-(2-hydroxy-2-(3-pyridinyl)ethyl)-2,5-di methyl-1H-pyrrole-3-carbonitrile

A suspension of sodium hydride (60% oil suspension, 80 mg) in dimethylformamide (5 ml) was cooled in an ice bath, and to the suspension was added the compound (0.40 g) produced in Example 105. The reaction mixture was stirred at the same temperature for 10 minutes. To the reaction mixture was added 3- (2-oxylanyl) pyridine (0.44 g), and the reaction mixture was stirred at room temperature for 16 hours and at 60 °C for 3 hours. The reaction mixture was poured into water, extracted with ethyl acetate. The ethyl acetate layer was washed with water and saturated brine, dried over magnesium sulfate and concentrated. The residue was purified by column chromatography (carrier: silicagel, eluant: ethyl acetate-methanol) to obtain the titled compound (0.17 g) as colorless crystals.
IR (KBr): ν 2213, 1609, 1426 cm⁻¹.
¹H-NMR (CDCl₃) δ: 2.23 (3H, s), 2.43 (3H, s), 2.51 (1H, d, *J*=3.6 Hz), 3.96-4.19 (2H, m), 4.99-5.08 (1H, m), 7.36 (1H, dd, *J*=7.6, 5.2 Hz), 7.47 (2H, d, *J*=8.4 Hz), 7.66-7.72 (3H, m), 8.60-8.65 (2H, m).

### Example 249

### Production of ethyl 4-(4-cyanophenyl)-2-(4-fluorobenzyl)-1,5-dimethyl-1H-pyrrol e-3-carboxylate

By using the compound (0.13 g) produced in Example 215 and methyl iodide (0.022 ml), the reaction and purification were carried out in the same manner as Reference Example 24 to obtain the titled compound (0.05 g) as colorless crystals.
IR (KBr) : ν 2226, 1692, 1607, 1508, 1291, 1221, 1190, 1154, 1042, 839 cm⁻¹.
¹H-NMR (CDCl₃) δ: 0.98 (3H, t, *J*=7.2 Hz), 2.08 (3H, s), 3.35 (3H, s), 4.12 (2H, q, *J*=7.2 Hz), 4.41 (2H, s), 6.97 (2H, t, *J*=8.7 Hz), 7.14 (2H, dd, *J*=8.7, 5.4 Hz), 7.35 (2H, d, *J*=8.4 Hz), 7.63 (2H, d, J=8.4 Hz).

### Example 250

### Production of 4-(4-cyanophenyl)-5-formyl-2-methyl-1-(3-pyridinylmethyl)-1 H-pyrrole-3-carbonitrile

Dimethylformamide (5 ml) was cooled in an ice bath, and phosphorous oxychloride (0.98 ml) was added. The reaction mixture was stirred for 30 minutes, and to the reaction mixture was added the compound (1.00 g) produced in Example 247. The reaction mixture was stirred at 80 °C for 14 hours, poured into water. The reaction mixture was neutralized with saturated sodium bicarbonate water and extracted with ethyl acetate. The ethyl acetate layer was washed with water and saturated brine, successively, dried over magnesium sulfate and concentrated. The residue was purified by column chromatography (carrier: silicagel, eluant: hexane-ethyl acetate) to obtain the titled compound (0.30 g) as colorless crystals.
IR (KBr): ν 2228, 1663, 1478, 1429, 866 cm⁻¹.
¹H-NMR (CDCl₃) δ: 2.51 (3H, s), 5.73 (2H, s), 7.29-7.43 (2H, m), 7.62 (2H, d, *J*=8.8 Hz), 7.81 (2H, d, *J*=8.8 Hz), 8.40 (1H, d, *J*=1.6 Hz), 8.58 (1H, dd, *J*=4.8, 1.6 Hz), 9.51 (1H, s).

### Example 251

### Production of 4-(4-cyanophenyl)-5-(hydroxymethyl)-2-methyl-1-(3-pyridinyl methyl)-1H-pyrrole-3-carbonitrile

The compound (51 mg) produced in Example 250 was dissolved in methanol (1.5 ml) and tetrahydrofuran (0.5 ml), and to the solution was added sodium boron hydride (8 mg). The reaction mixture was stirred at room temperature for 30 minutes and concentrated. The residue was diluted with saturated brine and extracted with ethyl acetate. The ethyl acetate layer was dried over magnesium sulfate and concentrated to obtain the titled compound (50 mg) as colorless crystals.
IR (KBr): ν 3202, 2224, 1609, 1427, 1024, 862, 710, 559 cm⁻¹.
¹H-NMR (CDCl₃+ DMSO-*d*₆) δ:2.35 (3H, s), 4.45 (2H, d, *J*=4.8 Hz), 4.63 (1H, t, *J*=4.8 Hz), 5.36 (2H, s), 7.28-7.31 (2H, m), 7.67 (2H, d, *J*=8.8 Hz), 7.73 (2H, d, *J*=8.8 Hz), 8.39 (1H, s), 8.56 (1H, t, *J*=3.4 Hz).

Formulas of the compounds produced in Reference Examples and Examples mentioned above are shown in the following tables 1 to 31.

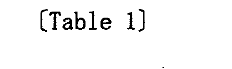

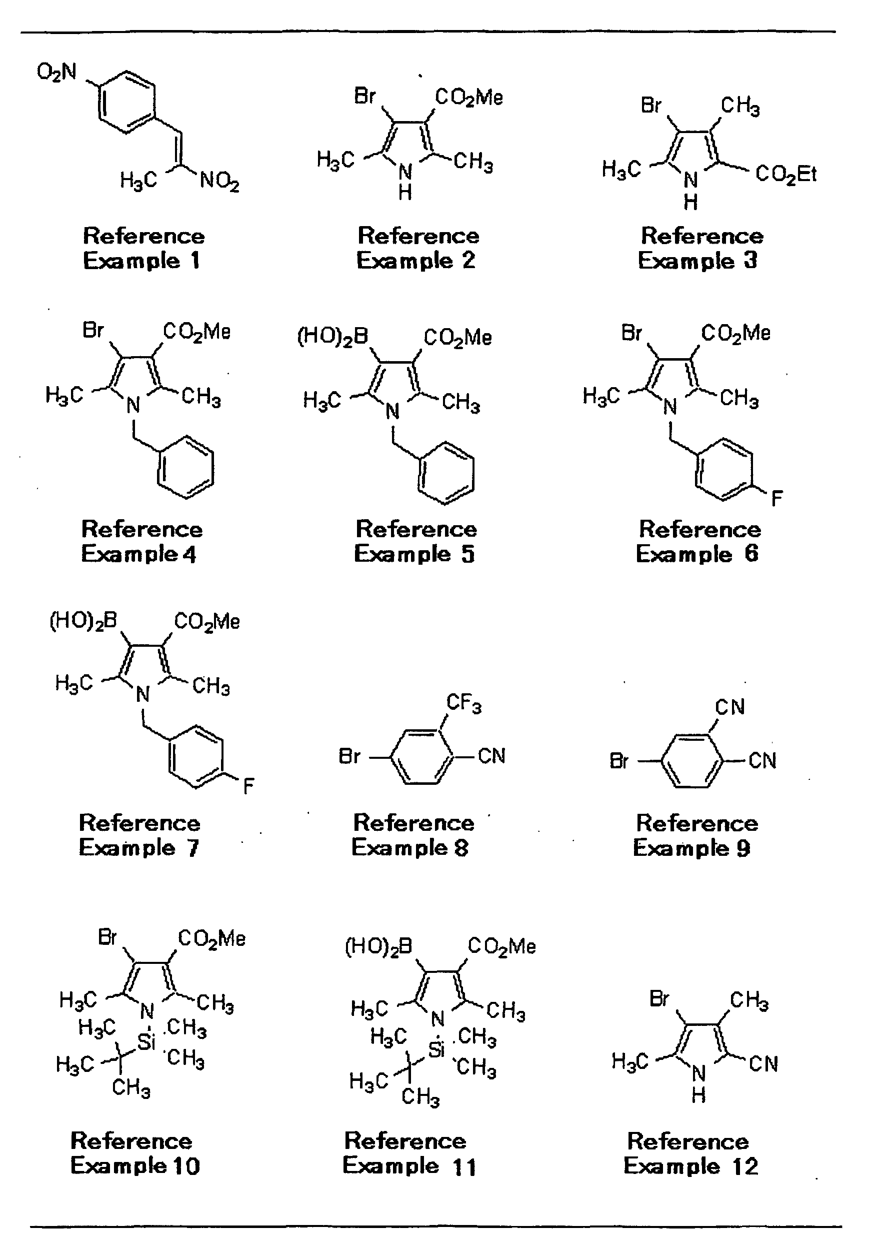

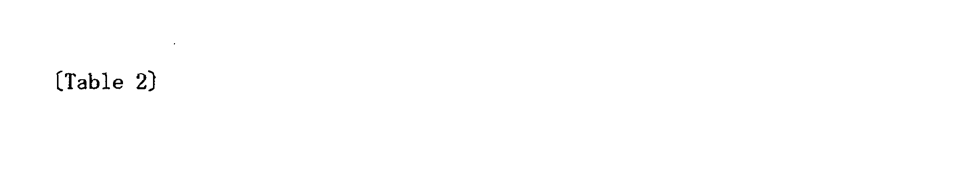

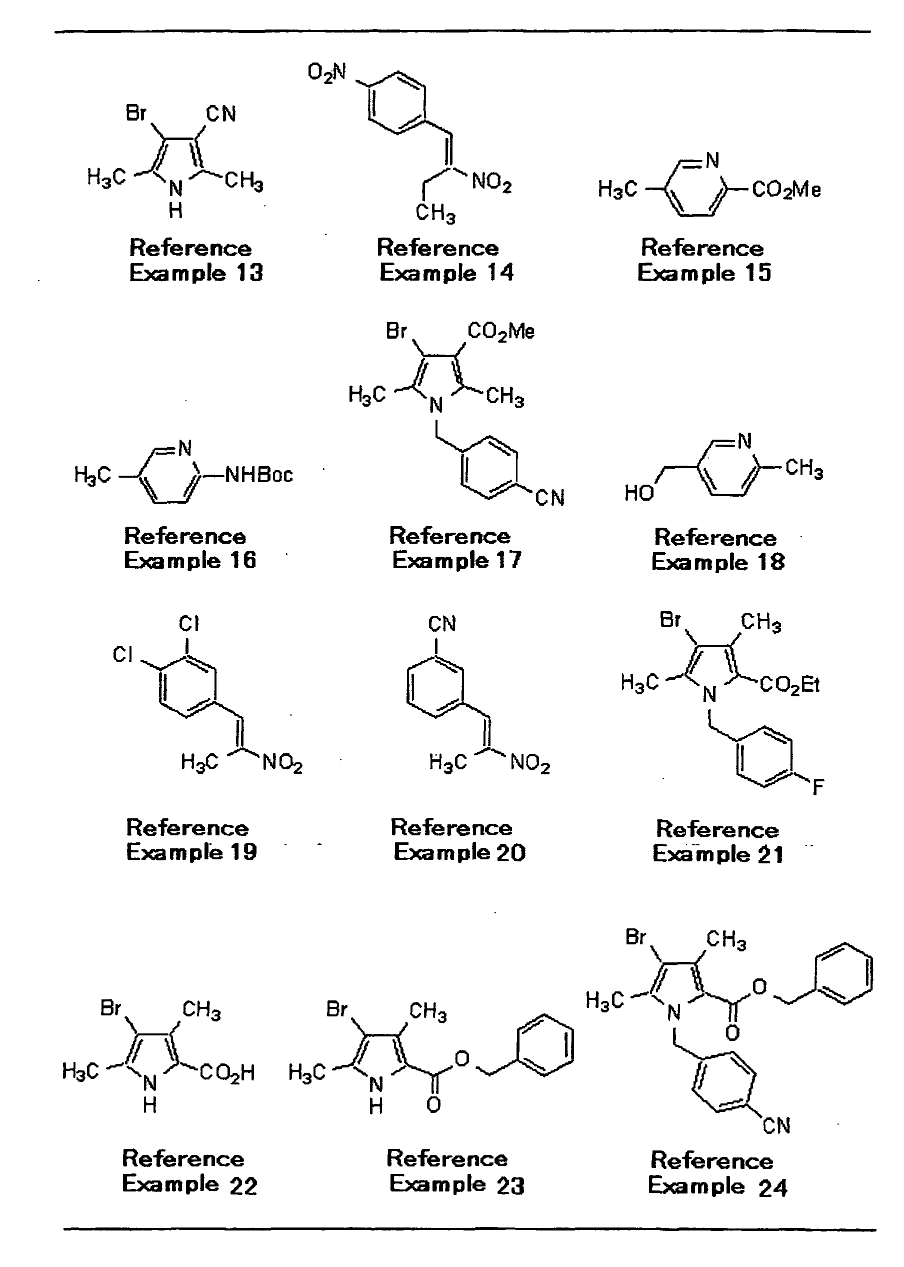

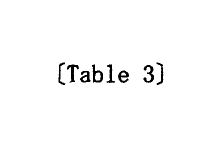

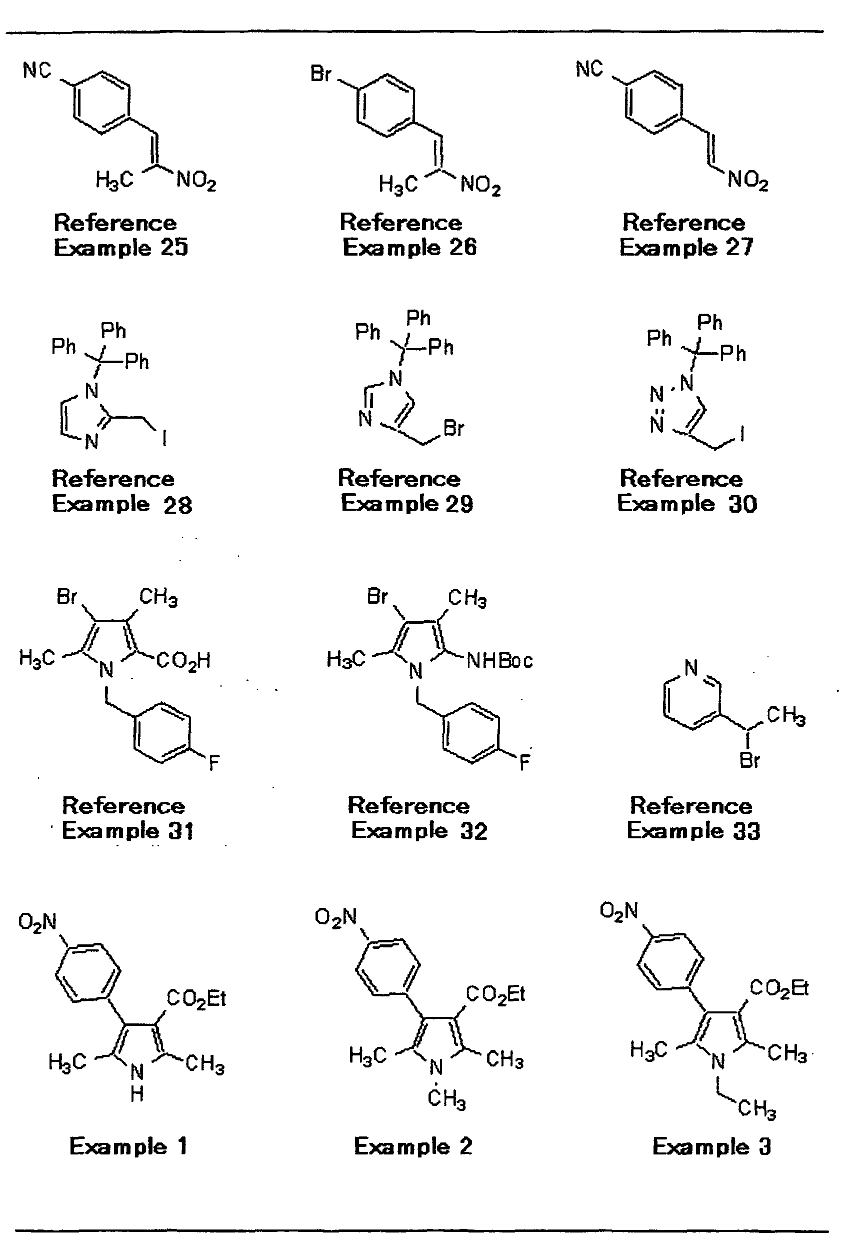

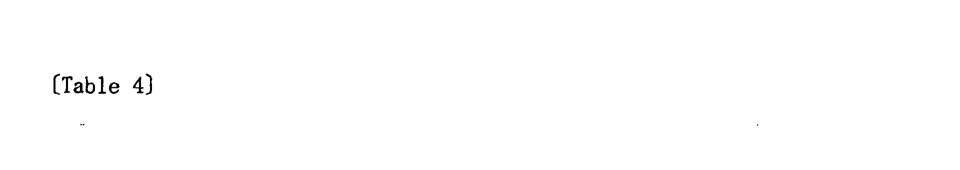

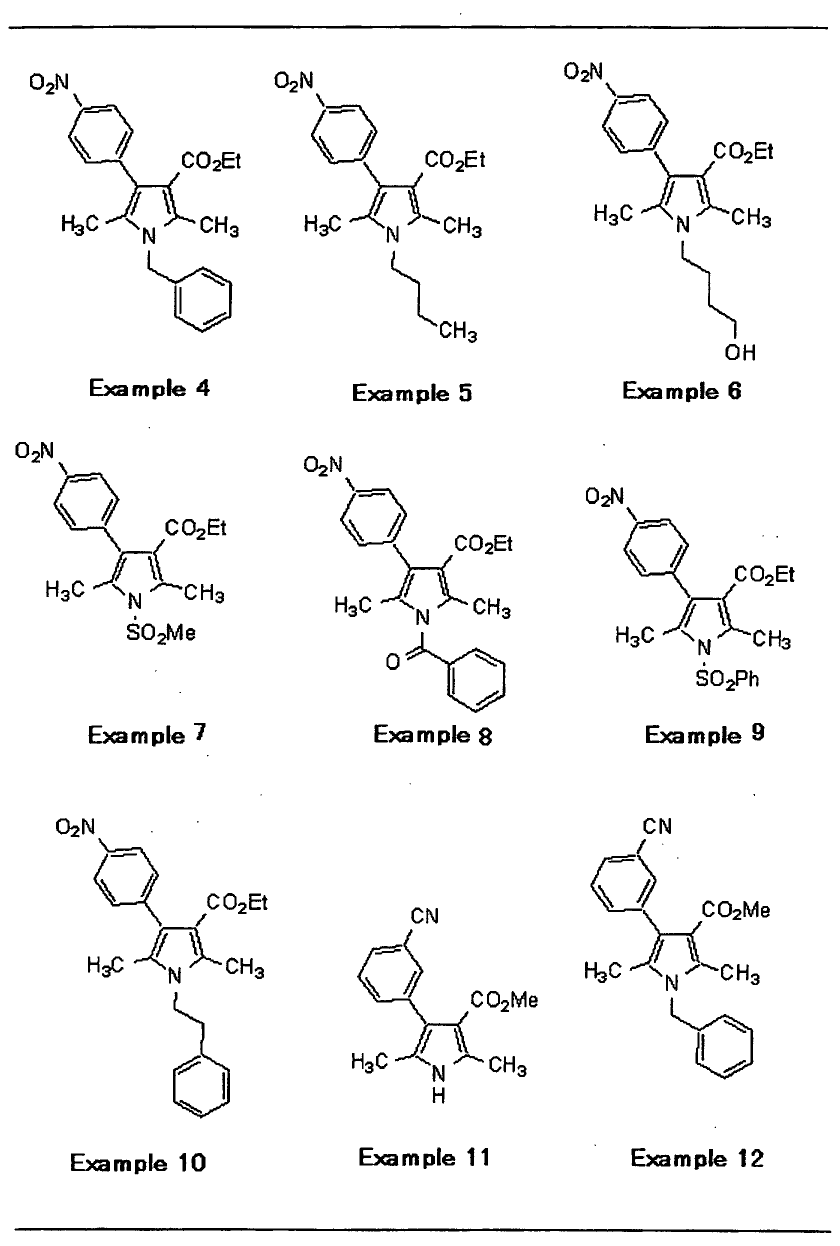

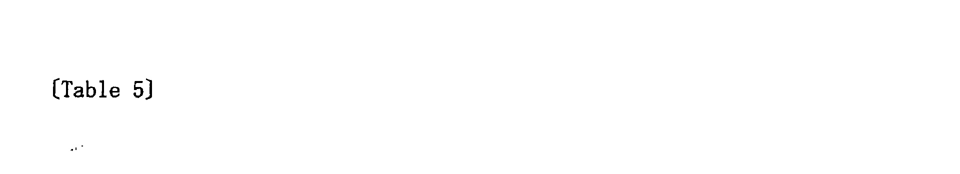

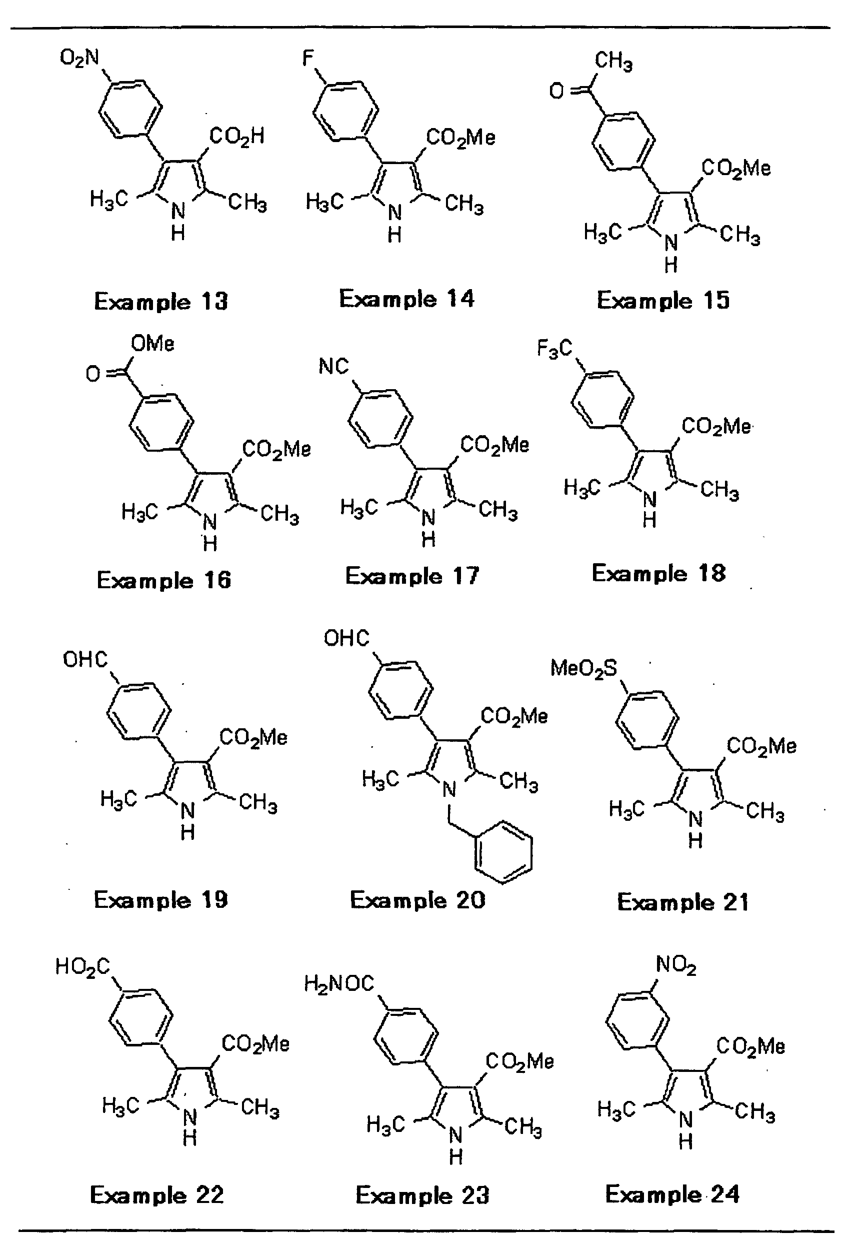

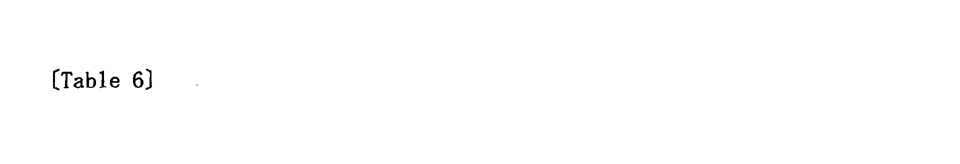

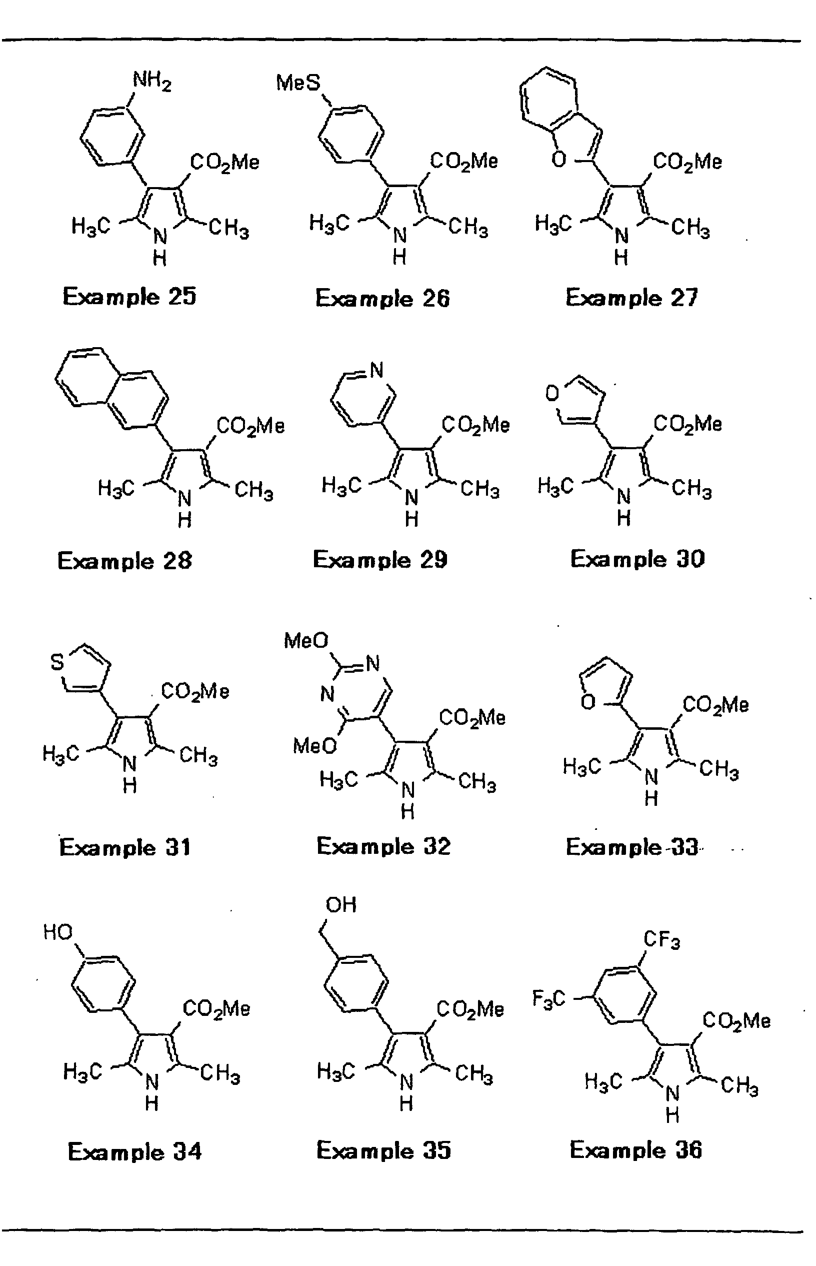

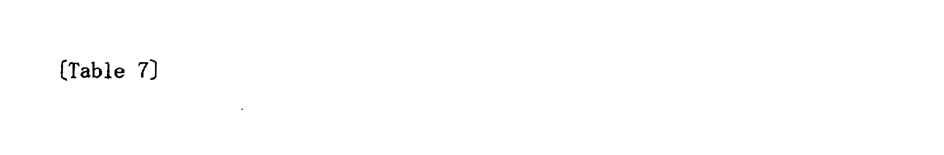

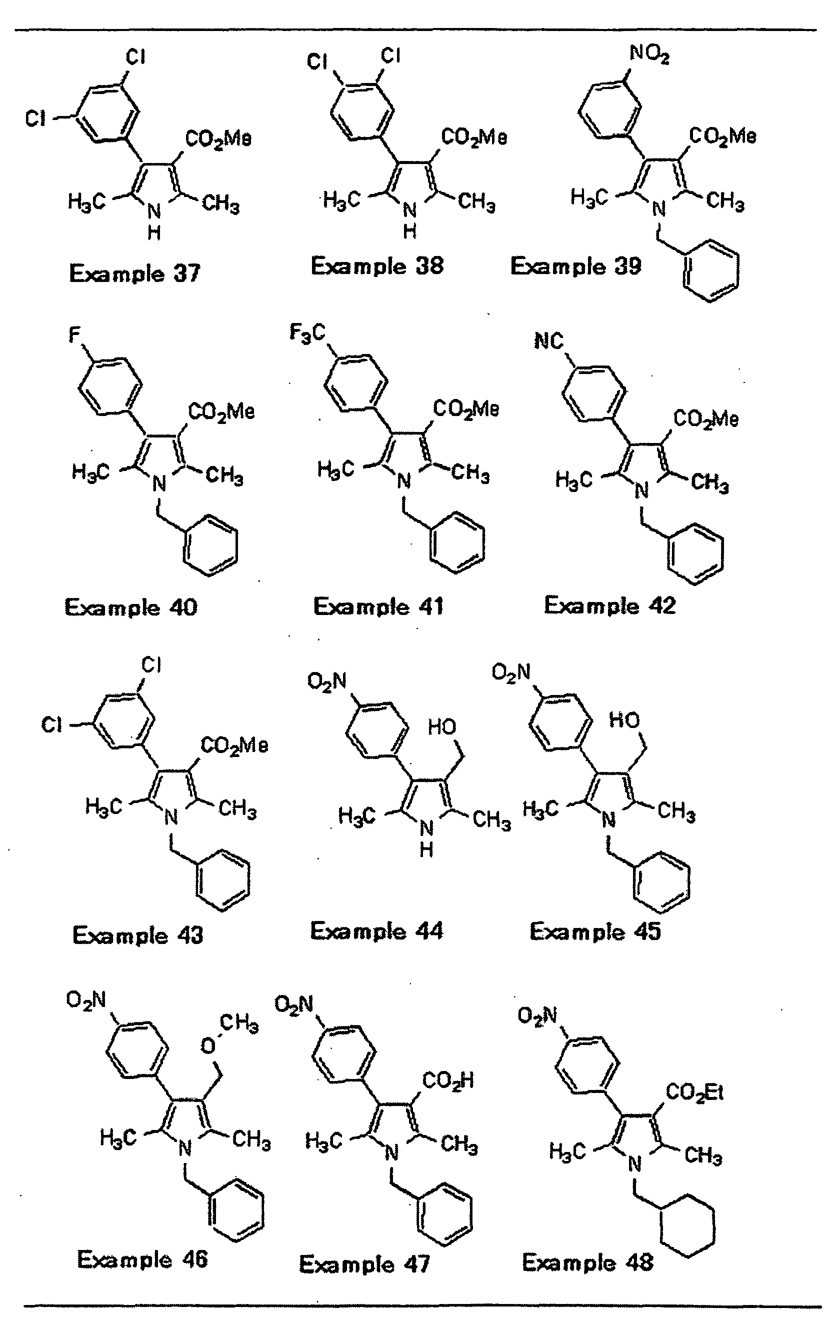

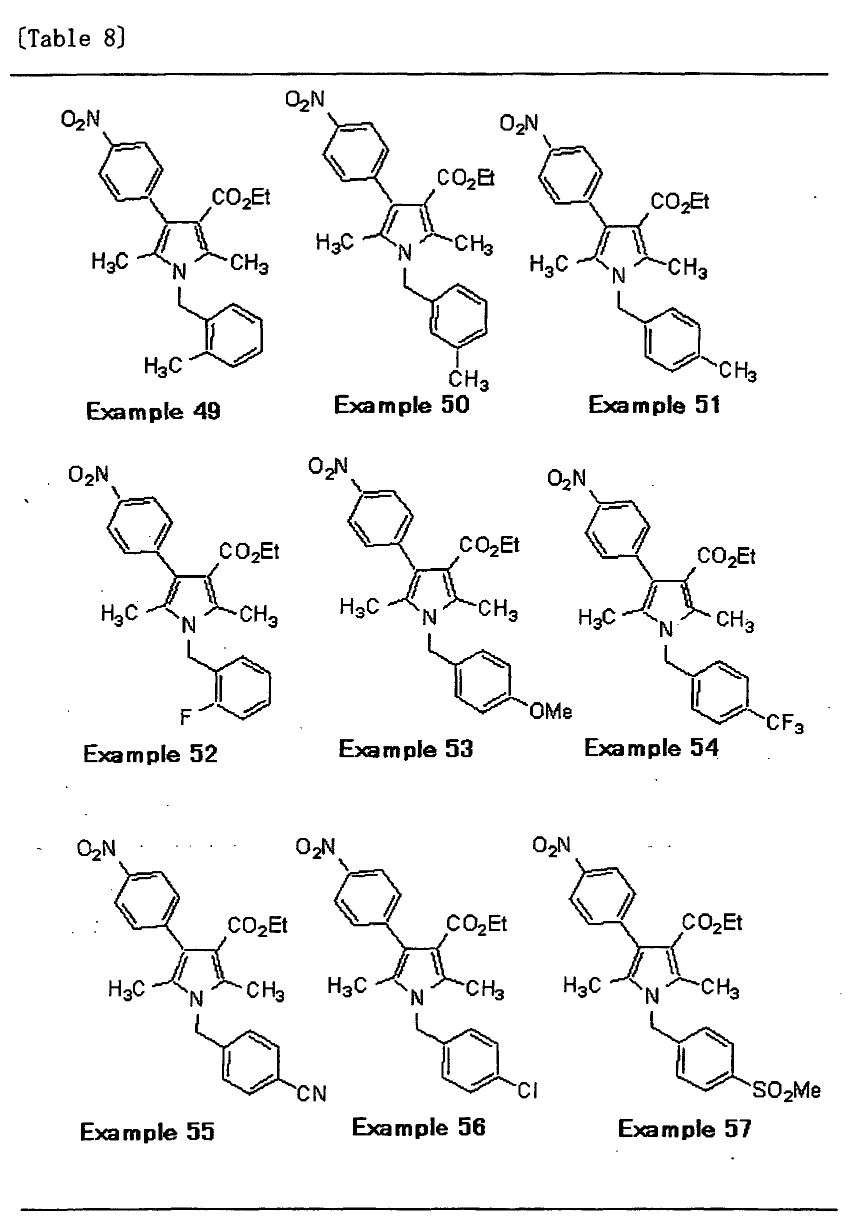

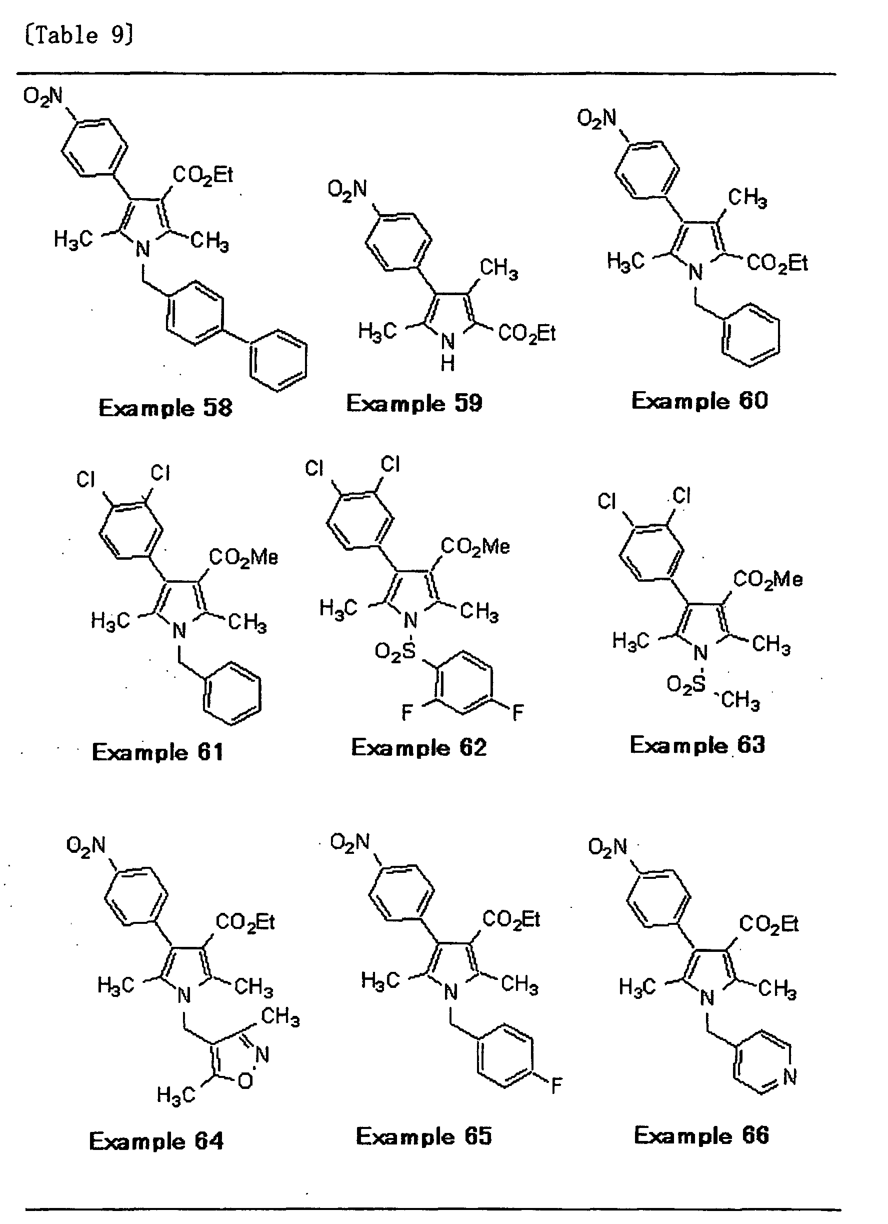

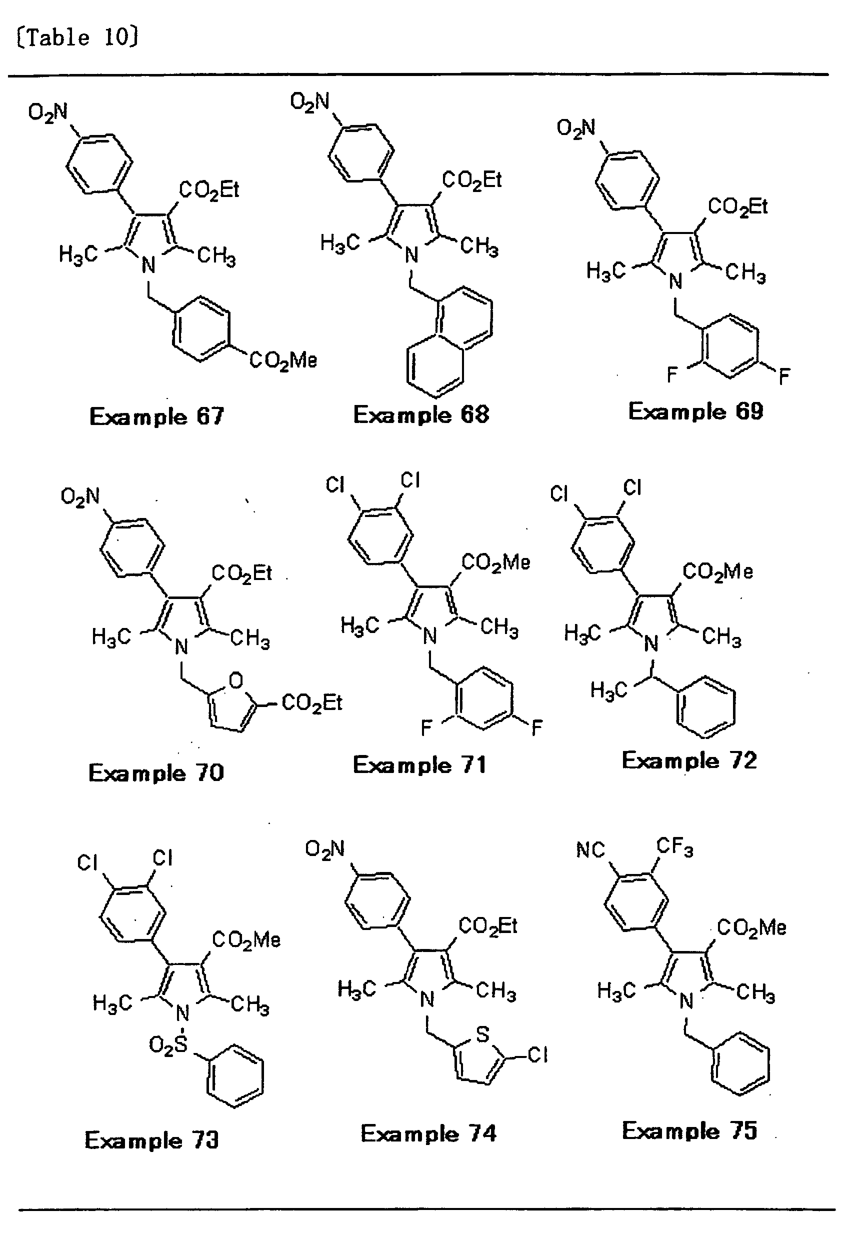

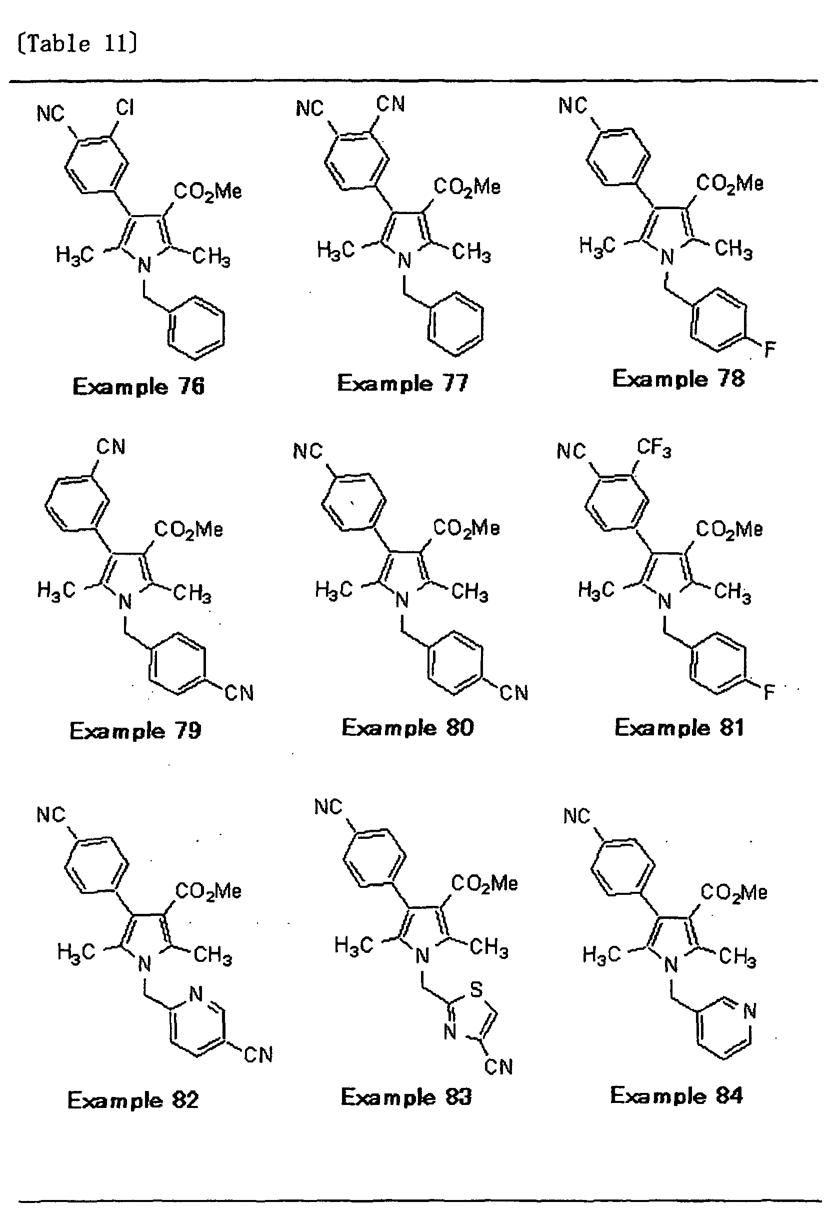

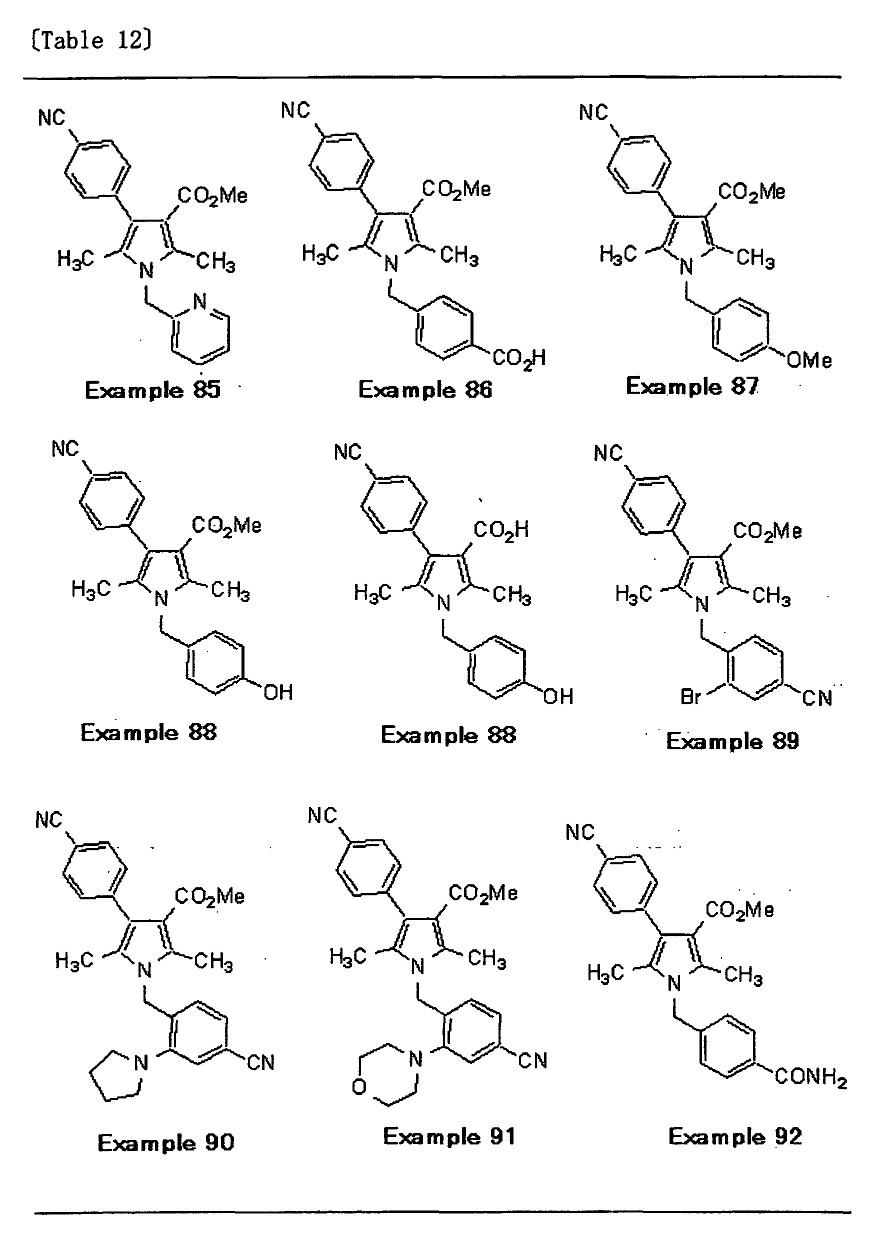

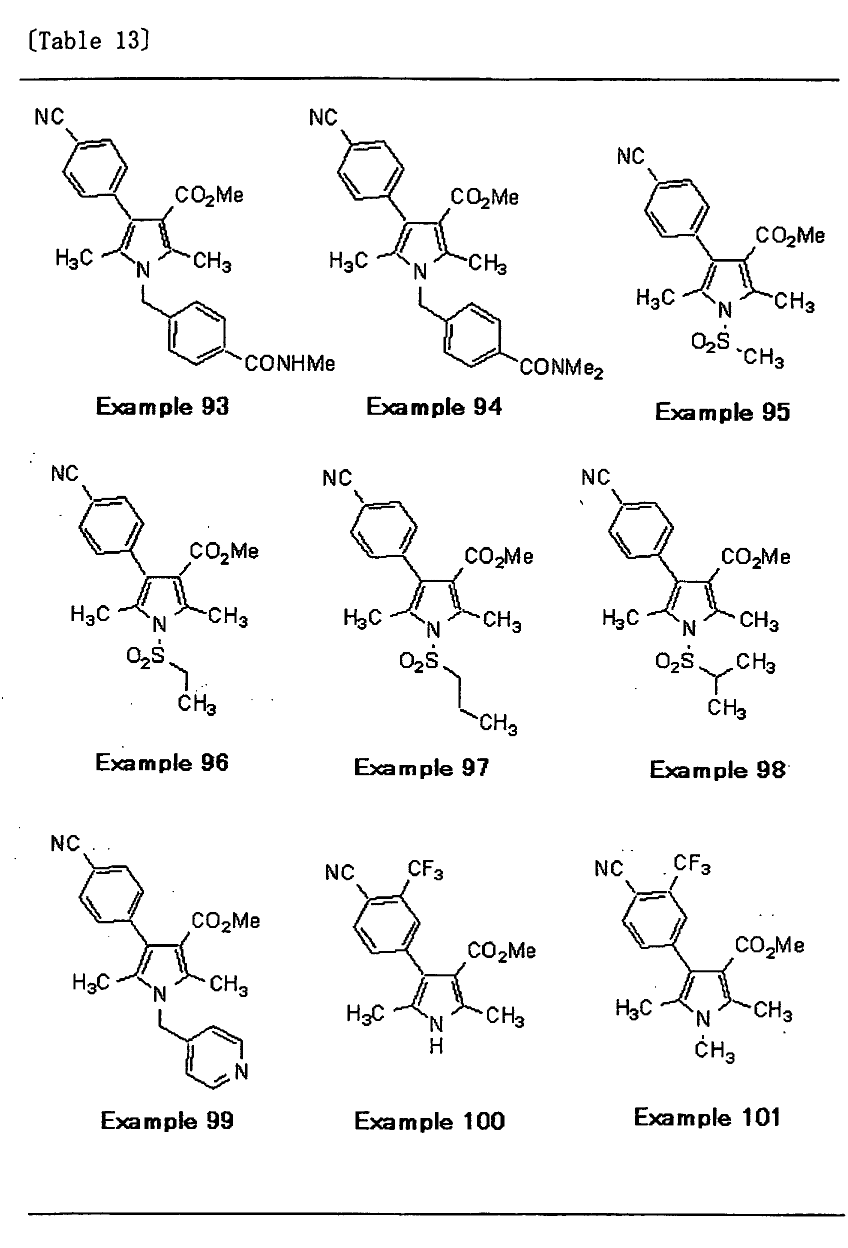

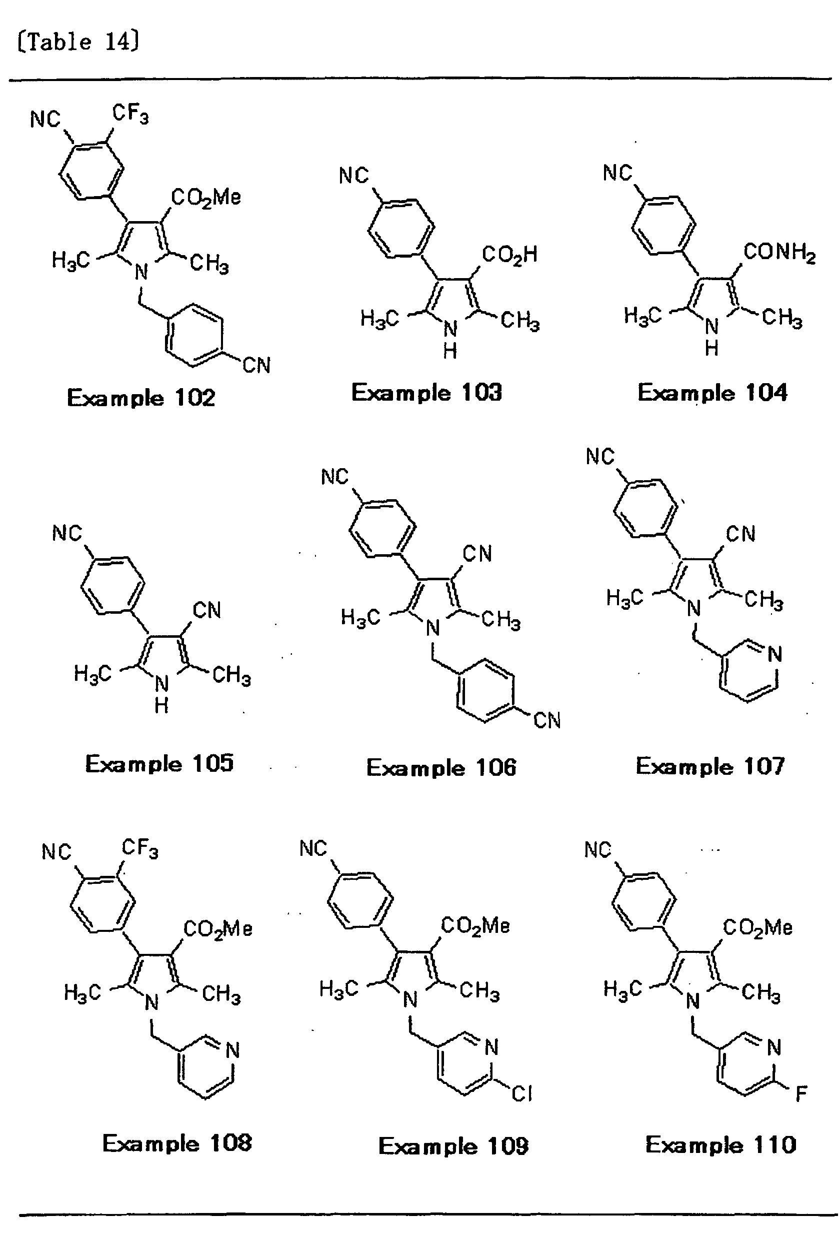

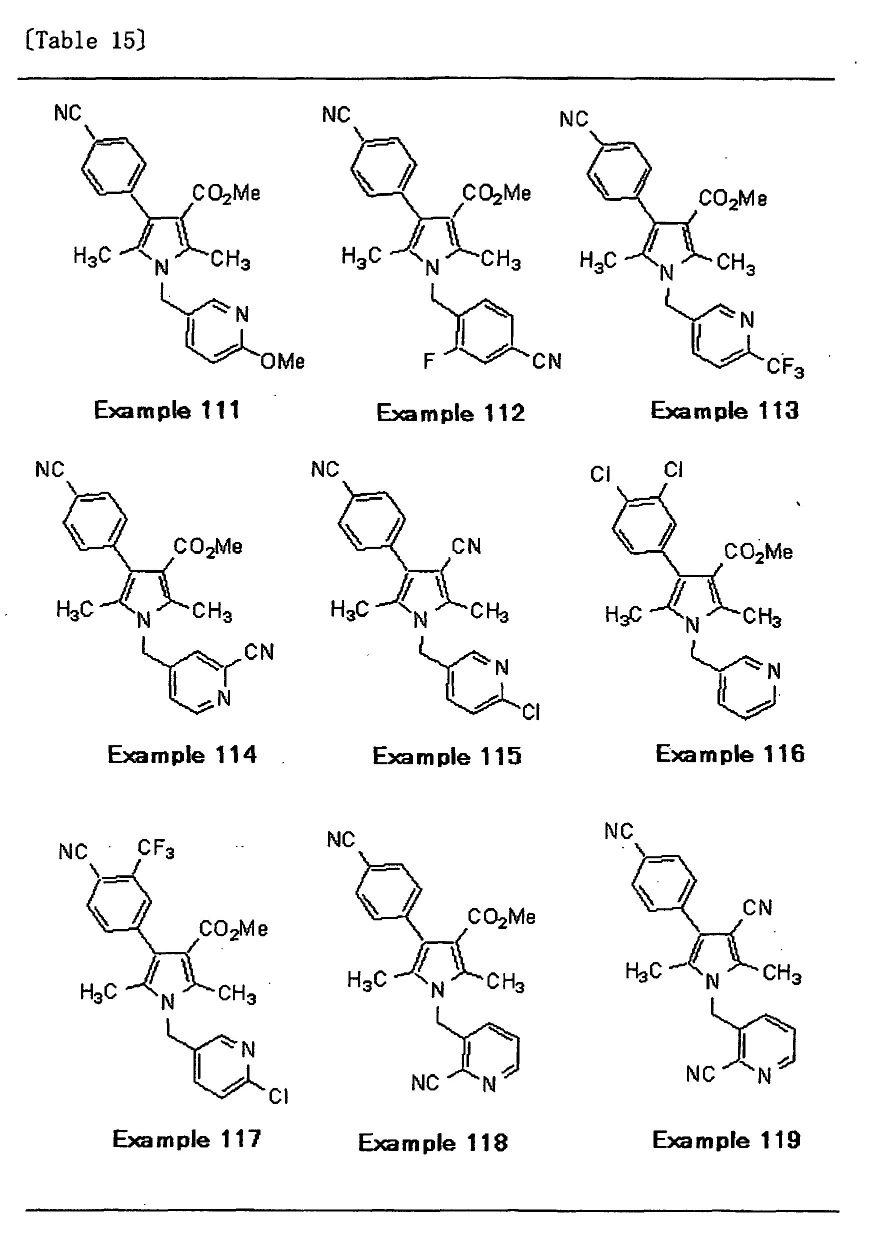

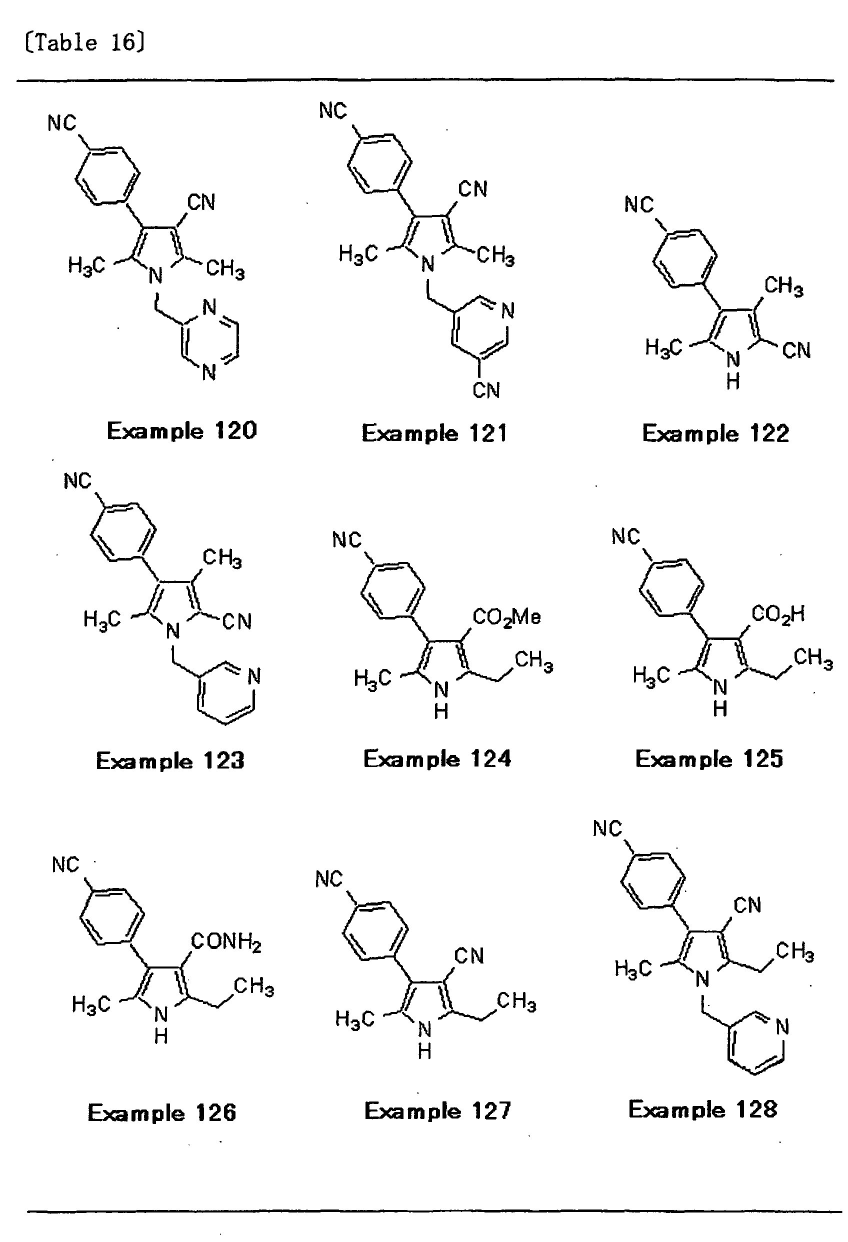

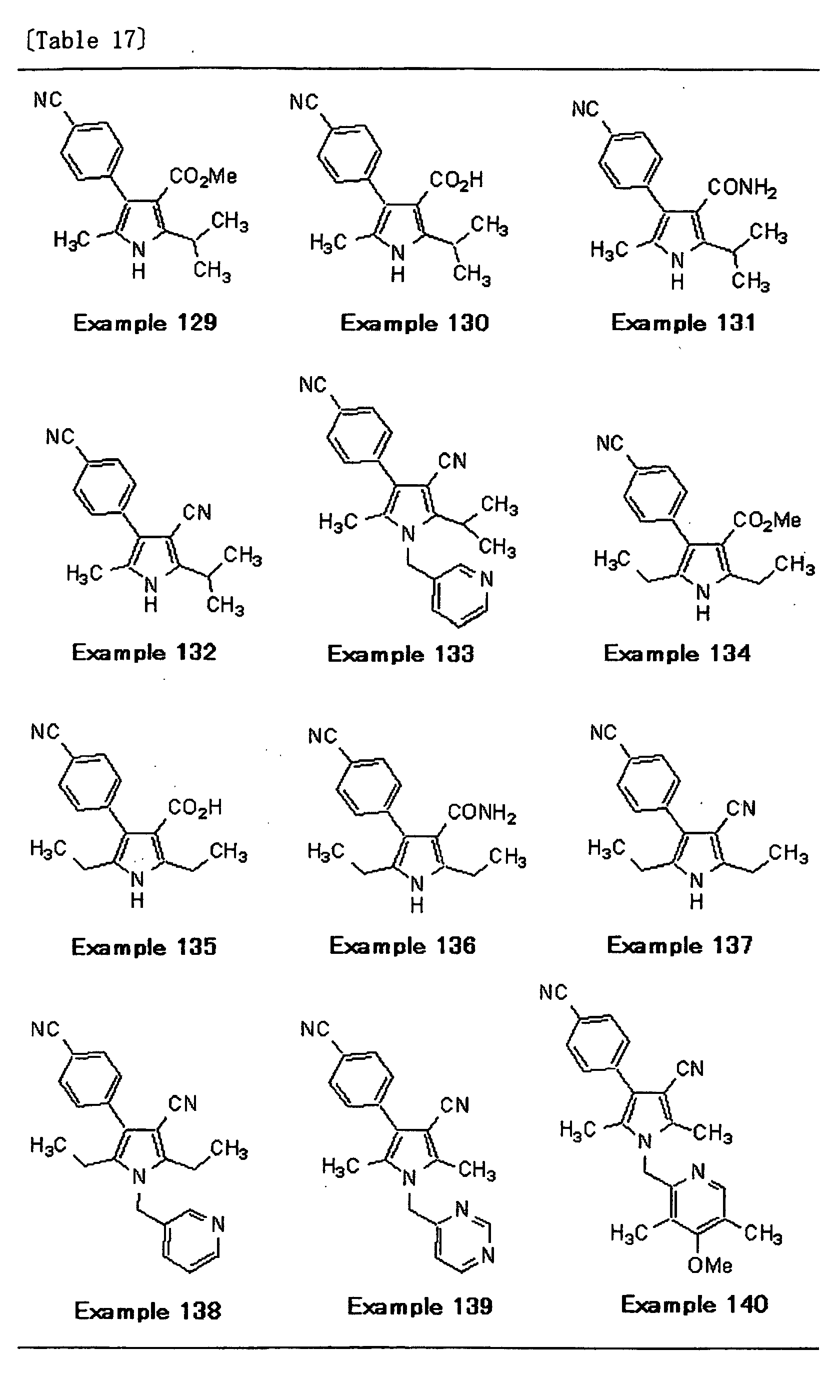

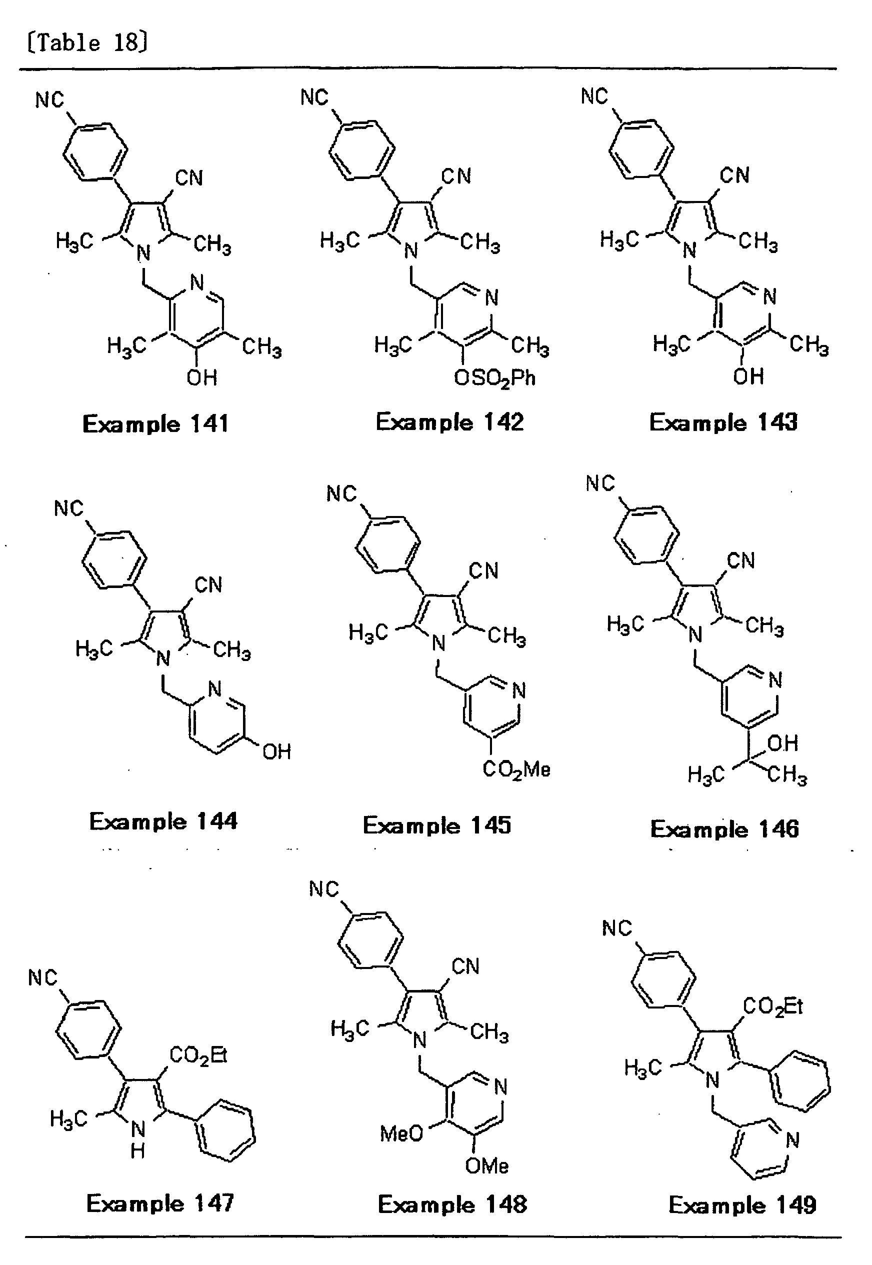

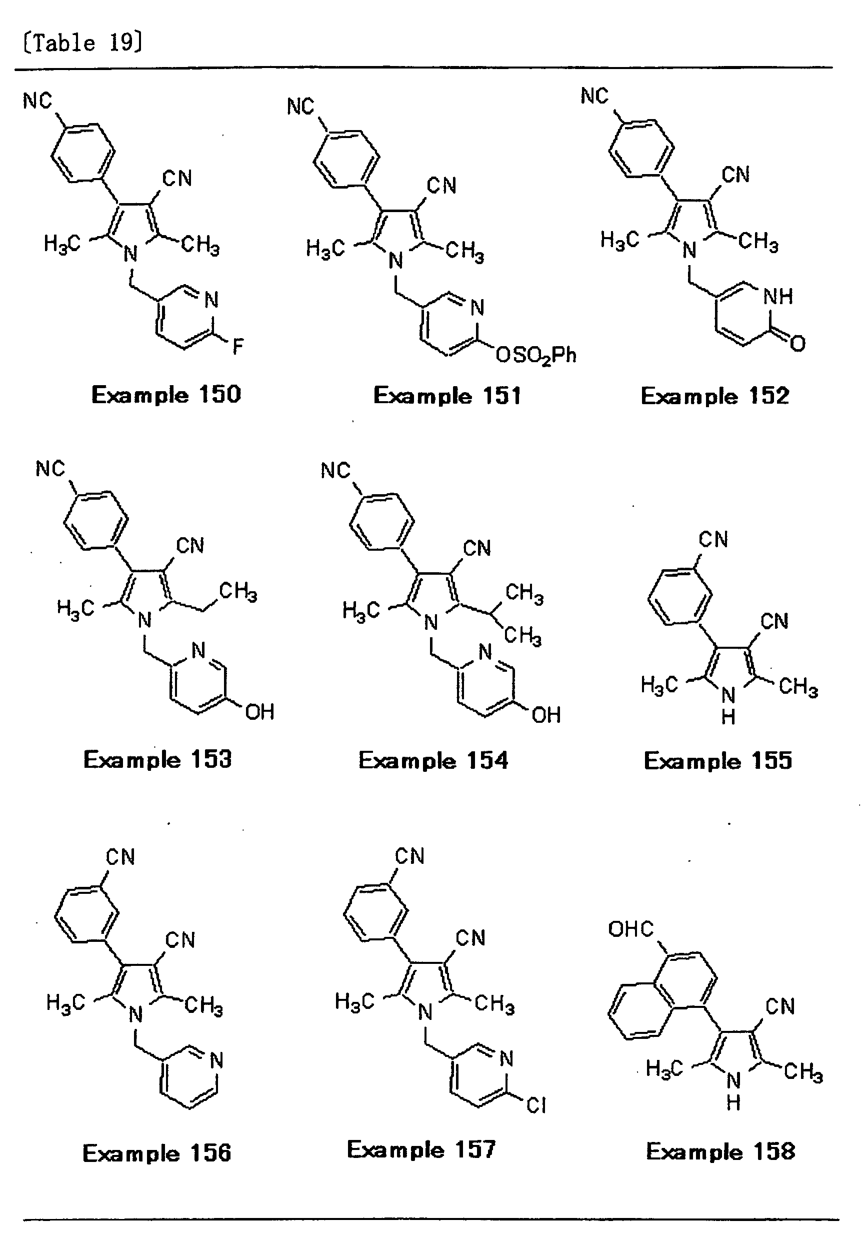

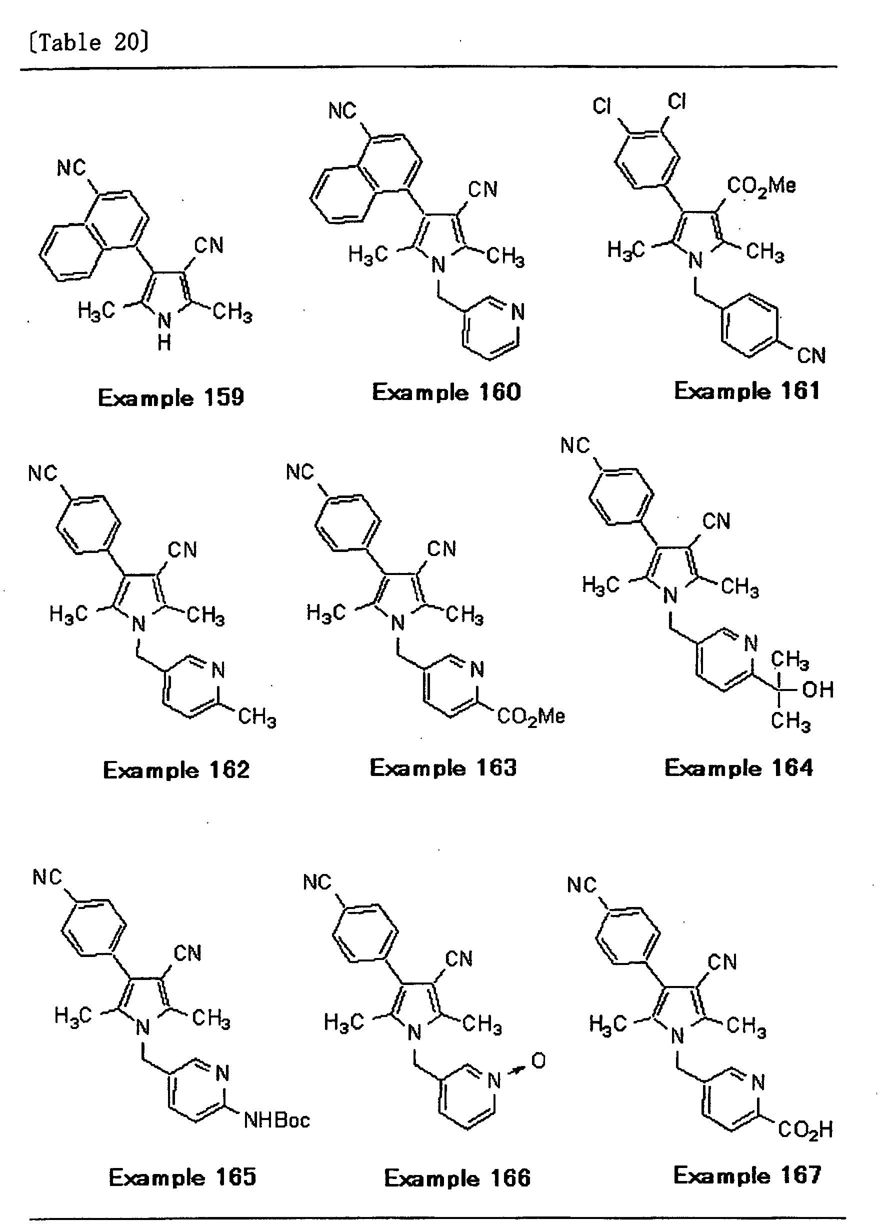

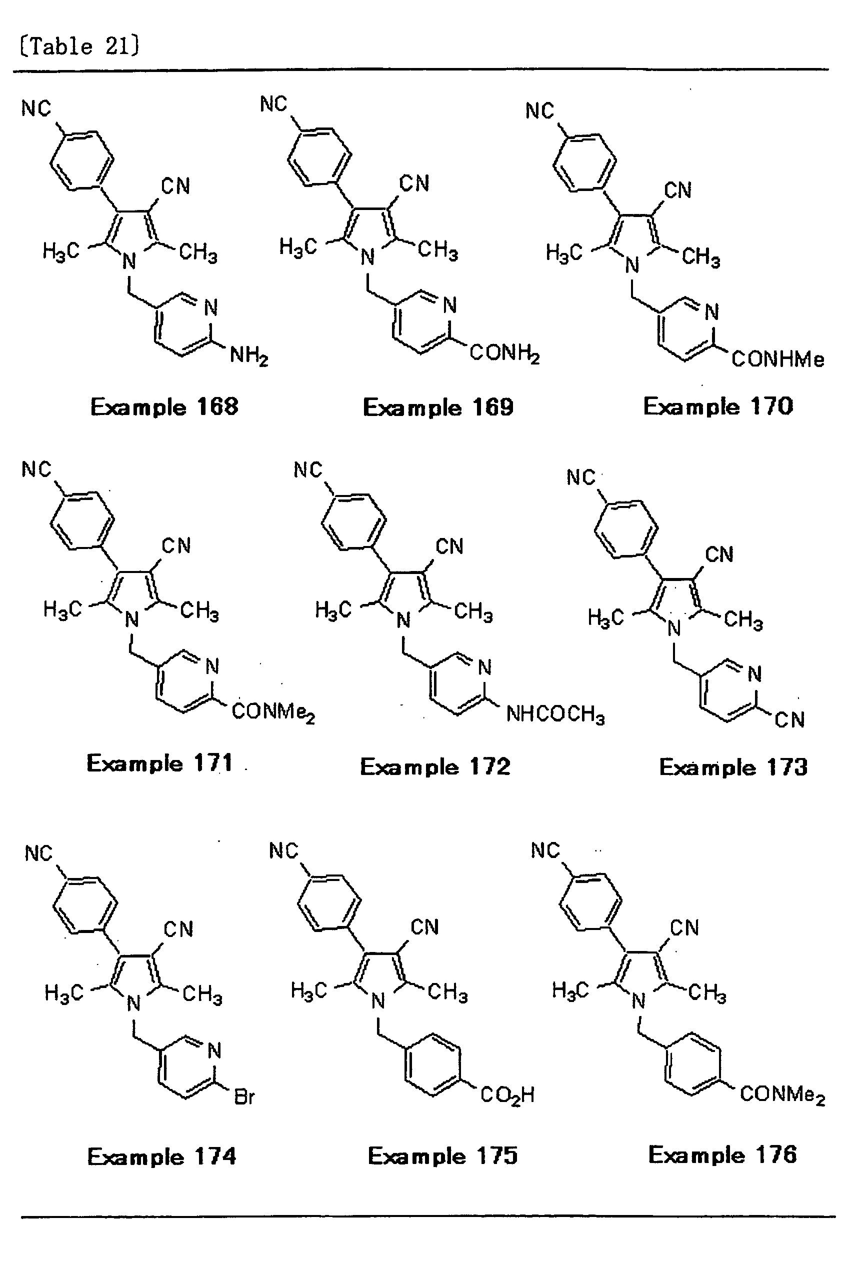

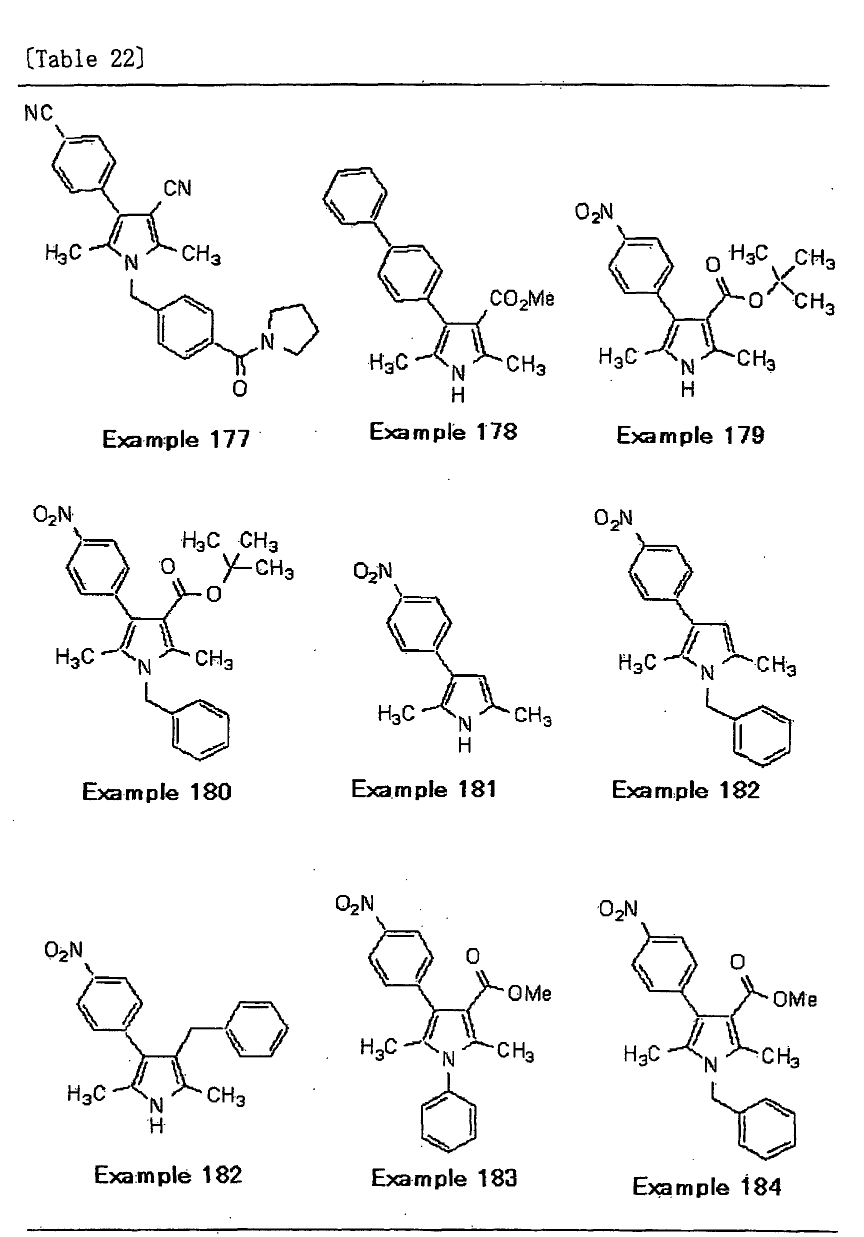

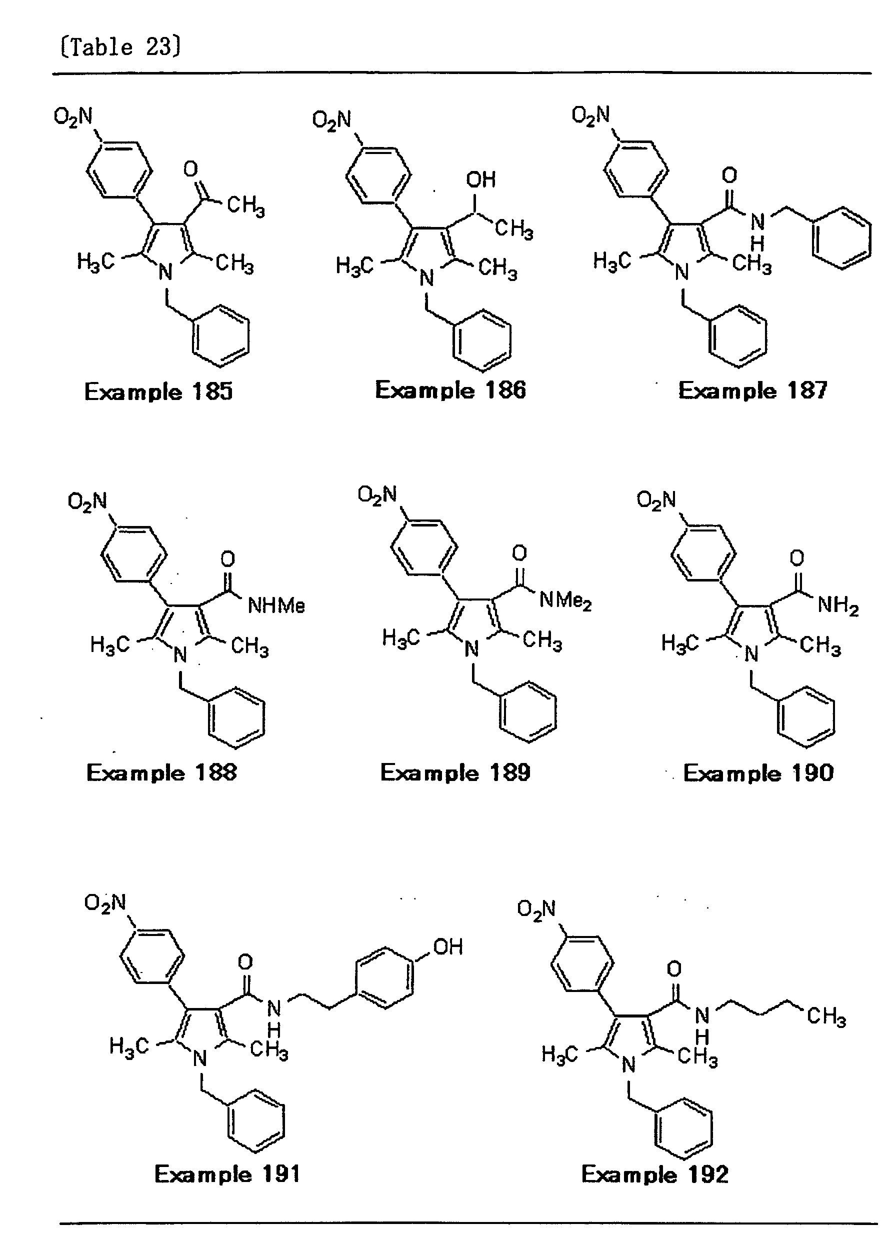

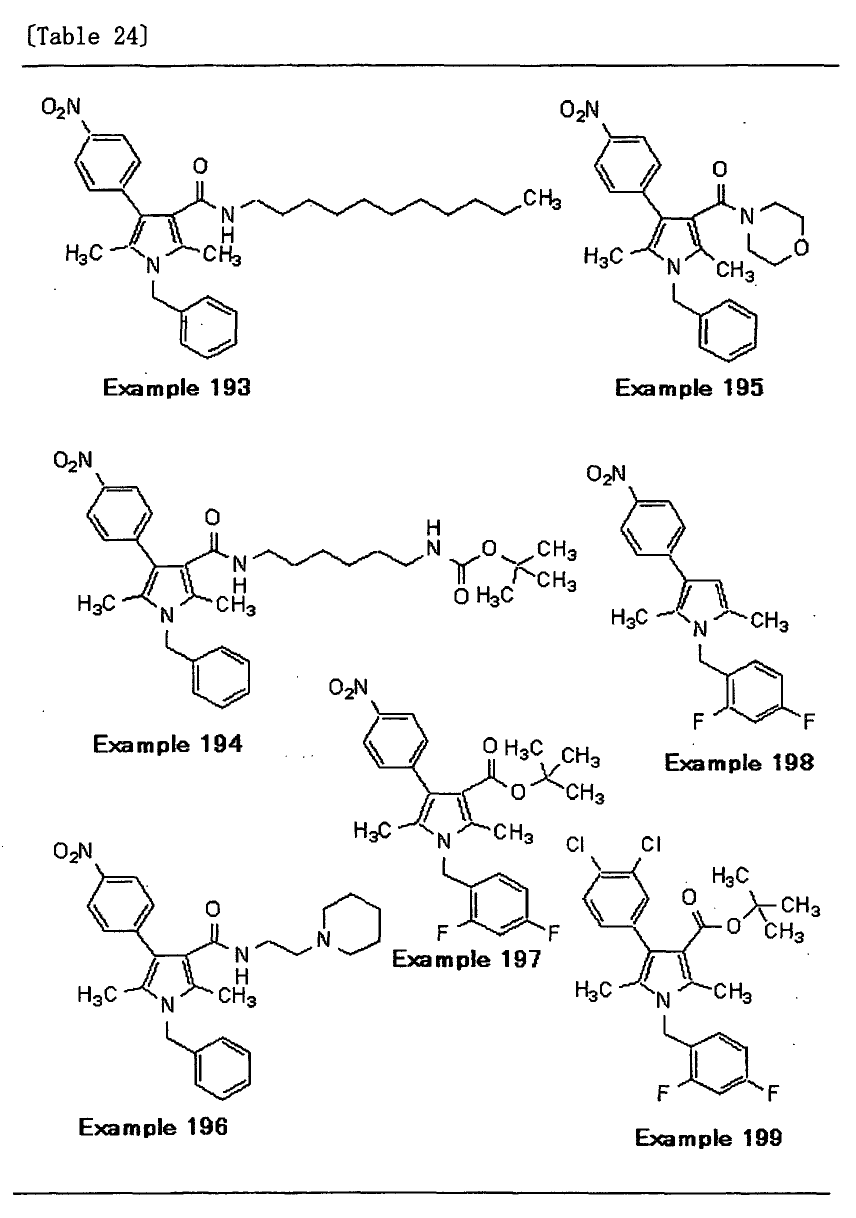

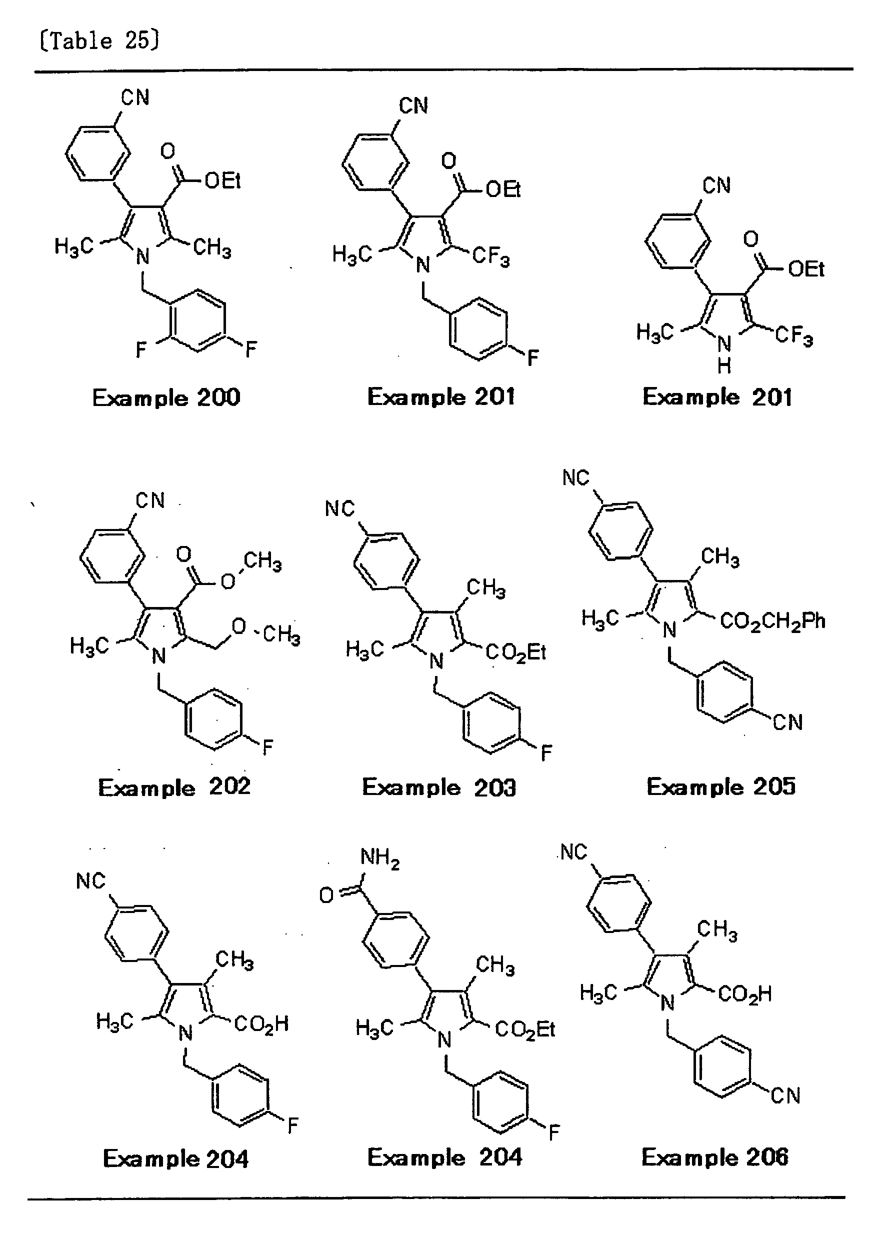

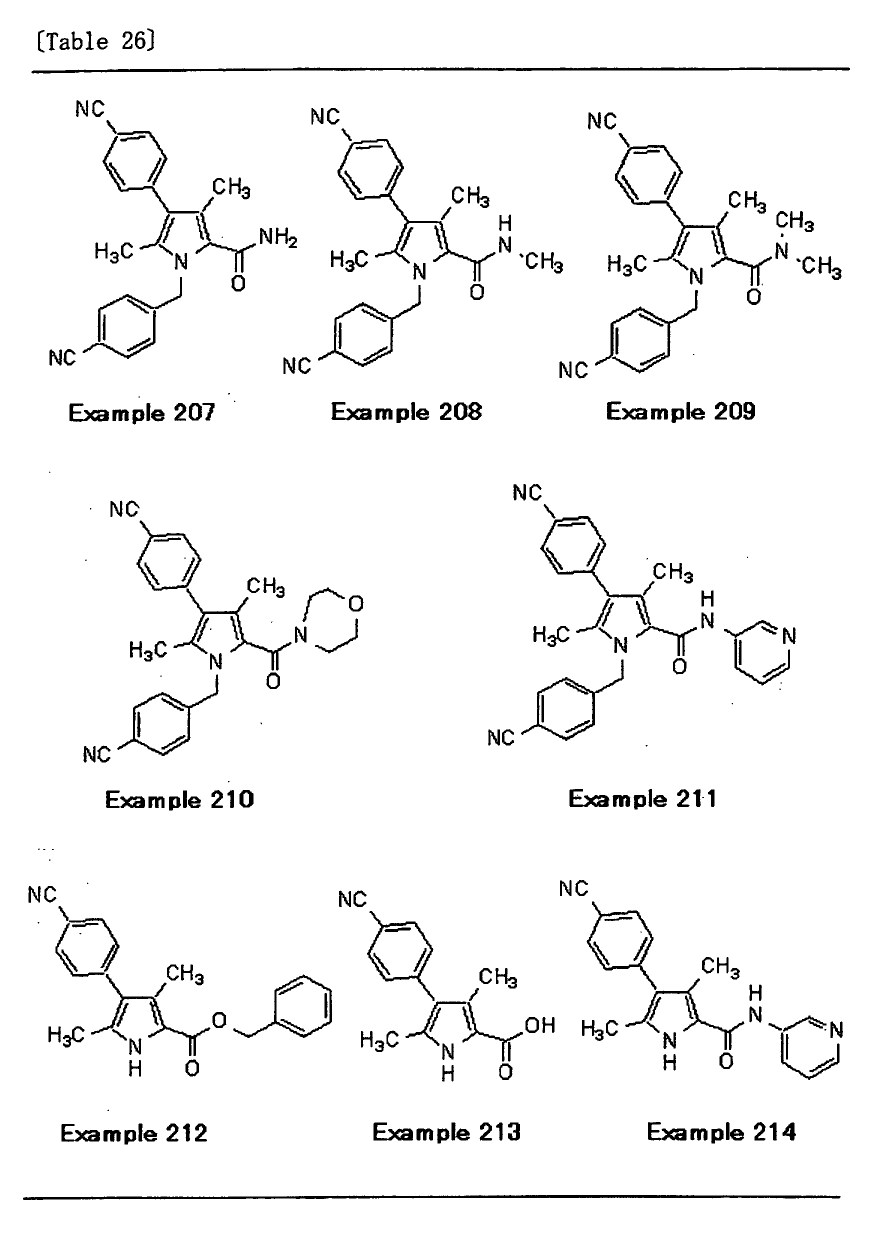

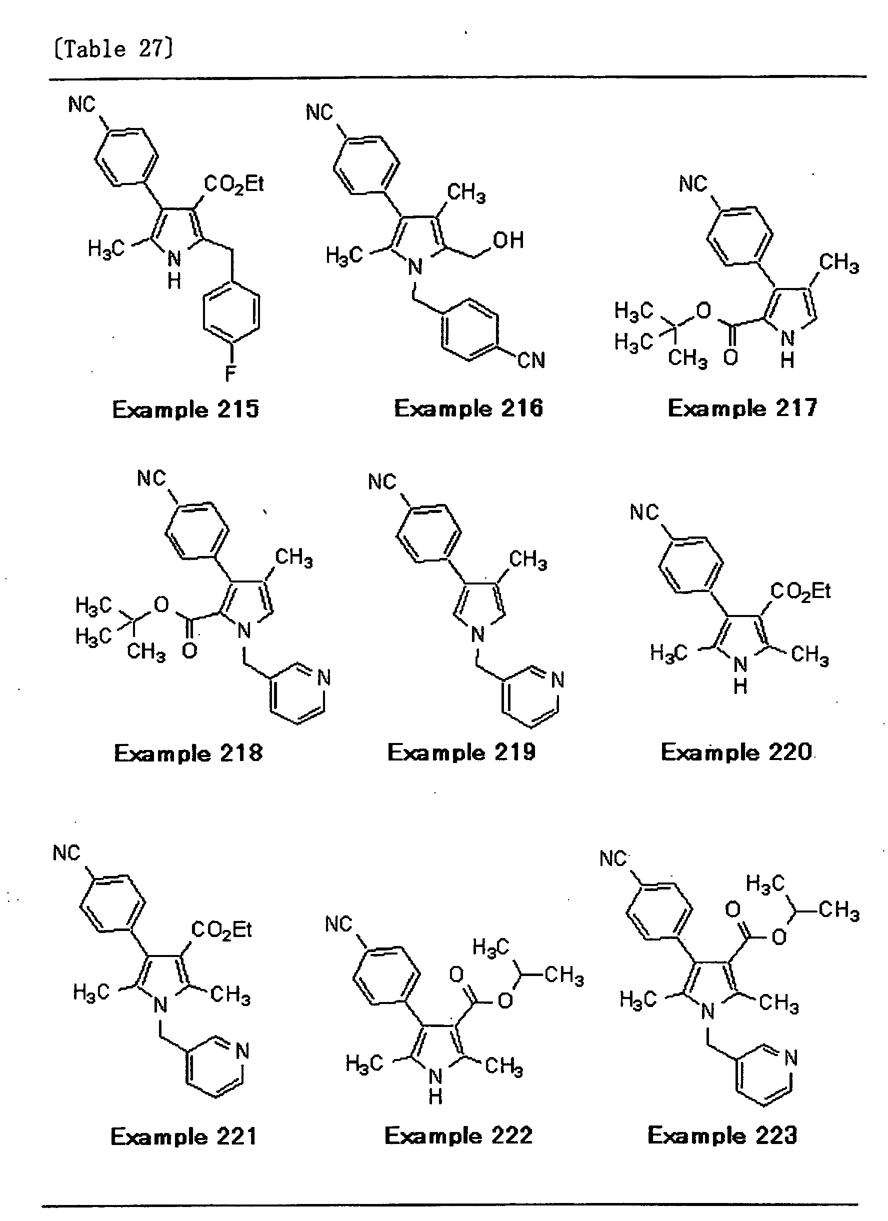

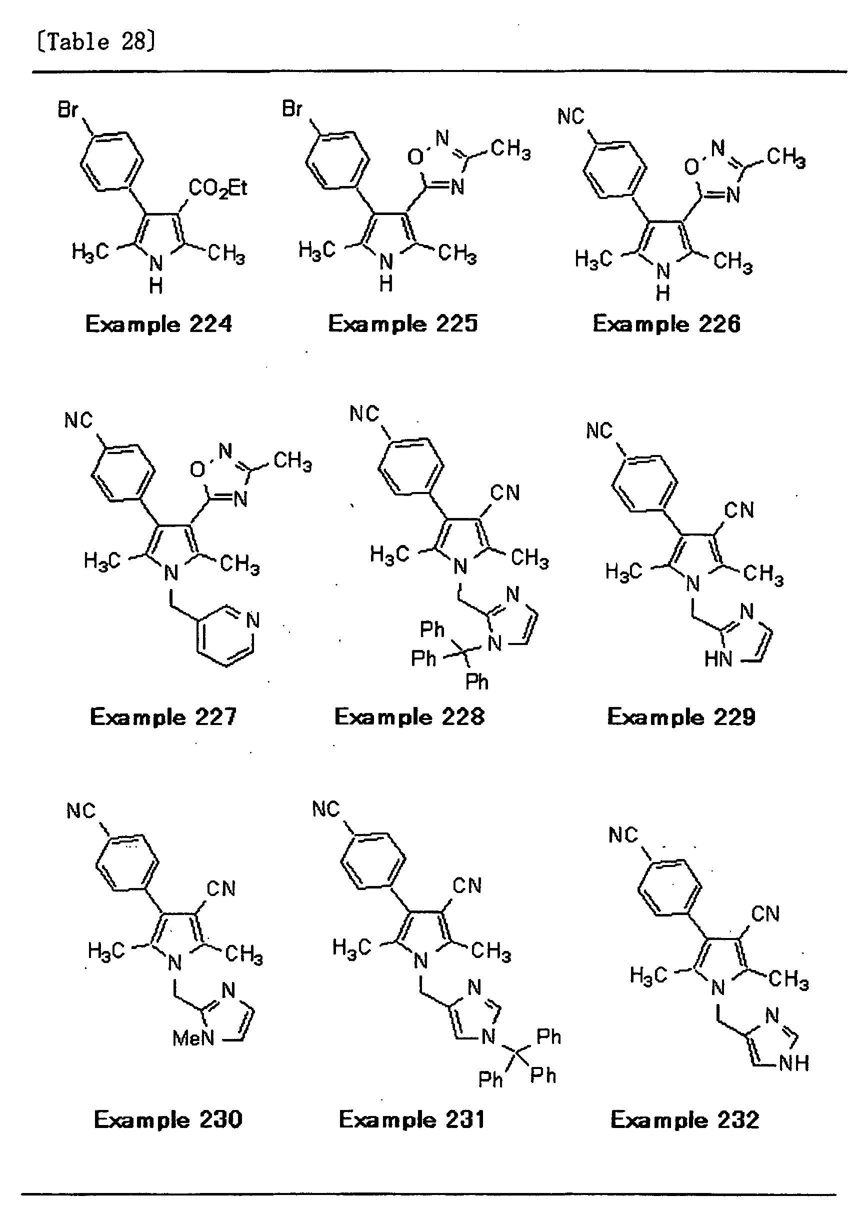

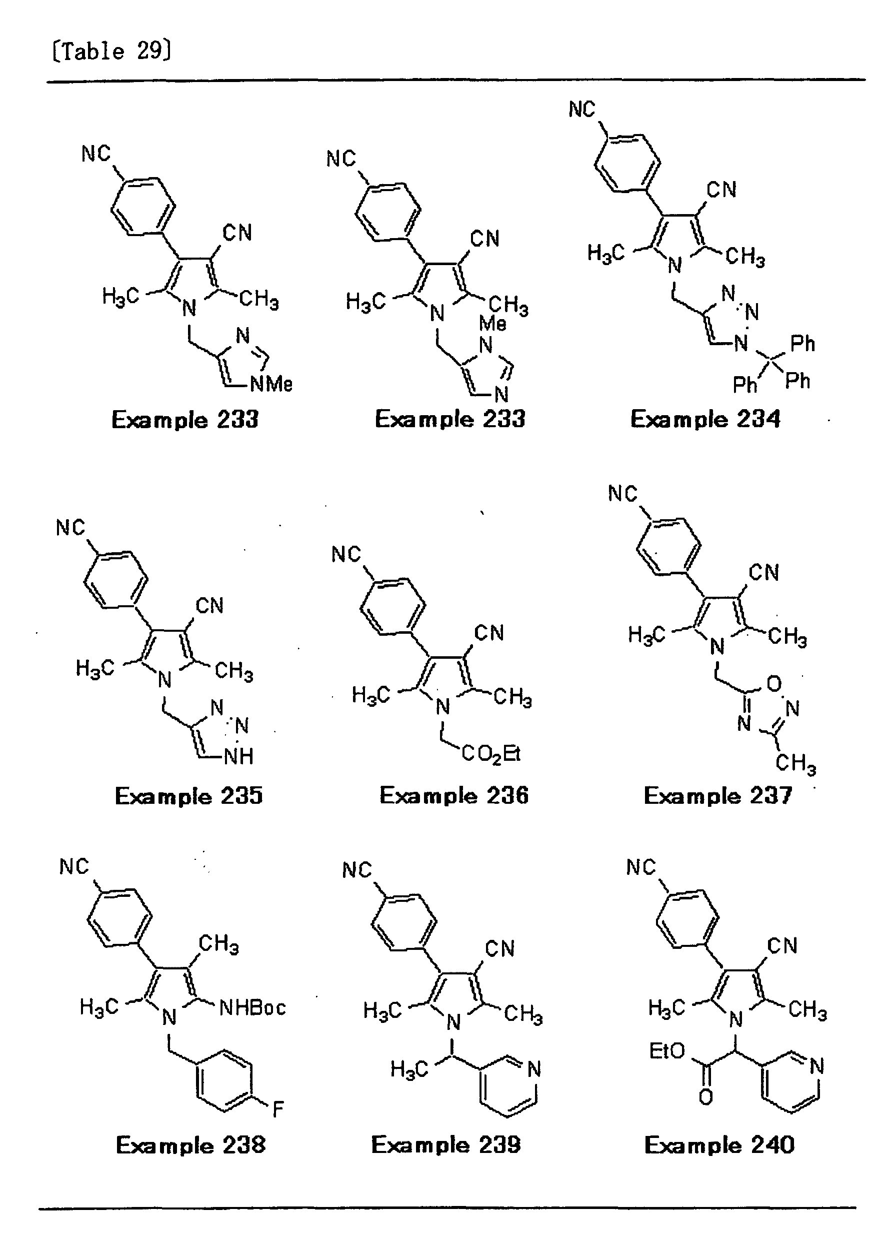

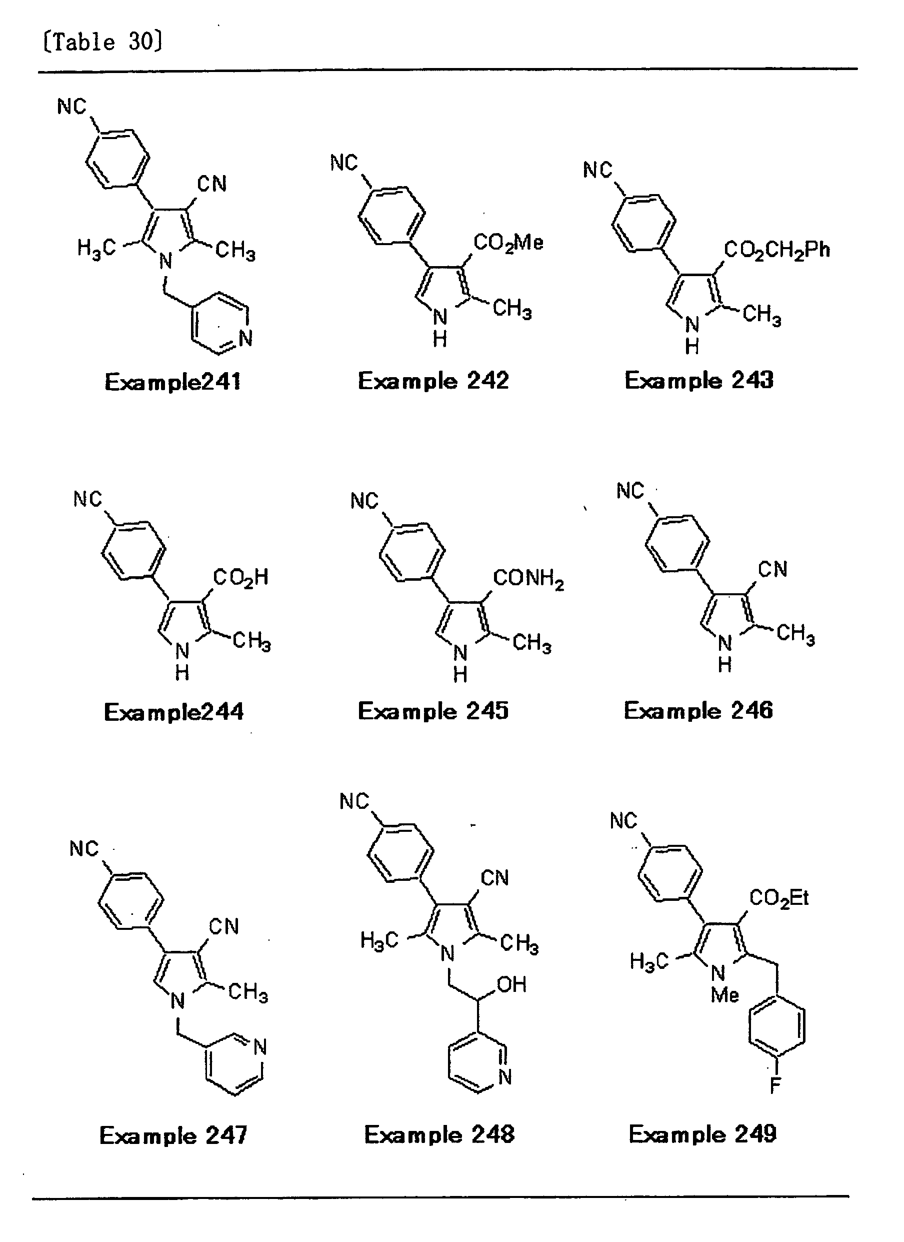

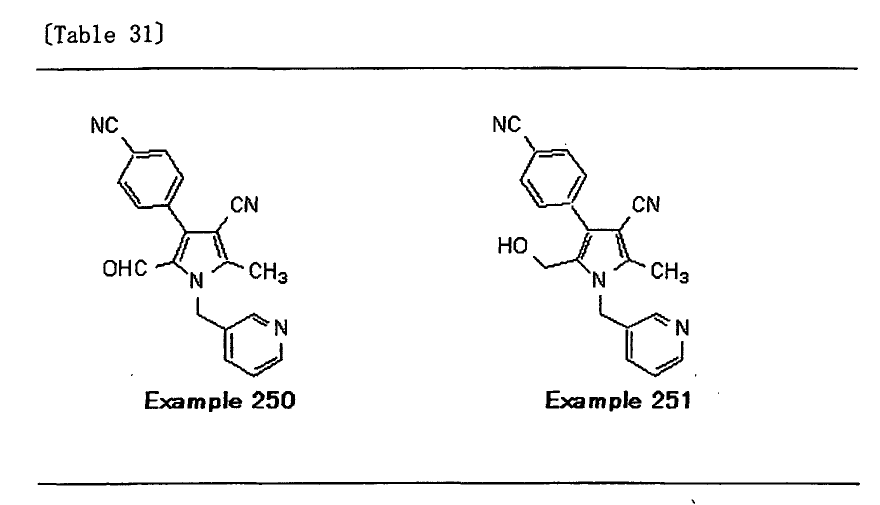

| Pharmaceutical preparation 1 | | |
|---|---|---|
| (1) | Compound of Example 4 | 5.0 mg |
| (2) | Sodium chloride | 20.0 mg |
| (3) | Distilled water To make total amount | 2 ml |

The compound of Example 4 (5. 0 mg) and sodiumchloride (20.0 mg) are dissolved in distilled water, and to the solution is added water to make the total volume 2 ml. The solution is filtered, and filled into an ampoule (content: 2 ml) under sterilized conditions. The ampoule is sterilized and sealed to obtain an injectable solution.

| Pharmaceutical preparation 2 | | |
|---|---|---|
| (1) | Compound of Example 4 | 50 mg |
| (2) | Lactose | 34 mg |
| (3) | Corn starch | 10.6 mg |
| (4) | Corn starch (in paste form) | 5 mg |
| (5) | Magnesium stearate | 0.4 mg |
| (6) | Carboxymethyl cellulose calcium | 20 mg |
| | Total 120 mg | |

In accordance with conventional methods, the above (1) to (6) were mixed and tabletted by means of a tabletting machine to obtain a tablet.

| Pharmaceutical Preparation 3 | | |
|---|---|---|
| (1) | Compound of Example 4 | 5.0 mg |
| (2) | Sodium chloride | 20.0 mg |
| (3) | Distilled water To make the total amount | 2 ml |

The compound of Example 4 (5.0 mg) and sodium chloride (20.0 mg) are dissolved in distilled water, and to the solution is added water to make the total volume 2.0 ml. The solution is filtered, and filled into an ampoule (content: 2 ml) under sterilized conditions. The ampoule is sterilized, and sealed to obtain an injectable solution.

| Pharmaceutical preparation 4 | | |
|---|---|---|
| (1) | Compound of Example 11 | 5.0 mg |
| (2) | Sodium chloride | 20.0 mg |
| (3) | Distilled water To make the total amount | 2 ml |

The compound of Example 11 (5.0mg) and sodium chloride (20.0 mg) are dissolved in distilled water, and to the solution is added water to make the total volume 2 ml. The solution is filtered, and filled into an ampoule (content: 2 ml) under sterilized conditions. The ampoule is sterilized, and sealed to obtain an injectable solution.

| Pharmaceutical preparation 5 | | |
|---|---|---|
| (1) | Compound of Example 11 | 50 mg |
| (2) | Lactose | 34 mg |
| (3) | Corn starch | 10.6 mg |
| (4) | Corn starch (paste) | 5 mg |
| (5) | Magnesium stearate | 0.4 mg |
| (6) | Carboxymethylcellulose calcium | 20 mg |
| | Total | 120 mg |

In accordance with conventional methods, the above (1) to (6) were mixed and tabletted by means of a tabletting machine to obtain tablets.

| Pharmaceutical preparation 6 | | |
|---|---|---|
| (1) | Compound of Example 11 | 5.0 mg |
| (2) | Sodium chloride | 20.0 mg |
| (3) | Distilled water To make the total amount | 2 ml |

The compound of Example 11 (5.0 mg) and sodium chloride (20.0 mg) are dissolved in distilled water, and to the solution is added water to make the total volume 2 ml. The solution is filtered, and filled into an ampoule (content: 2 ml) under sterilized conditions. The ampoule is sterilized, and sealed to obtain an injectable solution.

| Pharmaceutical preparation 7 | | |
|---|---|---|
| (1) | Compound of Example 12 | 5.0 mg |
| (2) | Sodium chloride | 20.0 mg |
| (3) | Distilled water To make the total amount | 2 ml |

The compound of Example 12 (5.0 mg) and sodium chloride (20.0 mg) are dissolved in distilled water, and to the solution is added water to make the total volume 2 ml. The solution is filtered, and filled into an ampoule (content: 2 ml) under sterilized conditions. The ampoule is sterilized, and sealed to prepare an injectable solution.

| Pharmaceutical preparation 8 | | |
|---|---|---|
| (1) | Compound of Example 12 | 50 mg |
| (2) | Lactose | 34 mg |
| (3) | Corn starch | 10.6 mg |
| (4) | Corn starch (paste) | 5 mg |
| (5) | Magnesium stearate | 0.4 mg |
| (6) | Carboxymethylcellulose calcium | 20 mg |
| | Total | 120 mg |

In accordance with conventional methods, the above (1) to (6) are mixed and tabletted by means of a tabletting machine to obtain tablets.

| Pharmaceutical preparation 9 | | |
|---|---|---|
| (1) | Compound of Example 12 | 5.0 mg |
| (2) | Sodium chloride | 20.0 mg |
| (3) | Distilled water To make the total amount | 2 ml |

The compound of Example 12 (5.0mg) and sodium chloride (20.0 mg) are dissolved in distilled water, and to the solution is added water to make the total volume 2 ml. The solution is filtered, and filled into an ampoule (content: 2 ml) under sterilized conditions. The ampoule is sterilized, and sealed to prepare an injectable solution.

### Test Example 1 AR-binding inhibition test (wild type, LNCaP type)

A solution of wild type or LNCaP type mutant androgen receptor, radio labeled Mibolerone (final concentration 3 nM) and a compound (final concentration 0.001 to 10 µM) were mixed. The mixture was incubated at 4 °C for 3 hours, and by the dextran/charcoal method, B (Bound) and F (Free) Mibolerone were separated. The radioactivity of B was measured and IC₅₀ was calculated. The results are shown in Table 32.

**[Table 32]**

| Compound | wild | type | LNCaP | type |
|---|---|---|---|---|
| Compound of Example | 1 | | 190 nM | 200 nM |
| Compound of Example | 4 | | 7.8 nM | 7.5 nM |
| Compound of Example | 80 | 40 nM | | 68 nM |
| Bicalutamide | | | 95 nM | 305 nM |

As is clear from Table 32, the compound of the present invention showed a strong affinity to both the wild type and LNCaP type mutant androgen receptor.

### Test Example 2 Inhibition test of the compound of the present invention on prostate-specific antigen (PSA) production in various prostate cancer cells including mutants

Human prostate cancer cells LNCaP-FGC were seeded in a 24-well plate at a concentration of 40, 000 cells/ml/well or in a 96-well plate at a concentration of 5000 cells/100 µl/well. On the following day, testosterone (final concentration 1 ng/ml) and a compound (final concentration 0.01 to 10 µM) were added. Three days after the addition, the concentration of PSA in the supernatant of the culture solution was measured by ELISA, and IC₅₀ was calculated. The results are shown in Table 33.

**[Table 33]**

| | |
|---|---|
| Compound of Example 1 | 392 nM |
| Compound of Example 4 | 87 nM |
| Compound of Example 80 | 34 nM |
| Bicalutamide | 823 nM |

As is clear from Table 33, the compounds of the present invention showed a strong PSA production suppressing activity. Test Example 3 AR transcription inhibition test

Cos-7 cells were inoculated in a flask at 5, 000, 000 cells, and incubated in a culture solution (DMEM+10% Dextran Charcoal (DCC)-Fetal Bovine Serum (FBS) + 2 mM glutamine) for 24 hours. A vector DNA in which a mutation type AR (W741C) gene was inserted and a vector DNA combined with luciferase gene downstream of the androgen responsive promoter were cotransfected by the liposome method. Two hours later, the culture medium was replaced and incubation was further carried out for 3 hours. DHT (Dihydrotestosterone) at a final concentration 0.04µM and a test compound at a final concentration of 1µM were added, and incubation was further carried out for 24 hours. By measuring luciferase activity, AR transcription inhibitory activity of the test compound was examined. The results are shown in Table 34 as inhibition rate (%) relative to the control.

**[Table 34]**

| Inhibition rate(%) | |
|---|---|
| Compound of Example 1 | 64.3 |
| Compound of Example 4 | 20.1 |
| Compound of Example 80 | 80.1 |
| Bicalutamide | 2.1 |

As is clear from Table 34, while bicalutamide showed almost no transcription inhibitory activity in mutant type AR (W741C), compounds of the present invention showed a strong transcription inhibitory activity.

### Industrial Applicability

The compound (I) or salt thereof of the present invention has a superior antagonistic effect against normal androgen receptor and (or) a mutant androgen receptor, and is useful as a prophylactic and therapeutic agent against hormone-sensitive cancers at androgen-dependent and (or) androgen-independent stages.

## Claims

1. An androgen receptor antagonistic agent containing a compound of the formula: 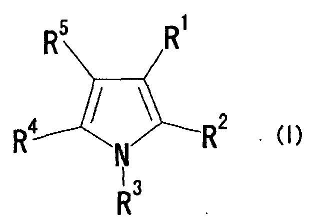 wherein, R¹ is a hydrogen atom, a group binding through a carbon atom, a group binding through a nitrogen atom, a group binding through an oxygen atom or a group binding through a sulfur atom, R² is a hydrogen atom, a group binding through a carbon atom, a group binding through a nitrogen atom, a group binding through an oxygen atom or a group binding through a sulfur atom, R³ is a hydrogen atom, a hydrocarbon group which may have substituent (s) , an acyl group or a heterocyclic group which may have substituent(s), R⁴ is a hydrogen atom, a group binding through a carbon atom, a group binding through a nitrogen atom, a group binding through an oxygen atom or a group binding through a sulfur atom, R⁵ is a cyclic group which may have substituent (s), or a salt thereof, or its prodrug.

2. An agent as claimed in claim 1, wherein R¹ is a hydrogen atom, a cyano group, a hydrocarbon group which may have substituent(s), a group represented by the formula: -COOR⁶¹ (wherein R⁶¹ is a hydrogen atom or a hydrocarbon group which may have substituent (s)), a group represented by the formula: - CONR⁷¹R⁸¹ (wherein R⁷¹ and R⁸¹ are the same or different, each of them is a hydrogen atom or a hydrocarbon group which may have substituent(s), and R⁷¹ and R⁸¹ are combined with each other together with the adjacent nitrogen atom to form a cyclic group which may have substituent(s)), a group represented by the formula: -COR⁹¹ (wherein R⁹¹ is a hydrogen atom or a hydrocarbon group which may have substituent (s)) or a group represented by the formula: -OR¹³¹ (wherein R¹³¹ is a hydrogen atom or a hydrocarbon group which may have substituent(s)).

3. An agent as claimed in claim 1, wherein R¹ is a cyano group or a group represented by the formula: -COOR⁶¹ (wherein R⁶¹ is a hydrocarbon group which may have substituent(s)).

4. An agent as claimed in claim 1, wherein R² is a hydrogen atom, a cyano group, a hydrocarbon group which may have substituent(s), a group represented by the formula: -COOR⁶²: (wherein R⁶² is a hydrocarbon group which may have substituent (s)), a group represented by the formula: -CONR⁷²R⁸² (wherein R⁷² and R⁸² are the same or different, each of them is a hydrogen atom or a hydrocarbon group which may have substituent (s), R⁷² and R⁸² are combined with each other together with the adjacent nitrogen atom to form a cyclic group which may have substituent (s)), a group represented by the formula: -COR⁹² (wherein R⁹² is a hydrogen atom or a hydrocarbon group which may have substituent (s)), or a group represented by the formula: -OR¹³² (wherein R¹³² is a hydrogen atom or a hydrocarbon group which may have substituent(s)).

5. An agent as claimed in claim 1, wherein R² is a C₁₋₆ alkyl group which may have substituent(s).

6. An agent as claimed in claim 1, wherein R³ is a C₁₋₆ alkyl group which may have substituent (s) , a C₇₋₁₅ aralkyl group which may have substituent (s) , a heterocyclic group-C₁₋₆ alkyl group which may have substituent (s) , a C₁₋₆ alkyl group-sulfonyl group which may have substituent (s) , a C₆₋₁₄ aryl group-sulfonyl group which may have substituent(s), a heterocyclic group-sulfonyl group which may have substituent (s), a C₁₋₆ alkyl group-carbonyl group which may have substituent (s) , a C₆₋₁₄ aryl group-carbonyl group which may have substituent(s) or a heterocyclic group-carbonyl group which may have substituent(s).

7. An agent as claimed in claim 1, wherein R³ is a benzyl group which may have substituent(s) or a pyridylmethyl group which may have substituent(s).

8. An agent as claimed in claim 1, wherein R³ is a heterocyclic group-methyl group which may have substituent(s)).

9. An agent as claimed in claim 1, wherein R⁴ is a hydrogen atom, a cyano group, a hydrocarbon group which may have substituent(s), a group represented by the formula: -COOR⁶⁴ (wherein R⁶⁴ is a hydrogen atom or a hydrocarbon group which may have substituent(s)), a group represented by the formula: -CONR⁷⁴R⁸⁴ (wherein R⁷⁴ and R⁸⁴ are the same or different, each of them is a hydrogen atom or a hydrocarbon group which may have substituent (s), R⁷⁴ and R⁸⁴ are combined to each other together with the adjacent nitrogen atom to form a cyclic group which may have substituent(s)), a group represented by the formula: - COR⁹⁴ (wherein R⁹⁴ is a hydrogen atom or a hydrocarbon group which may have substituent(s)) or a group represented by the formula: -OR¹³⁴ (wherein R¹³⁴ is a hydrogen atom or a hydrocarbon group which may have substituent(s)).

10. An agent as claimed in claim 1, wherein R⁴ is a C₁₋₆ alkyl group which may have substituent(s).

11. An agent as claimed in claim 1, wherein R⁵ is a cyclic hydrocarbon group which may have substituent(s).

12. An agent as claimed in claim 1, wherein R⁵ is a C₆₋₁₄ aryl group which may have substituent(s) or a heterocyclic group which may have substituent(s).

13. An agent as claimed in claim 1, wherein R⁵ is a C₆₋₁₄ aryl group which has at least one substituent selected from a group consisting of a cyano group and a nitro group.

14. An agent as claimed in claim 1, wherein R¹ is a cyano group, a C₁₋₆ alkyl group which may have hydroxy, a group represented by the formula: -COOR^{6'1} (wherein R^{6'1} is a C₁₋₆ alkyl group), a group represented by the formula: -CONR^{7'1}R^{8'1} (wherein R^{7'1} and R^{8'1} are the same or different, each of them is a hydrogen atom, a C₁₋₆ alkyl group or a C₆₋₁₀ aryl group, R^{7'1} and R^{8'1} are taken together to form a cyclic group which may have substituent (s)), a group represented by the formula: -COR^{9"1} (wherein R^{9"1} is ₐ C₁₋₆ alkyl group, a C₃₋₈ cyclo alkyl group or a heterocyclic group which may have substituent(s)), R² and R⁴ are the same or different, and each of them is a hydrogen atom, a cyano group or a C₁₋₆ alkyl group, R³ is a hydrogen atom or a hydrocarbon group which may have substituent(s) or a heterocyclic group which may have substituent(s), R⁵ is an aryl group which may have substituent(s), a C₃₋₈ cyclo alkyl group which may have substituent(s) or a heterocyclic group which may have substituent(s).

15. An agent as claimed in claim 1, wherein R¹ is a cyano group or a group represented by the formula: -COOR^{6'1} (wherein R^{6'1} is a C₁₋₆ alkyl group), R² is a C₁₋₆ alkyl group which may have substituent(s), R³ is a C₁₋₆ alkyl group which may have substituent(s), a C₇₋₁₅ aralkyl group which may have substituent (s), a C₁₋₆ alkyl group-sulfonyl group which may have substituent(s) or a C₆₋₁₄ aryl group-carbonyl group which may have substituent(s), a R⁴ is a C₁₋₆ alkyl group which may have substituent(s), R⁵ is a C₆₋₁₄ aryl group which may have substituent(s).

16. An agent as claimed in claim 1, wherein R¹ is a cyano group or a group represented by the formula: -COOR^{6'1} (wherein R^{6'1} is a C₁₋₆ alkyl group), R² is a C₁₋₆ alkyl group, R³ is a C₁₋₆ alkyl group which may have hydroxy, a C₇₋₁₅ aralkyl group, a C₁₋₆ alkyl group-sulfonyl group or a C₆₋₁₄ aryl group-carbonyl group, R⁴ is a C₁₋₆ alkyl group, and R⁵ is a C₆₋₁₄ aryl group which may have a nitro group, a cyano group or a C₁₋₃ acyl group.

17. An agent as claimed in claim 1, wherein the compound is a group represented by the formula: 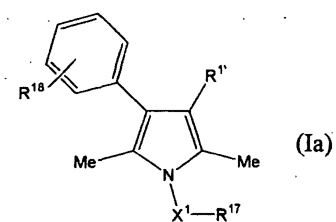 wherein R^{1'} is a cyano group, a C₁₋₆ alkyl group, a group represented by the formula: -COOR^{6'1'} (wherein R^{6'1'} is a C₁₋₆ alkyl group), a group represented by the formula: -CONR^{7'1'}R^{8'1'} (wherein R^{7'1'} and R^{8'1'} are the same or different, and each of them is a hydrogen atom, a C₁₋₆ alkyl group or a C₆₋₁₀ aryl group) , a group represented by the formula: -C(OH)R^{161'}R^{16'1'} (R^{161'} and R^{16'1'} are the same or different, and each of them is a hydrogen atom or a C₁₋₆ alkyl) or a group represented by the formula: - COR^{9''1'} (wherein R^{9''1'} is a C₁₋₆ alkyl group, a C₃₋₈ cyclo alkyl group or a heterocyclic group which may have substituent (s)), R¹⁷ is a C₆₋₁₄ aryl group which may have substituent(s) or a heterocyclic group which may have substituent (s), R¹⁸ is a nitro group, a C₁₋₆ alkyl group which may have substituent(s), a halogen atom, a C₁₋₆ alkoxy group, a cyano group, a carboxyl group, a carboxylic acid ester group, a hydroxy group or an amide group, X¹ is a bivalent group in which number of straight-chained carbon atoms is 1 to 5 which may have substituent (s) ; or a salt thereof, or its prodrug.

18. An agent as claimed in claim 1, wherein the compound is represented by the formula: 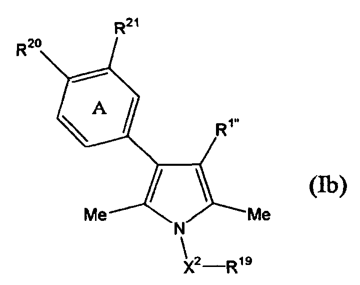 wherein R^{1"} is a cyano group or a group represented by the formula: -COOR^{6"1"} (wherein R^{6"1"} is a methyl group or a ethyl group), R¹⁹ is a C₆₋₁₄ aryl group which may have substituent(s) or a heterocyclic group which may have substituent (s), at least one of R²⁰ and R²¹ is a nitro group or a cyano group, the ring A may further have substituent(s), and X² is a bivalent group in which the number of straight-chained carbon atom(s) is 1 to 5 which may have substituent(s); or a salt thereof, or its prodrug.

19. An agent as claimed in claim 1, wherein the compound is one selected from the group consisting of (1) ethyl
1-benzyl-2,5-dimethyl-4-(4-nitrophenyl)-1H-pyrrole-3-carbox ylate or a salt thereof, (2) methyl
1-benzyl-4-(3-cyanophenyl)-2,5-dimethyl-1H-pyrrole-3-carbox ylate or a salt thereof, (3) methyl
1-benzyl-4-(4-formylphenyl)-2,5-dimethyl-1H-pyrrole-3-carbo xylate or a salt thereof, (4) methyl
4-(4-cyanophenyl)-2,5-dimethyl-1-(3-pyridylmethyl)-1H-pyrro le-3-carboxylate or a salt thereof, (5)
4-(4-cyanophenyl)-2,5-dimethyl-1-(3-pyridylmethyl)-1H-pyrro le-3-carbonitrile or a salt thereof, (6)
4-(4-cyanophenyl)-2,5-dimethyl-1-((6-methyl-3-pyridyl)methy l)-1H-pyrrole-3-carbonitrile or a salt thereof and (7)
4-(4-cyanophenyl)-2,5-dimethyl-1-((6-chloro-3-pyridyl) methyl)-1H-pyrrole-3-carbonitrile or a salt thereof.

20. An agent as claimed in claim 1, wherein the androgen receptor is a normal androgen receptor and/or a mutant androgen receptor.

21. An agent as claimed in claim 1, which is a prophylactic or therapeutic agent for hormone-sensitive cancer in androgen-dependent stage and/or androgen-independent stage.

22. An agent as claimed in claim 1, which is a prophylactic or therapeutic agent for prostate cancer.

23. A compound represented by the formula: 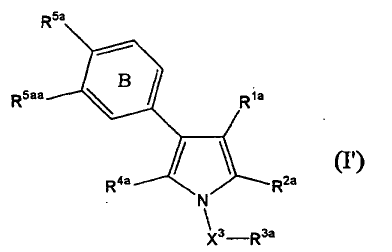 wherein R^{1a} is a hydrogen atom, a group binding through a carbon atom, a group binding through a nitrogen atom, a group binding through an oxygen atom or a group binding through a sulfur atom, R^{2a} is a hydrogen atom, a group binding through a carbon atom, a group binding through a nitrogen atom, a group binding through an oxygen atom or a group binding through a sulfur atom, R^{3a} is an aromatic ring group which may have substituent(s), R^{4a} is a hydrogen atom, a group binding through a carbon atom, a group binding through a nitrogen atom, a group binding through an oxygen atom or a group binding through a sulfur atom, at least one of R^{5a} and R^{5aa} is a nitro group, a cyano group or a formyl group, the ring B may further have substituent(s), and X³ is a bivalent group in which the number of straight-chained carbon atom(s) is 1 to 5, which may have substituent (s) ; (provided that (1) ethyl
4-(3-cyanophenyl)-1-(2-naphthylmethyl)-1H-pyrrole-3-carboxy late, (2) ethyl
4-(3-cyanophenyl)-1-(1-naphthylmethyl)-1H-pyrrole-3-carboxy late, (3) 1-benzyl-3-(3-nitrophenyl)-1H-pyrrole, (4)
1-benzyl-3-methyl-4-(4-nitrophenyl)-1H-pyrrole, (5) tert-butyl
4-ethyl-1-(4-nitrobenzyl)-3-(4-nitrophenyl)-1H-pyrrole-2-ca rboxylate, (6) tert-butyl
4-ethyl-1-(4-methoxybenzyl)-3-(4-nitrophenyl)-1H-pyrrole-2-carboxylate, (7) tert-butyl
1-benzyl-4-ethyl-3-(4-nitrophenyl)-1H-pyrrole-2-carboxylate, (8) tert-butyl
1-benzyl-4-methyl-3-(4-nitrophenyl)-1H-pyrrole-2-carboxylat e, (9) methyl
[4-(aminocarbonyl)-5-methyl-3-(4-nitrophenyl)-1-(2-pyridine - 2-ylethyl)-1H-pyrrole-2-yl]acetate, (10)
1-benzyl-N-{2'-[(tert-butylamino)sulfonyl]-1,1'-biphenyl-4-yl}-4-(3-cyanophenyl)-1H-pyrrole-3-carboxamide, (11)
3-{1-[(E)-1-cyano-2-(3,5-dichloro-1-methyl-1H-pyrazole-4-yl )-2-hydroxyethenyl]-1H-pyrrole-3-yl}benzonitrile, (12) methyl
4-(4-amino-3-nitrophenyl)-1-benzyl-2,5-dimethyl-1H-pyrrole-3-carboxylate, (13) methyl
1-benzoyl-4-(3-nitrophenyl)-1H-pyrrole-3-carboxylate, (14)
4-[1-benzyl-2-(2-furanyl)-5-phenyl-1H-pyrrole-3-yl]-benzoni trile and (15)
4-[1-benzyl-2,5-diphenyl-1H-pyrrole-3-yl]-benzonitrile are excluded) a salt thereof, or its prodrug.

24. A compound as claimed in claim 23, wherein X³ is a methylene group which may have substituent(s), R^{3a} is a C₆₋₁₄ aryl group which may have substituent (s) , or a 5- to 14-membered aromatic heterocyclic group which may have substituent(s) or a salt thereof, or its prodrug.

25. A compound as claimed in claim 23, wherein X³ is a methylene group which may have substituent(s), and R^{3a} is a 5- to 14-membered aromatic heterocyclic group which may have substituent(s) or a salt thereof, or its prodrug.

26. A compound represented by the formula: 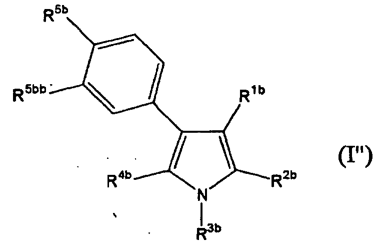 wherein R^{1b} is a cyano group or a group represented by the formula: -COOR^{6'1b} (wherein R^{6'1b} is a C₁₋₆ alkyl group), R^{2b} is a hydrogen atom or a C₁₋₆ alkyl group, R^{3b} is a C₁₋₆ alkyl group which may have substituent (s) , a C₇₋₁₅ aralkyl group which may have substituent (s), a 5- to 14-membered aromatic heterocyclic group-C₁₋₆ alkyl group, a group represented by the formula: - CO-R^{9'3b} (wherein R^{9'3b} is a C₁₋₆ alkyl group which may have substituent (s) , a C₆₋₁₄ aryl group which may have substituent (s) or a 5- to 14-membered aromatic heterocyclic group which may have substituent(s)) or a group represented by the formula: - SO₂-R^{12'3b} (wherein R^{12'3b} is a C₁₋₆ alkyl group which may have substituent (s), a C₆₋₁₄ aryl group which may have substituent (s) or a 5- to 14-membered aromatic heterocyclic group which may have substituent (s)), R^{4b} is a hydrogen atom or a C₁₋₆ alkyl group, at least one of R^{5b} and R^{5bb} is a nitro group, a cyano group or a formyl group; (provided (1) ethyl
4-(3-cyanophenyl)-1-(2-naphthylmethyl)-1H-pyrrole-3-carboxy late, (2) ethyl
4-(3-cyanophenyl)-1-(1-naphthylmethyl)-1H-pyrrole-3-carboxy late, (3) methyl
4-(4-amino-3-nitrophenyl)-1-benzyl-2,5-dimethyl-1H-pyrrole-3-carboxylate, (4) methyl
4-(4-amino-3-nitrophenyl)-1-butyl-2,5-dimethyl-1H-pyrrole-3 - carboxylate, (5) methyl
4-(4-amino-3-nitrophenyl)-1,2,5-trimethyl-1H-pyrrole-3-carb oxylate, (6) methyl
1-benzoyl-4-(3-nitrophenyl)-1H-pyrrole-3-carboxylate, (7) dimethyl 4-(3-nitrophenyl)-1H-pyrrole-1,3-dicarboxylate are excluded), its salt, or its prodrug.

27. A compound as claimed in claim 26, wherein R^{1b} is a cyano group or a group represented by the formula: -COOR^{6'1b} (wherein R^{6'1b} is a C₁₋₆ alkyl group), R^{2b} is a C₁₋₆ alkyl group, R^{3b} is a C₁₋₆ alkyl group which may have substituent (s) , a C₇₋₁₅ aralkyl group which may have substituent(s), a 5- to 14-membered aromatic heterocyclic group-C₁₋₆ alkyl group which may have substituent(s), a group represented by the formula: -CO-R^{9'3b} (wherein R^{9'3b} is a C₁₋₆ alkyl group which may have substituent (s), a C₆₋₁₄ aryl group which may have substituent (s) or a 5- to 14-membered aromatic heterocyclic group which may have substituent(s)) or a group represented by the formula: -SO₂-R^{12'3b} (wherein R^{12'3b} is a C₁₋₆ alkyl group which may have substituent (s), a C₆₋₁₄ aryl group which may have substituent (s) or a 5- to 14-membered aromatic heterocyclic group which may have substituent(s)), R^{4b} is a C₁₋₆ alkyl group, R^{5b} is a nitro group, a cyano group or a formyl group or a salt thereof, or its prodrug.

28. A compound as claimed in claim 26, wherein R^{3b} is a C₆₋₁₄ aryl-methyl group which may have substituent(s), or a 5- to 14-membered aromatic heterocyclic group-methyl group which may have substituent(s) or a salt thereof, or its prodrug.

29. A compound as claimed in claim 26, wherein R^{3b} is a 5- to 14-membered aromatic heterocyclic group-methyl group which may have substituent(s) a salt thereof, or its prodrug.

30. A compound as claimed in claim 26, wherein R^{1b} is a cyano group or a salt thereof, or its prodrug.

31. A compound as claimed in claim 26, wherein R^{3b} is a benzyl group which may have substituent (s) or a pyridylmethyl group which may have substituent (s) or a salt thereof, or its prodrug.

32. A compound selected from the group consisting of (1) ethyl 1-benzyl-2,5-dimethyl-4-(4-nitrophenyl)-1H-pyrrole-3-carbox ylate or its salt, (2) methyl
1-benzyl-4-(3-cyanophenyl)-2,5-dimethyl-1H-pyrrole-3-carbox ylate or its salt, (3) methyl
1-benzyl-4-(4-formylphenyl)-2,5-dimethyl-1H-pyrrole-3-carbo xylate or its salt, (4) methyl
4-(4-cyanophenyl)-2,5-dimethyl-1-(3-pyridylmethyl)-1H-pyrro le-3-carboxylate or its salt, (5)
4-(4-cyanophenyl)-2,5-dimethyl-1-(3-pyridylmethyl)-1H-pyrro le-3-carbonitrile or its salt, (6)
4-(4-cyanophenyl)-2,5-dimethyl-1-((6-methyl-3-pyridyl)methy 1)-1H-pyrrole-3-carbonitrile or its salt, and (7)
4-(4-cyanophenyl)-2,5-dimethyl-1-((6-chloro-3-pyridyl)methy 1)-1H-pyrrole-3-carbonitrile or its salt.

33. A medicament containing a compound defined in claim 23 or claim 26 or a salt thereof, or its prodrug.

34. A medicament which comprises a compound represented by the formula: 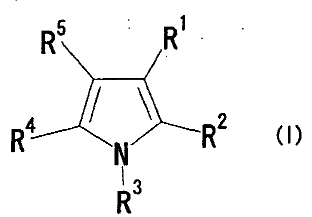 wherein R¹ is a hydrogen atom, a group binding through a carbon atom, a group binding through a nitrogen atom, a group binding through an oxygen atom or a group binding through a sulfur atom, R² is a hydrogen atom, a group binding through a carbon atom, a group binding through a nitrogen atom, a group binding through an oxygen atom or a group binding through a sulfur atom, R³ is a hydrogen atom, a hydrocarbon group which may have substituent (s), an acyl group or a heterocyclic group which may have substituent(s), R⁴ is a hydrogen atom, a group binding through a carbon atom, a group binding through a nitrogen atom, a group binding through a oxygen atom or a group binding through a sulfur atom, R⁵ is a cyclic group which may have substituent(s); or a salt thereof, or its prodrug and an anticancer drug in combination.

35. A medicament as claimed in claim 34, wherein the anticancer agent is an LH-RH derivative.

36. Aprophylactic or therapeutic agent for a cancer sensitive. to a hormone in androgen-dependent stage and/or androgen-independent stage comprising a mutant androgen receptor antagonistic drug.

37. A prophylactic or therapeutic agent for a cancer sensitive to a hormone in androgen-dependent stage and/or androgen-independent stage which comprises mutant androgen receptor antagonistic drug and an anticancer agent.

38. An agent as claimed in claim 37, wherein the anticancer agent is an LH-RH derivative.

39. Aprophylactic or therapeutic agent for a cancer sensitive to a hormone in androgen-dependent stage and/or androgen-independent stage containing a sensitivity-increased androgen receptor antagonistic drug.

40. Aprophylactic or therapeutic agent for a cancer sensitive to a hormone in androgen-dependent stage and/or androgen-independent stage which comprises a sensitivity-increased androgen receptor antagonistic drug and an anticancer agent.

41. A prophylactic or therapeutic agent as claimed in claim 40, wherein the anticancer agent is an LH-RH derivative.

42. A method for antagonizing androgen receptor, which comprises administering an effective amount of a compound of the formula: 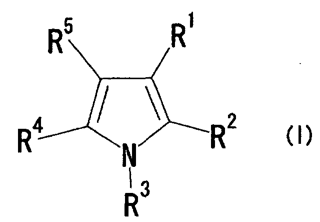 wherein R¹ is a hydrogen atom, a group binding through a carbon atom, a group binding through a nitrogen atom, a group binding through an oxygen atom or a group binding through a sulfur atom, R² is a hydrogen atom, a group binding through a carbon atom, a group binding through a nitrogen atom, a group binding through an oxygen atom or a group binding through a sulfur atom, R³ is a hydrogen atom, a hydrocarbon group which may have substituent (s) , an acyl group or a heterocyclic group which may have substituent(s), R⁴ is a hydrogen atom, a group binding through a carbon atom, a group binding through a nitrogen atom, a group binding through an oxygen atom or a group binding through a sulfur atom, R⁵ is a cyclic group which may have substituent (s); or a salt thereof, or its prodrug, to a mammal.

43. A method for preventing or treating a cancer sensitive to a hormone in androgen-dependent stage and/or androgen-independent stage, which comprises administering an effective amount of a compound represented by the formula: 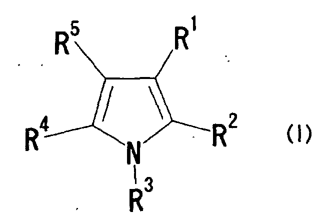 wherein R¹ is a hydrogen atom, a group binding through a carbon atom, a group binding through a nitrogen atom, a group binding through an oxygen atom or a group binding through a sulfur atom, R² a hydrogen atom, a group binding through a carbon atom, a group binding through a nitrogen atom, a group binding through an oxygen atom or a group binding through a sulfur atom, R³ is a hydrogen atom, a hydrocarbon group which may have substituent (s), an acyl group or a heterocyclic group which may have substituent(s), R⁴ is a hydrogen atom, a group binding through a carbon atom, a group binding through a nitrogen atom, a group binding through an oxygen atom or a group binding through a sulfur atom, R⁵ is a cyclic group which may have substituent (s) ; or a salt thereof, or its prodrug, to a mammal.

44. A method for preventing or treating prostate cancer, which comprises administering an effective amount of a compound represented by the formula: 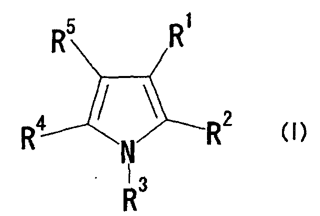 wherein R¹ is a hydrogen atom, a group binding through a carbon atom, a group binding through a nitrogen atom, a group binding through an oxygen atom or a group binding through a sulfur atom, R² is a hydrogen atom, a group binding through a carbon atom, a group binding through a nitrogen atom, a group binding through an oxygen atom or a group binding through a sulfur atom, R³ is a hydrogen atom, a hydrocarbon group which may have substituent (s) , an acyl group or a heterocyclic group which may have substituent(s), R⁴ is a hydrogen atom, a group binding through a carbon atom, a group binding through a nitrogen atom, a group binding through an oxygen atom or a group binding through a sulfur atom, R⁵ is a cyclic group which may have substituent (s) ; or a salt thereof, or its prodrug, to a mammal.

45. Amethod as claimed in claim 43 and 44, in which an effective amount of an anticancer agent is further administered.

46. A method as claimed in claim 45, wherein the anticancer agent is an LH-RH derivative.

47. A method for preventing or treating a cancer sensitive to a hormone in androgen-dependent stage and/or androgen-independent stage, which comprise administering an effective amount of mutant androgen receptor antagonistic drug to a mammal.

48. A method as claimed in claim 47, in which an effective amount of an anticancer agent is further administered.

49. A method as claimed in claim 48, wherein the anticancer agent is an LH-RH derivative.

50. A method for preventing or treating a cancer sensitive to a hormone in androgen-dependent stage and/or androgen-independent stage, which comprises administering an effective amount of a sensitivity-increased androgen receptor antagonistic drug to a mammal.

51. A method as claimed in claim 50, in which an effective amount of an anticancer agent is further administered.

52. A method as claimed in claim 51, wherein the anticancer agent is an LH-RH derivative.
